(19) Europäisches Patentamt
European Patent Office
Office européen des brevets

(11) EP 4 174 055 A1

(12) EUROPEAN PATENT APPLICATION

(43) Date of publication:
**03.05.2023 Bulletin 2023/18**

(21) Application number: **22204291.3**

(22) Date of filing: **28.10.2022**

(51) International Patent Classification (IPC):
***C07D 209/88*** *(2006.01)* ***C07D 307/91*** *(2006.01)*
***C07D 333/76*** *(2006.01)* ***C07C 211/61*** *(2006.01)*
***H10K 50/15*** *(2023.01)*

(52) Cooperative Patent Classification (CPC):
**C07D 209/88; C07C 211/61; C07D 307/91; C07D 333/76; H10K 85/633;** C07C 2601/14; C07C 2602/42; C07C 2603/74; C07C 2603/94; H10K 50/15; H10K 59/38

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **29.10.2021 KR 20210146503**
**05.01.2022 KR 20220001805**

(71) Applicant: **Samsung Display Co., Ltd.**
**Yongin-si, Gyeonggi-do 17113 (KR)**

(72) Inventors:
- **KIM, Minji**
  **Hwaseong-si (KR)**
- **PAK, Hankyu**
  **Suwon-si (KR)**
- **KIM, Dongjun**
  **Suwon-si (KR)**
- **KIM, Chaeyeong**
  **Yongin-si (KR)**
- **PARK, Hyunbin**
  **Suwon-si (KR)**
- **YOO, Byeongwook**
  **Hwaseong-si (KR)**
- **LEE, Jeongmin**
  **Hwaseong-si (KR)**
- **JEONG, Eunjae**
  **Hwaseong-si (KR)**
- **CHO, Seowon**
  **Asansi (KR)**
- **JO, Sohee**
  **Cheonan-si (KR)**
- **CHOI, Jiyong**
  **Cheonan-si (KR)**
- **HAN, Sanghyun**
  **Hwaseong-si (KR)**

(74) Representative: **Russell, Tim et al**
**Venner Shipley LLP**
**200 Aldersgate**
**London EC1A 4HD (GB)**

# (54) LIGHT EMITTING DEVICE AND AMINE COMPOUND FOR LIGHT EMITTING DEVICE

(57) A light emitting device includes a first electrode, a second electrode on the first electrode, and at least one functional layer between the first electrode and the second electrode wherein the at least one functional layer comprises an amine compound represented by Formula 1.

[Formula 1]

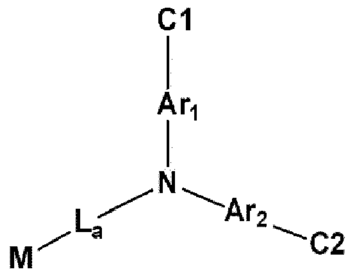



Processed by Luminess, 75001 PARIS (FR)

## Description

### CROSS-REFERENCE TO RELATED APPLICATIONS

This application claims priority to and the benefit of Korean Patent Application Nos. 10-2021-0146503, filed on October 29, 2021 and 10-2022-0001805, filed on January 5, 2022, the entire contents of each of which are hereby incorporated by reference.

### BACKGROUND

#### 1. Field

Embodiments of the present disclosure herein relate to an amine compound used in a light emitting device, and, for example, to an amine compound used in a hole transport region, and a light emitting device including the same.

#### 2. Related Art

Recently, the development of an organic electroluminescence display as an image display is being actively conducted. The organic electroluminescence display is different from a liquid crystal display and is a so-called a self-luminescent type display in which holes and electrons injected from a first electrode and a second electrode recombine in an emission layer so that a light emitting material including an organic compound in the emission layer emits light to achieve a display.

In the application of an organic electroluminescence device to a display, the decrease of a driving voltage and the increase of emission efficiency and life of the organic electroluminescence device are desired, and development of materials for an organic electroluminescence device stably achieving the requirements is being continuously conducted.

In addition, in order to achieve an organic electroluminescence device having high efficiency, development of a material for a hole transport region is being conducted.

### SUMMARY

The invention is defined by the claims.

Embodiments of the present disclosure provide a light emitting device having improved emission efficiency and device life.

Embodiments of the present disclosure also provide an amine compound which may improve the emission efficiency and device life of a light emitting device.

An embodiment of the present disclosure provides a light emitting device including a first electrode, a second electrode on the first electrode, and at least one functional layer between the first electrode and the second electrode, wherein the at least one fucntiuonal layer comprises an amine compound represented by Formula 1 below.

Formula 1

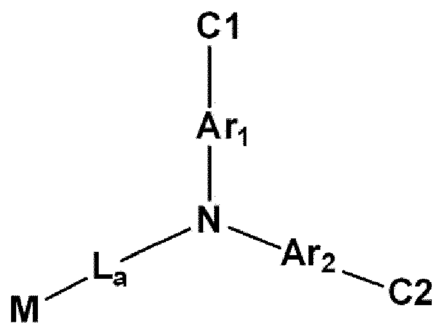

In Formula 1, An and $Ar_2$ are each independently a substituted or unsubstituted arylene group of 6 to 30 ring-forming carbon atoms, or a substituted or unsubstituted heteroarylene group of 2 to 30 ring-forming carbon atoms, $L_a$ is a direct linkage, a substituted or unsubstituted arylene group of 6 to 30 ring-forming carbon atoms, or a substituted or unsubstituted heteroarylene group of 2 to 30 ring-forming carbon atoms, C1 and C2 are each independently a substituted or unsubstituted adamantyl group, a substituted or unsubstituted cyclohexyl group, a substituted or unsubstituted bicycloheptanyl group, a substituted or unsubstituted bicyclooctanyl group, or a substituted or unsubstituted triphenylsilyl

group, a case where both C1 and C2 are substituted or unsubstituted adamantyl groups is excluded, and M is represented by any one selected from among Formula 1-a to Formula 1-c below.

Formula 1-a

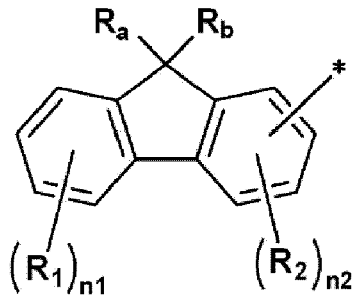

Formula 1-b

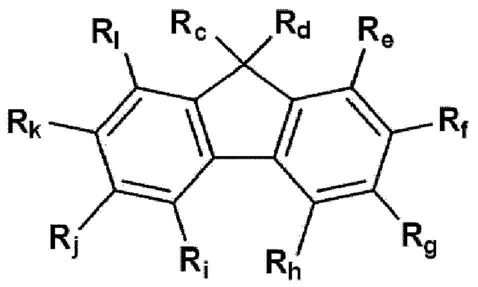

Formula 1-c

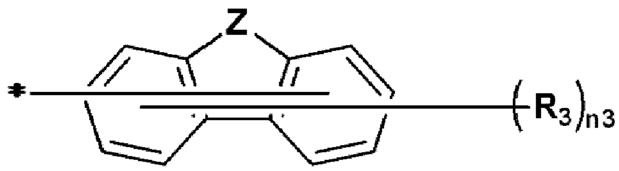

In Formula 1-a to Formula 1-c, Z is $NR_4$, O, or S, $R_1$ and $R_2$ are each independently a substituted or unsubstituted aryl group of 6 to 30 ring-forming carbon atoms, or a substituted or unsubstituted heteroaryl group of 2 to 30 ring-forming carbon atoms, and $R_a$ to $R_l$, $R_3$, and $R_4$ are each independently a hydrogen atom, a deuterium atom, a halogen atom, a substituted or unsubstituted alkyl group of 1 to 20 carbon atoms, a substituted or unsubstituted alkenyl group of 2 to 20 carbon atoms, a substituted or unsubstituted aryl group of 6 to 30 ring-forming carbon atoms, or a substituted or unsubstituted heteroaryl group of 2 to 30 ring-forming carbon atoms, or combined with an adjacent group to form a ring, in Formula 1-b, any one selected from among $R_c$ to $R_l$ is a part connected with Formula 1, in Formula 1-a and Formula 1-c, -* is a part connected with Formula 1, in a case where M in Formula 1 is represented by Formula 1-b or Formula 1-c, at least one selected from among C1 and C2 in Formula 1 is a bicycloheptanyl group, n1 is an integer of 0 to 4, n2 is an integer of 0 to 3, where the sum of n1 and n2 is 1 or more, and n3 is an integer of 0 to 7.

In an embodiment, the at least one functional layer may include an emission layer, a hole transport region between the first electrode and the emission layer, and an electron transport region between the emission layer and second electrode, and the hole transport region may include the amine compound.

In an embodiment, the amine compound represented by Formula 1 may be a monoamine compound.

In an embodiment, the amine compound represented by Formula 1 may be represented by any one selected from among Formula 2-1 to Formula 2-8 below.

Formula 2-1

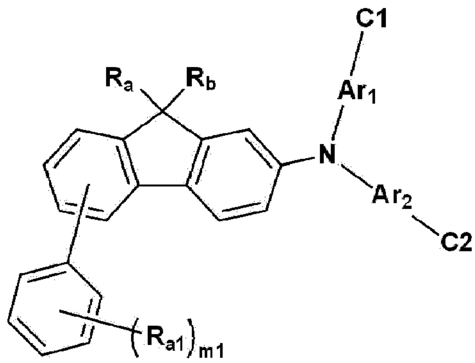

Formula 2-2

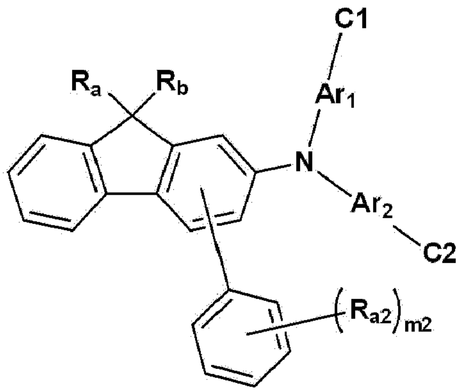

Formula 2-3

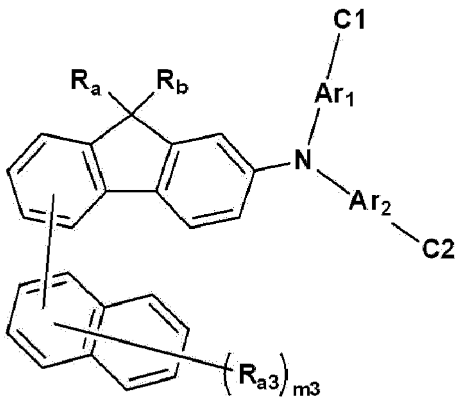

Formula 2-4

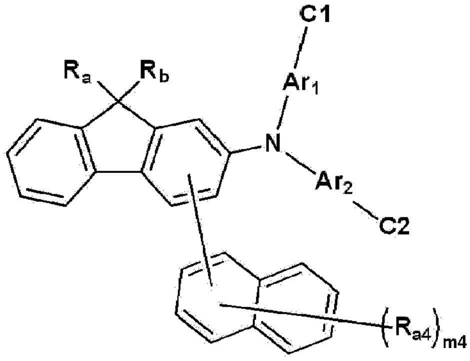

Formula 2-5

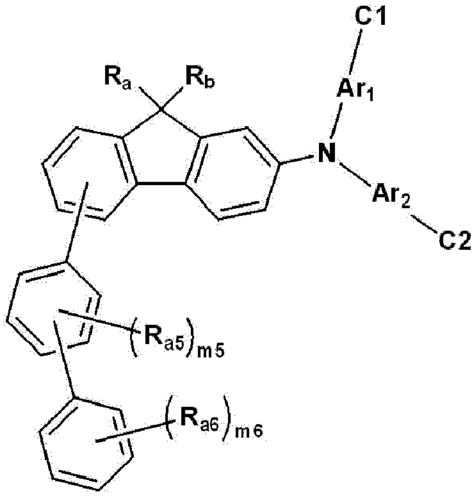

Formula 2-6

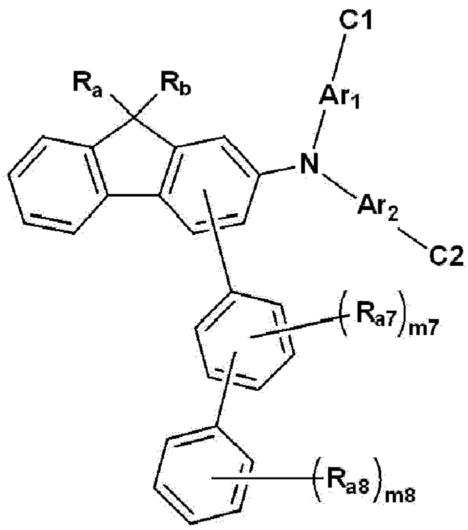

Formula 2-7

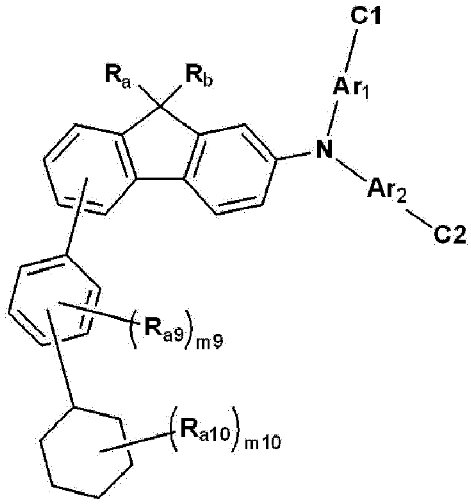

Formula 2-8

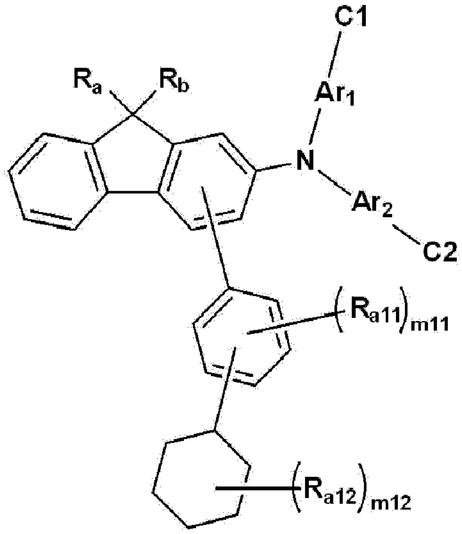

In Formula 2-1 to Formula 2-8, $R_{a1}$ to $R_{a12}$ are each independently a hydrogen atom, a deuterium atom, a halogen atom, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms, m1, m2, m6, and m8 are each independently an integer of 0 to 5, m3 and m4 are each independently an integer of 0 to 7, m5, m7, m9, and m11 are each independently an integer of 0 to 4, and m10, and m12 are each independently an integer of 0 to 11.

In Formula 2-1 to Formula 2-8, the same description of $R_a$, $R_b$, $Ar_1$, $Ar_2$, C1 and C2 provided with respect to Formula 1 and Formula 1-a may be applied.

In an embodiment, the amine compound represented by Formula 1 may be represented by Formula 3-1 or Formula 3-2 below.

Formula 3-1

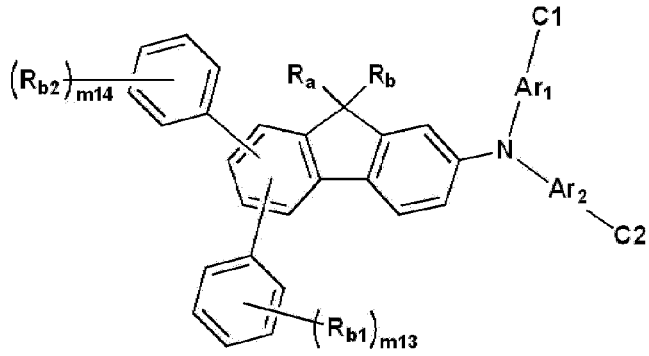

Formula 3-2

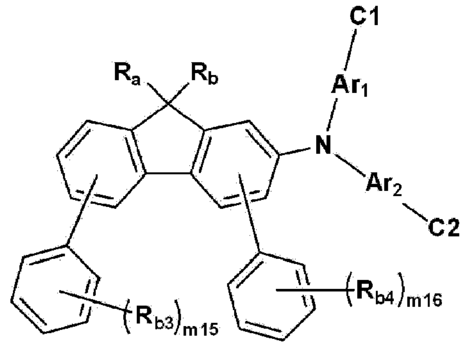

In Formula 3-1 and Formula 3-2, $R_{b1}$ to $R_{b4}$ are each independently a hydrogen atom, a deuterium atom, a halogen atom, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms, and m13 to m16 are each independently an integer of 0 to 5.

In Formula 3-1 and Formula 3-2, the same description of $R_a$, $R_b$, $Ar_1$, $Ar_2$, C1 and C2 provided with respect to Formula 1 and Formula 1-a may be applied.

In an embodiment, the amine compound represented by Formula 1 is represented by any one selected from among Formula 4-1 to Formula 4-15 below.

Formula 4-1

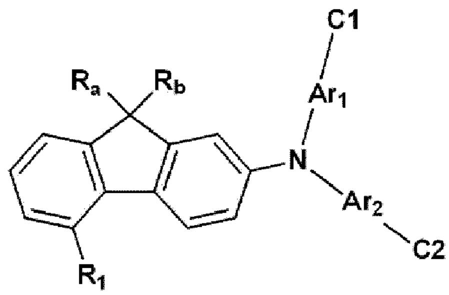

Formula 4-2

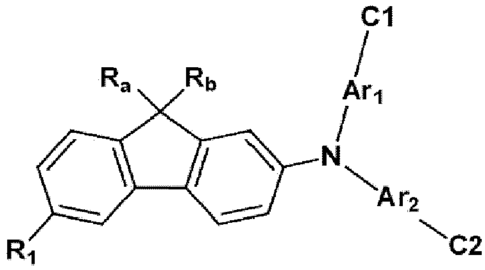

Formula 4-3

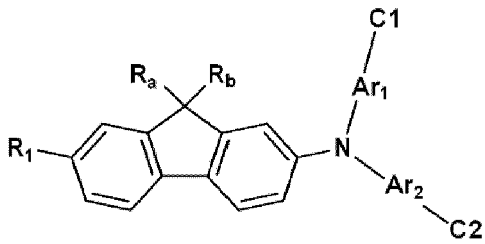

Formula 4-4

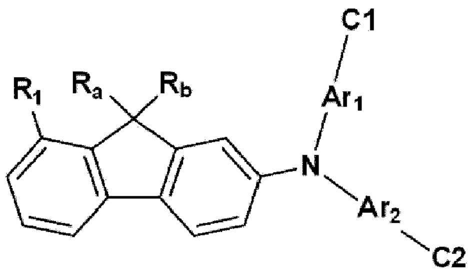

Formula 4-5

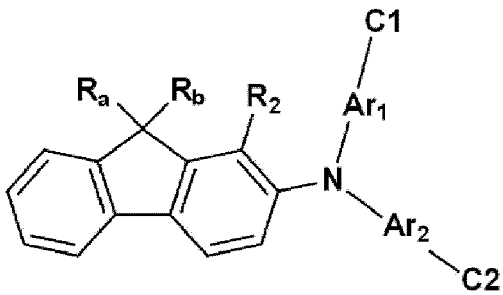

Formula 4-6

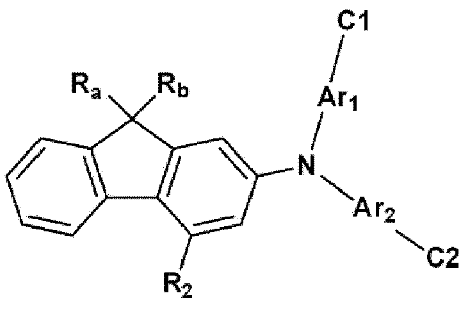

Formula 4-7

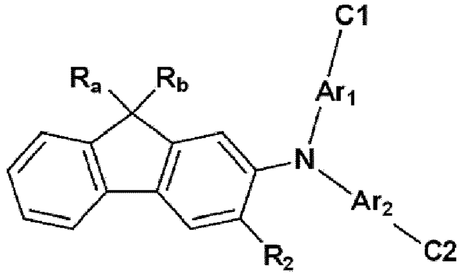

Formula 4-8

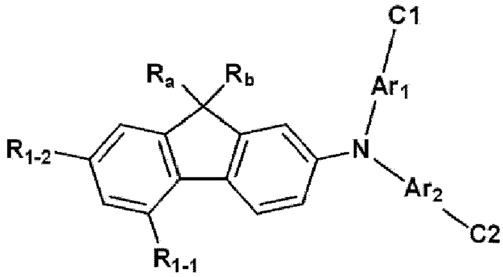

Formula 4-9

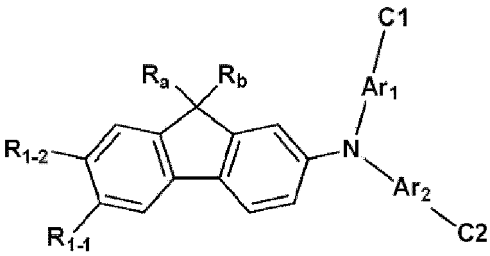

Formula 4-10

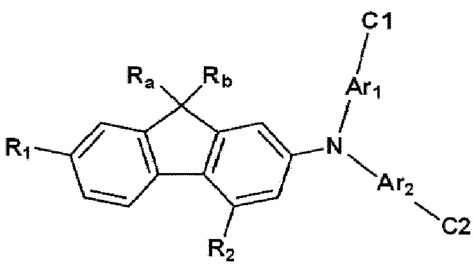

Formula 4-11

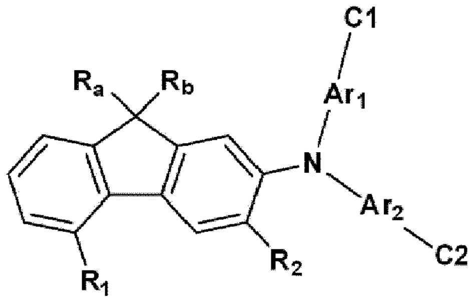

Formula 4-12

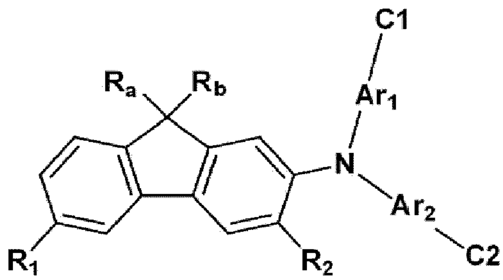

Formula 4-13

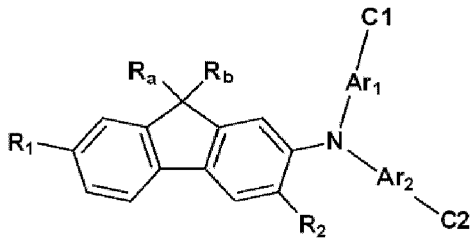

Formula 4-14

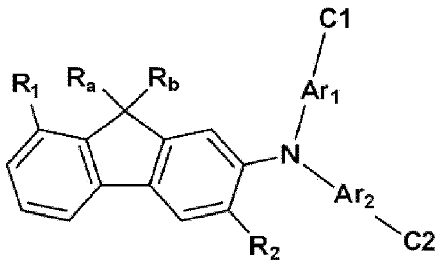

Formula 4-15

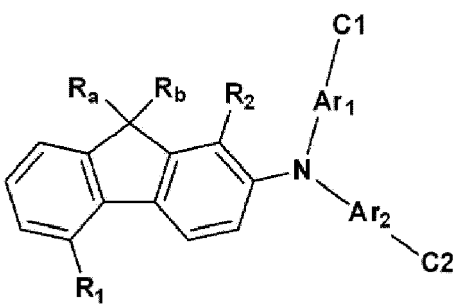

In Formula 4-1 to Formula 4-15, $R_{1-1}$ and $R_{1-2}$ may be each independently a substituted or unsubstituted aryl group of 6 to 30 ring-forming carbon atoms, or a substituted or unsubstituted heteroaryl group of 2 to 30 ring-forming carbon atoms.

In Formula 4-1 to Formula 4-15, the same description of $R_1$, $R_2$, $R_a$, $R_b$, $Ar_1$, $Ar_2$, C1, and C2 provided with respect to Formula 1 and Formula 1-a may be applied.

In an embodiment, the amine compound represented by Formula 1 may be represented by any one selected from among Formula 5-1 to Formula 5-3 below.

Formula 5-1

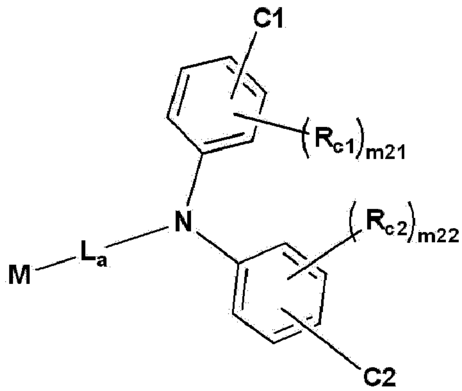

Formula 5-2

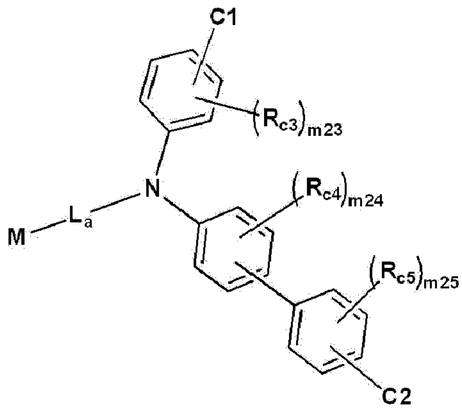

Formula 5-3

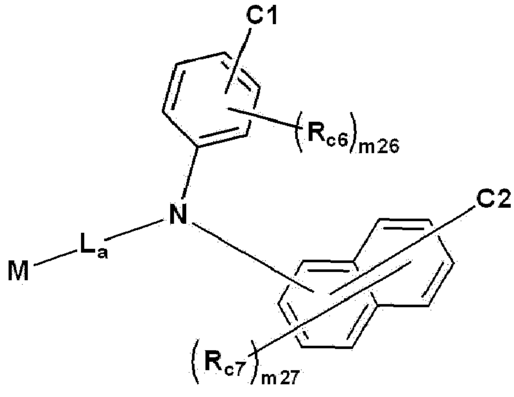

In Formula 5-1 to Formula 5-3, $R_{c1}$ to $R_{c7}$ are each independently a hydrogen atom, a deuterium atom, a halogen atom, a substituted or unsubstituted alkyl group of 1 to 20 carbon atoms, a substituted or unsubstituted aryl

group of 6 to 30 ring-forming carbon atoms, or a substituted or unsubstituted heteroaryl group of 2 to 30 ring-forming carbon atoms, m21 to m26 are each independently an integer of 0 to 4, and m27 is an integer of 0 to 6.

In Formula 5-1 to Formula 5-3, the same description of M, $L_a$, C1, and C2 provided with respect to Formula 1 may be applied.

In an embodiment, the amine compound represented by Formula 1 may be represented by any one selected from among Formula 6-1 to Formula 6-8 below.

Formula 6-1

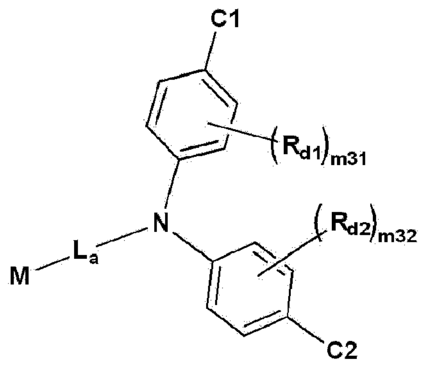

Formula 6-2

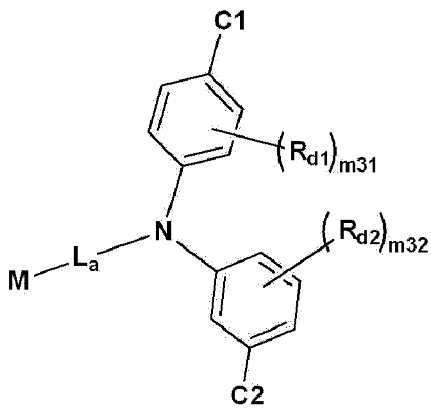

Formula 6-3

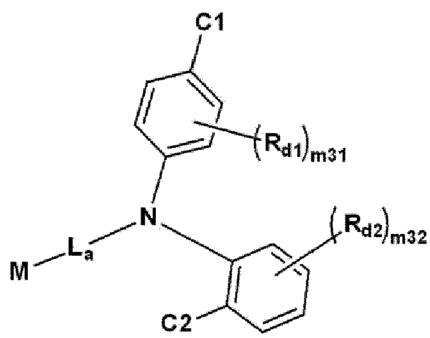

Formula 6-4

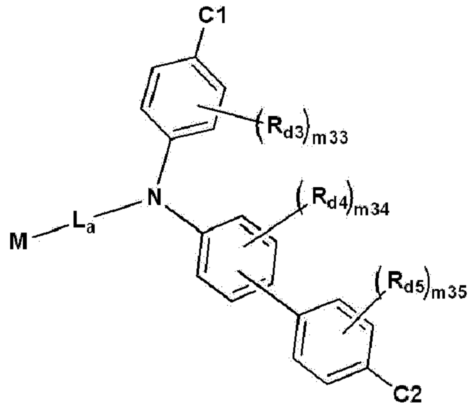

Formula 6-5

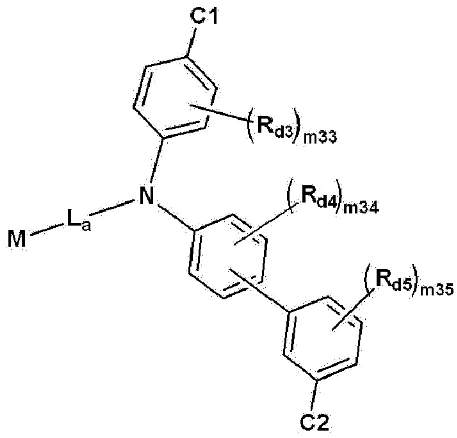

Formula 6-6

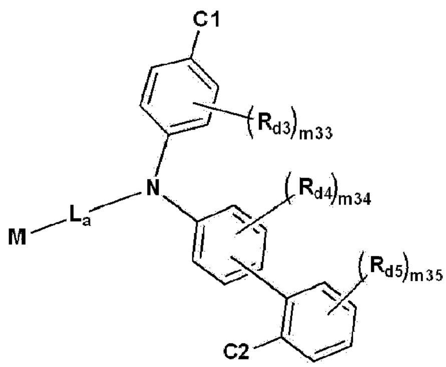

Formula 6-7

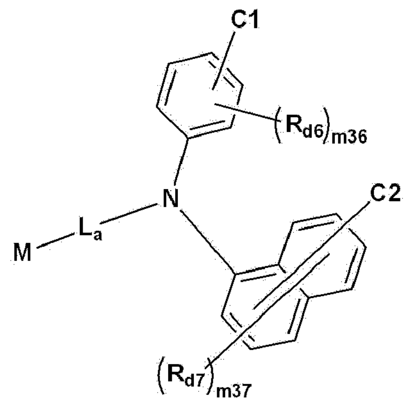

Formula 6-8

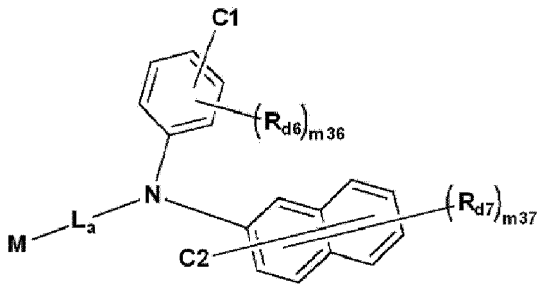

In Formula 6-1 to Formula 6-8, $R_{d1}$ to $R_{d7}$ are each independently a hydrogen atom, a deuterium atom, a halogen atom, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms, m31 to m36 are each independently an integer of 0 to 4, and m37 is an integer of 0 to 6.

In Formula 6-1 to Formula 6-8, the same description of M, $L_a$, C1, and C2 provided with respect to Formula 1 may be applied.

In an embodiment, C1 and C2 may be each independently represented by any one selected from among Formula 7-1 to Formula 7-8 below.

Formula 7-1

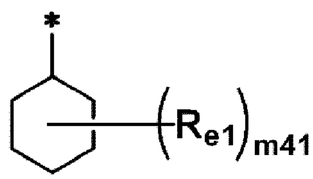

Formula 7-2

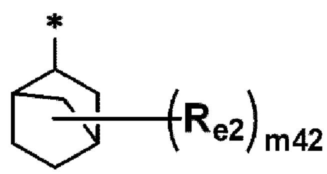

Formula 7-3

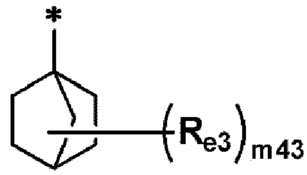

Formula 7-4

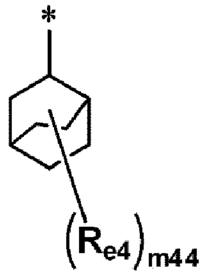

Formula 7-5

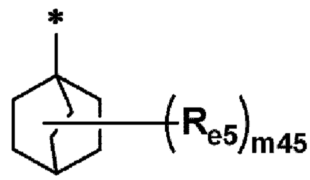

Formula 7-6

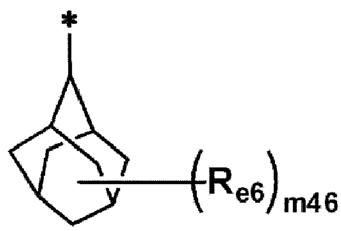

Formula 7-7

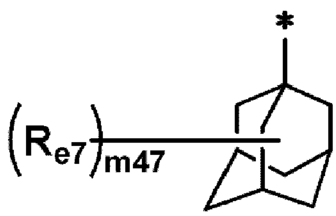

Formula 7-8

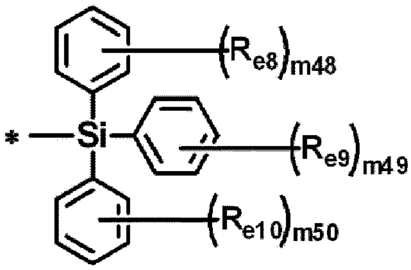

In Formula 7-1 to Formula 7-8, $R_{e1}$ to $R_{e10}$ are each independently a hydrogen atom, a deuterium atom, a halogen atom, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms, m41 to m43 are each independently an integer of 0 to 11, m44 and m45 are each independently an integer of 0 to 13, m46 and m47 are each independently an integer of 0 to 15, and m48 to m50 are each independently an integer of 0 to 5.

In an embodiment, the amine compound represented by Formula 1 may be represented by any one selected from among Formula 8-1 to Formula 8-5 below.

Formula 8-1

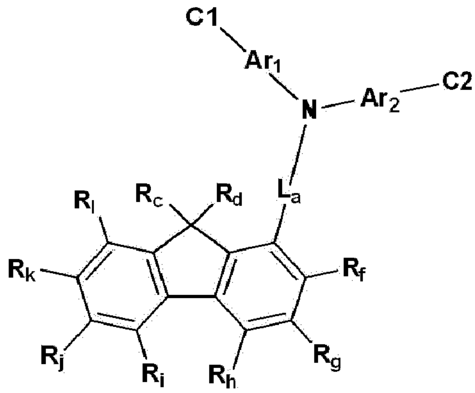

Formula 8-2

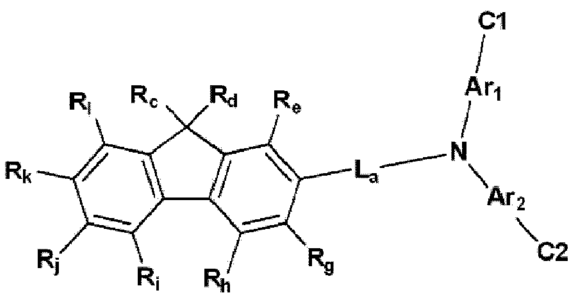

Formula 8-3

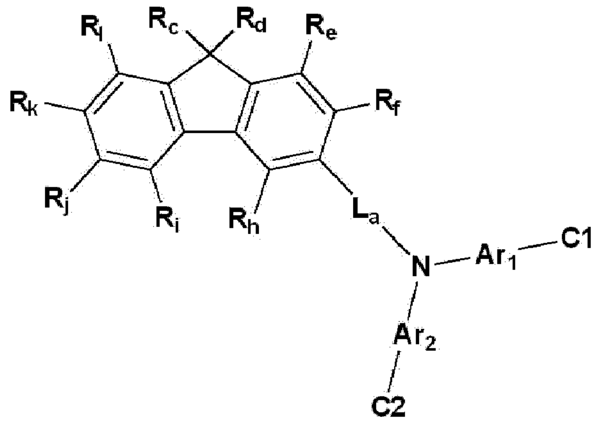

Formula 8-4

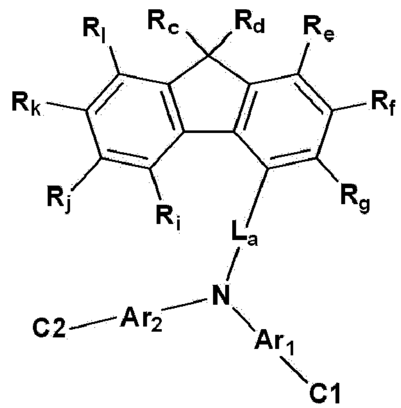

Formula 8-5

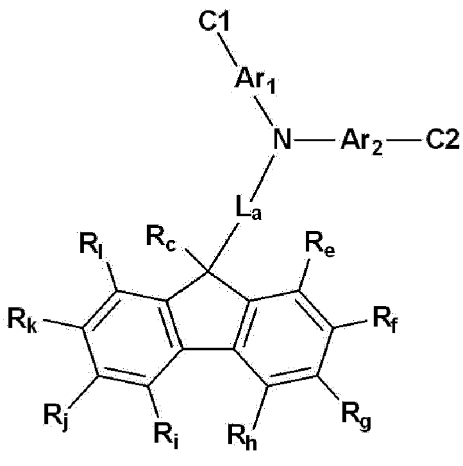

In Formula 8-1 to Formula 8-5, at least one selected from among C1 and C2 may be a substituted or unsubstituted bicycloheptanyl group.

In Formula 8-1 to Formula 8-5, the same description of $Ar_1$, $Ar_2$, $L_a$, C1, C2, and $R_c$ to $R_l$ provided with respect to Formula 1 and Formula 1-b may be applied.

In an embodiment, the amine compound represented by Formula 1 may be represented by any one selected from among Formula 9-1 to Formula 9-4 below.

Formula 9-1

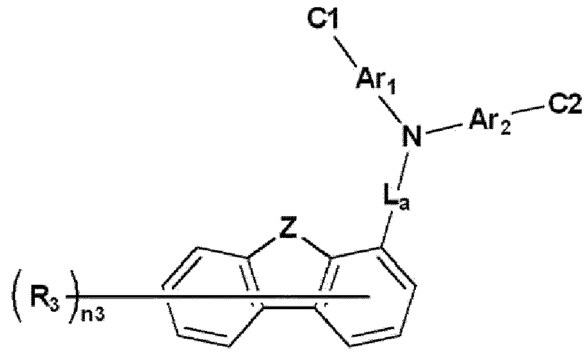

Formula 9-2

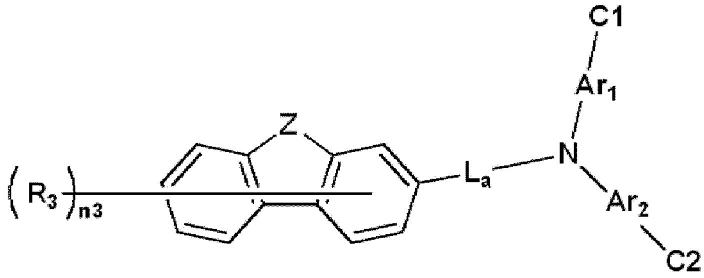

Formula 9-3

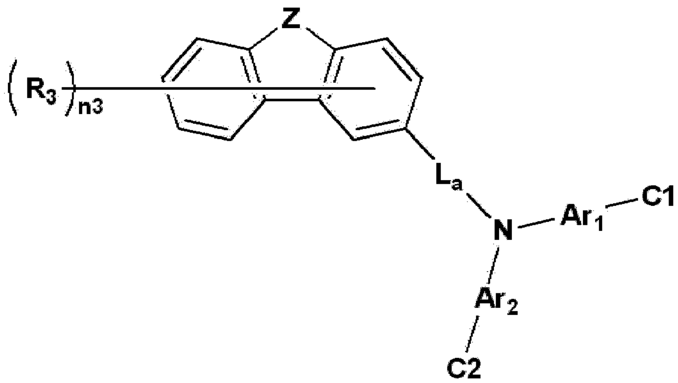

Formula 9-4

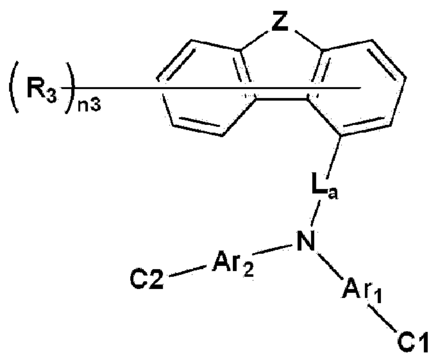

In Formula 9-1 to Formula 9-4, at least one selected from among C1 and C2 may be a substituted or unsubstituted

bicycloheptanyl group.

In Formula 9-1 to Formula 9-4, the same description of Z, $R_3$, $Ar_1$, $Ar_2$, $L_a$, n3, C1, and C2 provided with respect to Formula 1 and Formula 1-c may be applied.

In an embodiment, the amine compound may be at least one selected from among the compounds represented in Compound Group 1.

In an embodiment, the at least one functional layer may include an emission layer, a first hole transport layer between the first electrode and the emission layer, a second hole transport layer between the first hole transport layer and the emission layer, and an electron transport region between the emission layer and the second electrode, and the first hole transport layer may include the amine compound.

In an embodiment, the at least one functional layer may further include a third hole transport layer between the second hole transport layer and the emission layer, and the third hole transport layer may include the amine compound.

In an embodiment, the second hole transport layer may include an amine derivative compound represented by Formula 10 below.

Formula 10

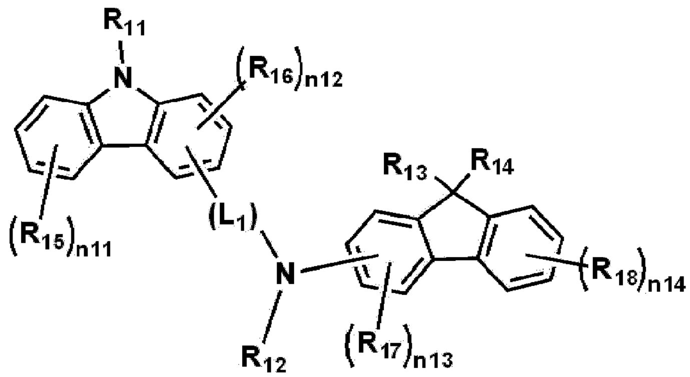

In Formula 10, $L_1$ is a direct linkage, a substituted or unsubstituted arylene group of 6 to 30 ring-forming carbon atoms, or a substituted or unsubstituted heteroarylene group of 2 to 30 ring-forming carbon atoms, $R_{11}$ to $R_{14}$ are each independently a substituted or unsubstituted alkyl group of 1 to 10 carbon atoms, a substituted or unsubstituted alkenyl group of 2 to 10 carbon atoms, a substituted or unsubstituted aryl group of 6 to 30 ring-forming carbon atoms, or a substituted or unsubstituted heteroaryl group of 2 to 30 ring-forming carbon atoms, or combined with an adjacent group to form a ring, $R_{15}$ to $R_{18}$ are each independently a hydrogen atom, a deuterium atom, a halogen atom, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms, or combined with an adjacent group to form a ring, n11 and n14 are each independently an integer of 0 to 4, and n12 and n13 are each independently an integer of 0 to 3.

In an embodiment, the amine derivative compound represented by Formula 10 may be represented by Formula 11-1 or Formula 11-2 below.

Formula 11-1

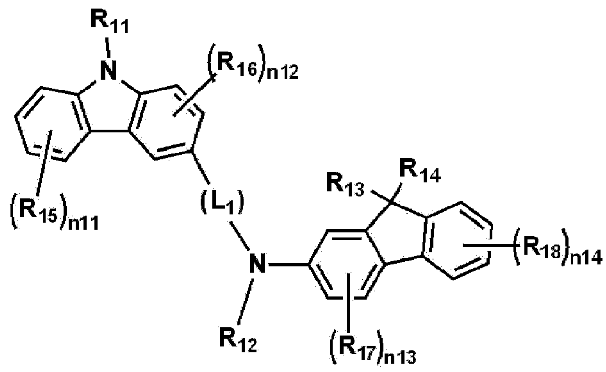

Formula 11-2

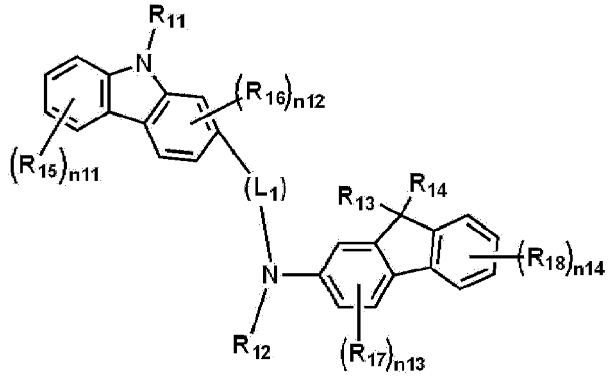

In Formula 11-1 and Formula 11-2, the same description of $L_1$, $R_{11}$ to $R_{14}$, $R_{15}$ to $R_{18}$, and n11 to n14 provided with respect to Formula 10 may be applied.

In an embodiment, the amine derivative compound represented by Formula 10 may be represented by any one selected from among compounds represented in Compound Group 2.

In an embodiment, the at least one functional layer may include an emission layer, a hole transport layer between the first electrode and the emission layer, and a hole transport auxiliary layer between the hole transport layer and the emission layer, and the hole transport auxiliary layer may include the amine compound.

In an embodiment, the hole transport auxiliary layer may include a first hole transport auxiliary layer on the hole transport layer, and a second hole transport auxiliary layer on the first hole transport auxiliary layer, and the first hole transport auxiliary layer may include the amine compound.

In an embodiment, a refractive index of the first hole transport auxiliary layer in a wavelength range of about 450 nm to about 700 nm may be about 1.55 to about 1.80, and a refractive index of the second hole transport auxiliary layer in a wavelength range of about 450 nm to about 700 nm may be about 1.65 to about 1.90.

In an embodiment, an absolute value of the highest occupied molecular orbital (HOMO) energy level of the second hole transport auxiliary layer may be greater than an absolute value of the HOMO energy level of the first hole transport auxiliary layer.

An amine compound according to an embodiment of the present disclosure is represented by Formula 1 above.

## BRIEF DESCRIPTION OF THE FIGURES

The accompanying drawings are included to provide a further understanding of the subject matter of the present disclosure and are incorporated in and constitute a part of this specification. The drawings illustrate embodiments of the present disclosure and, together with the description, serve to explain principles of the present disclosure. In the drawings:

FIG. 1 is a plan view of a display apparatus according to an embodiment of the present disclosure;
FIG. 2 is a cross-sectional view of a display apparatus according to an embodiment of the present disclosure;
FIG. 3 is a cross-sectional view schematically showing a light emitting device according to an embodiment of the present disclosure;
FIG. 4 is a cross-sectional view schematically showing a light emitting device according to an embodiment of the present disclosure;
FIG. 5 is a cross-sectional view schematically showing a light emitting device according to an embodiment of the present disclosure;
FIG. 6 is a cross-sectional view schematically showing a light emitting device according to an embodiment of the present disclosure;
FIG. 7 is a cross-sectional view schematically showing a light emitting device according to an embodiment of the present disclosure;
FIG. 8 is a cross-sectional view schematically showing a light emitting device according to an embodiment of the present disclosure;
FIG. 9 and FIG. 10 are respective cross-sectional views of display apparatuses according to embodiments;
FIG. 11 is a cross-sectional view showing a display apparatus according to an embodiment; and
FIG. 12 is a cross-sectional view showing a display apparatus according to an embodiment.

## DETAILED DESCRIPTION

The subject matter of the present disclosure may have various modifications and may be embodied in different forms, and example embodiments will be explained in more detail with reference to the accompany drawings. The subject matter of the present disclosure may, however, be embodied in different forms and should not be construed as limited to the embodiments set forth herein. Rather, the present disclosure is intended to cover all modifications, equivalents, and substituents which are included in the spirit and technical scope of the appended claims, and equivalents thereof.

Like reference numerals refer to like elements throughout. In the drawings, the dimensions of structures may be exaggerated for clarity of illustration. It will be understood that, although the terms first, second, etc. may be used herein to describe various elements, these elements should not be limited by these terms. These terms are only used to distinguish one element from another element. Thus, a first element could be termed a second element without departing from the spirit and scope of the present disclosure. Similarly, a second element could be termed a first element. As used herein, the singular forms are intended to include the plural forms as well, unless the context clearly indicates otherwise.

In the present description, it will be further understood that the terms "comprises" and/or "comprising," when used in this specification, specify the presence of stated features, numerals, steps, operations, elements, parts, or the combination thereof, but do not preclude the presence or addition of one or more other features, numerals, steps, operations, elements, parts, or the combination thereof.

In the present description, when a layer, a film, a region, a plate, etc. is referred to as being "on" or "above" another part, it can be "directly on" the other part, or intervening layers may also be present. On the contrary, when a layer, a film, a region, a plate, etc. is referred to as being "under" or "below" another part, it can be "directly under" the other part, or intervening layers may also be present. Also, when an element is referred to as being "on" another element, it can be under the other element.

In the present description, the term "substituted or unsubstituted" corresponds to substituted or unsubstituted with at least one substituent selected from the group consisting of a deuterium atom, a halogen atom, a cyano group, a nitro group, an amine group, a silyl group, an oxy group, a thio group, a sulfinyl group, a sulfonyl group, a carbonyl group, a boron group, a phosphine oxide group, a phosphine sulfide group, an alkyl group, an alkenyl group, an alkynyl group, an alkoxy group, a hydrocarbon ring group, an aryl group, and a heterocyclic group. In addition, each of the exemplified substituents may be substituted or unsubstituted. For example, a biphenyl group may be interpreted as an aryl group or a phenyl group substituted with a phenyl group.

In the present description, the term "forming a ring via the combination with an adjacent group" may mean forming a substituted or unsubstituted hydrocarbon ring, or a substituted or unsubstituted heterocycle via the combination with an adjacent group. The hydrocarbon ring includes an aliphatic hydrocarbon ring and an aromatic hydrocarbon ring. The heterocycle includes an aliphatic heterocycle and an aromatic heterocycle. The hydrocarbon ring and the heterocycle may be monocycles or polycycles. In addition, the ring formed via the combination with an adjacent group may be combined with another ring to form a spiro structure.

In the present description, the term "adjacent group" may mean a substituent substituted for an atom which is directly combined with an atom substituted with a corresponding substituent, another substituent substituted for an atom which is substituted with a corresponding substituent, or a substituent sterically positioned at the nearest position to a corresponding substituent. For example, in 1,2-dimethylbenzene, two methyl groups may be interpreted as "adjacent groups" to each other, and in 1,1-diethylcyclopentene, two ethyl groups may be interpreted as "adjacent groups" to each other. In addition, in 4,5-dimethylphenanthrene, two methyl groups may be interpreted as "adjacent groups" to each other.

In the present description, the halogen atom may be a fluorine atom, a chlorine atom, a bromine atom or an iodine atom.

In the present description, the alkyl group may be a linear, branched or cyclic type. The carbon number of the alkyl group may be 1 to 50, 1 to 30, 1 to 20, 1 to 10, or 1 to 6. Examples of the alkyl group may include methyl, ethyl, n-propyl, isopropyl, n-butyl, s-butyl, t-butyl, i-butyl, 2-ethylbutyl, 3,3-dimethylbutyl, n-pentyl, i-pentyl, neopentyl, t-pentyl, cyclopentyl, 1-methylpentyl, 3-methylpentyl, 2-ethylpentyl, 4-methyl-2-pentyl, n-hexyl, 1-methylhexyl, 2-ethylhexyl, 2-butylhexyl, cyclohexyl, 4-methylcyclohexyl, 4-t-butylcyclohexyl, n-heptyl, 1-methylheptyl, 2,2-dimethylheptyl, 2-ethylheptyl, 2-butylheptyl, n-octyl, t-octyl, 2-ethyloctyl, 2-butyloctyl, 2-hexyloctyl, 3,7-dimethyloctyl, cyclooctyl, n-nonyl, n-decyl, adamantyl, 2-ethyldecyl, 2-butyldecyl, 2-hexyldecyl, 2-octyldecyl, n-undecyl, n-dodecyl, 2-ethyldodecyl, 2-butyldodecyl, 2-hexyldocecyl, 2-octyldodecyl, n-tridecyl, n-tetradecyl, n-pentadecyl, n-hexadecyl, 2-ethylhexadecyl, 2-butylhexadecyl, 2-hexylhexadecyl, 2-octylhexadecyl, n-heptadecyl, n-octadecyl, n-nonadecyl, n-eicosyl, 2-ethyleicosyl, 2-butyleicosyl, 2-hexyleicosyl, 2-octyleicosyl, n-henicosyl, n-docosyl, n-tricosyl, n-tetracosyl, n-pentacosyl, n-hexacosyl, n-heptacosyl, n-octacosyl, n-nonacosyl, n-triacontyl, etc., without limitation.

In the present description, a cycloalkyl group may be a cyclic alkyl group. The carbon number of the cycloalkyl group may be 3 to 50, 3 to 30, 3 to 20, or 3 to 10. Examples of the cycloalkyl group include a cyclopropyl group, a cyclobutyl group, a cyclopentyl group, a cyclohexyl group, a 4-methylcyclohexyl group, a 4-t-butylcyclohexyl group, a

cycloheptyl group, a cyclooctyl group, a cyclononyl group, a cyclodecyl group, a norbornyl group, a 1-adamantyl group, a 2-adamantyl group, an isobornyl group, a bicycloheptanyl group, a bicyclooctanyl group, etc., without limitation.

In the present description, an aryl group may be an optional functional group or substituent derived from an aromatic hydrocarbon ring. The aryl group may be a monocyclic aryl group or a polycyclic aryl group. The carbon number for forming rings in the aryl group may be 6 to 30, 6 to 20, or 6 to 15. Examples of the aryl group may include phenyl, naphthyl, fluorenyl, anthracenyl, phenanthryl, biphenyl, terphenyl, quaterphenyl, quinquephenyl, sexiphenyl, triphenylenyl, pyrenyl, benzofluoranthenyl, chrysenyl, etc., without limitation.

In the present description, a fluorenyl group may be substituted, and two substituents may be combined with each other to form a spiro structure. Examples of a substituted fluorenyl group are as follows, but an embodiment of the present disclosure is not limited thereto.

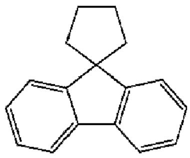
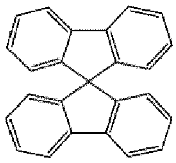
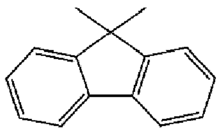
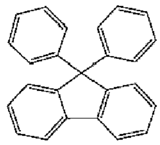

In the present description, a heteroaryl group may include one or more selected from among B, O, N, P, Si and S as heteroatoms. If the heteroaryl group includes two or more heteroatoms, two or more heteroatoms may be the same or different. The heteroaryl group may be a monocyclic heterocyclic group or polycyclic heterocyclic group. The carbon number for forming rings of the heteroaryl group may be 2 to 30, 2 to 20, or 2 to 10. Examples of the heteroaryl group may include thiophene, furan, pyrrole, imidazole, pyridine, bipyridine, pyrimidine, triazine, triazole, acridyl, pyridazine, pyrazinyl, quinoline, quinazoline, quinoxaline, phenoxazine, phthalazine, pyrido pyrimidine, pyrido pyrazine, pyrazino pyrazine, isoquinoline, indole, carbazole, N-arylcarbazole, N-heteroarylcarbazole, N-alkylcarbazole, benzoxazole, benzoimidazole, benzothiazole, benzocarbazole, benzothiophene, dibenzothiophene, thienothiophene, benzofuran, phenanthroline, thiazole, isooxazole, oxazole, oxadiazole, thiadiazole, phenothiazine, dibenzosilole, dibenzofuran, etc., without limitation.

In the present description, the explanation of the aryl group may be applied to an arylene group except that the arylene group is a divalent group. The explanation on the heteroaryl group may be applied to a heteroarylene group except that the heteroarylene group is a divalent group.

In the present description, a silyl group may include an alkyl silyl group and an aryl silyl group. Examples of the silyl group include a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group, etc., without limitation.

In the present description, the carbon number of an amine group is not specifically limited, but may be 1 to 30. The amine group may include an alkyl amine group and an aryl amine group. Examples of the amine group include a methylamine group, a dimethylamine group, a phenylamine group, a diphenylamine group, a naphthylamine group, a 9-methyl-anthracenylamine group, a triphenylamine group, etc., without limitation.

In the present description, a direct linkage may be a single bond.

In the present description, "-*" means a position to be connected.

Hereinafter, embodiments of the present disclosure will be further explained by referring to the drawings.

FIG. 1 is a plan view showing an embodiment of a display apparatus DD. FIG. 2 is a cross-sectional view of a display apparatus DD of an embodiment. FIG. 2 is a cross-sectional view showing a part corresponding to line I-I' in FIG. 1.

The display apparatus DD may include a display panel DP and an optical layer PP on the display panel DP. The display panel DP includes light emitting devices ED-1, ED-2 and ED-3. The display apparatus DD may include multiple light emitting devices ED-1, ED-2 and ED-3. The optical layer PP may be on the display panel DP and control reflected light from external light at the display panel DP. The optical layer PP may include, for example, a polarization layer or a color filter layer. In some embodiments, different from the drawings, the optical layer PP may be omitted in the display apparatus DD of an embodiment.

A base substrate BL may be on the optical layer PP. The base substrate BL may be a member providing a base surface that the optical layer PP is on. The base substrate BL may be a glass substrate, a metal substrate, a plastic substrate, etc. However, an embodiment of the present disclosure is not limited thereto, and the base substrate BL may be an inorganic layer, an organic layer or a composite material layer. In addition, different from the drawings, the base substrate BL may be omitted in an embodiment.

The display apparatus DD according to an embodiment may further include a plugging layer. The plugging layer may be between a display device layer DP-ED and a base substrate BL. The plugging layer may be an organic layer. The plugging layer may include at least one selected from among an acrylic resin, a silicon-based resin and an epoxy-based resin.

The display panel DP may include a base layer BS, a circuit layer DP-CL provided on the base layer BS and a display device layer DP-ED. The display device layer DP-ED may include a pixel definition layer PDL, light emitting devices ED-1, ED-2 and ED-3 in the pixel definition layer PDL, and an encapsulating layer TFE on the light emitting devices ED-1, ED-2 and ED-3.

The base layer BS may be a member providing a base surface that the display device layer DP-ED is on. The base layer BS may be a glass substrate, a metal substrate, a plastic substrate, etc. However, an embodiment of the present disclosure is not limited thereto, and the base layer BS may be an inorganic layer, an organic layer or a composite material layer.

In an embodiment, the circuit layer DP-CL is on the base layer BS, and the circuit layer DP-CL may include multiple transistors. Each of the transistors may include a control electrode, an input electrode, and an output electrode. For example, the circuit layer DP-CL may include switching transistors and driving transistors for driving the light emitting devices ED-1, ED-2 and ED-3 of the display device layer DP-ED.

Each of the light emitting devices ED-1, ED-2 and ED-3 may have the structures of light emitting devices ED of embodiments according to FIG. 3 to FIG. 8, which will be further explained herein below. Each of the light emitting devices ED-1, ED-2 and ED-3 may include a first electrode EL1, a hole transport region HTR, emission layers EML-R, EML-G and EML-B, an electron transport region ETR, and a second electrode EL2.

FIG. 2 shows an embodiment where the emission layers EML-R, EML-G and EML-B of light emitting devices ED-1, ED-2 and ED-3 are in opening portions OH defined in a pixel definition layer PDL, and a hole transport region HTR, an electron transport region ETR and a second electrode EL2 are provided as common layers in all light emitting devices ED-1, ED-2 and ED-3. However, an embodiment of the present disclosure is not limited thereto. Different from FIG. 2, in an embodiment, the hole transport region HTR and the electron transport region ETR may be patterned and provided in the opening portions OH defined in the pixel definition layer PDL. For example, in an embodiment, the hole transport region HTR, the emission layers EML-R, EML-G and EML-B, and the electron transport region ETR of the light emitting devices ED-1, ED-2 and ED-3 may be patterned by an ink jet printing method and provided.

An encapsulating layer TFE may cover the light emitting devices ED-1, ED-2 and ED-3. The encapsulating layer TFE may encapsulate the display device layer DP-ED. The encapsulating layer TFE may be a thin film encapsulating layer. The encapsulating layer TFE may be one layer or a stacked layer of multiple layers. The encapsulating layer TFE includes at least one insulating layer. The encapsulating layer TFE according to an embodiment may include at least one inorganic layer (hereinafter, referred to as an encapsulating inorganic layer). In addition, the encapsulating layer TFE according to an embodiment may include at least one organic layer (hereinafter, encapsulating organic layer) and at least one encapsulating inorganic layer.

The encapsulating inorganic layer protects the display device layer DP-ED from moisture/oxygen, and the encapsulating organic layer protects the display device layer DP-ED from foreign materials such as dust particles. The encapsulating inorganic layer may include silicon nitride, silicon oxy nitride, silicon oxide, titanium oxide, and/or aluminum oxide, without specific limitation. The encapsulating organic layer may include an acrylic compound, an epoxy-based compound, etc. The encapsulating organic layer may include a photopolymerizable organic material, without specific limitation.

The encapsulating layer TFE may be on the second electrode EL2 and may be provided while filling the opening portion OH.

Referring to FIG. 1 and FIG. 2, the display apparatus DD may include a non-luminous area NPXA and luminous areas PXA-R, PXA-G and PXA-B. The luminous areas PXA-R, PXA-G and PXA-B may be areas that emit light produced from the light emitting devices ED-1, ED-2 and ED-3, respectively. The luminous areas PXA-R, PXA-G and PXA-B may be spaced apart from each other on a plane.

The luminous areas PXA-R, PXA-G and PXA-B may be areas spaced apart by the pixel definition layer PDL. The non-luminous areas NPXA may be areas between neighboring luminous areas PXA-R, PXA-G and PXA-B and may be areas corresponding to the pixel definition layer PDL. In the present disclosure, each of the luminous areas PXA-R, PXA-G and PXA-B may correspond to each pixel. The pixel definition layer PDL may divide the light emitting devices ED-1, ED-2 and ED-3. The emission layers EML-R, EML-G and EML-B of the light emitting devices ED-1, ED-2 and ED-3 may be respectively provided and divided in the opening portions OH defined in the pixel definition layer PDL.

The luminous areas PXA-R, PXA-G and PXA-B may be divided into multiple groups according to the color of light produced from the light emitting devices ED-1, ED-2 and ED-3. In the display apparatus DD of an embodiment, shown in FIG. 1 and FIG. 2, three luminous areas PXA-R, PXA-G and PXA-B that emit red light, green light and blue light, respectively, are illustrated as an embodiment. For example, the display apparatus DD of an embodiment may include a red luminous area PXA-R, a green luminous area PXA-G and a blue luminous area PXA-B, which are spaced apart from each other.

In the display apparatus DD according to an embodiment, multiple light emitting devices ED-1, ED-2 and ED-3 may emit light having different wavelength regions. For example, in an embodiment, the display apparatus DD may include a first light emitting device ED-1 emitting red light, a second light emitting device ED-2 emitting green light, and

a third light emitting device ED-3 emitting blue light. In some embodiments, the red luminous area PXA-R, the green luminous area PXA-G, and the blue luminous area PXA-B of the display apparatus DD may correspond to the first light emitting device ED-1, the second light emitting device ED-2, and the third light emitting device ED-3, respectively.

However, an embodiment of the present disclosure is not limited thereto, and the first to third light emitting devices ED-1, ED-2 and ED-3 may emit light in the same wavelength region, or at least one thereof may emit light in a different wavelength region. For example, all the first to third light emitting devices ED-1, ED-2 and ED-3 may emit blue light.

The luminous areas PXA-R, PXA-G and PXA-B in the display apparatus DD according to an embodiment may be arranged in a stripe shape. Referring to FIG. 1, multiple red luminous areas PXA-R, multiple green luminous areas PXA-G and multiple blue luminous areas PXA-B may be arranged along a second directional axis DR2. In addition, the red luminous area PXA-R, the green luminous area PXA-G and the blue luminous area PXA-B may be arranged by turns along a first directional axis DR1.

In FIG. 1 and FIG. 2, the areas of the luminous areas PXA-R, PXA-G and PXA-B are shown similar, but an embodiment of the present disclosure is not limited thereto. The areas of the luminous areas PXA-R, PXA-G and PXA-B may be different from each other according to the wavelength region of light emitted. The areas of the luminous areas PXA-R, PXA-G and PXA-B may mean areas on a plane defined by the first directional axis DR1 and the second directional axis DR2.

The arrangement type of the luminous areas PXA-R, PXA-G and PXA-B is not limited to the configuration shown in FIG. 1, and the arrangement order of the red luminous areas PXA-R, the green luminous areas PXA-G and the blue luminous areas PXA-B may be provided in various suitable combinations according to the properties of display quality required or desired for the display apparatus DD. For example, the arrangement type of the luminous areas PXA-R, PXA-G and PXA-B may be a PENTILE® arrangement structure (e.g., an RGBG matrix, RGBG structure, or RGBG matrix structure), or a diamond arrangement structure. PENTILE® is a duly registered trademark of Samsung Display Co., Ltd.

In addition, the areas of the luminous areas PXA-R, PXA-G and PXA-B may be different from each other. For example, in an embodiment, the area of the green luminous area PXA-G may be smaller than the area of the blue luminous area PXA-B, but an embodiment of the present disclosure is not limited thereto.

Hereinafter, FIG. 3 to FIG. 8 are cross-sectional views schematically showing light emitting devices according to embodiments. The light emitting device ED according to an embodiment may include a first electrode EL1, a second electrode EL2 facing the first electrode EL1, and at least one functional layer between the first electrode EL1 and the second electrode EL2. The at least one functional layer may include a hole transport region HTR, an emission layer EML, and an electron transport region ETR stacked in order. In some embodiments, the light emitting device ED of an embodiment may include a first electrode EL1, a hole transport region HTR, an emission layer EML, an electron transport region ETR, and a second electrode EL2 stacked in order.

When compared with FIG. 3, FIG. 4 shows the cross-sectional view of a light emitting device ED of an embodiment, wherein a hole transport region HTR includes a hole injection layer HIL and a hole transport layer HTL, and an electron transport region ETR includes an electron injection layer EIL and an electron transport layer ETL. In addition, when compared with FIG. 3, FIG. 5 shows the cross-sectional view of a light emitting device ED of an embodiment, wherein a hole transport region HTR includes a hole injection layer HIL, a hole transport layer HTL, and an electron blocking layer EBL, and an electron transport region ETR includes an electron injection layer EIL, an electron transport layer ETL, and a hole blocking layer HBL. When compared with FIG. 4, FIG. 6 shows the cross-sectional view of a light emitting device ED of an embodiment, including a capping layer CPL on the second electrode EL2. When compared with FIG. 4, FIG. 7 shows the cross-sectional view of a light emitting device ED of an embodiment, including multiple hole transport layers HTL1, HTL2 and HTL3. When compared with FIG. 4, FIG. 8 shows the cross-sectional view of a light emitting device ED of an embodiment, wherein a hole transport region HTR includes a hole transport auxiliary layer HTAL between an emission layer EML and a hole transport layer HTL.

The light emitting device ED of an embodiment may include the amine compound of an embodiment, which will be further explained herein below, in at least one functional layer such as a hole transport region HTR, an emission layer EML, and an electron transport region ETR.

The first electrode EL1 has conductivity (e.g., electrical conductivity). The first electrode EL1 may be formed using a metal material, a metal alloy and/or a conductive compound. The first electrode EL1 may be an anode or a cathode. However, an embodiment of the present disclosure is not limited thereto. In addition, the first electrode EL1 may be a pixel electrode. The first electrode EL1 may be a transmissive electrode, a transflective electrode, or a reflective electrode. The first electrode EL1 may include at least one selected from among Ag, Mg, Cu, Al, Pt, Pd, Au, Ni, Nd, Ir, Cr, Li, Ca, LiF, Mo, Ti, W, In, Sn, and Zn, compounds of two or more selected therefrom, mixtures of two or more selected therefrom, or oxides thereof.

If the first electrode EL1 is the transmissive electrode, the first electrode EL1 may include a transparent metal oxide such as indium tin oxide (ITO), indium zinc oxide (IZO), zinc oxide (ZnO), and indium tin zinc oxide (ITZO). If the

first electrode EL1 is the transflective electrode or the reflective electrode, the first electrode EL1 may include Ag, Mg, Cu, Al, Pt, Pd, Au, Ni, Nd, Ir, Cr, Li, Ca, LiF/Ca (a stacked structure of LiF and Ca), LiF/Al (a stacked structure of LiF and Al), Mo, Ti, W, compounds thereof, or mixtures thereof (for example, a mixture of Ag and Mg). Also, the first electrode EL1 may have a structure including multiple layers including a reflective layer or a transflective layer formed using the above materials, and a transmissive conductive layer formed using ITO, IZO, ZnO, or ITZO. For example, the first electrode EL1 may include a three-layer structure of ITO/Ag/ITO. However, an embodiment of the present disclosure is not limited thereto. The first electrode EL1 may include the above-described metal materials, combinations of two or more metal materials selected from the above-described metal materials, or oxides of the above-described metal materials. The thickness of the first electrode EL1 may be from about 700 Å to about 10,000 Å. For example, the thickness of the first electrode EL1 may be from about 1,000 Å to about 3,000 Å.

The hole transport region HTR is provided on the first electrode EL1. The hole transport region HTR may include at least one of a hole injection layer HIL, a hole transport layer HTL, a buffer layer or an emission auxiliary layer, or an electron blocking layer EBL.

The hole transport region HTR may have a single layer formed using a single material, a single layer formed using multiple different materials, or a multilayer structure including multiple layers formed using multiple different materials.

For example, the hole transport region HTR may have the structure of a single layer of a hole injection layer HIL or a hole transport layer HTL, and may have a structure of a single layer formed using a hole injection material and a hole transport material. In some embodiments, the hole transport region HTR may have a structure of a single layer formed using multiple different materials, or a structure stacked from the first electrode EL1 of hole injection layer HIL/hole transport layer HTL, hole injection layer HIL/hole transport layer HTL/buffer layer, hole injection layer HIL/buffer layer, hole transport layer HTL/buffer layer, or hole injection layer HIL/hole transport layer HTL/electron blocking layer EBL, without limitation.

If the hole transport region HTR includes the hole transport layer HTL, the hole transport layer HTL may have a single layer formed using a single material, a single layer formed using multiple different materials, or a multiple structure having multiple layers having multiple different materials. If the hole transport layer HTL has a multiple structure (e.g., a multilayer structure), as shown in FIG. 7, the hole transport layer HTL may include first to third hole transport layers HTL1, HTL2 and HTL3. The hole transport layer HTL may include a first hole transport layer HTL1 on the first electrode EL1, a second hole transport layer HTL2 on the first hole transport layer HTL1, and a third hole transport layer HTL3 on the second hole transport layer HTL2. In the light emitting device ED of an embodiment, the hole transport layer HTL may include multiple hole transport layers HTL1, HTL2 and HTL3 in the order of first hole transport layer HTL1/second hole transport layer HTL2/third hole transport layer HTL3 in a thickness direction.

Referring to FIG. 7, in the light emitting device ED of an embodiment, the hole transport region HTR may include a first hole transport layer HTL1 adjacent to a hole injection layer HIL, a third hole transport layer HTL3 adjacent to an emission layer EML, and a second hole transport layer HTL2 between the first hole transport layer HTL1 and the third hole transport layer HTL3. Each of the first hole transport layer HTL1 and the third hole transport layer HTL3 may include the amine compound of an embodiment, represented by Formula 1. The second hole transport layer HTL2 may include an amine derivative compound of an embodiment, represented by Formula 10.

In an embodiment, the first hole transport layer HTL1 and the third hole transport layer HTL3 may be layers having a refractive index smaller than that of the second hole transport layer HTL2. The first refractive index of the first hole transport layer HTL1 may be smaller than the second refractive index of the second hole transport layer HTL2, and the third refractive index of the third hole transport layer HTL3 may be smaller than the second refractive index of the second hole transport layer HTL2.

The hole transport region HTR may include the first to third hole transport layers HTL1, HTL2 and HTL3. Based on the second hole transport layer HTL2 having a relatively higher refractive index when compared to the first and third hole transport layers HTL1 and HTL3, the first hole transport layer HTL1 may be under the second hole transport layer HTL2, and the third hole transport layer HTL3 may be on the second hole transport layer HTL2. In the light emitting device ED of an embodiment, the hole transport region HTR may include multiple hole transport layers HTL1, HTL2 and HTL3 in the order of hole transport layer having a low refractive index/hole transport layer having a high refractive index/hole transport layer having a low refractive index in a thickness direction.

At a wavelength of about 460 nm, a difference between the first refractive index of the first hole transport layer HTL1 and the second refractive index of the second hole transport layer HTL2 may be greater than about 0.1. For example, at a wavelength of about 460 nm, a difference between the first refractive index and the second refractive index may be about 0.2 or more. In addition, at a wavelength of about 460 nm, a difference between the third refractive index of the third hole transport layer HTL3 and the second refractive index of the second hole transport layer HTL2 may be greater than about 0.1. For example, at a wavelength of about 460 nm, a difference between the third refractive index and the second refractive index may be about 0.2 or more.

At a wavelength of about 460 nm, the first refractive index of the first hole transport layer HTL1 and the third

refractive index of the third hole transport layer HTL3 may be about 1.30 to about 1.80 for each. In addition, at a wavelength of about 460 nm, the second refractive index of the second hole transport layer HTL2 may be about 1.85 to about 2.40. For example, the first refractive index of the first hole transport layer HTL1 and the third refractive index of the third hole transport layer HTL3 may be about 1.40 to about 1.60 for each, and the second refractive index of the second hole transport layer HTL2 may be about 1.90 to about 2.00.

The thickness of the hole transport region HTR may be about 300 Å to about 15,000 Å, for example, the thickness of the hole transport region HTR may be about 300 Å to about 5,000 Å. The thicknesses D1, D2 and D3 of the first to third hole transport layers HTL1, HTL2 and HTL3, included in the hole transport region HTR may be about 100 Å to about 1,000 Å for each.

The thickness ratio (D1:D2:D3) of the first to third hole transport layers HTL1, HTL2 and HTL3, included in the hole transport region HTR may be about 0.1:0.8:0.1 to about 0.45:0.1:0.45. For example, in an embodiment, the thickness (D1) of the first hole transport layer HTL1 and the thickness (D3) of the third hole transport layer HTL3 may be substantially the same, and the thickness (D2) of the second hole transport layer HTL2 may be different from the thickness (D1) of the first hole transport layer HTL1 and the thickness (D3) of the third hole transport layer HTL3. However, an embodiment of the present disclosure is not limited thereto, and the thickness (D1) of the first hole transport layer HTL1 and the thickness (D3) of the third hole transport layer HTL3 may be different from each other. The thickness ratio (D1:D2:D3) of the first to third hole transport layers HTL1, HTL2 and HTL3 may be controlled to a suitable or optimal range according to the wavelength region of light emitted from the emission layer EML, the display quality required or desired for the display apparatus DD (FIG. 2), and the type of the hole transport materials used in each of the hole transport layers HTL1, HTL2 and HTL3 of the hole transport region HTR.

For example, in the light emitting device ED of an embodiment, if the emission layer EML emits blue light having a central wavelength in a wavelength region of about 430 nm to about 470 nm, the thickness ratio (D1 :D2:D3) of the first to third hole transport layers HTL1, HTL2 and HTL3 may be 1:1:1.

The light emitting device ED of an embodiment may include multiple hole transport layers HTL1, HTL2 and HTL3 in the order of hole transport layer having a low refractive index/hole transport layer having a high refractive index/hole transport layer having a low refractive index, and may show improved emission efficiency properties. The light emitting device ED of an embodiment includes the hole transport layers HTL1, HTL2 and HTL3 of the hole transport region HTR, having refractive index differences to minimize or reduce the destructive interference and extinction of light emitted from internal functional layers and to provide or improve constructive interference by the hole transport layers HTL1 HTL2 and HTL3, having refractive index differences, thereby showing high light extraction efficiency.

In some embodiments, the first hole transport layer HTL1 may be directly on the first electrode EL1. In addition, the third hole transport layer HTL3 may be directly under the emission layer EML.

In the present description, when a layer, a film, a region, a plate, etc. is referred to as being "directly on" the other part, it can mean that intervening layers, films, regions, plates, etc. are not present. For example, the "directly on" means that two layers are provided without using an additional member such as an adhesion member therebetween.

At a wavelength of about 460 nm in the light emitting device ED of an embodiment, the refractive index of the first electrode EL1 may be about 1.80 to about 2.40. For example, the refractive index of the first electrode EL1 may be about 1.90 to about 2.00. In some embodiments, the refractive index of the first electrode EL1 may be greater than the first refractive index of the first hole transport layer HTL1, and the refractive index difference at about 460 nm between adjacent first hole transport layer HTL1 and first electrode EL1 may be greater than about 0.1.

In addition, in the light emitting device ED of an embodiment, the refractive index of the emission layer EML at a wavelength of about 460 nm may be about 1.80 to about 2.24. For example, the refractive index of the emission layer EML may be about 1.90 to about 2.00. In some embodiments, the refractive index of the emission layer EML may be greater than the third refractive index of the third hole transport layer HTL3, and the refractive index difference at about 460 nm between adjacent third hole transport layer HTL3 and emission layer EML may be greater than about 0.1.

In some embodiments, the light emitting device ED of an embodiment includes a hole transport region HTR which includes hole transport layers HTL1 and HTL3, having refractive index differences from neighboring first electrode EL1 or emission layer EML, and may show high light extraction efficiency properties and improved emission efficiency properties.

The first hole transport layer HTL1 and the third hole transport layer HTL3 may each independently include an amine compound represented by Formula 1. The amine compound represented by Formula 1 may have a refractive index value of about 1.30 to about 1.80 at a wavelength of about 460 nm. Each of the first hole transport layer HTL1 and the third hole transport layer HTL3 may be formed using any one of the amine compounds represented by Formula 1, or mixtures thereof.

Referring to FIG. 8, the hole transport region HTR may further include a hole transport auxiliary layer HTAL between the emission layer EML and the hole transport layer HTL. The hole transport auxiliary layer HTAL may include a first hole transport auxiliary layer HTAL1 on the hole transport layer HTL, and a second hole transport auxiliary layer HTAL2 between the first hole transport auxiliary layer HTAL1 and the emission layer EML. The first hole transport

auxiliary layer HTAL1 may include the amine compound of an embodiment, represented by Formula 1.

In an embodiment, the first hole transport auxiliary layer HTAL1 may be a layer having a smaller refractive index than the second hole transport auxiliary layer HTAL2. The fourth refractive index of the first hole transport auxiliary layer HTAL1 may be smaller than the fifth refractive index of the second hole transport auxiliary layer HTAL2.

At a wavelength of about 450 nm to about 700 nm, the difference between the fourth refractive index of the first hole transport auxiliary layer HTAL1 and the fifth refractive index of the second hole transport auxiliary layer HTAL2 may be greater than about 0.1. For example, at a wavelength of about 450 nm to about 700 nm, the difference between the fourth refractive index and the fifth refractive index may be about 0.2 or more. At a wavelength of about 450 nm to about 700 nm, the fourth refractive index of the first hole transport auxiliary layer HTAL1 may be about 1.55 to about 1.80. In addition, at a wavelength of about 450 nm to about 700 nm, the fifth refractive index of the second hole transport auxiliary layer HTAL2 may be about 1.65 to about 1.90.

In an embodiment, the first hole transport auxiliary layer HTAL1 may be directly on the hole transport layer HTL. In addition, the second hole transport auxiliary layer HTAL2 may be directly under the emission layer EML.

In an embodiment, the second hole transport auxiliary layer HTAL2 may have the highest occupied molecular orbital (HOMO) energy level which is deeper than that of the first hole transport auxiliary layer HTAL1.

In an embodiment, the HOMO energy levels of the hole transport layer HTL, the first hole transport auxiliary layer HTAL1, and the second hole transport auxiliary layer HTAL2 may satisfy Equation 1 below.

Equation 1

$$HOMO_{HTL} \geq HOMO_{HTAL1} > HOMO_{HTAL2}$$

In Equation 1, $HOMO_{HTL}$ represents the HOMO energy level of the hole transport layer HTL, $HOMO_{HTAL1}$ represents the HOMO energy level of the first hole transport auxiliary layer HTAL1, and $HOMO_{HTAL2}$ represents the HOMO energy level of the second hole transport auxiliary layer HTAL2.

Referring to Equation 1, the HOMO energy level of the first hole transport auxiliary layer HTAL1 may be the same as the HOMO energy level of the hole transport layer HTL or deeper than the HOMO energy level of the hole transport layer. In addition, the HOMO energy level of the second hole transport auxiliary layer HTAL2 may be deeper than the HOMO energy level of the first hole transport auxiliary layer HTAL1. Because the first hole transport auxiliary layer HTAL1 which is adjacent to the hole transport layer HTL has the same as or deeper than the HOMO energy level of the hole transport layer HTL, holes may be easily injected. Because the second hole transport auxiliary layer HTAL2 has a deeper HOMO energy level than the first hole transport auxiliary layer HTAL1, the energy barrier of the emission layer EML becomes small, and holes may easily move to the emission layer EML.

In an embodiment, the HOMO energy level of the first hole transport auxiliary layer HTAL1 may be about -5.05 eV to about -5.30 eV. In addition, the HOMO energy level of the second hole transport auxiliary layer HTAL2 may be about -5.20 eV to about -5.40 eV. The light emitting device ED of an embodiment includes first and second hole transport auxiliary layers HTAL1 and HTAL2, having different energy levels, and hole transport capacity is improved, and excellent emission efficiency and improved device-life characteristics may be shown.

The first hole transport auxiliary layer HTAL1 may include the amine compound represented by Formula 1. The amine compound represented by Formula 1 may have the HOMO energy level of about -5.05 eV to about -5.30 eV. The first hole transport auxiliary layer HTAL1 may be formed using any one selected from among the amine compounds represented by Formula 1, or mixtures thereof.

In the light emitting device ED of an embodiment, the hole transport region HTR may include the amine compound of an embodiment. In the light emitting device ED of an embodiment, the hole transport layer HTL may include the amine compound represented by Formula 1. In addition, as shown in FIG. 7, if the light emitting device ED includes multiple hole transport layers HTL1, HTL2 and HTL3, the first hole transport layer HTL1 and/or the third hole transport layer HTL3 may include the amine compound represented by Formula 1.

The amine compound of an embodiment has a structure in which an amine group is connected with a first fused ring core and essentially includes a first substituent and a second substituent in a molecular structure. In an embodiment, the first fused ring may be a substituted or unsubstituted fluorenyl group, a substituted or unsubstituted dibenzofuran group, a substituted or unsubstituted dibenzothiophene group, or a substituted or unsubstituted carbazole group. In an embodiment, the first substituent and the second substituent may be each independently one substituent selected from among a substituted or unsubstituted adamantyl group, a substituted or unsubstituted cyclohexyl group, a substituted or unsubstituted cycloheptanyl group, a substituted or unsubstituted bicyclooctanyl group, and a substituted or unsubstituted triphenylsilyl group. In the present disclosure, the first fused ring may be any one substituent selected from among Formula 1-a to Formula 1-c, which will be further explained herein below.

The amine compound of an embodiment includes an amine group, and has a structure in which the nitrogen

atom of the amine group is connected with one carbon atom selected from among the carbon atoms composing the first fused ring. The first fused ring and the nitrogen atom of the amine group may be directly linked, or combined via a first connecting group. The first substituent and the second substituent may be combined with the amine group of the amine compound of an embodiment via a second connecting group and a third connecting group, respectively. In an embodiment, the first connecting group to the third connecting group may be substituted or unsubstituted arylene groups of 6 to 30 ring-forming carbon atoms, or substituted or unsubstituted heteroarylene groups of 2 to 30 ring-forming carbon atoms. Because the amine compound of an embodiment, having such a structure shows high thermal properties, if applied to the light emitting device ED of an embodiment, the improvement of device life and efficiency may be achieved, holes and electrons in an emission layer EML may be balanced due to excellent charge transport capacity, the deposition temperature may be reduced during the deposition of the device, and thin film stability may be improved. In addition, the amine compound of an embodiment includes the first substituent and the second substituent, and the intermolecular interaction may be reduced, and low packing density may be achieved. Accordingly, the amine compound of an embodiment may have a low refractive index, and through diversely changing the combination of the first substituent and the second substituent, the refractive index of the molecule may be changed.

The amine compound of an embodiment may include at least one third substituent connected with the first fused ring, or may include a structure in which at least one selected from among the first substituent and the second substituent, which is connected with the nitrogen atom of the amine group, is a substituted or unsubstituted bicycloheptanyl group.

The amine compound of an embodiment may include at least one third substituent connected with the first fused ring. The third substituent may be a group connected with at least one benzene ring selected from among the benzene rings composing the first fused ring to expand a $\pi$-conjugation structure. For example, the third substituent may be a substituent having at least one or more $\pi$ bonds which may be connected with the first fused ring and conjugated. In an embodiment, the third substituent may be a substituted or unsubstituted aryl group of 6 to 30 ring-forming carbon atoms, or a substituted or unsubstituted heteroaryl group of 2 to 30 ring-forming carbon atoms. By introducing at least one third substituent to the first fused ring, the $\pi$-conjugation may be expanded widely, the molecular stability of a polaron state may be improved, and accordingly, the device life and efficiency may be improved. In addition, the total molecular weight of the amine compound may be increased due to the third substituent, and there are benefits of markedly increasing the glass transition temperature. In the present disclosure, the third substituent may be the substituent represented by $R_1$ and $R_2$ in Formula 1-a.

Otherwise, the amine compound of an embodiment may include a structure in which at least one selected from among the first substituent and the second substituent connected with the nitrogen atom of the amine group is a substituted or unsubstituted bicycloheptanyl group. For example, the amine compound of an embodiment may include a first substituent which is one substituent selected from a substituted or unsubstituted adamantyl group, a substituted or unsubstituted cyclohexyl group, a substituted or unsubstituted bicycloheptanyl group, a substituted or unsubstituted bicyclooctanyl group, and a substituted or unsubstituted triphenylsilyl group, and a second substituent of a substituted or unsubstituted bicycloheptanyl group, connected with the nitrogen atom. In the amine compound of an embodiment, because at least one selected from among the first substituent and the second substituent includes the substituted or unsubstituted bicycloheptanyl group, the amine compound according to an embodiment, represented by Formula 1 may have high glass transition temperature properties, and due to such high glass transition temperature properties, excellent properties of heat resistance and durability may be shown.

The amine compound of an embodiment is represented by Formula 1 below.

Formula 1

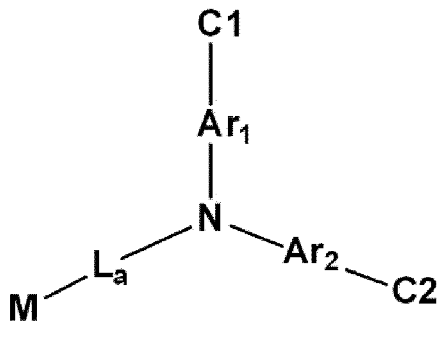

In Formula 1, An and $Ar_2$ are each independently a substituted or unsubstituted arylene group of 6 to 30 ring-forming carbon atoms, or a substituted or unsubstituted heteroarylene group of 2 to 30 ring-forming carbon atoms. In an embodiment, An and $Ar_2$ may be each independently a substituted or unsubstituted phenylene group, a substituted or unsubstituted divalent biphenyl group, or a substituted or unsubstituted divalent naphthyl group. For example, An and $Ar_2$ may be each independently an unsubstituted phenylene group, an unsubstituted divalent biphenyl group, or an unsubstituted divalent naphthyl group.

In Formula 1, $L_a$ is a direct linkage, a substituted or unsubstituted arylene group of 6 to 30 ring-forming carbon atoms, or a substituted or unsubstituted heteroarylene group of 2 to 30 ring-forming carbon atoms. In an embodiment, $L_a$ may be a direct linkage, or a substituted or unsubstituted arylene group of 6 to 30 ring-forming carbon atoms. For example, $L_a$ may be a direct linkage, or a substituted or unsubstituted phenylene group.

In Formula 1, C1 and C2 are each independently a substituted or unsubstituted adamantyl group, a substituted or unsubstituted cyclohexyl group, a substituted or unsubstituted bicycloheptanyl group, a substituted or unsubstituted bicyclooctanyl group, or a substituted or unsubstituted triphenylsilyl group. In some embodiments, the amine compound of an embodiment may include one first substituent selected from a substituted or unsubstituted adamantyl group, a substituted or unsubstituted cyclohexyl group, a substituted or unsubstituted bicycloheptanyl group, a substituted or unsubstituted bicyclooctanyl group, or a substituted or unsubstituted triphenylsilyl group combined with $Ar_1$, and one second substituent selected from a substituted or unsubstituted adamantyl group, a substituted or unsubstituted cyclohexyl group, a substituted or unsubstituted bicycloheptanyl group, a substituted or unsubstituted bicyclooctanyl group, or a substituted or unsubstituted triphenylsilyl group combined with $Ar_2$. For example, C1 and C2 may be each independently a substituted or unsubstituted adamantyl group, a substituted or unsubstituted cyclohexyl group, a substituted or unsubstituted bicyclo[2,2,1]heptanyl group, a substituted or unsubstituted bicyclo[2,2,2]octanyl group, or a substituted or unsubstituted triphenylsilyl group. In an embodiment, C1 and C2 may be the same or different. However, a case where both C1 and C2 are substituted or unsubstituted adamantyl groups is excluded from Formula 1.

In Formula 1, M is represented by any one selected from among Formula 1-a to Formula 1-c below. The amine compound represented by Formula 1 may include a first fused ring which is any one selected from Formula 1-a to Formula 1-c below. The amine group in the amine compound represented by Formula 1 may be connected with the first fused ring which is any one selected from Formula 1-a to Formula 1-c below.

Formula 1-a

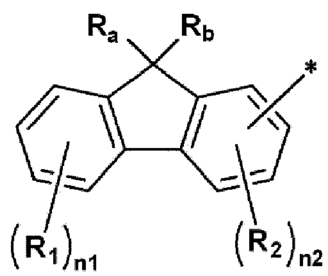

Formula 1-b

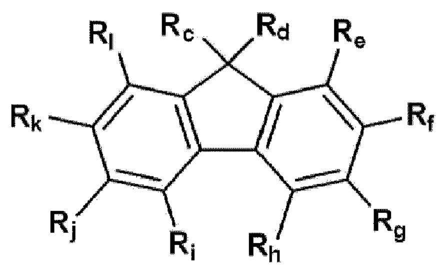

Formula 1-c

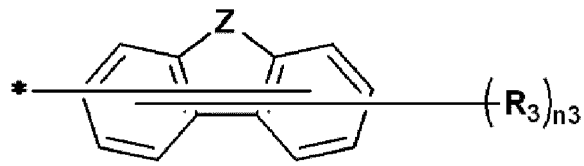

In Formula 1-c, Z is $NR_4$, O, or S.

In Formula 1-a, $R_1$ and $R_2$ are each independently a substituted or unsubstituted aryl group of 6 to 30 ring-forming carbon atoms, or a substituted or unsubstituted heteroaryl group of 2 to 30 ring-forming carbon atoms. In an embodiment, $R_1$ and $R_2$ may be each independently a substituted or unsubstituted aryl group of 6 to 30 ring-forming carbon atoms. For example, $R_1$ and $R_2$ may be each independently a substituted or unsubstituted phenyl group, a substituted or unsubstituted biphenyl group, or a substituted or unsubstituted naphthyl group. $R_1$ and $R_2$ may be each independently an unsubstituted phenyl group, an unsubstituted biphenyl group, or an unsubstituted naphthyl group.

In Formula 1-a to Formula 1-c, $R_a$ to $R_l$, $R_3$ and $R_4$ are each independently a hydrogen atom, a deuterium atom, a halogen atom, a substituted or unsubstituted alkyl group of 1 to 20 carbon atoms, a substituted or unsubstituted alkenyl group of 2 to 20 carbon atoms, a substituted or unsubstituted aryl group of 6 to 30 ring-forming carbon atoms, or a substituted or unsubstituted heteroaryl group of 2 to 30 ring-forming carbon atoms. Otherwise, each of $R_a$ to $R_l$, $R_3$ and $R_4$ is combined with an adjacent group to form a ring. In an embodiment, $R_a$ to $R_d$ may be each independently a substituted or unsubstituted alkyl group of 1 to 10 carbon atoms, or a substituted or unsubstituted aryl group of 6 to 20 ring-forming carbon atoms. In addition, each group of $R_a$ and $R_b$, and $R_c$ and $R_d$ may be combined with each other to form a ring. If $R_a$ and $R_b$ are combined with each other to form a ring, the substituent represented by Formula 1-a may have a spiro structure. In addition, if $R_c$ and $R_d$ are combined with each other to form a ring, the substituent represented by Formula 1-b may have a spiro structure. In an embodiment, $R_a$ to $R_l$, $R_3$ and $R_4$ may be each independently a hydrogen atom.

In Formula 1-b, any one selected from among $R_c$ to $R_l$ is a part connected with Formula 1. In some embodiments, in Formula 1-b, in $R_c$ to $R_l$, the remaining substituents excluding the substituent combined with the nitrogen atom of the amine compound represented by Formula 1 may be each independently a hydrogen atom, a deuterium atom, a halogen atom, a substituted or unsubstituted alkyl group of 1 to 20 carbon atoms, a substituted or unsubstituted alkenyl group of 2 to 20 carbon atoms, a substituted or unsubstituted aryl group of 6 to 30 ring-forming carbon atoms, or a substituted or unsubstituted heteroaryl group of 2 to 30 ring-forming carbon atoms, or combined with an adjacent group to form a ring. For example, in $R_c$ to $R_l$, the remaining substituents excluding the substituent combined with the nitrogen atom of the amine compound represented by Formula 1 may be a hydrogen atom.

In Formula 1-a and Formula 1-c, -* is a part connected with Formula 1.

In Formula 1-a, n1 is the number of $R_1$, and n1 is an integer of 0 to 4. n2 is the number of $R_2$ and is an integer of 0 to 3.

If n1 is 0, the amine compound of an embodiment may not be substituted with $R_1$. Formula 1-a where n1 is 4, and all of $R_1$ are hydrogen atoms, may be the same as Formula 1-a where n1 is 0. If n1 is an integer of 2 or more, multiple $R_1$ may all be the same, or at least one selected from among multiple $R_1$ may be different.

If n2 is 0, the amine compound of an embodiment may not be substituted with $R_2$. Formula 1-a where n2 is 3, and all of $R_2$ are hydrogen atoms, may be the same as Formula 1-a where n2 is 0. If n2 is an integer of 2 or more, multiple $R_2$ may all be the same, or at least one selected from among multiple $R_2$ may be different.

In Formula 1-a, the sum of n1 and n2 is 1 or more. If M is represented by Formula 1-a in Formula 1, the first fused ring represented by Formula 1-a in the amine compound of an embodiment may include at least one third substituent. In the amine compound of an embodiment, the first fused ring represented by Formula 1-a may include at least one substituent represented by $R_1$, or at least one substituent represented by $R_2$. In an embodiment, n1 and n2 may be each independently an integer of 0 to 2, and the sum of n1 and n2 may be 1 or 2. For example, if the sum of n1 and n2 is 1, n1 may be 1, and n2 may be 0 in Formula 1, or n1 may be 0, and n2 may be 1 in Formula 1. If the sum of n1 and n2 is 2, n1 and n2 of Formula 1 may be 1 each, or n1 may be 2, and n2 may be 0. However, an embodiment of the present disclosure is not limited thereto.

In Formula 1, if M is represented by Formula 1-b or Formula 1-c, at least one selected from among C1 and C2 in Formula 1 may be a substituted or unsubstituted bicycloheptanyl group. In some embodiments, if M is represented by Formula 1-b or Formula 1-c in Formula 1, the amine compound represented by Formula 1 may essentially include a substituted or unsubstituted bicycloheptanyl group. For example, the amine compound represented by Formula 1 may include one first substituent selected from a substituted or unsubstituted adamantyl group, a substituted or unsubstituted cyclohexyl group, a substituted or unsubstituted bicycloheptanyl group, a substituted or unsubstituted bicyclooctanyl group, or a substituted or unsubstituted triphenylsilyl group, combined with $Ar_1$, and a second substituent of a substituted or unsubstituted bicycloheptanyl group combined with $Ar_2$.

In Formula 1-c, n3 is an integer of 0 to 7. If n3 is 0, the amine compound of an embodiment may not be substituted with $R_5$. Formula 1-c where n3 is 7, and all of $R_3$ are hydrogen atoms, may be the same as Formula 1-c where n3 is 0. If n3 is an integer of 2 or more, multiple $R_3$ may all be the same, or at least one selected from among multiple $R_3$ may be different.

In an embodiment, the amine compound represented by Formula 1 may be represented by Formula 1-1 below.

Formula 1-1

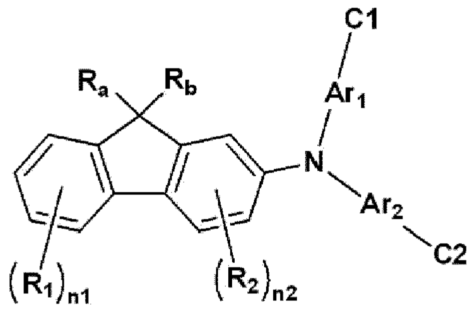

Formula 1-1 represents a case of the structure of Formula 1 where M is represented by Formula 1-a, and the carbon position of the first fused ring represented by Formula 1-a, to which the nitrogen atom of Formula 1 is bonded is specified. The amine compound of an embodiment, represented by Formula 1 is directly bonded to a fluorenyl group represented by Formula 1-a, and in this case, the nitrogen atom of the amine group may be connected with the carbon at position 2 or carbon at position 7 of the fluorenyl group represented by Formula 1-a. In some embodiments, the carbon numbers of the fluorenyl group are shown in Formula a shown below.

Formula a

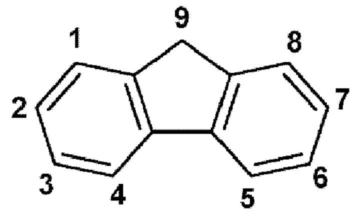

In Formula 1-1, the same description of $R_a$, $R_b$, $R_1$, $R_2$, n1, n2, $Ar_1$, $Ar_2$, C1 and C2 provided with respect to Formula 1 and Formula 1-a may be applied.

In an embodiment, the amine compound represented by Formula 1 may be represented by any one selected from among Formula 2-1 to Formula 2-8 below.

Formula 2-1

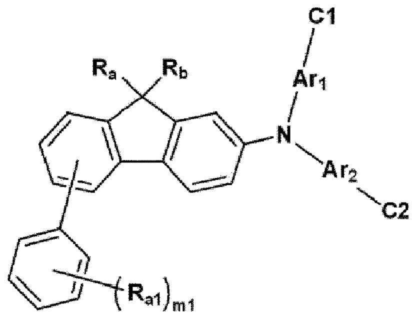

Formula 2-2

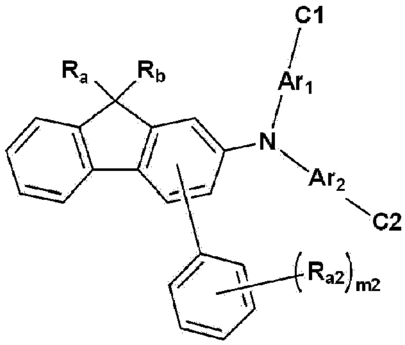

Formula 2-3

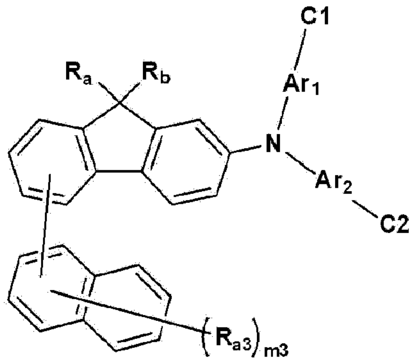

Formula 2-4

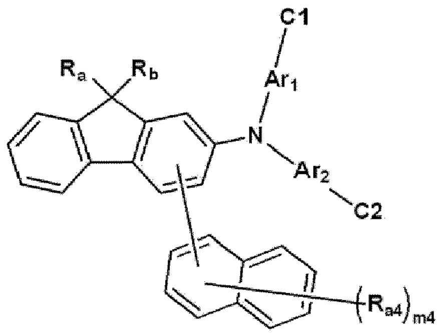

Formula 2-5

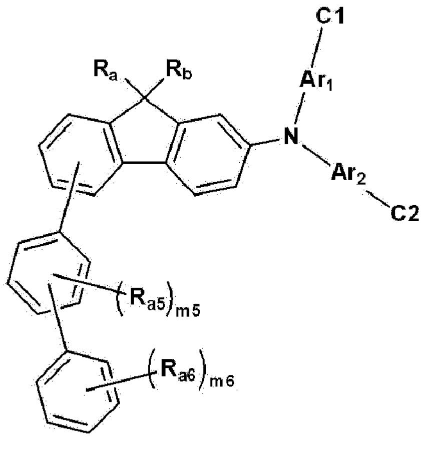

Formula 2-6

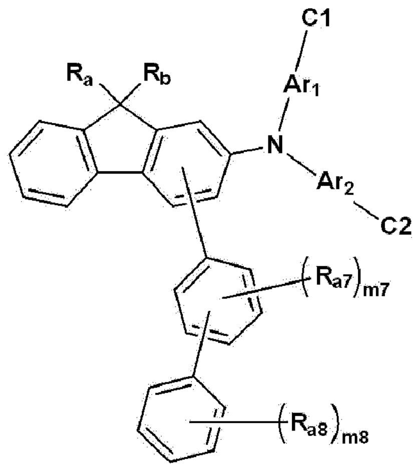

Formula 2-7

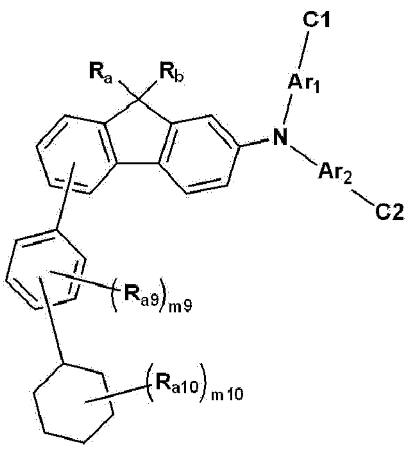

Formula 2-8

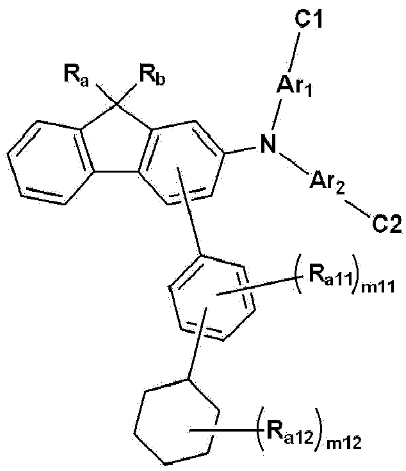

Formula 2-1 to Formula 2-8 represent cases of Formula 1 where M is represented by Formula 1-a, the carbon position of the first fused ring structure represented by Formula 1-a to which the nitrogen atom of Formula 1 is bonded is specified, and the number and type of substituents connected with the substituent represented by Formula 1-a are specified. For example, in the structure of Formula 1-a, the number and type of $R_1$ and $R_2$ are specified.

Formula 2-1 represents a case of Formula 1 where M is represented by Formula 1-a, n1 is 1, and n2 is 0 in Formula 1-a, and the substituent represented by $R_1$ is a substituted or unsubstituted phenyl group. Formula 2-2 represents a case where n1 is 0, and n2 is 1 in Formula 1-a, and the substituent represented by $R_2$ is a substituted or unsubstituted phenyl group. Formula 2-3 represents a case of Formula 1 where n1 is 1, and n2 is 0 in Formula 1-a, and the substituent represented by $R_1$ is a substituted or unsubstituted naphthyl group. Formula 2-4 represents a case where n1 is 0, and n2 is 1 in Formula 1-a, and the substituent represented by $R_2$ is a substituted or unsubstituted naphthyl group. Formula 2-5 represents a case where n1 is 1, and n2 is 0 in Formula 1-a, and the substituent represented by $R_1$ is a substituted or unsubstituted biphenyl group. Formula 2-6 represents a case where n1 is 0, and n2 is 1 in Formula 1-a, and the substituent represented by $R_2$ is a substituted or unsubstituted biphenyl group. Formula 2-7 represents a case where n1 is 1, and n2 is 0 in Formula 1-a, and the substituent represented by $R_1$ is a substituted or unsubstituted cyclohexylphenyl group. Formula 2-8 represents a case where n1 is 0, and n2 is 1 in Formula 1-a, and the substituent represented by $R_2$ is a substituted or unsubstituted cyclohexylphenyl group.

In Formula 2-1 to Formula 2-8, $R_{a1}$ to $R_{a12}$ are each independently a hydrogen atom, a deuterium atom, a halogen atom, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms. For example, $R_{a1}$ to $R_{a12}$ may be each independently a hydrogen atom, or a substituted or unsubstituted phenyl group.

In Formula 2-1 to Formula 2-8, m1, m2, m6, and m8 are each independently an integer of 0 to 5, m3 and m4 are each independently an integer of 0 to 7, m5, m7, m9, and m11 are each independently an integer of 0 to 4, and m10 and m12 are each independently an integer of 0 to 11.

In the cases where m1, m2, m6, and m8 are 0, the amine compounds of embodiments may be unsubstituted with $R_{a1}$, $R_{a2}$, $R_{a6}$, and $R_{a8}$, respectively. The cases where m1, m2, m6, and m8 are 5, and $R_{a1}$, $R_{a2}$, $R_{a6}$, and $R_{a8}$ are hydrogen atoms, may be the same as the cases of Formula 2-1, Formula 2-2, Formula 2-5 and Formula 2-6, where m1, m2, m6, and m8 are 0. If m1, m2, m6, and m8 are integers of 2 or more, each of multiple $R_{a1}$, $R_{a2}$, $R_{a6}$, and $R_{a8}$ may all be the same, or at least one selected from among multiple $R_{a1}$, $R_{a2}$, $R_{a6}$, and $R_{a8}$ may be different.

In the cases where m5, m7, m9, and m11 are 0, the amine compounds of embodiments may be unsubstituted with $R_{a5}$, $R_{a7}$, $R_{a9}$, and $R_{a11}$, respectively. The cases where m5, m7, m9, and m11 are 4, and $R_{a5}$, $R_{a7}$, $R_{a9}$, and $R_{a11}$ are hydrogen atoms, may be the same as the cases of Formula 2-5 to Formula 2-8, where m5, m7, m9, and m11 are 0. If m5, m7, m9, and m11 are integers of 2 or more, each of multiple $R_{a5}$, $R_{a7}$, $R_{a9}$, and $R_{a11}$ may all be the same, or at least one selected from among multiple $R_{a5}$, $R_{a7}$, $R_{a9}$, and $R_{a11}$ may be different.

The cases where m3 and m4 are 0, may mean the amine compounds of embodiments unsubstituted with $R_{a3}$ and $R_{a4}$, respectively. The cases where m3 and m4 are 7, and $R_{a3}$ and $R_{a4}$ are all hydrogen atoms, may be the same as the cases of Formula 2-3 and Formula 2-4 where m3 and m4 are 0. If m3 and m4 are integers of 2 or more, multiple $R_{a3}$ and $R_{a4}$ may all be the same, or at least one selected from among multiple $R_{a3}$ and $R_{a4}$ may be different.

The cases where m10 and m12 are 0, may mean the amine compounds of embodiments unsubstituted with $R_{a10}$ and $R_{a12}$, respectively. The cases where m10 and m12 are 11, and $R_{a10}$ and $R_{a12}$ are all hydrogen atoms may be the same as the cases of Formula 2-7 and Formula 2-8 where m10 and m12 are 0. If m10 and m12 are integers of 2 or more, multiple $R_{a10}$ and $R_{a12}$ may all be the same, or at least one selected from among multiple $R_{a10}$ and $R_{a12}$ may be different.

In Formula 2-1 to Formula 2-8, the same description of $R_a$, $R_b$, $Ar_1$, $Ar_2$, C1 and C2 referring to Formula 1 and Formula 1-a may be applied.

In an embodiment, the amine compound represented by Formula 1 may be represented by Formula 3-1 or Formula 3-2 below.

Formula 3-1

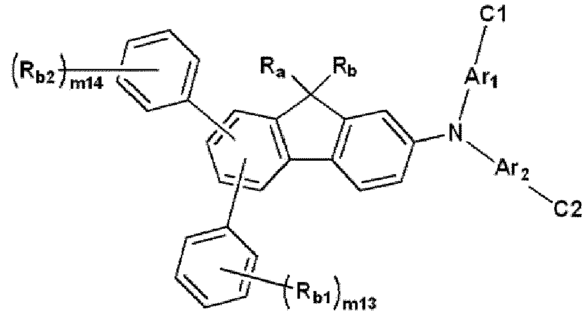

Formula 3-2

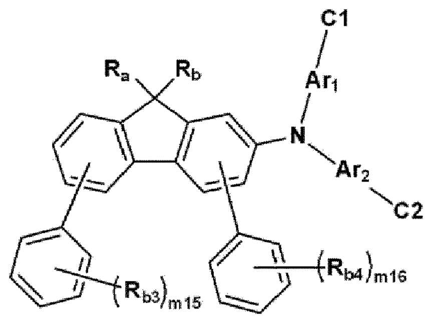

Formula 3-1 and Formula 3-2 represent the structure of Formula 1 where M is represented by Formula 1-a, the carbon position of the first fused ring represented by Formula 1-a to which the nitrogen atom of Formula 1 is bonded is specified, and the number and type of substituents connected with the substituent represented by Formula 1-a are specified. For example, in the structure of Formula 1-a, the number and type of $R_1$ and $R_2$ are specified.

Formula 3-1 represents a case where n1 is 2, and n2 is 0 in Formula 1-a, and all substituents represented by $R_1$ are substituted or unsubstituted phenyl groups. Formula 3-2 represents a case where n1 and n2 are 1 in Formula 1-a, and the substituent represented by $R_1$ is a substituted or unsubstituted phenyl group, and the substituent represented by $R_2$ is a substituted or unsubstituted phenyl group.

In Formula 3-1 and Formula 3-2, $R_{b1}$ to $R_{b4}$ are each independently a hydrogen atom, a deuterium atom, a halogen atom, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms. For example, $R_{b1}$ to $R_{b4}$ may be a hydrogen atom.

In Formula 3-1 and Formula 3-2, m13 to m16 are each independently an integer of 0 to 5. If m13 to m16 are 0, the amine compounds of embodiments may be unsubstituted with $R_{b1}$ to $R_{b4}$, respectively. In Formula 3-1 and Formula 3-2, the cases where m13 to m16 are 5, and $R_{b1}$ to $R_{b4}$ are hydrogen atoms, may be the same as Formula 3-1 and Formula 3-2, where m13 to m16 are 0, respectively. If m13 to m16 are integers of 2 or more, each of multiple $R_{b1}$ to $R_{b4}$ may all be the same, or at least one selected from among multiple $R_{b1}$ to $R_{b4}$ may be different.

In Formula 3-1 and Formula 3-2, the same description of $R_a$, $R_b$, $Ar_1$, $Ar_2$, C1 and C2 referring to Formula 1 and Formula 1-a may be applied.

In an embodiment, the amine compound represented by Formula 1 may be represented by any one selected from among Formula 4-1 to Formula 4-15 below.

Formula 4-1

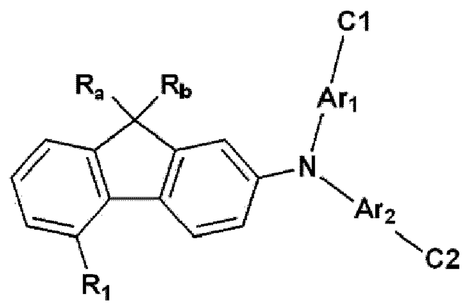

Formula 4-2

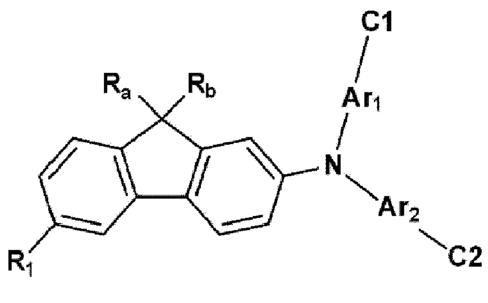

Formula 4-3

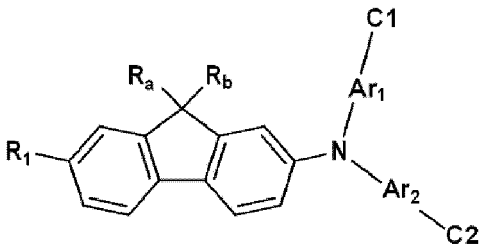

Formula 4-4

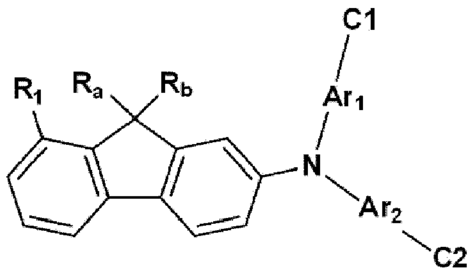

Formula 4-5

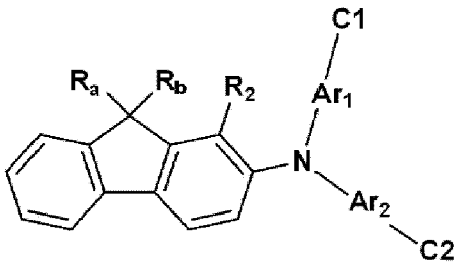

Formula 4-6

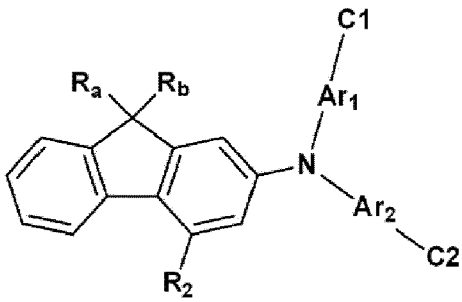

Formula 4-7

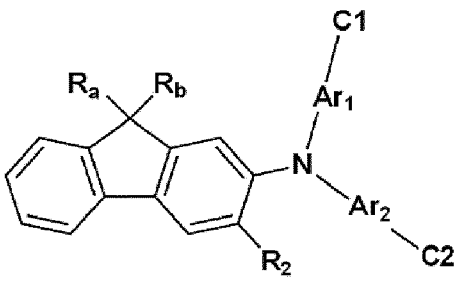

Formula 4-8

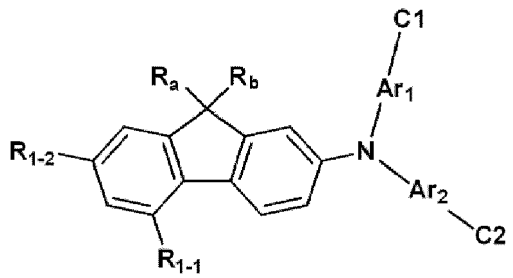

Formula 4-9

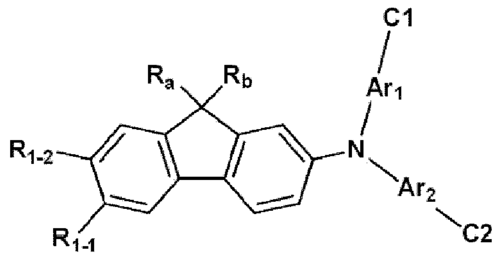

Formula 4-10

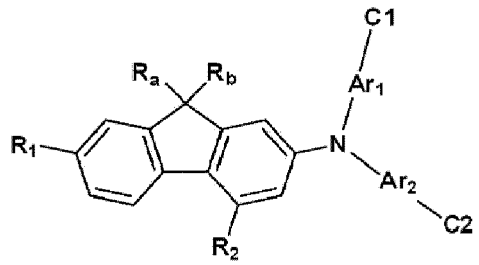

Formula 4-11

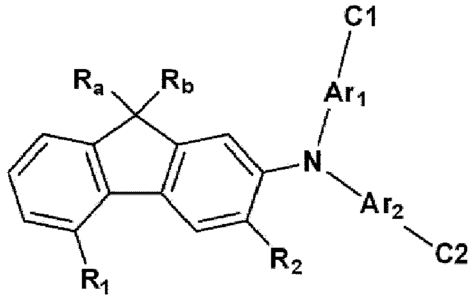

Formula 4-12

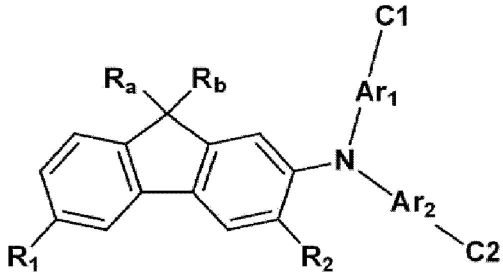

Formula 4-13

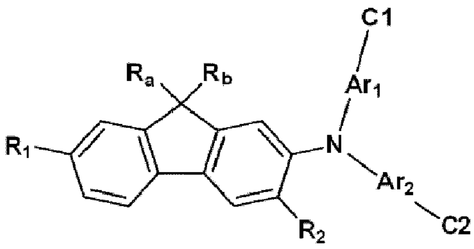

Formula 4-14

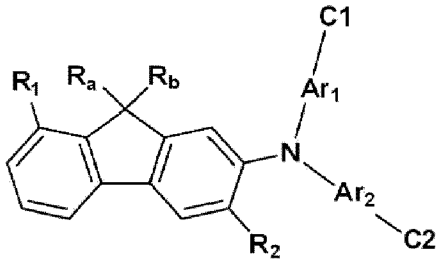

Formula 4-15

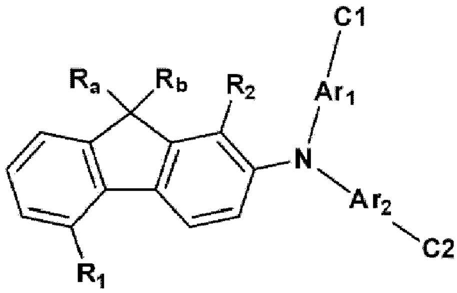

Formula 4-1 to Formula 4-15 represent the structure of Formula 1 where M is represented by Formula 1-a, and the number and connecting position of substituents connected with the first fused ring represented by Formula 1-a are specified. For example, in the first fused ring structure represented by Formula 1-a, the number and connecting positions of $R_1$ and $R_2$ are specified.

In Formula 4-8 and Formula 4-9, $R_{1-1}$ and $R_{1-2}$ are each independently a substituted or unsubstituted aryl group of 6 to 30 ring-forming carbon atoms, or a substituted or unsubstituted heteroaryl group of 2 to 30 ring-forming carbon atoms. For example, $R_{1-1}$ and $R_{1-2}$ may be each independently a substituted or unsubstituted phenyl group, a substituted or unsubstituted biphenyl group, or a substituted or unsubstituted naphthyl group.

In Formula 4-1 to Formula 4-15, the same description of $R_1$, $R_2$, $R_a$, $R_b$, $Ar_1$, $Ar_2$, C1, and C2 referring to Formula 1 and Formula 1-a may be applied.

In an embodiment, the amine compound represented by Formula 1 may be represented by any one selected from among Formula 5-1 to Formula 5-3 below.

Formula 5-1

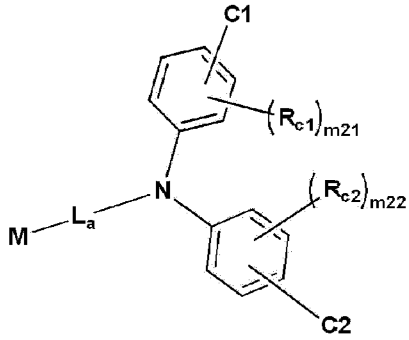

Formula 5-2

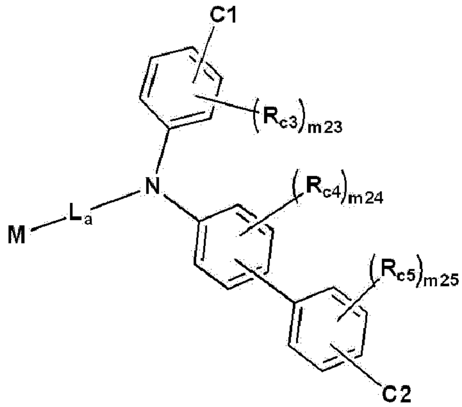

Formula 5-3

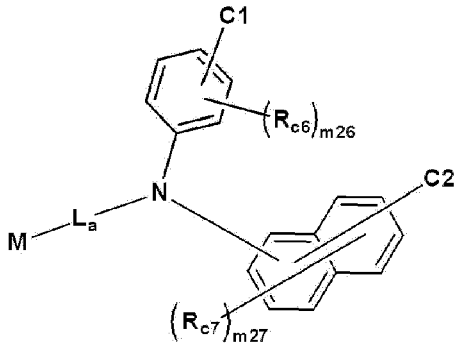

Formula 5-1 to Formula 5-3 represent Formula 1 where An and $Ar_2$ are specified as particular structures. Formula 5-1 represents Formula 1 where $Ar_1$ and $Ar_2$ are each independently a substituted or unsubstituted phenylene group. Formula 5-2 represents Formula 1 where $Ar_1$ is a substituted or unsubstituted phenylene group, and $Ar_2$ is a substituted or unsubstituted divalent biphenyl group. Formula 5-3 represents Formula 1 where $Ar_1$ is a substituted or unsubstituted phenylene group, and $Ar_2$ is a substituted or unsubstituted divalent naphthyl group.

C1 and C2 included in the amine compound of an embodiment may not be directly bonded to the amine group but may be bonded to the nitrogen atom of the amine group via $Ar_1$ and $Ar_2$. In an embodiment, $Ar_1$ and $Ar_2$ connecting C1 and C2 to the nitrogen atom of the amine group may be each independently a substituted or unsubstituted phenylene group, a substituted or unsubstituted divalent biphenyl group, or a substituted or unsubstituted divalent naphthyl group.

In Formula 5-1 to Formula 5-3, $R_{c1}$ to $R_{c7}$ may be each independently a hydrogen atom, a deuterium atom, a halogen atom, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms. In an embodiment, $R_{c1}$ to $R_{c7}$ may be each independently a hydrogen atom or a substituted or unsubstituted phenyl group.

In Formula 5-1 to Formula 5-3, m21 to m26 are each independently an integer of 0 to 4, and m27 is an integer

of 0 to 6.

If m21 to m26 are 0, the amine compounds of embodiments may be unsubstituted with $R_{c1}$ to $R_{c6}$, respectively. The cases where m21 to m26 are 4, and $R_{c1}$ to $R_{c6}$ are hydrogen atoms, may be the same as Formula 5-1 to Formula 5-3, where m21 to m26 are 0. If m21 to m26 are integers of 2 or more, each of multiple $R_{c1}$ to $R_{c6}$ may be the same, or at least one selected from among multiple $R_{c1}$ to $R_{c6}$ may be different.

If m27 is 0, the amine compound of an embodiment may be unsubstituted with $R_{c7}$. Formula 4-3 where m27 is 6, and all $R_{c7}$ are hydrogen atoms, may be the same as Formula 5-3 where m27 is 0. If m27 is an integer of 2 or more, multiple $R_{c7}$ may all be the same, or at least one selected from among multiple $R_{c7}$ may be different.

The same description of M, $L_a$, C1, and C2 referring to Formula 1 may be applied.

In an embodiment, the amine compound represented by Formula 1 may be represented by any one selected from among Formula 6-1 to Formula 6-8 below.

Formula 6-1

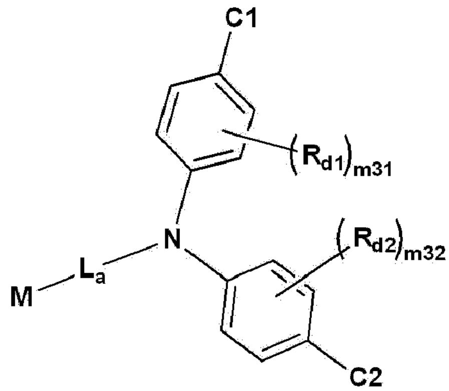

Formula 6-2

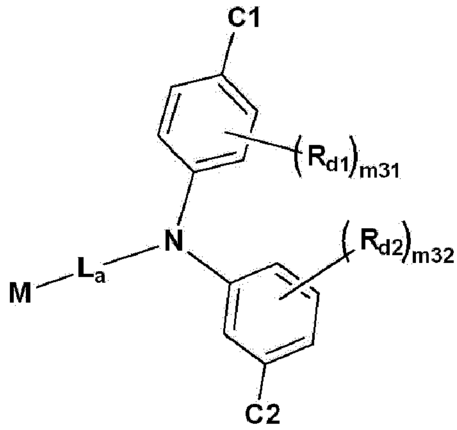

Formula 6-3

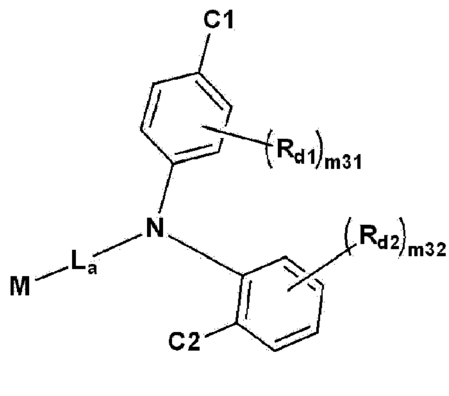

Formula 6-4

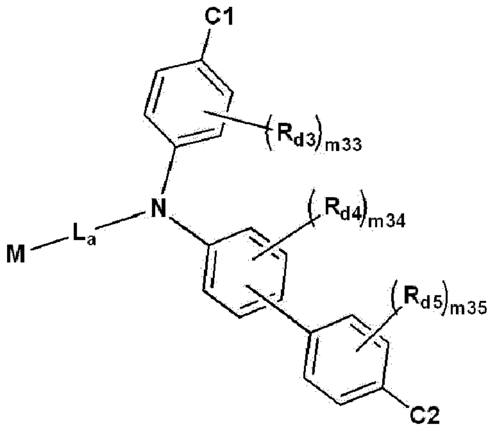

Formula 6-5

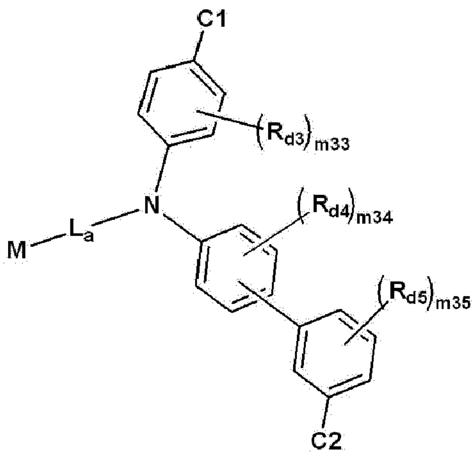

Formula 6-6

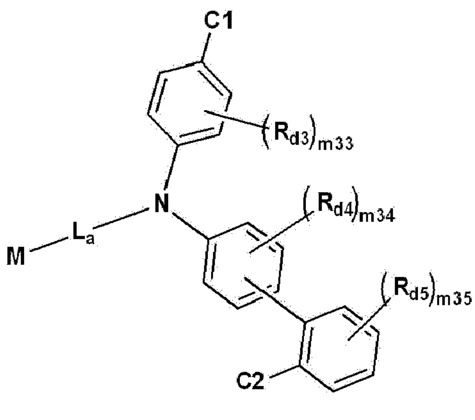

Formula 6-7

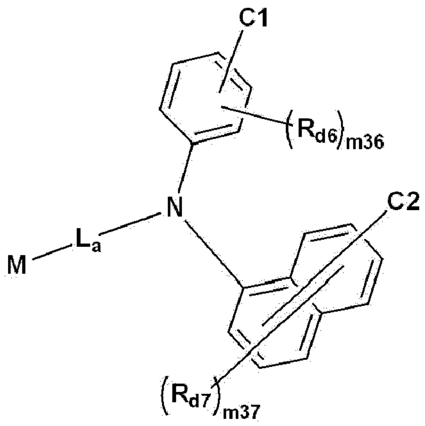

Formula 6-8

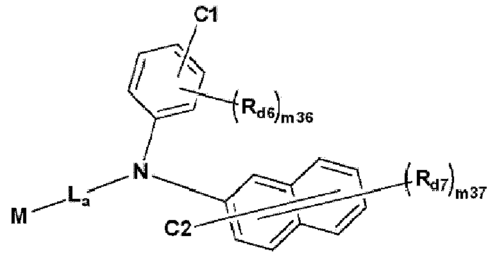

Formula 6-1 to Formula 6-8 represent Formula 1 where $Ar_1$ and $Ar_2$ are specified as particular structures, and the positions of C1 and C2 connected with $Ar_1$ and $Ar_2$, respectively, are specified.

In Formula 6-1 to Formula 6-8, $R_{d1}$ to $R_{d7}$ may be each independently a hydrogen atom, a deuterium atom, a halogen atom, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms. For example, $R_{d1}$ to $R_{d7}$ may be each independently a hydrogen atom, or a substituted or unsubstituted phenyl group.

In Formula 6-1 to Formula 6-8, m31 to m36 are each independently an integer of 0 to 4, and m37 is an integer of 0 to 6.

If m31 to m36 are 0, the amine compounds of embodiments may be unsubstituted with $R_{d1}$ to $R_{d6}$, respectively. The cases where m31 to m36 are 4, and $R_{d1}$ to $R_{d6}$ are all hydrogen atoms, may be the same as Formula 6-1 to Formula 6-8 where m31 to m36 are 0. If m31 to m36 are integers of 2 or more, each of multiple $R_{d1}$ to $R_{d6}$ may be the same, or at least one of multiple $R_{d1}$ to $R_{d6}$ may be different.

If m37 is 0, the amine compound of an embodiment may be unsubstituted with $R_{d7}$. Formula 6-7 and Formula 6-8, where m37 is 6, and all $R_{d7}$ are hydrogen atoms may be the same as Formula 6-7 and Formula 6-8, where m37 is 0. If m37 is an integer of 2 or more, each of multiple $R_{d7}$ may be the same, or at least one selected from among multiple $R_{d7}$ may be different.

The same description of M, $L_a$, C1, and C2 referring to Formula 1 may be applied.

In an embodiment, C1 and C2 may be each independently represented by any one selected from among Formula 7-1 to Formula 7-8 below.

Formula 7-1

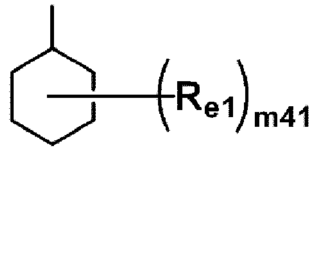

Formula 7-2

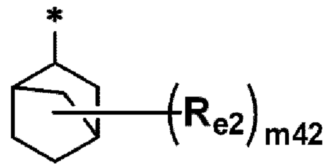

Formula 7-3

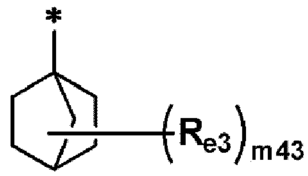

Formula 7-4

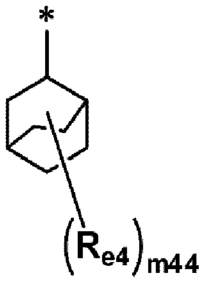

Formula 7-5

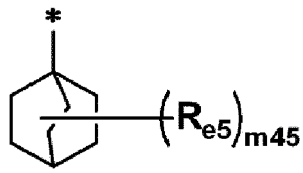

Formula 7-6

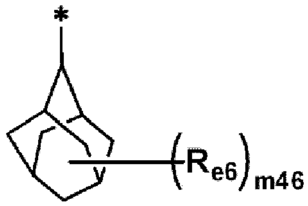

Formula 7-7

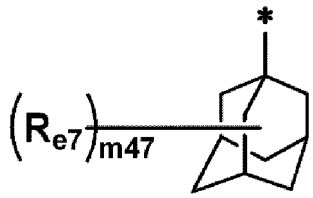

Formula 7-8

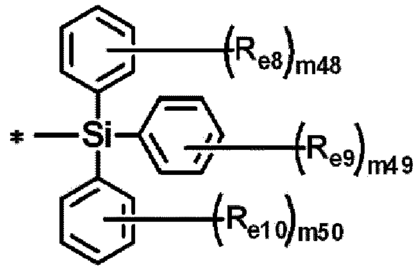

In Formula 7-1 to Formula 7-8, $Re_1$ to $Re_{10}$ are each independently a hydrogen atom, a deuterium atom, a halogen atom, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms. For example, $R_{e1}$ to $Re_{10}$ may be a hydrogen atom.

In Formula 7-1 to Formula 7-8, m41 to m43 are each independently an integer of 0 to 11, m44 and m45 are each independently an integer of 0 to 13, m46 and m47 are each independently an integer of 0 to 15, and m48 to m50 are each independently an integer of 0 to 5.

If m41 to m43 are 0, the amine compounds of embodiments may be unsubstituted with $R_{e1}$ to $R_{e3}$, respectively. The cases where m41 to m43 are 11, and $R_{e1}$ to $R_{e3}$ are hydrogen atoms, may be the same as Formula 7-1 to Formula 7-3 where m41 to m43 are 0, respectively. If m41 to m43 are integers of 2 or more, each of multiple $R_{e1}$ to $R_{e3}$ may all be the same, or at least one selected from among multiple $R_{e1}$ to $R_{e3}$ may be different.

If m44 and m45 are 0, the amine compounds of embodiments may be unsubstituted with $R_{e4}$ and $R_{e5}$, respectively. The cases where m44 and m45 are 13, and $R_{e4}$ and $R_{e5}$ are hydrogen atoms, may be the same as Formula 7-4 and Formula 7-5 where m44 and m45 are 0, respectively. If m44 and m45 are integers of 2 or more, each of multiple $R_{e4}$ and $R_{e5}$ may all be the same, or at least one selected from among multiple $R_{e4}$ and $R_{e5}$ may be different.

If m46 and m47 are 0, the amine compounds of embodiments may be unsubstituted with $R_{e6}$ and $R_{e7}$, respectively. The cases where m46 and m47 are 15, and $R_{e6}$ and $R_{e7}$ are hydrogen atoms, may be the same as Formula 7-6 and Formula 7-7 where m46 and m47 are 0, respectively. If m46 and m47 are integers of 2 or more, each of multiple $R_{e6}$ and $R_{e7}$ may all be the same, or at least one selected from among multiple $R_{e6}$ and $R_{e7}$ may be different.

If m48 to m50 are 0, the amine compounds of embodiments may be unsubstituted with $R_{e8}$ to $R_{e10}$, respectively. The cases where m48 to m50 are 5, and $R_{e8}$ to $Re_{10}$ are hydrogen atoms, may be the same as Formula 7-8 where m48 to m50 are 0. If m48 to m50 are integers of 2 or more, each of multiple $R_{e8}$ to $Re_{10}$ may all be the same, or at least one selected from among multiple $R_{e8}$ to $Re_{10}$ may be different.

In an embodiment, the amine compound represented by Formula 1 may be represented by any one selected from among Formula 8-1 to Formula 8-5 below.

Formula 8-1

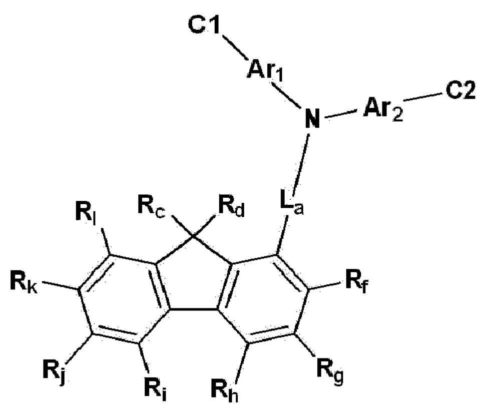

Formula 8-2

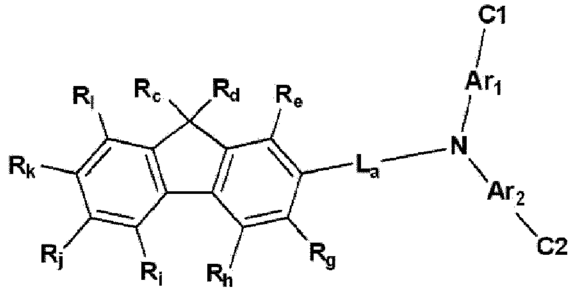

Formula 8-3

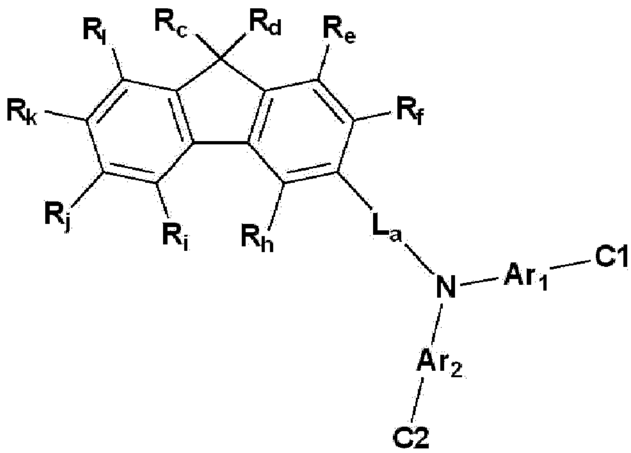

Formula 8-4

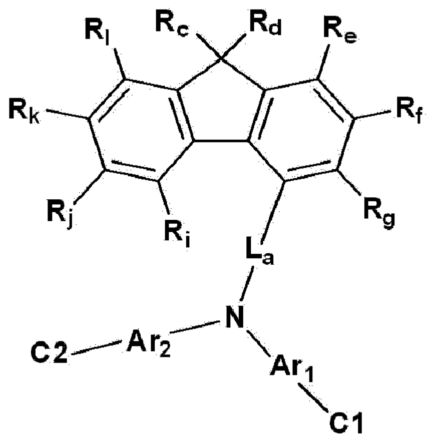

Formula 8-5

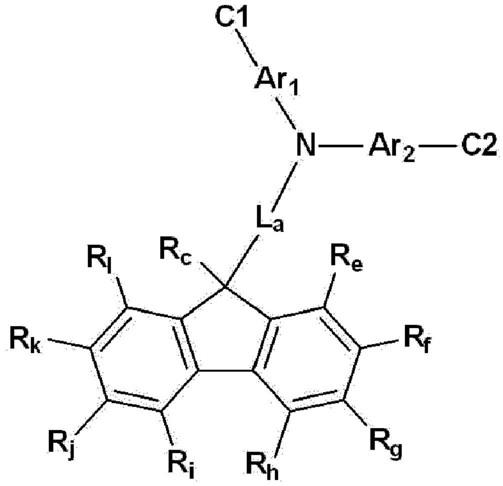

Formula 8-1 to Formula 8-5 represent the structure of Formula 1 where M is represented by Formula 1-b, and the position where the nitrogen atom of Formula 1 is connected with the substituent represented by Formula 1-b is specified.

In Formula 8-1 to Formula 8-5, at least one selected from among C1 and C2 may be a substituted or unsubstituted bicycloheptanyl group.

In Formula 8-1 to Formula 8-5, the same description of $Ar_1$, $Ar_2$, $L_a$, C1, C2, and $R_c$ to $R_l$ referring to Formula 1 and Formula 1-b may be applied.

In an embodiment, the amine compound represented by Formula 1 may be represented by any one selected from among Formula 9-1 to Formula 9-4 below.

Formula 9-1

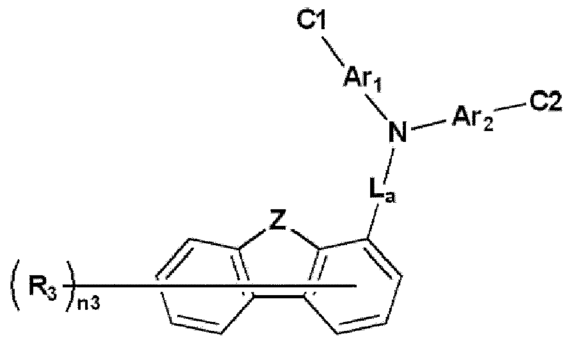

Formula 9-2

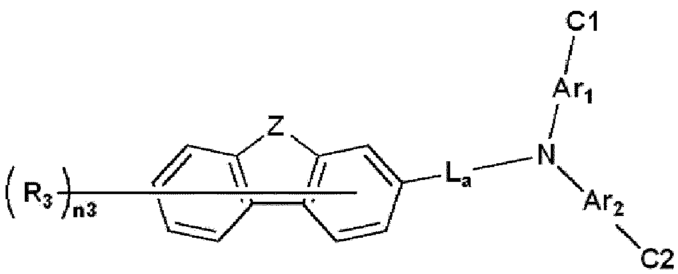

Formula 9-3

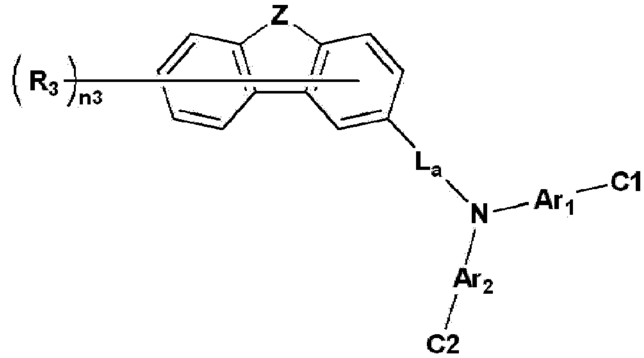

Formula 9-4

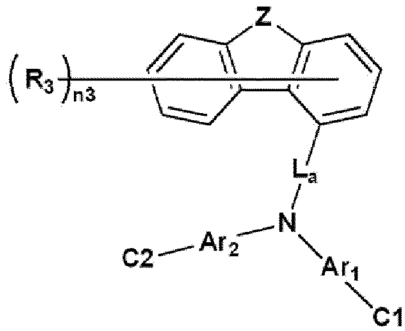

Formula 9-1 to Formula 9-4 represent the structure of Formula 1 where M is represented by Formula 1-c, and the position where the nitrogen atom of Formula 1 is connected with the substituent represented by Formula 1-c is specified.

In Formula 9-1 to Formula 9-4, at least one selected from among C1 and C2 may be a substituted or unsubstituted bicycloheptanyl group.

In Formula 9-1 to Formula 9-4, the same description of Z, $R_3$, $Ar_1$, $Ar_2$, $L_a$, n3, C1, and C2 referring to Formula 1 and Formula 1-c may be applied.

In the amine compound of an embodiment, represented by Formula 1, $Ar_1$ and $Ar_2$ may be each independently represented by any one selected from among Formulae L-1 to L-15 below.

Formula L-1

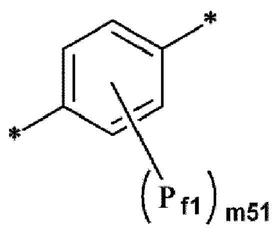

Formula L-2

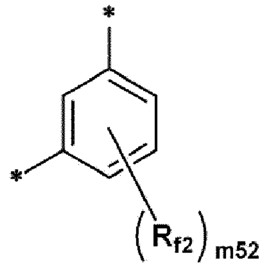

Formula L-3

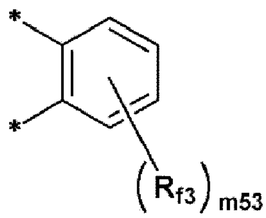

Formula L-4

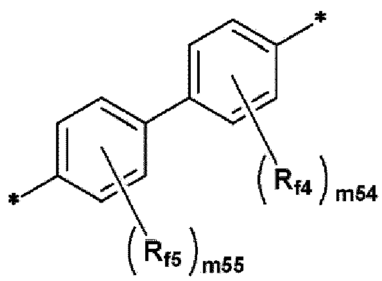

Formula L-5

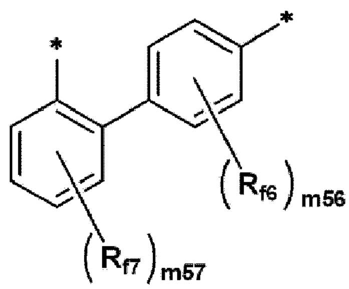

Formula L-6

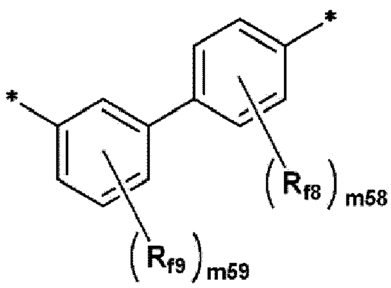

Formula L-7

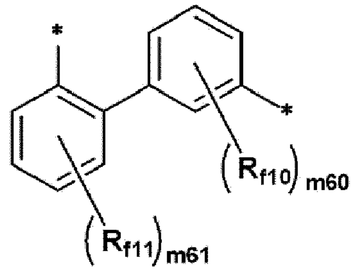

Formula L-8

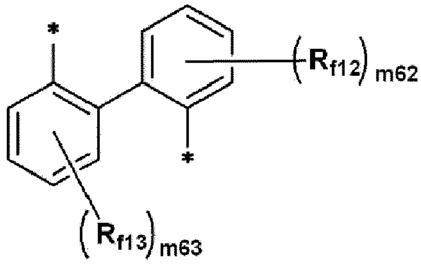

Formula L-9

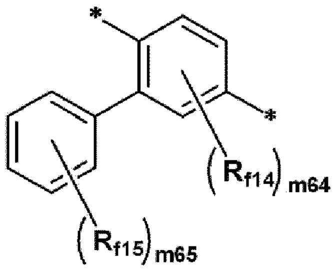

Formula L-10

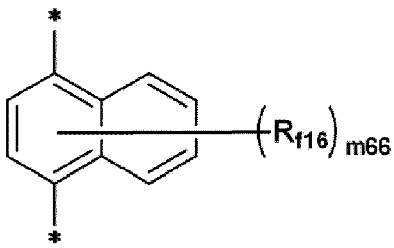

Formula L-11

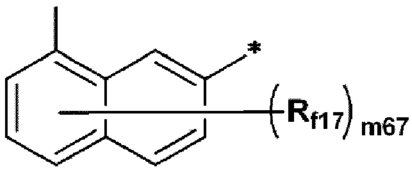

Formula L-12

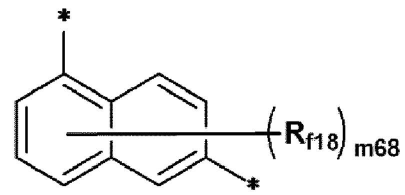

Formula L-13

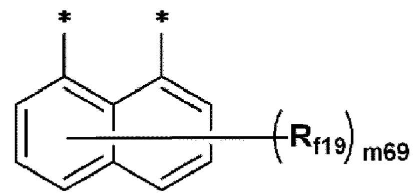

Formula L-14

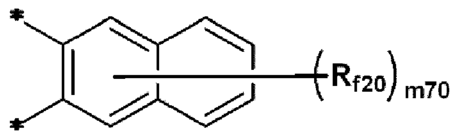

Formula L-15

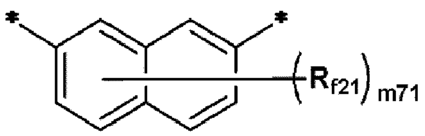

In Formula L-1 to Formula L-15, $R_{f1}$ to $R_{f21}$ may be each independently a hydrogen atom, a deuterium atom, a halogen atom, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms.

In Formula L-1 to Formula L-15, m51 to m63 are each independently an integer of 0 to 4, m64 is an integer of 0 to 3, m65 is an integer of 0 to 5, and m66 to m71 are each independently an integer of 0 to 6.

If m51 to m63 are 0, the amine compounds of embodiments may be unsubstituted with $R_{f1}$ to $R_{f13}$. The cases where m51 to m63 are 4, and $R_{f1}$ to $R_{f13}$ are all hydrogen atoms, may be the same as the cases where m51 to m63 are 0. If m51 to m63 are integers of 2 or more, each of multiple $R_{f1}$ to $R_{f13}$ may be the same, or at least one of multiple $R_{f1}$ to $R_{f13}$ may be different.

If m64 is 0, the amine compound of an embodiment may be unsubstituted with $R_{f14}$. The case where m64 is 3, and $R_{f14}$ are all hydrogen atoms, may be the same as the case where m64 is 0. If m64 is an integer of 2 or more, multiple $R_{f14}$ may all be the same, or at least one of multiple $R_{f14}$ may be different.

If m65 is 0, the amine compound of an embodiment may be unsubstituted with $R_{f15}$. The case where m65 is 5, and $R_{f15}$ are all hydrogen atoms, may be the same as the case where m65 is 0. If m65 is an integer of 2 or more, multiple $R_{f15}$ may all be the same, or at least one of multiple $R_{f15}$ may be different.

If m66 to m71 are 0, the amine compounds of embodiments may be unsubstituted with $R_{f16}$ to $R_{f21}$, respectively. The cases where m66 to m71 are 6, and $R_{f16}$ to $R_{f21}$ are all hydrogen atoms, may be the same as the cases where m66 to m71 are 0. If m66 to m71 are integers of 2 or more, each of multiple $R_{f16}$ to $R_{f21}$ may be the same, or at least one of multiple $R_{f16}$ to $R_{f21}$ may be different.

In an embodiment, the amine compound represented by Formula 1 may be represented by any one selected from among the compounds in Compound Group 1 below. The hole transport region HTR may include at least one selected from the compounds represented in Compound Group 1 below.

## Compound Group 1

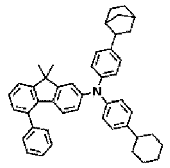
1

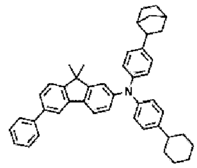
2

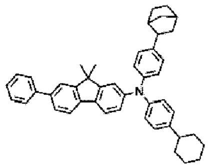
3

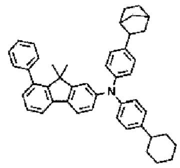
4

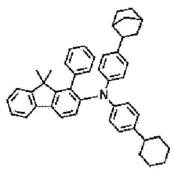
5

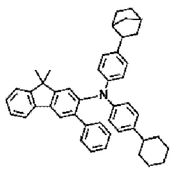
6

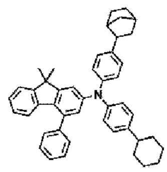
7

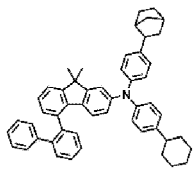
8

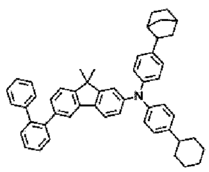
9

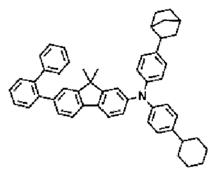
10

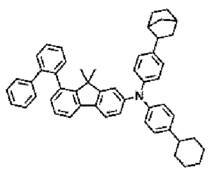
11

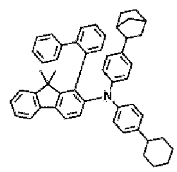
12

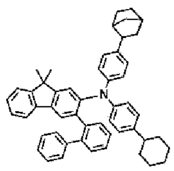
13

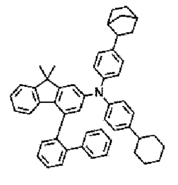
14

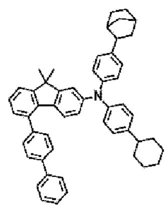
15

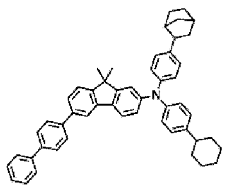
16

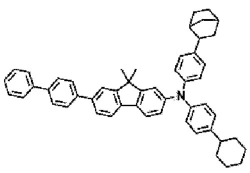
17

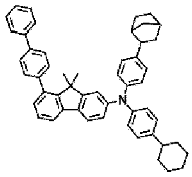
18

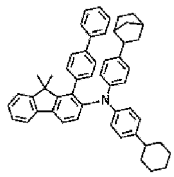
19

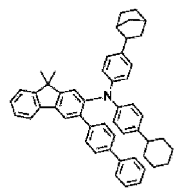
20

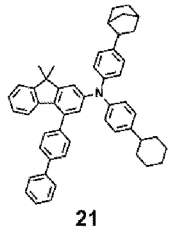
21

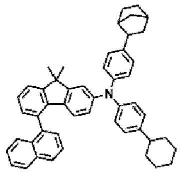
22

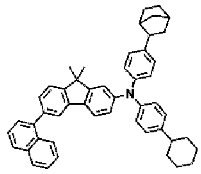
23

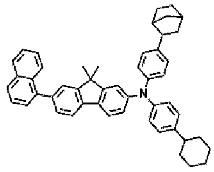
24

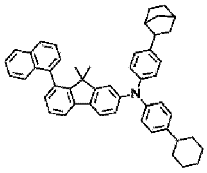
25

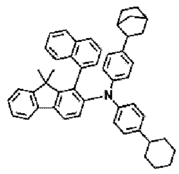
26

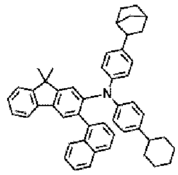
27

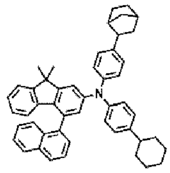
28

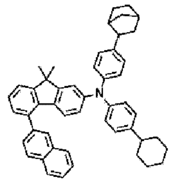
29

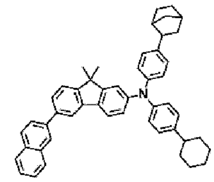
30

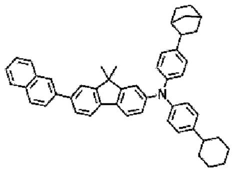
31

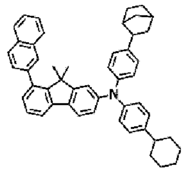
32

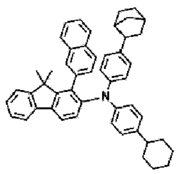
33

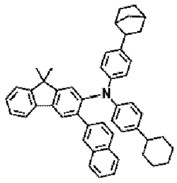
34

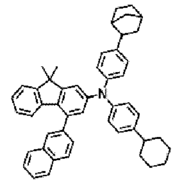
35

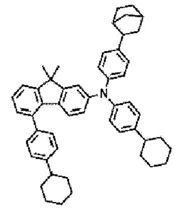
36

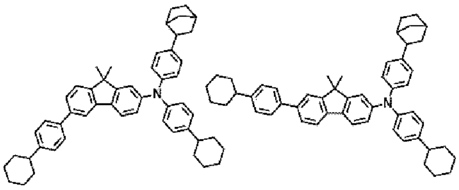
37

38

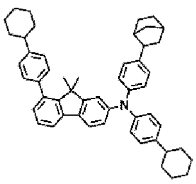
39

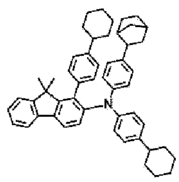
40

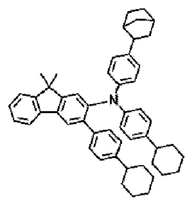
41

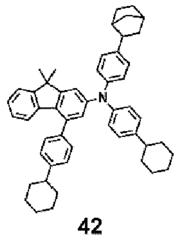
42

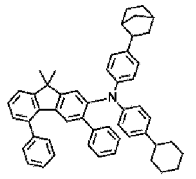
43

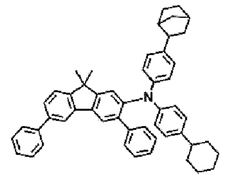
44

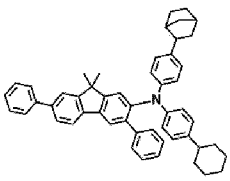
45

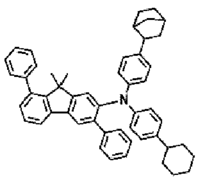
46

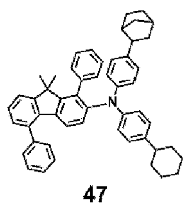
47

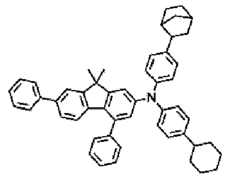
48

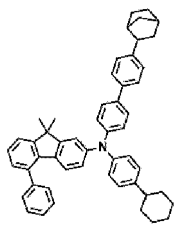
49

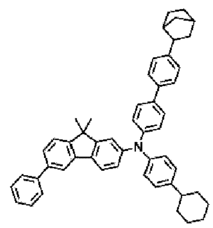
50

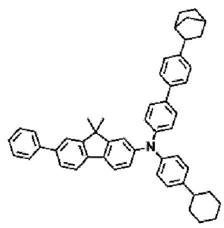
51

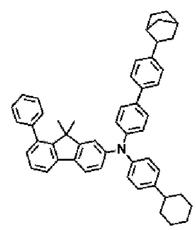
52

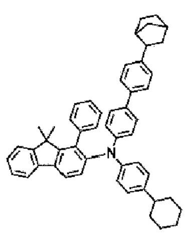
53

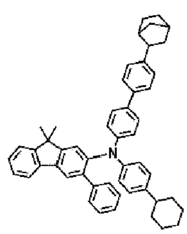
54

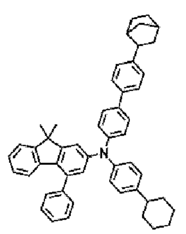
55

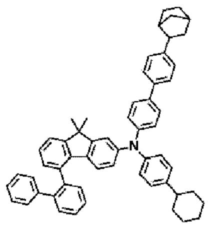
56

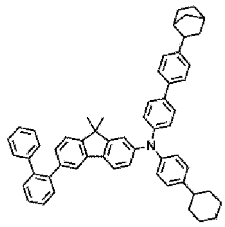
57

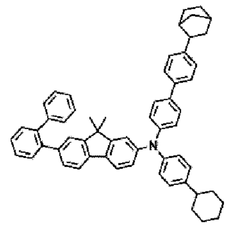
58

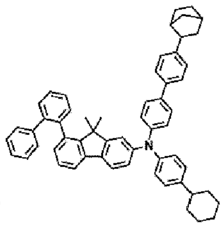
59

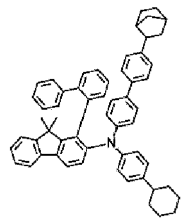
60

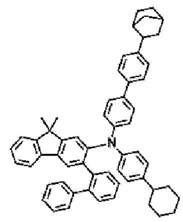
61

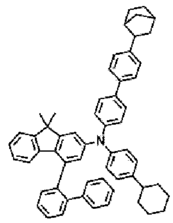
62

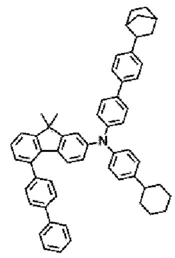
63

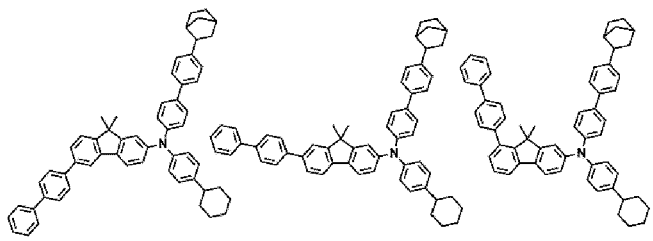
64

65

66

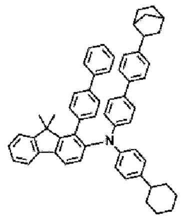
67

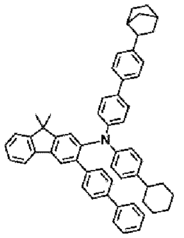
68

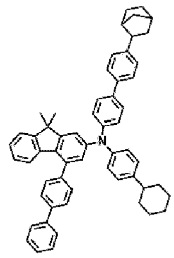
69

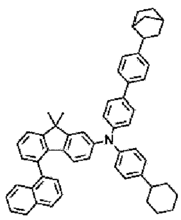
70

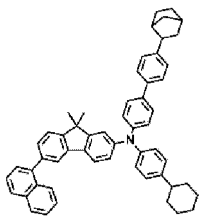
71

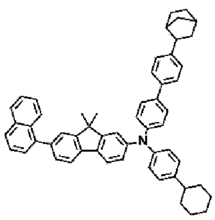
72

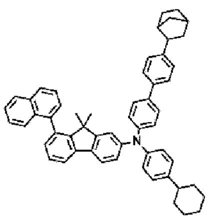
73

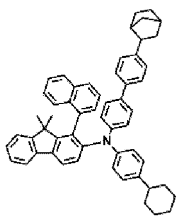
74

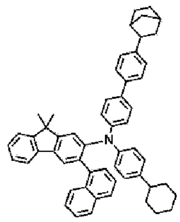
75

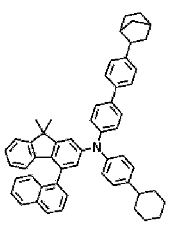
76

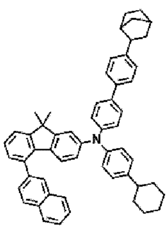
77

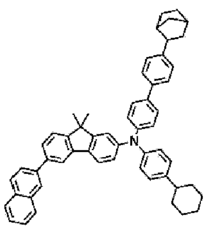
78

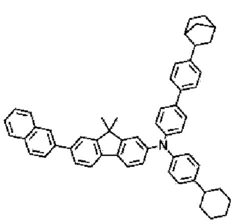
79

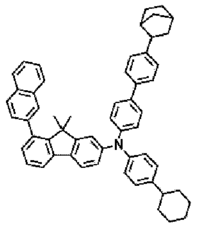
80

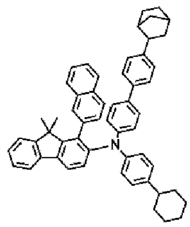
81

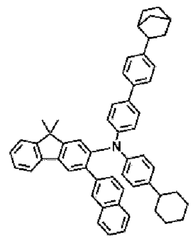
82

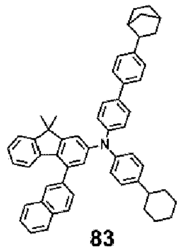
83

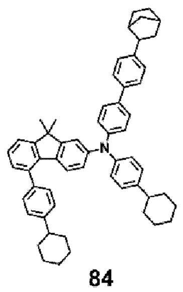
84

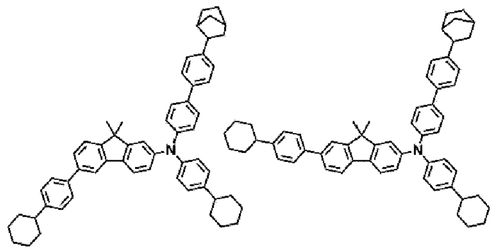
85

86

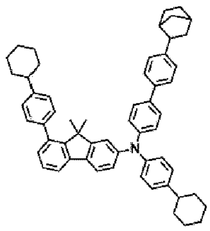
87

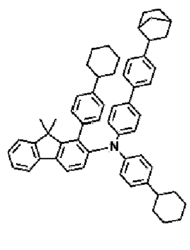
88

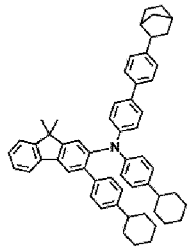
89

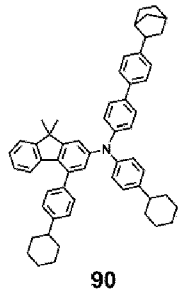
90

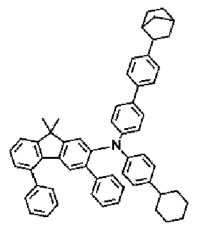
91

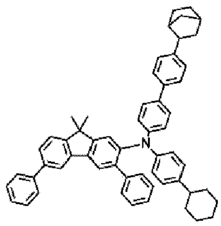
92

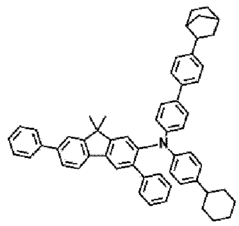
93

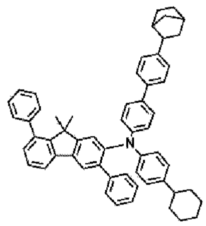
94

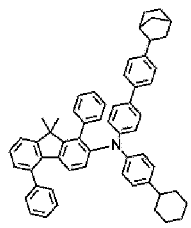
95

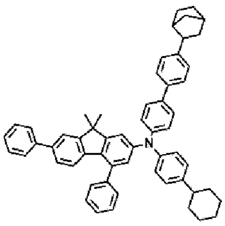
96

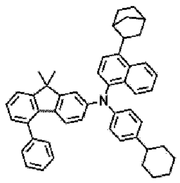
97

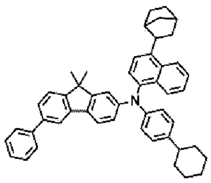
98

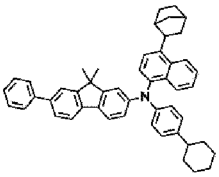 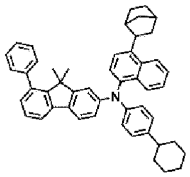 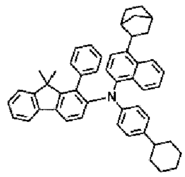 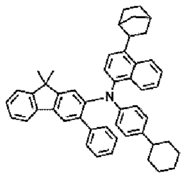

99 100 101 102

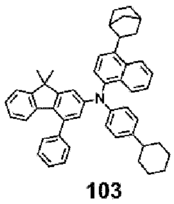 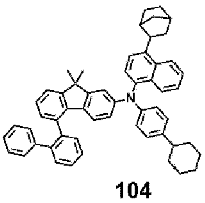 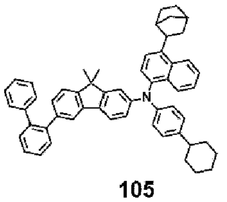 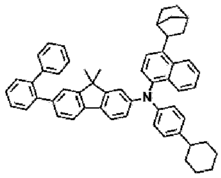

103 104 105 106

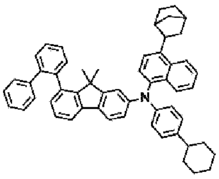 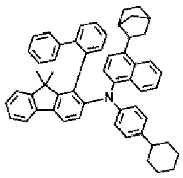 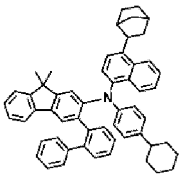 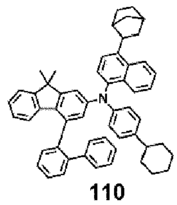

107 108 109 110

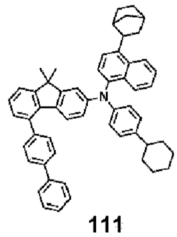 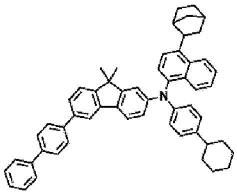 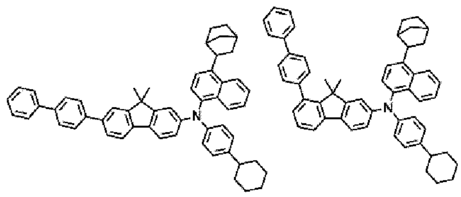

111 112 113 114

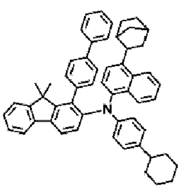 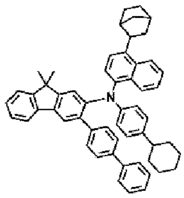 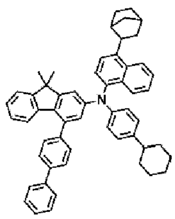 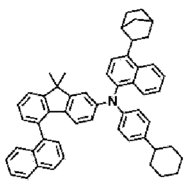

115 116 117 118

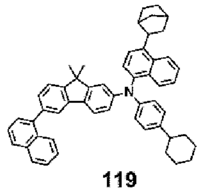
119

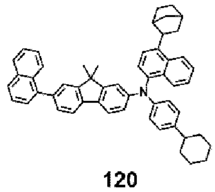
120

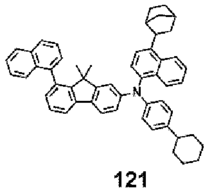
121

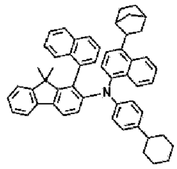
122

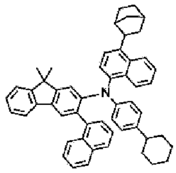
123

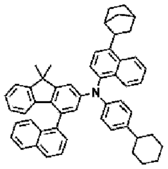
124

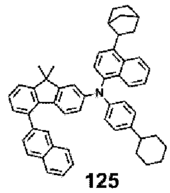
125

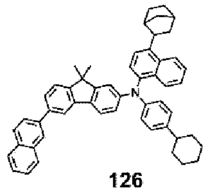
126

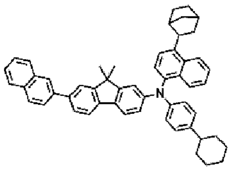
127

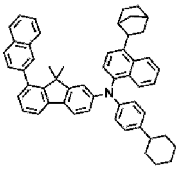
128

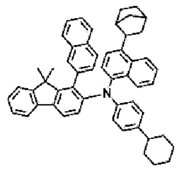
129

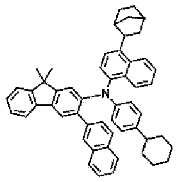
130

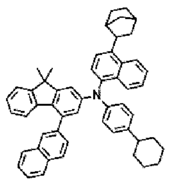
131

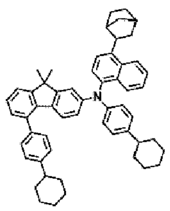
132

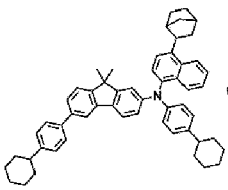
133

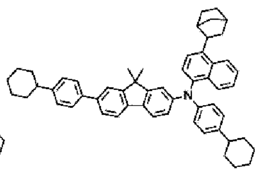
134

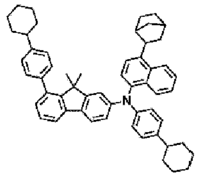
135

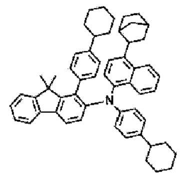
136

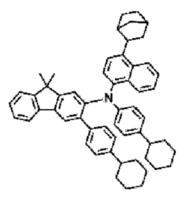
137

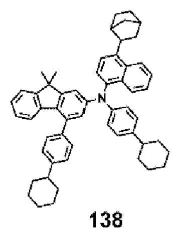
138

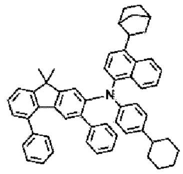
139

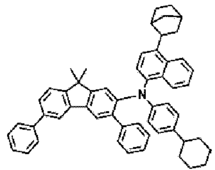
140

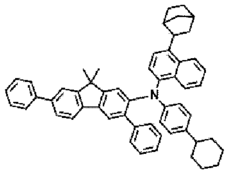
141

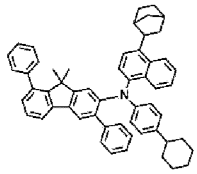
142

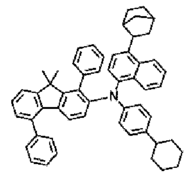
143

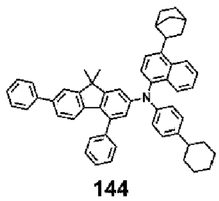
144

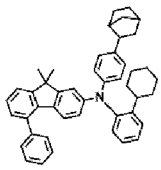
145

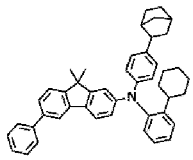
146

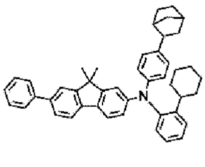
147

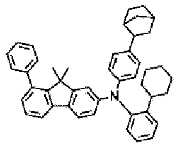
148

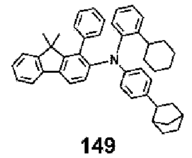
149

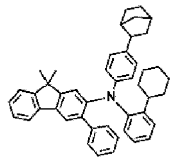
150

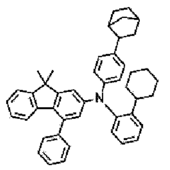
151

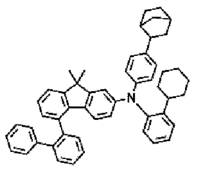
152

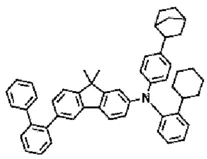
153

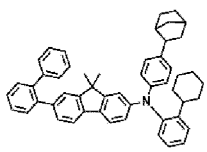
154

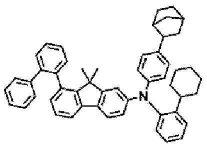
155

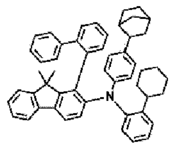
156

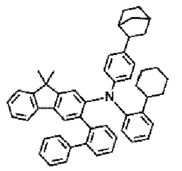
157

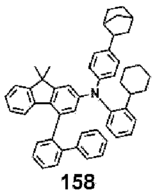
158

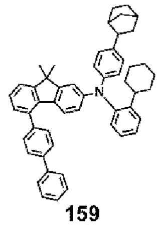
159

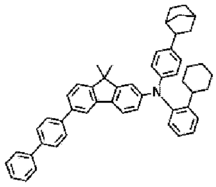
160

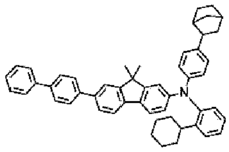
161

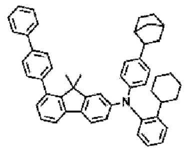
162

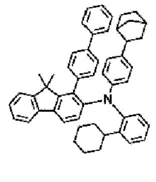
163

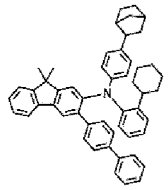
164

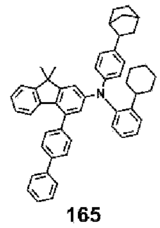
165

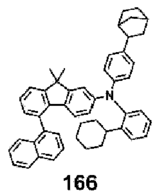
166

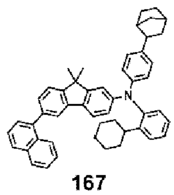
167

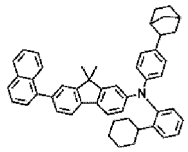
168

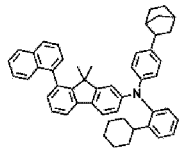
169

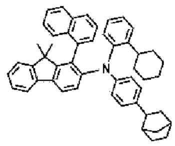
170

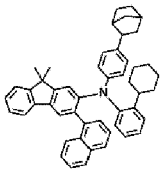
171

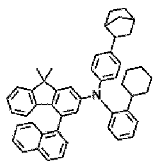
172

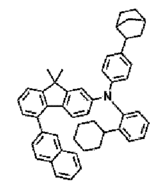
173

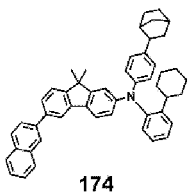
174

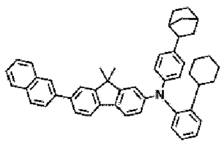
175

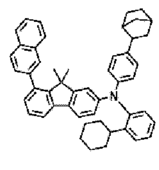
176

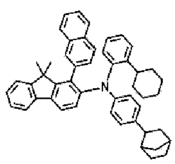
177

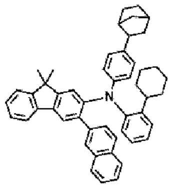 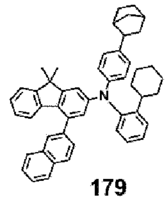 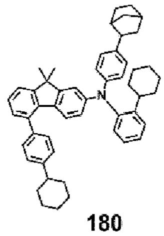 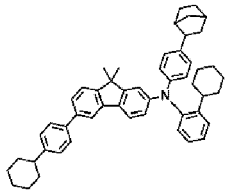

178 179 180 181

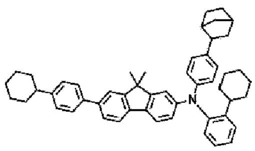 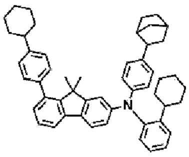 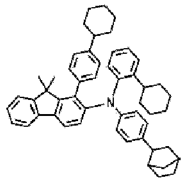 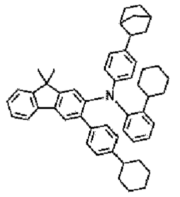

182 183 184 185

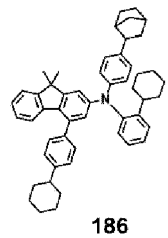 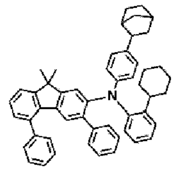 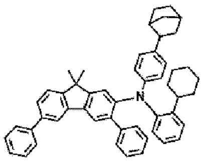 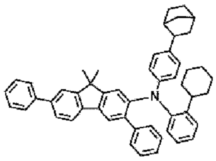

186 187 188 189

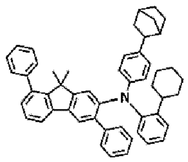 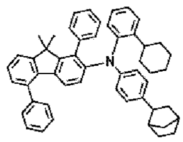 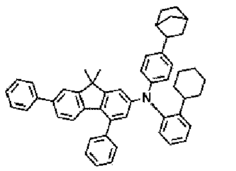 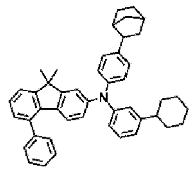

190 191 192 193

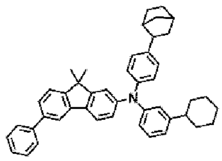 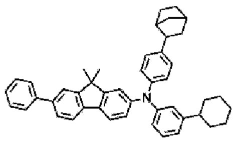 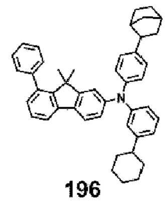

194 195 196

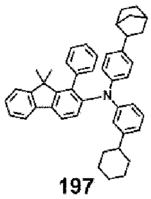
197

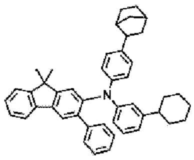
198

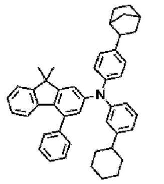
199

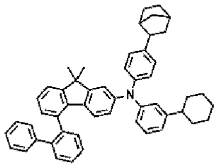
200

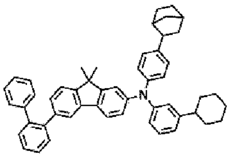
201

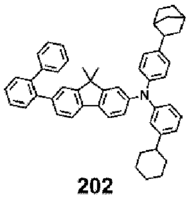
202

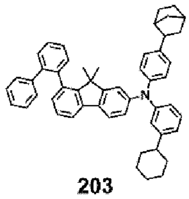
203

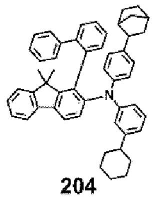
204

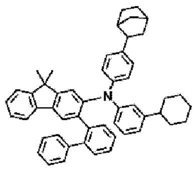
205

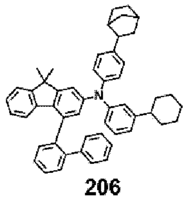
206

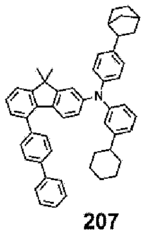
207

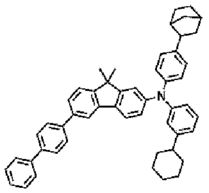
208

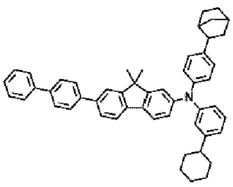
209

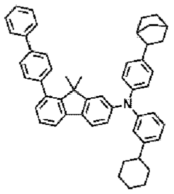
210

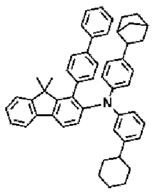
211

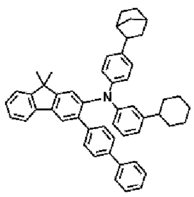
212

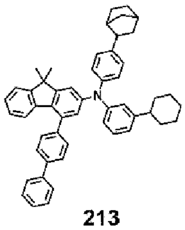
213

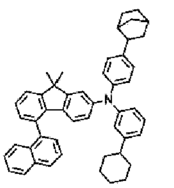
214

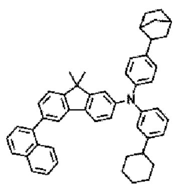
215

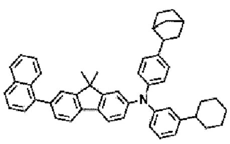
216

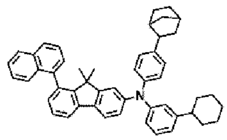
217
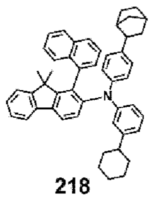
218
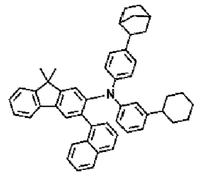
219
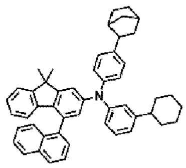
220

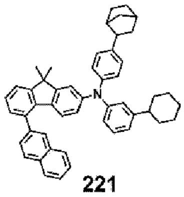
221
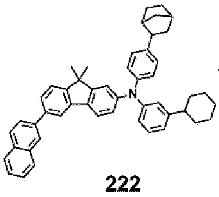
222
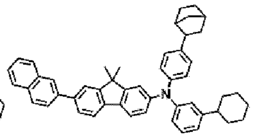
223
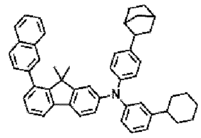
224

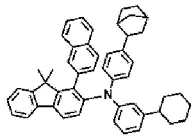
225
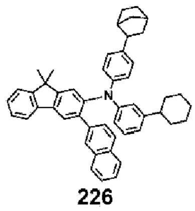
226
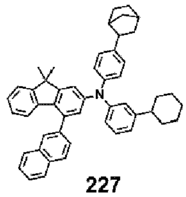
227
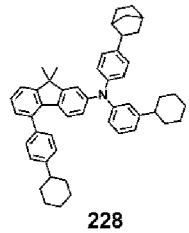
228

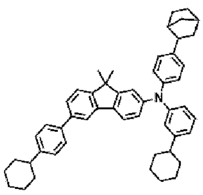
229
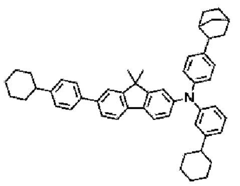
230
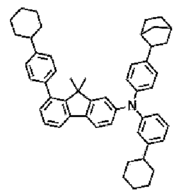
231
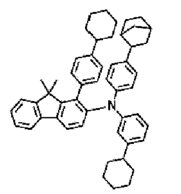
232

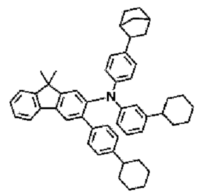
233
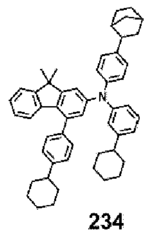
234
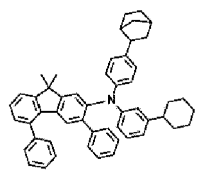
235

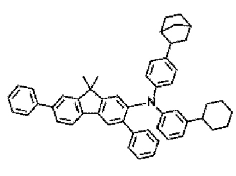
236

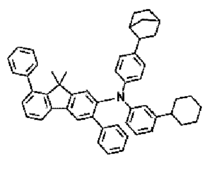
237

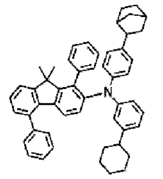
238

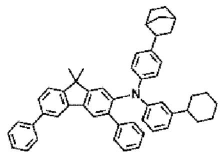
239

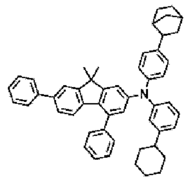
240

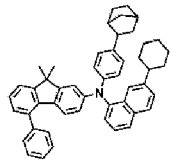
241

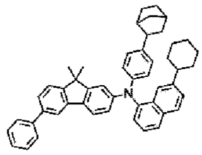
242

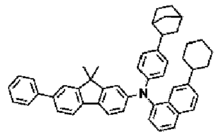
243

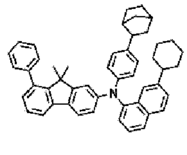
244

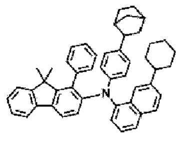
245

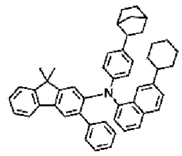
246

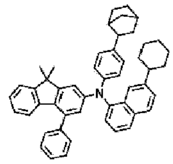
247

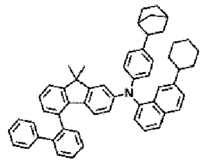
248

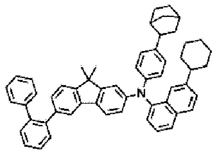
249

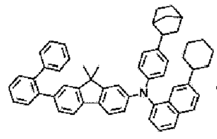
250

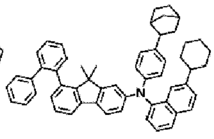
251

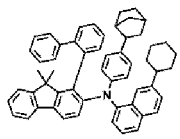
252

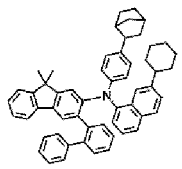
253

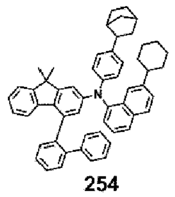
254

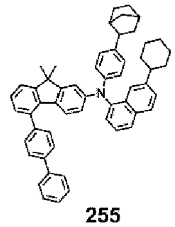
255

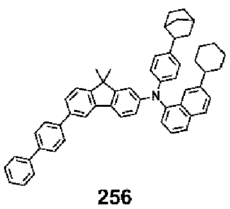
256

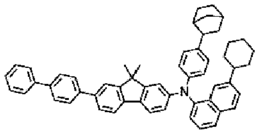
257

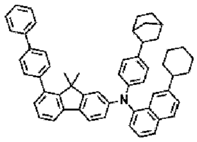
258

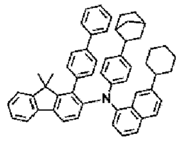
259

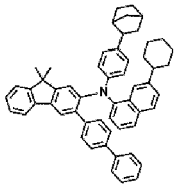
260

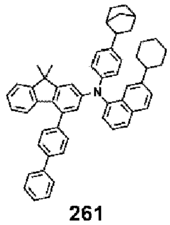
261

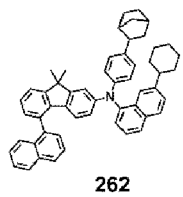
262

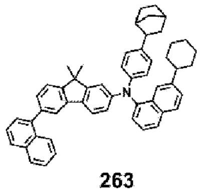
263

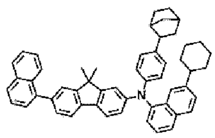
264

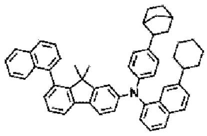
265

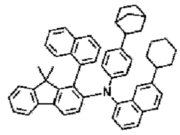
266

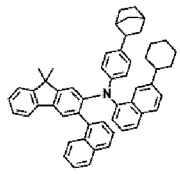
267

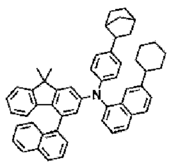
268

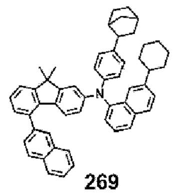
269

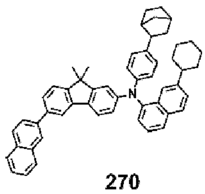
270

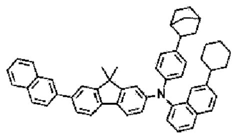
271

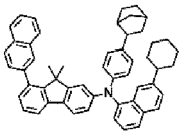
272

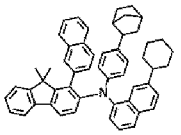
273

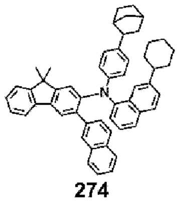
274

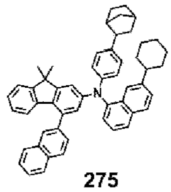
275

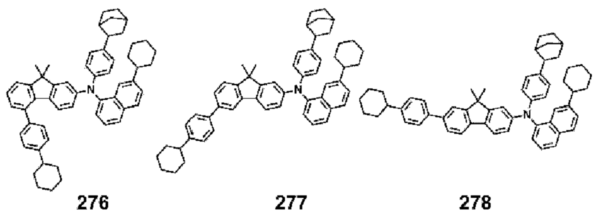

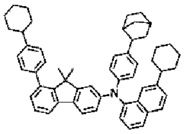

276 277 278 279

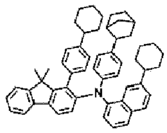

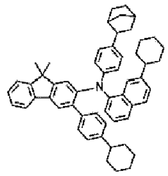

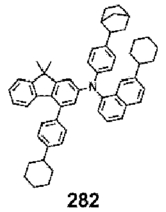

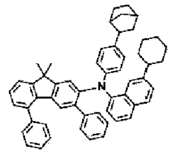

280 281 282 283

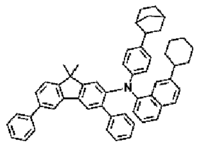

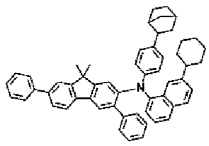

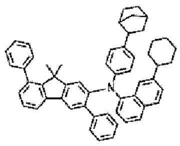

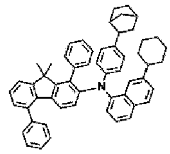

284 285 286 287

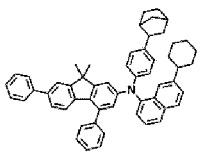

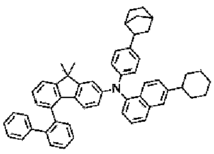

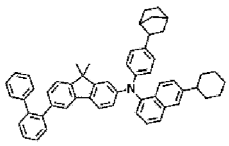

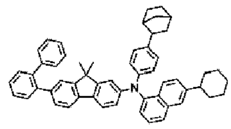

288 289 290 291

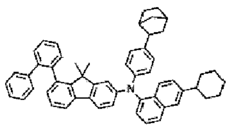

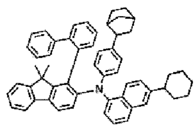

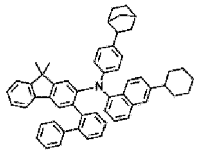

292 293 294

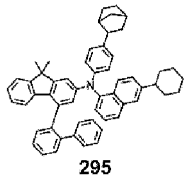
295

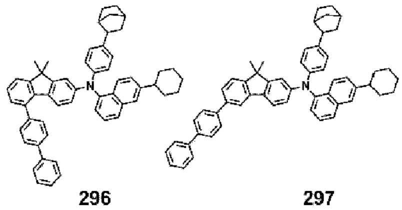
296 297

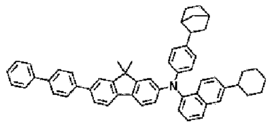
298

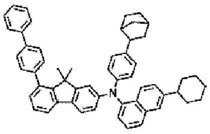
299

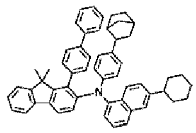
300

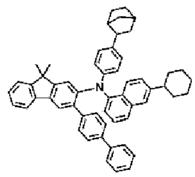
301

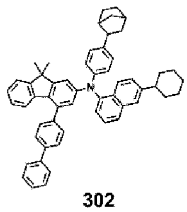
302

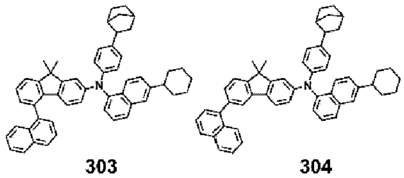
303 304

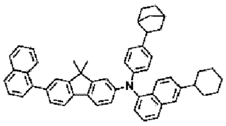
305

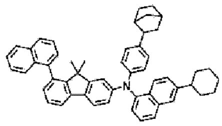
306

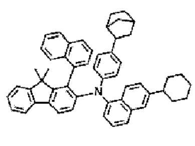
307

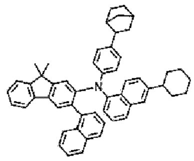
308

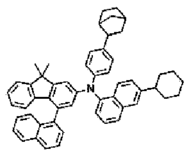
309

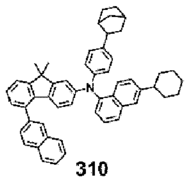
310

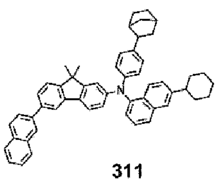
311

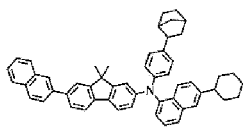
312

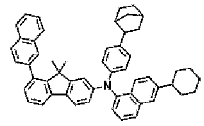
313

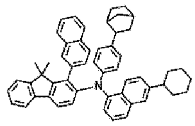
314

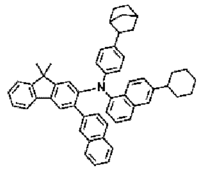
315

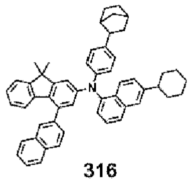
316

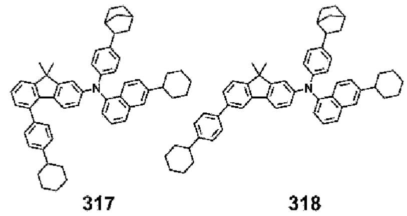
317

318

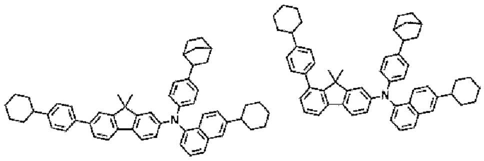
319

320

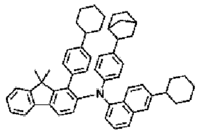
321

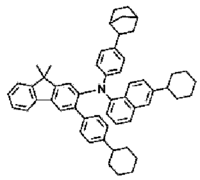
322

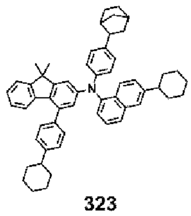
323

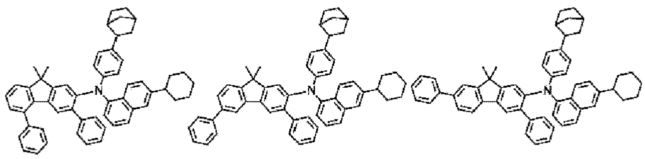
324

325

326

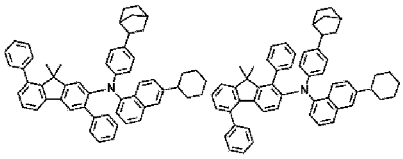
327

328

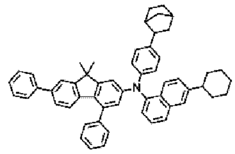
329

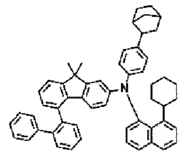
330

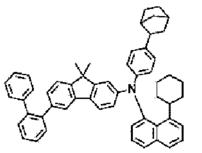
331

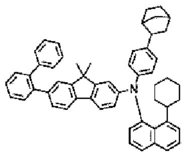
332

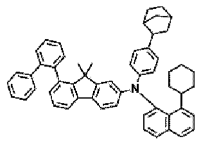
333

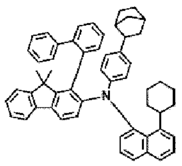
334

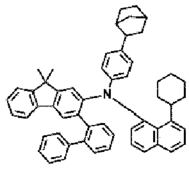
335

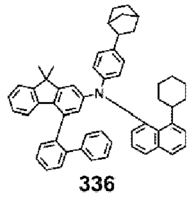
336

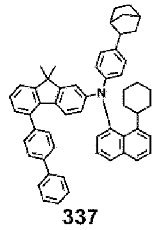
337

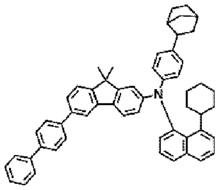
338

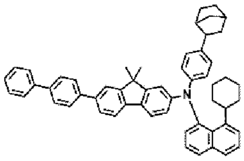
339

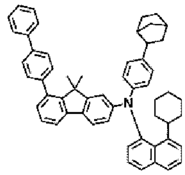
340

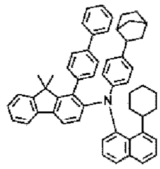
341

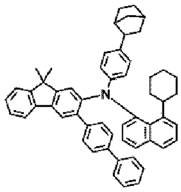
342

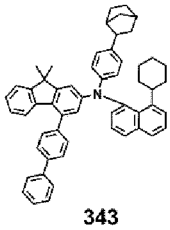
343

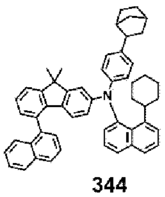
344

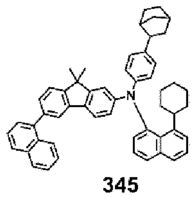
345

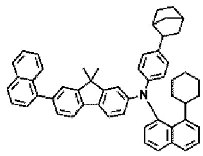
346

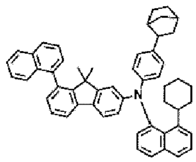
347

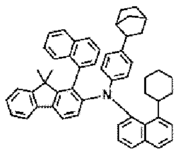
348

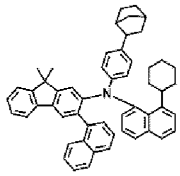
349

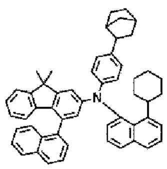
350

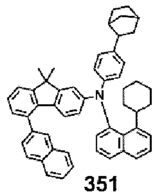
351

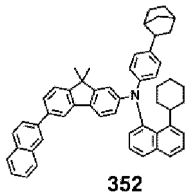
352

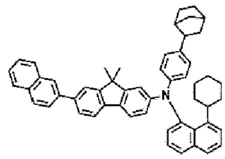
353

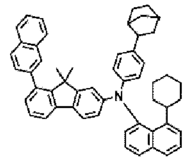 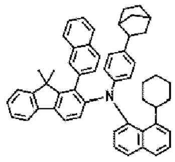 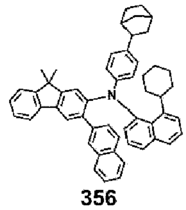 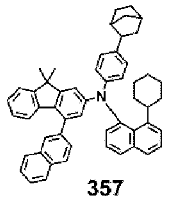

354 355 356 357

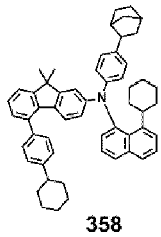 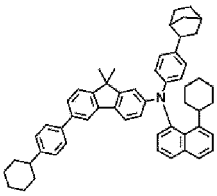 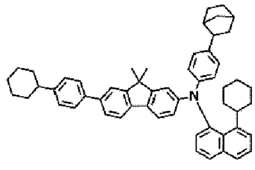 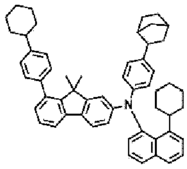

358 359 360 361

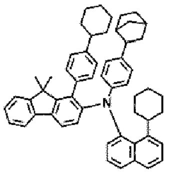 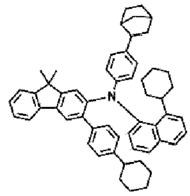 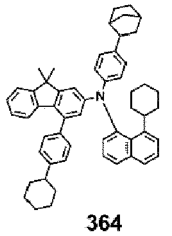 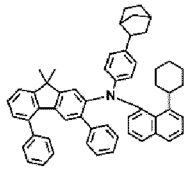

362 363 364 365

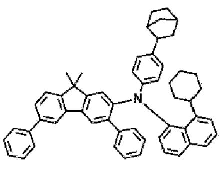 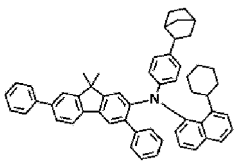 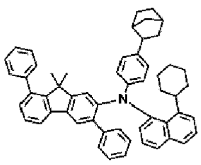 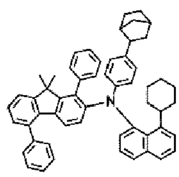

366 367 368 369

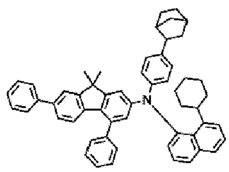 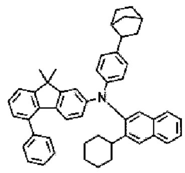 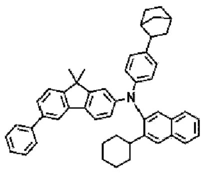

370 371 372

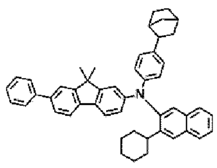
373
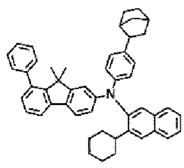
374
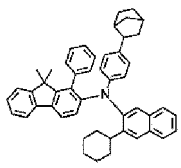
375
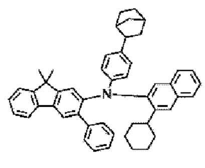
376

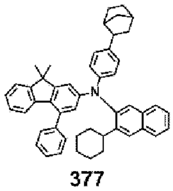
377
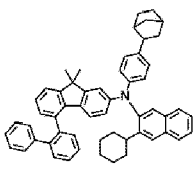
378
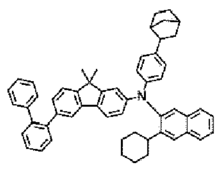
379
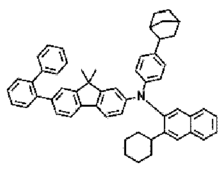
380

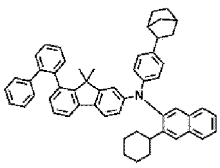
381
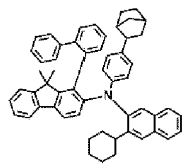
382
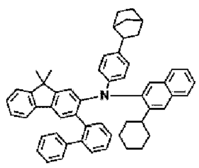
383
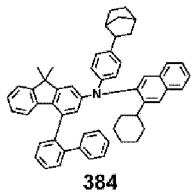
384

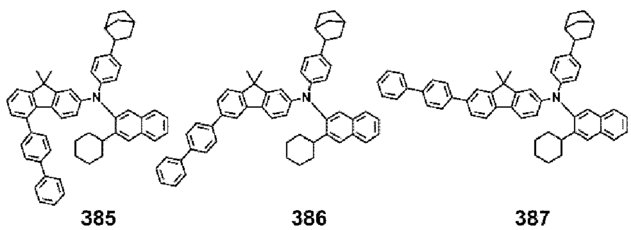
385
386
387
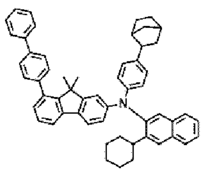
388

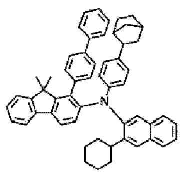
389
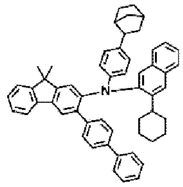
390
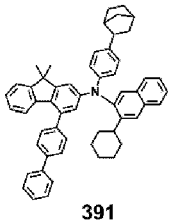
391
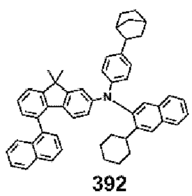
392

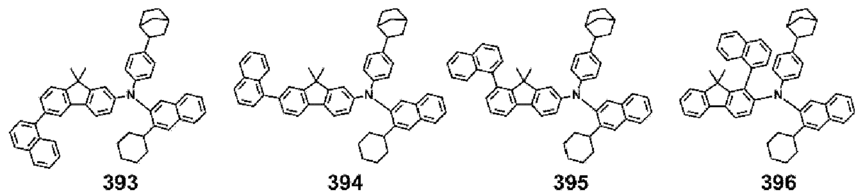

393 394 395 396

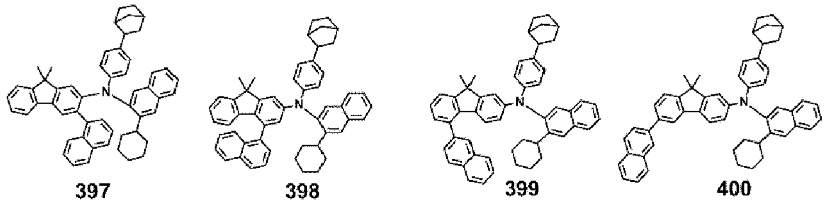

397 398 399 400

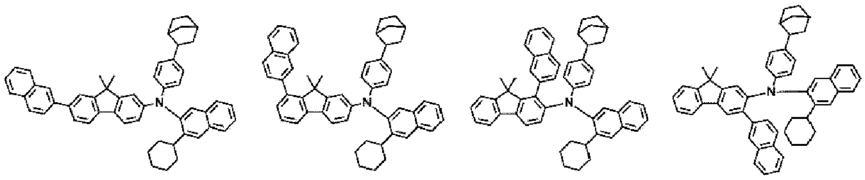

401 402 403 404

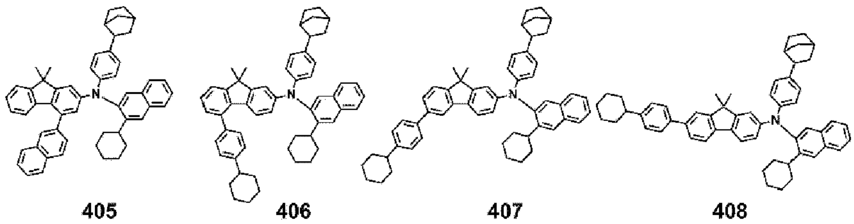

405 406 407 408

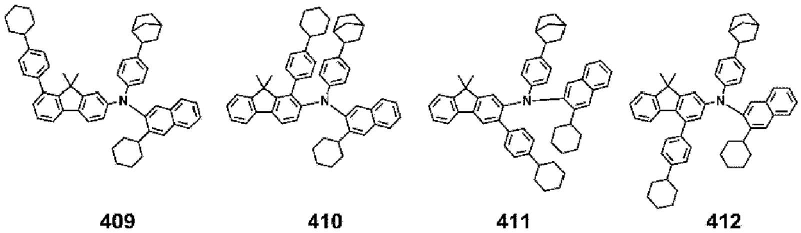

409 410 411 412

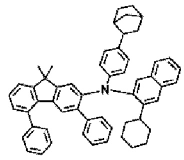
413
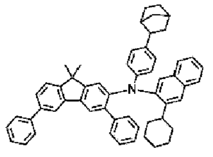
414
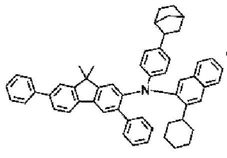
415
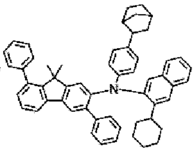
416

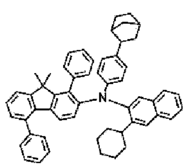
417
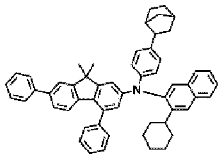
418
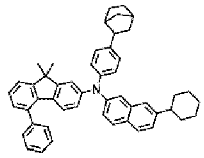
419
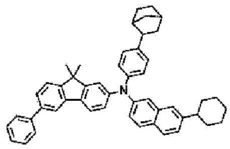
420

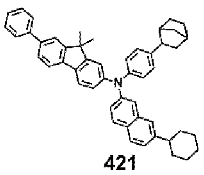
421
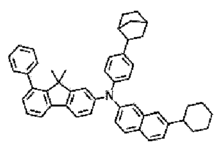
422
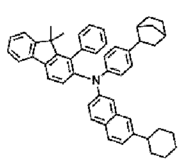
423
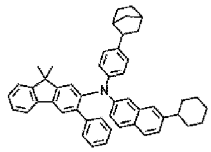
424

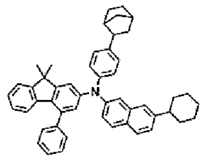
425
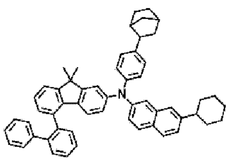
426
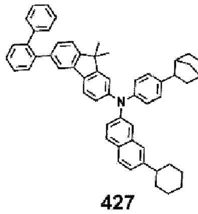
427
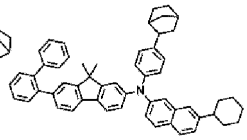
428

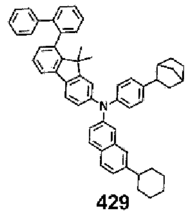
429
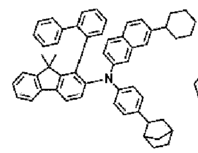
430
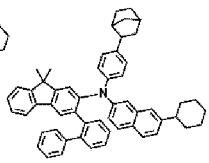
431

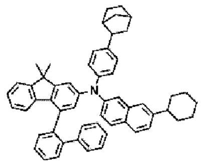
432
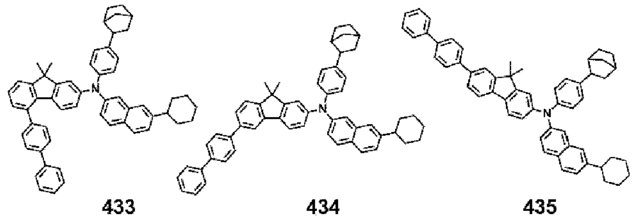
433 434 435

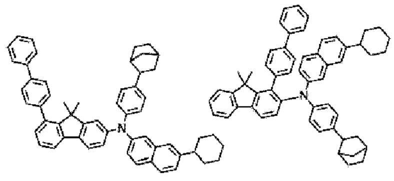
436 437
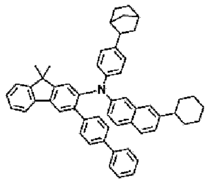
438
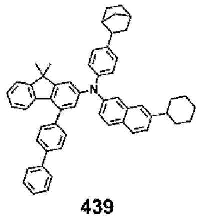
439

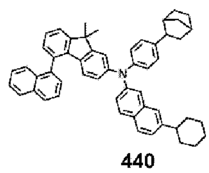
440
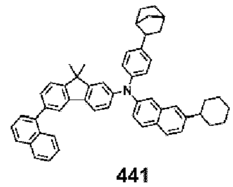
441
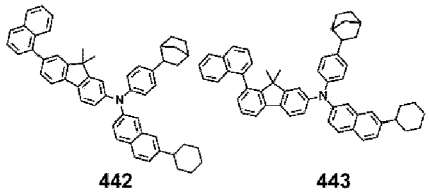
442 443

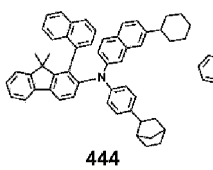
444
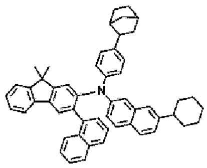
445
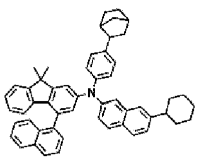
446
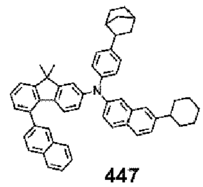
447

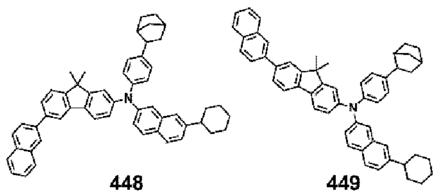
448 449
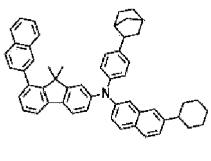
450
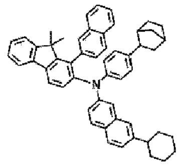
451

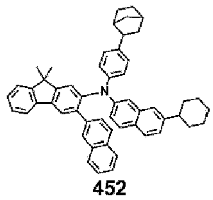

452

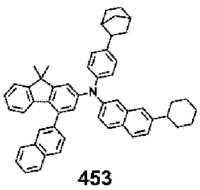

453

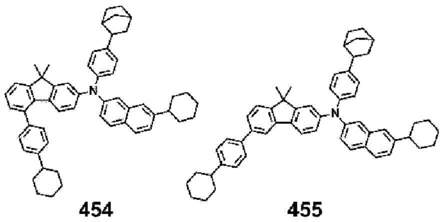

454 455

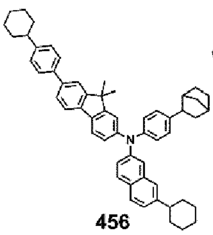

456

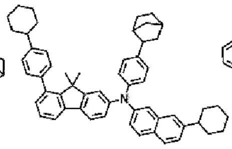

457

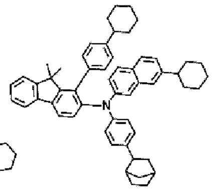

458

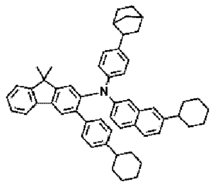

459

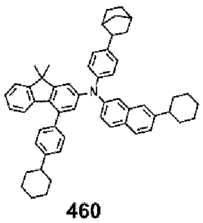

460

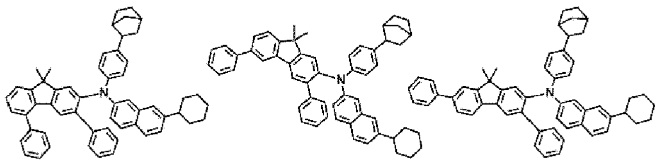

461 462 463

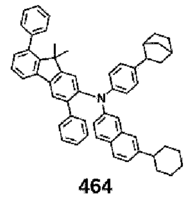

464

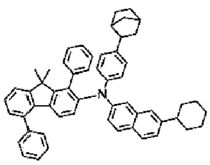

465

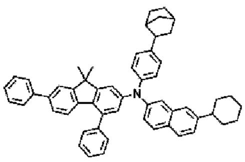

466

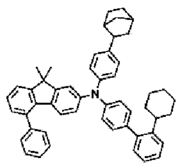

467

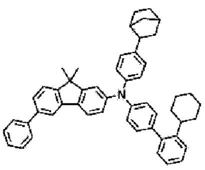

468

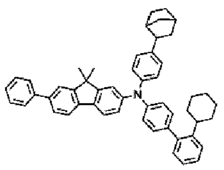

469

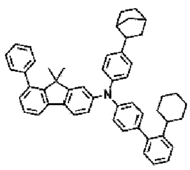

470

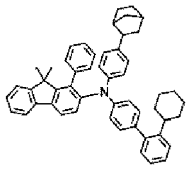
471
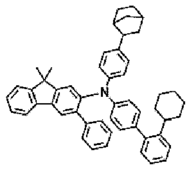
472
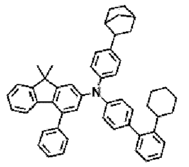
473
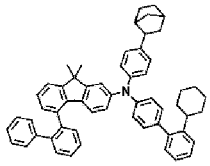
474

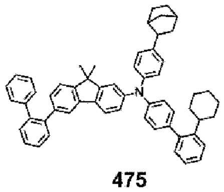
475
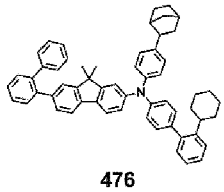
476
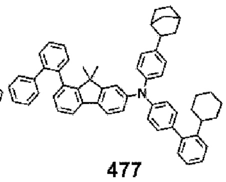
477
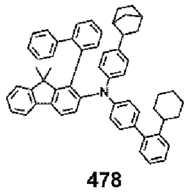
478

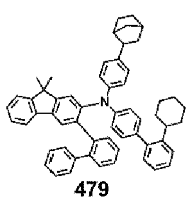
479
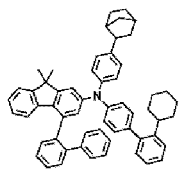
480
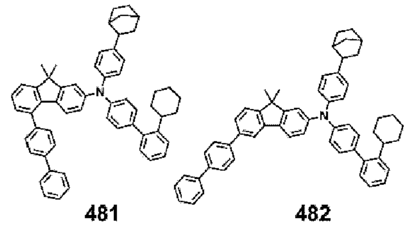
481
482

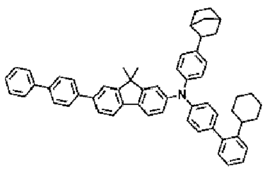
483
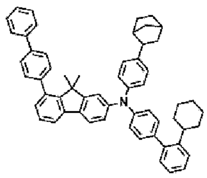
484
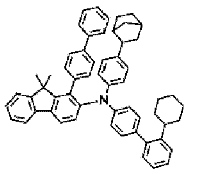
485
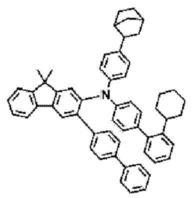
486

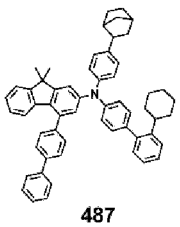
487
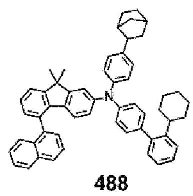
488
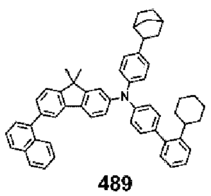
489
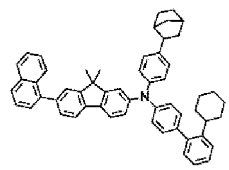
490

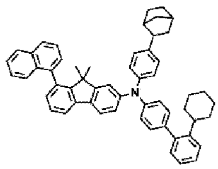
491
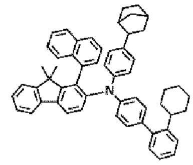
492
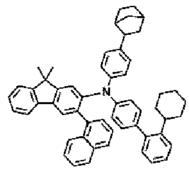
493
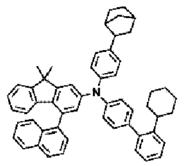
494

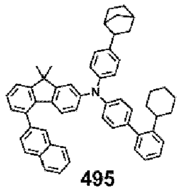
495
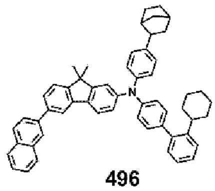
496
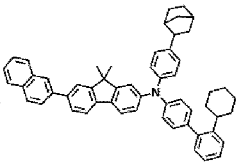
497
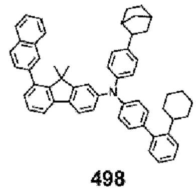
498

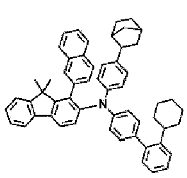
499
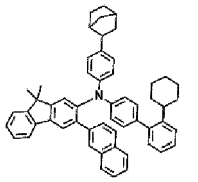
500
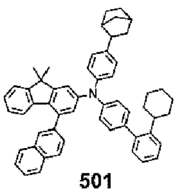
501
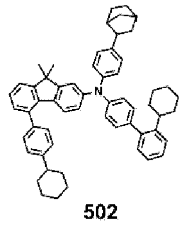
502

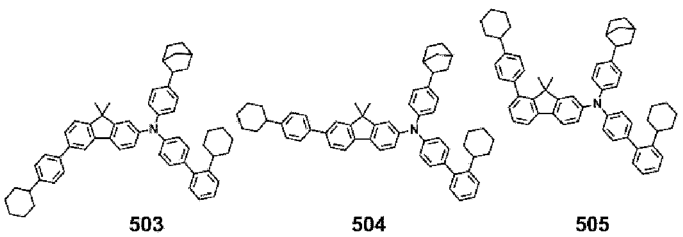
503
504
505
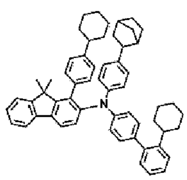
506

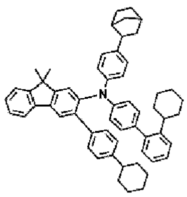
507
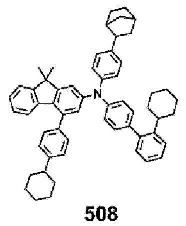
508
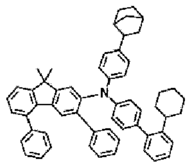
509

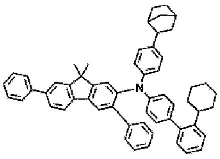

510

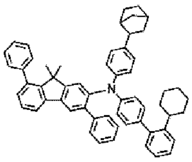

511

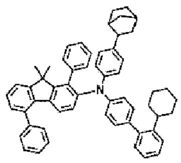

512

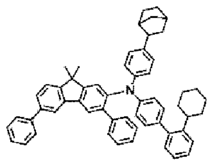

513

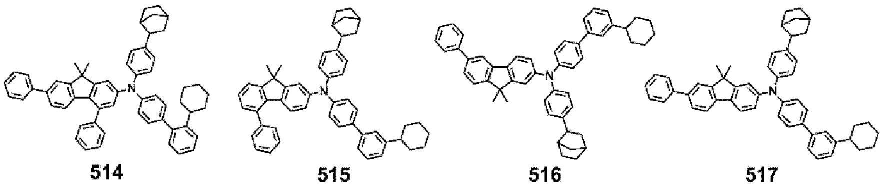

514 515 516 517

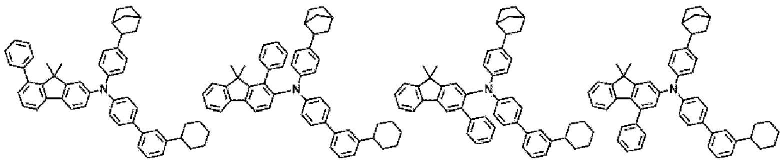

518 519 520 521

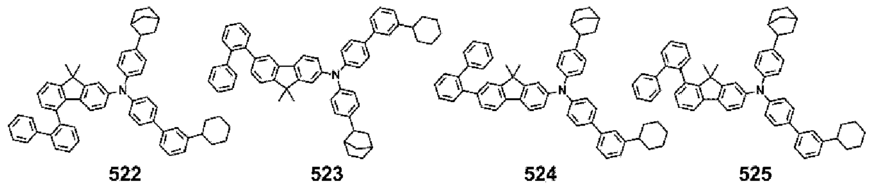

522 523 524 525

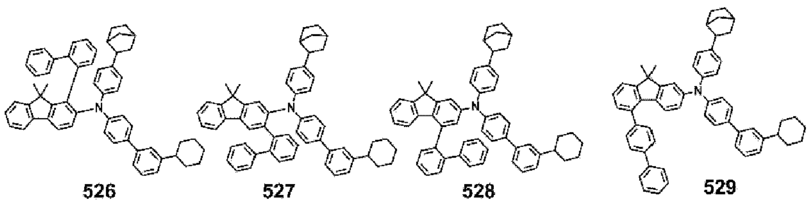

526 527 528 529

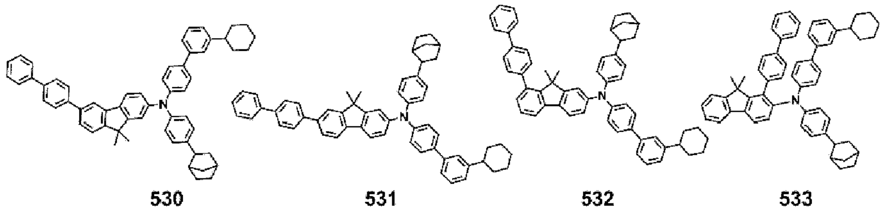

530 531 532 533

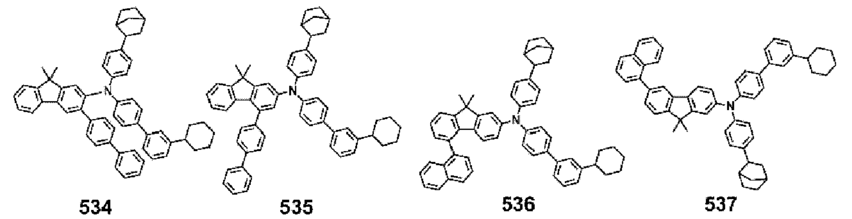

534 535 536 537

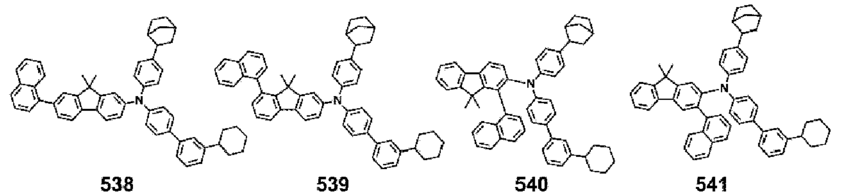

538 539 540 541

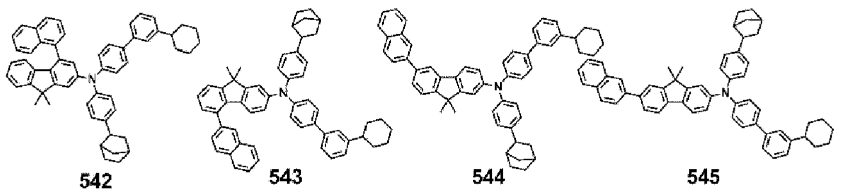

542 543 544 545

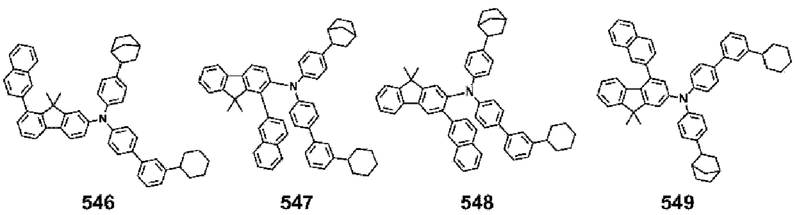

546 547 548 549

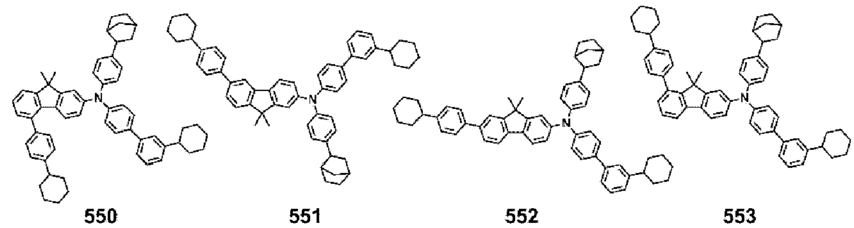

550 551 552 553

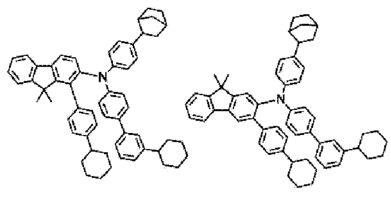

554 555

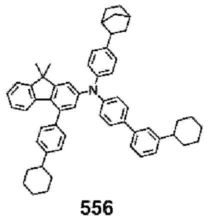

556

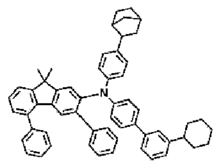

557

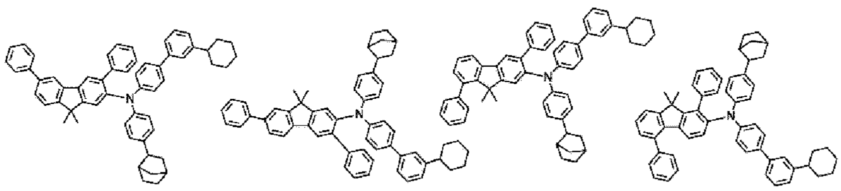

558 559 560 561

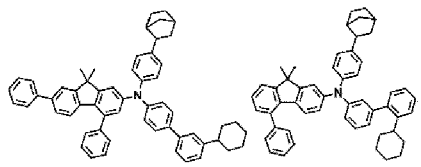

562 563

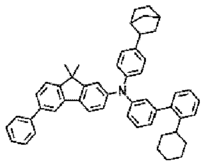

564

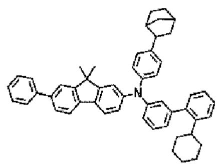

565

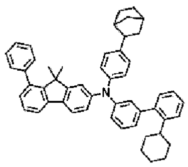

566

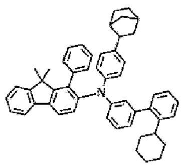

567

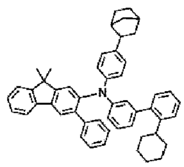

568

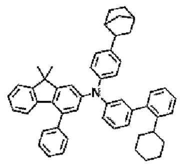
569
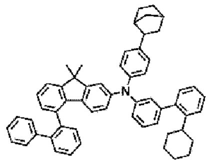
570
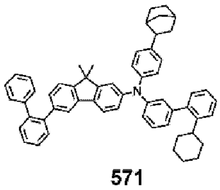
571
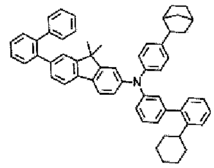
572

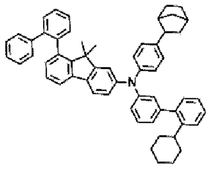
573
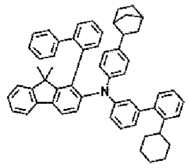
574
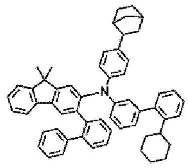
575
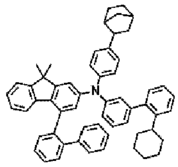
576

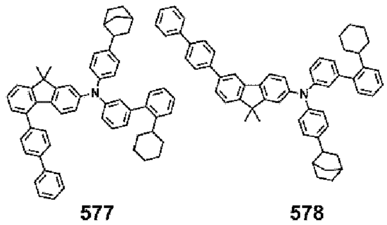
577
578
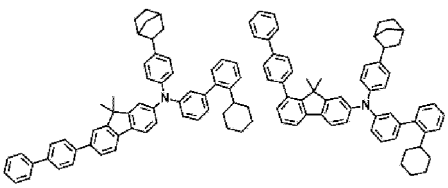
579
580

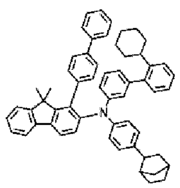
581
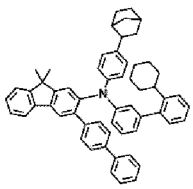
582
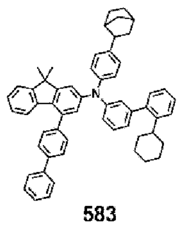
583
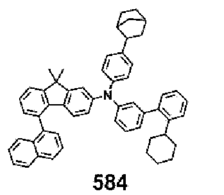
584

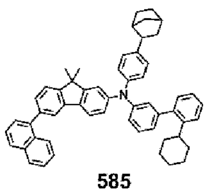
585
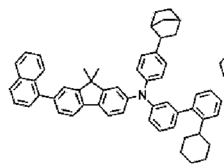
586
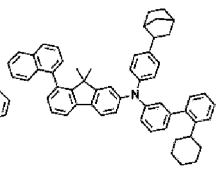
587
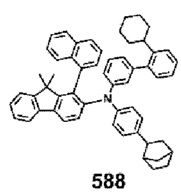
588

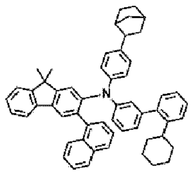
589

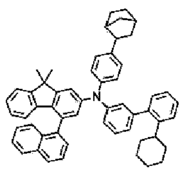
590

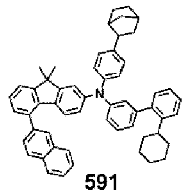
591

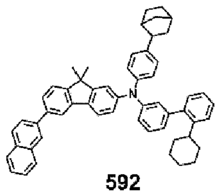
592

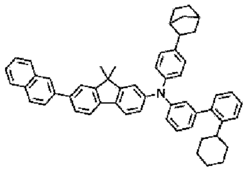
593

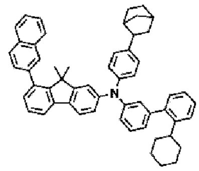
594

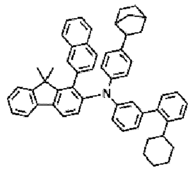
595

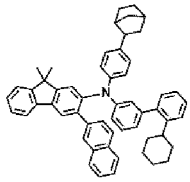
596

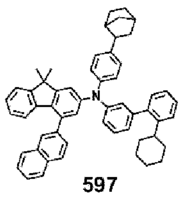
597

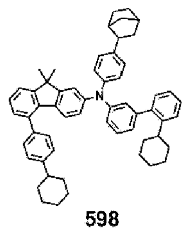
598

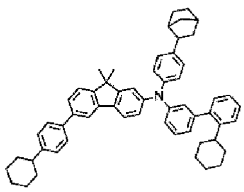
599

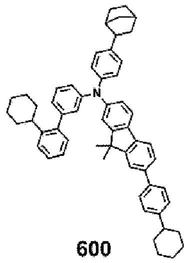
600

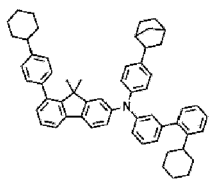
601

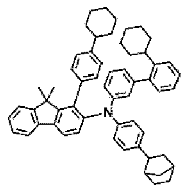
602

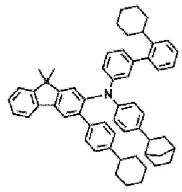
603

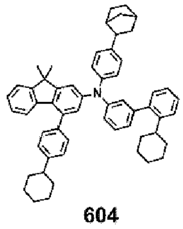
604

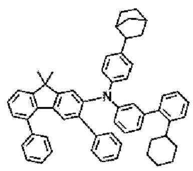
605

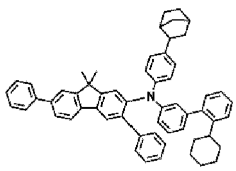
606

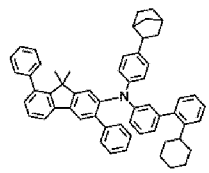
607

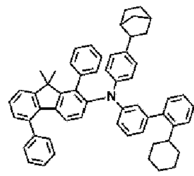
608
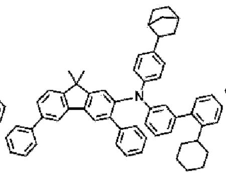
609
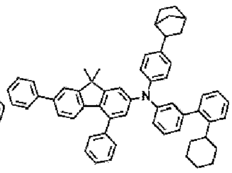
610
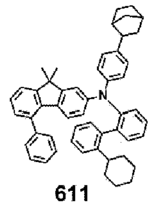
611
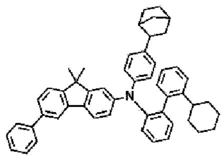
612
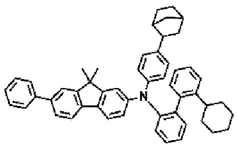
613
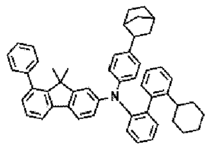
614
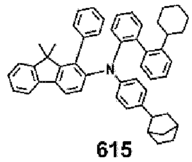
615
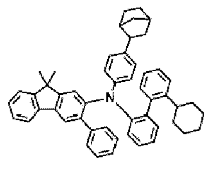
616
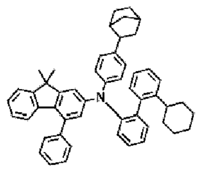
617
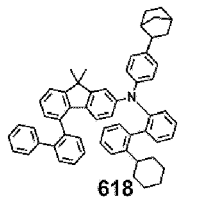
618
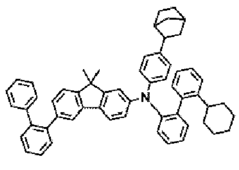
619
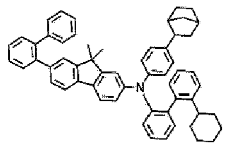
620
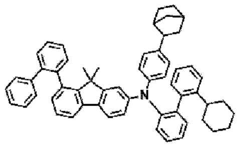
621
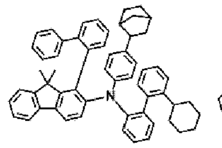
622
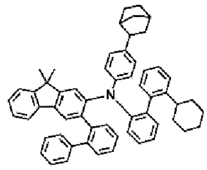
623
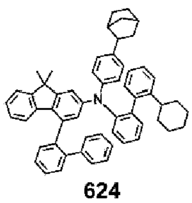
624
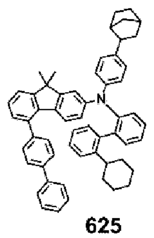
625
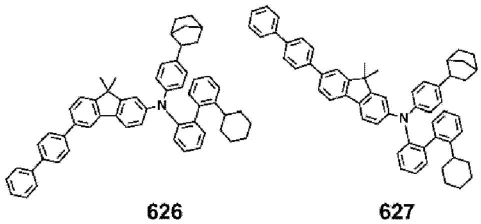
626

627

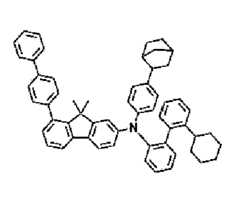
628
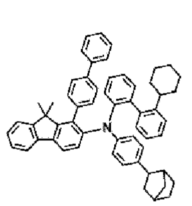
629
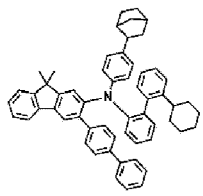
630
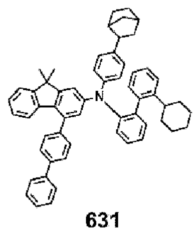
631

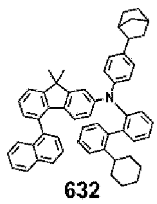
632
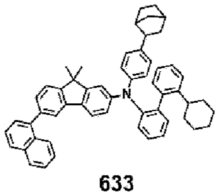
633
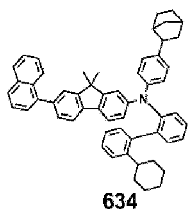
634
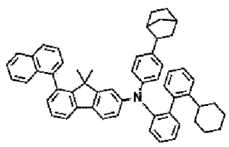
635

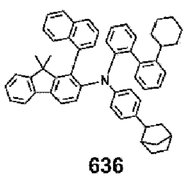
636
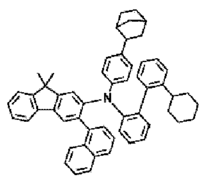
637
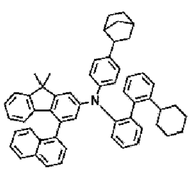
638
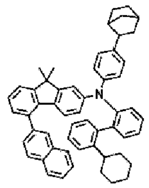
639

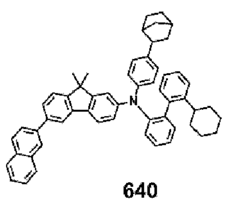
640
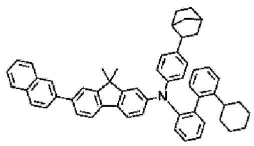
641
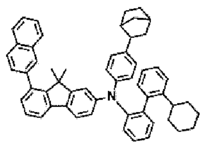
642
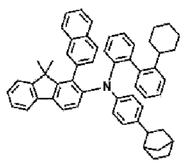
643

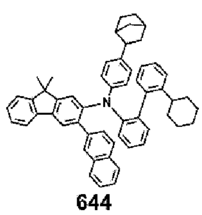
644
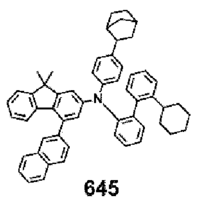
645
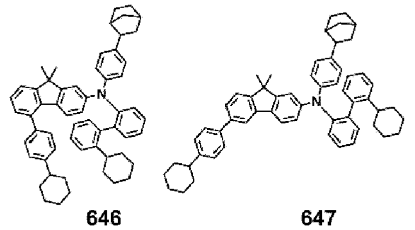
646

647

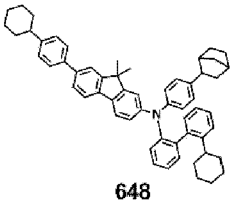
648
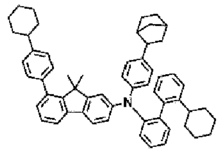
649
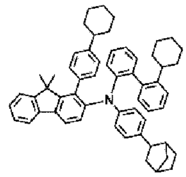
650
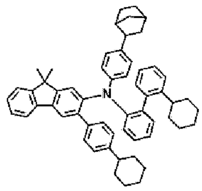
651

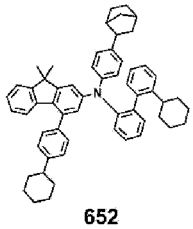
652
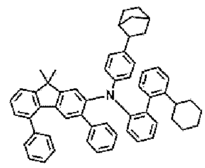
653
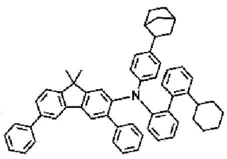
654
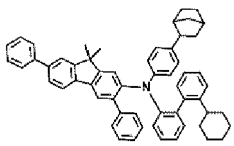
655

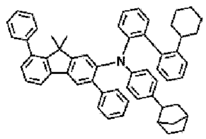
656
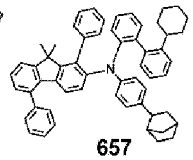
657
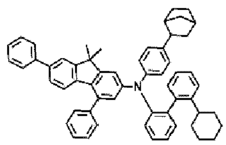
658
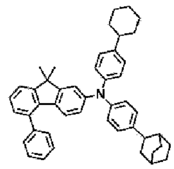
659

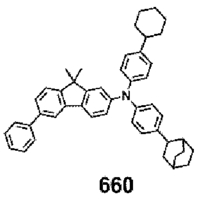
660
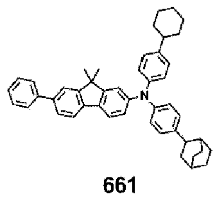
661
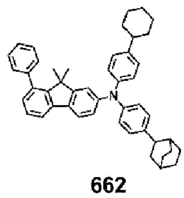
662
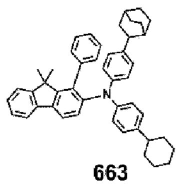
663

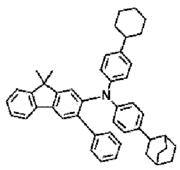
664
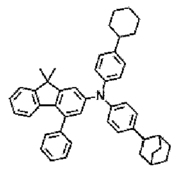
665
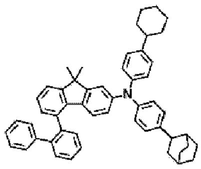
666

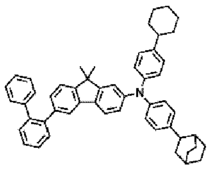
667

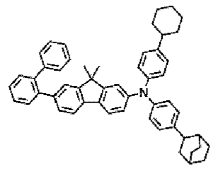
668

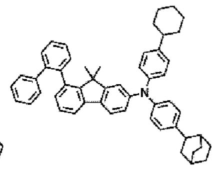
669

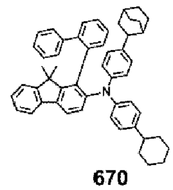
670

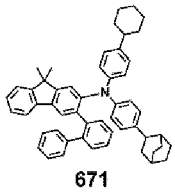
671

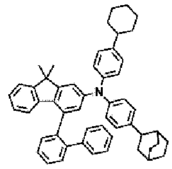
672

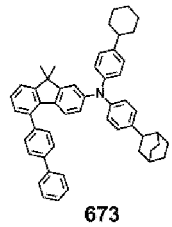
673

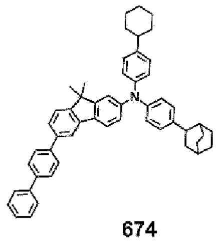
674

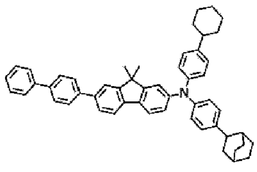
675

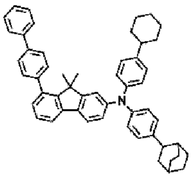
676

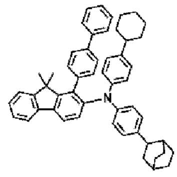
677

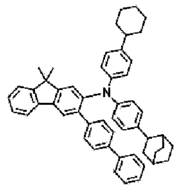
678

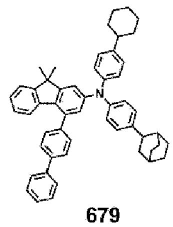
679

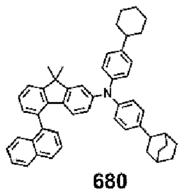
680

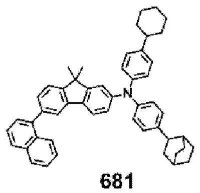
681

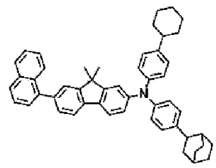
682

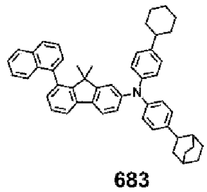
683

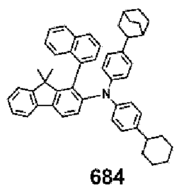
684

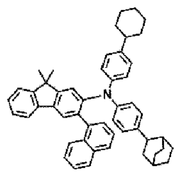
685

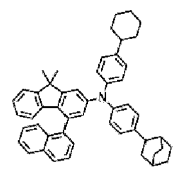
686

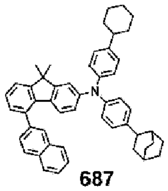
687
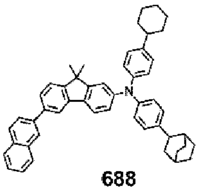
688
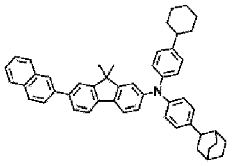
689
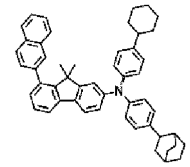
690

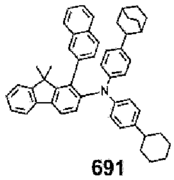
691
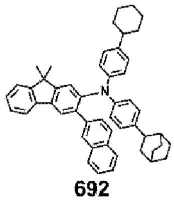
692
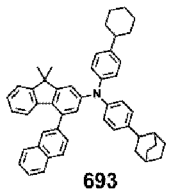
693
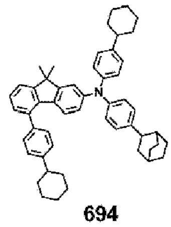
694

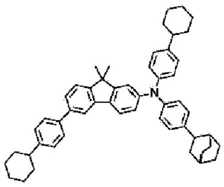
695
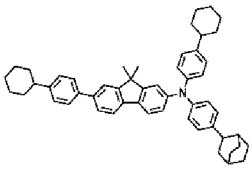
696
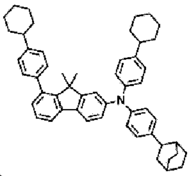
697
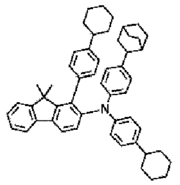
698

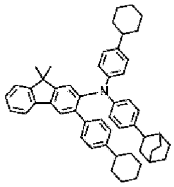
699
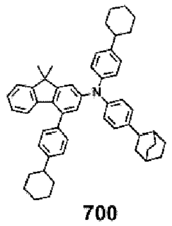
700
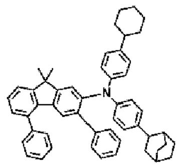
701
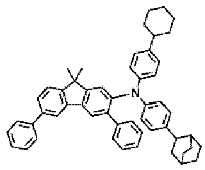
702

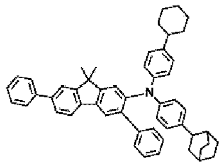
703
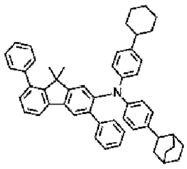
704
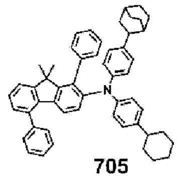
705

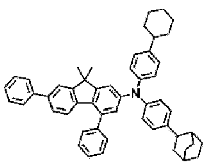

706

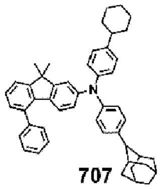

707

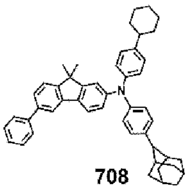

708

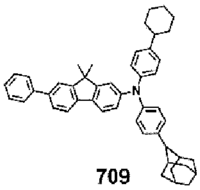

709

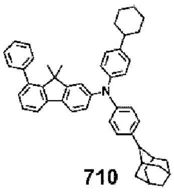

710

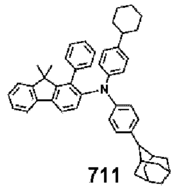

711

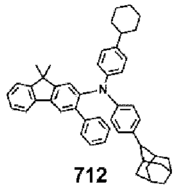

712

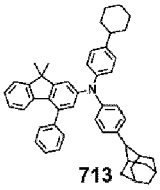

713

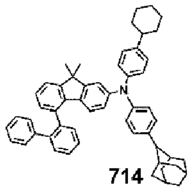

714

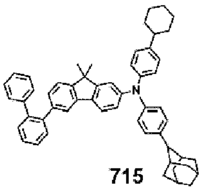

715

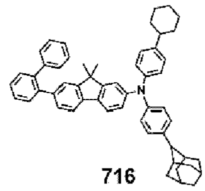

716

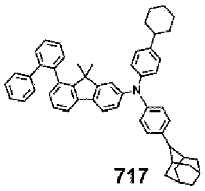

717

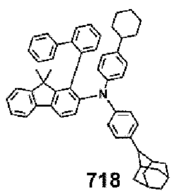

718

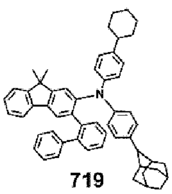

719

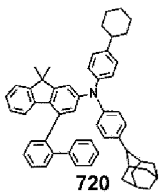

720

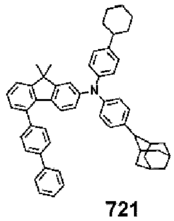

721

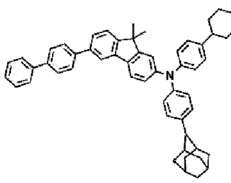

722

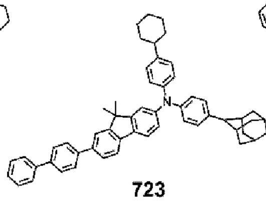

723

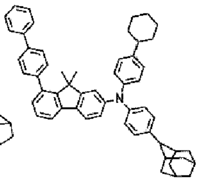

724

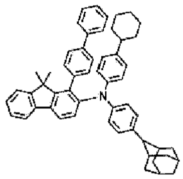

725

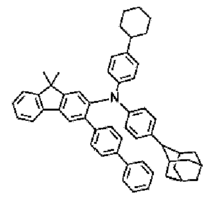
726
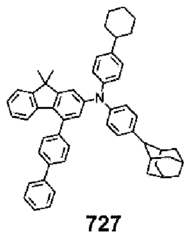
727
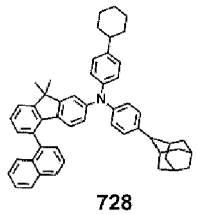
728
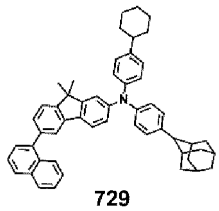
729

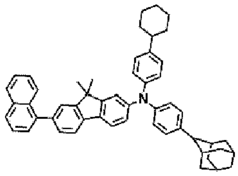
730
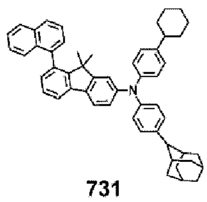
731
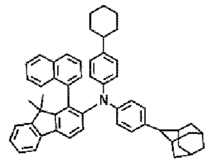
732
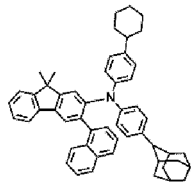
733

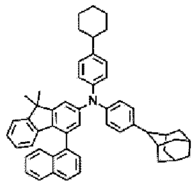
734
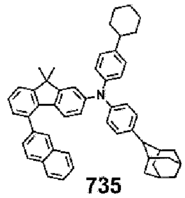
735
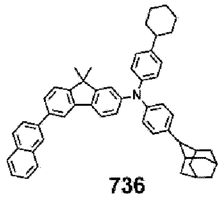
736
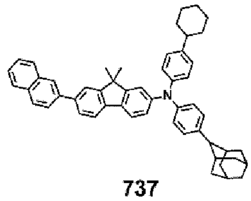
737

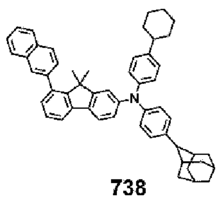
738
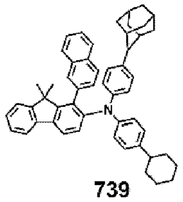
739
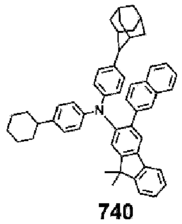
740
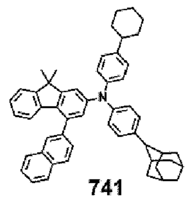
741

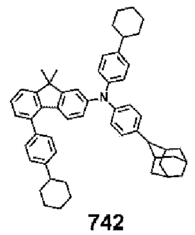
742
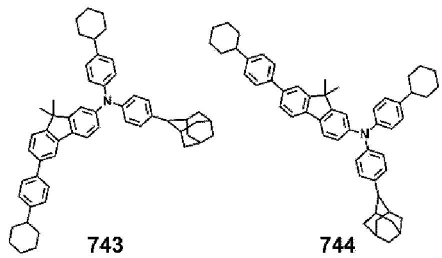
743
744
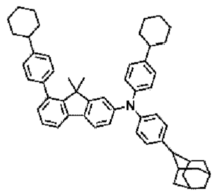
745

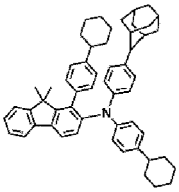
746
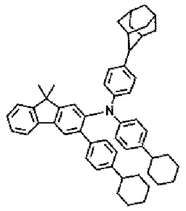
747
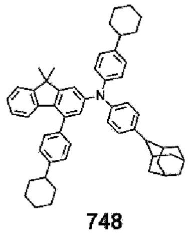
748
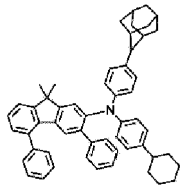
749

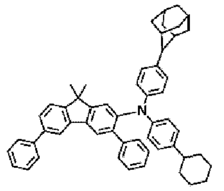
750
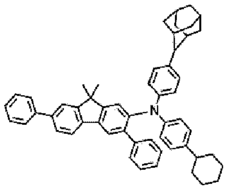
751
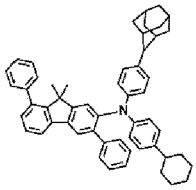
752
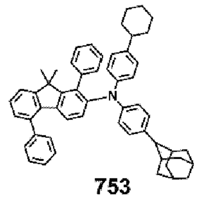
753

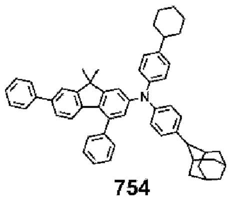
754
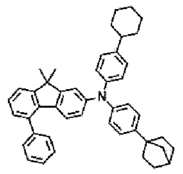
755
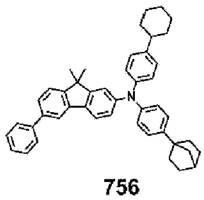
756
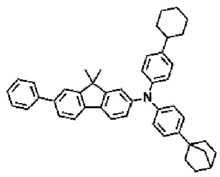
757

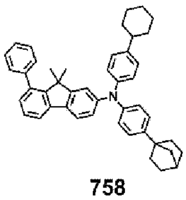
758
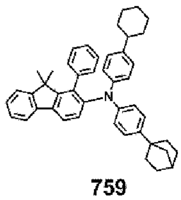
759
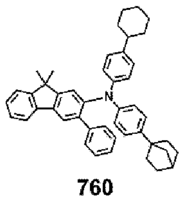
760
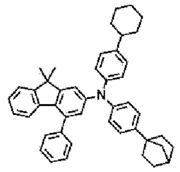
761

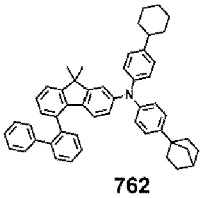
762
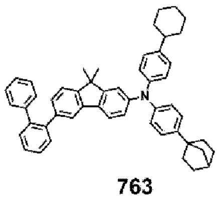
763
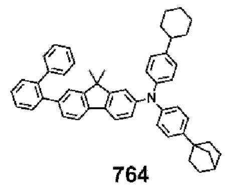
764

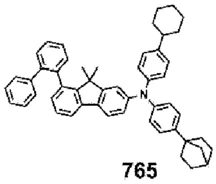
765
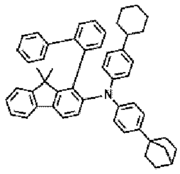
766
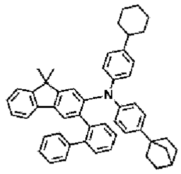
767
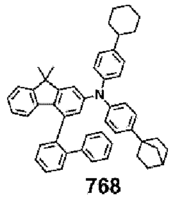
768

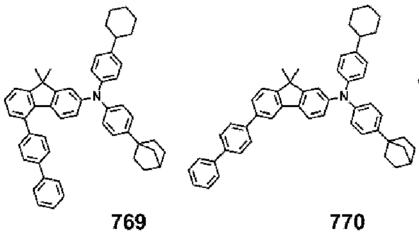
769

770
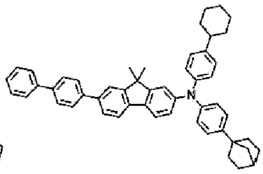
771
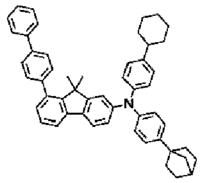
772

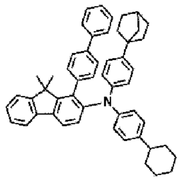
773
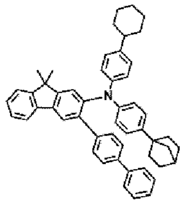
774
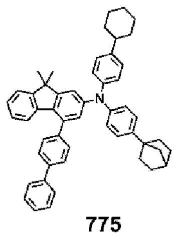
775
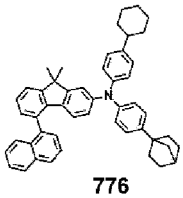
776

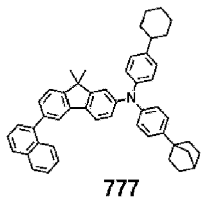
777
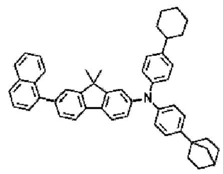
778
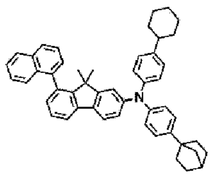
779
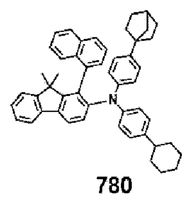
780

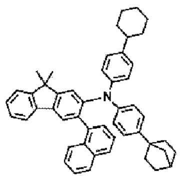
781
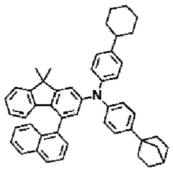
782
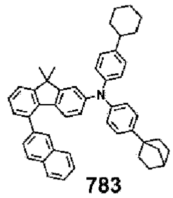
783
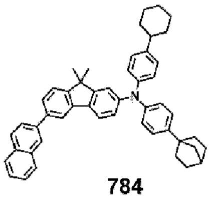
784

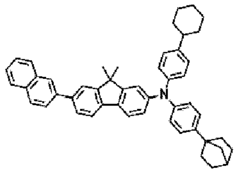

785

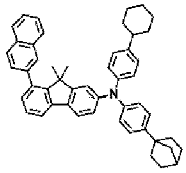

786

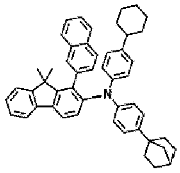

787

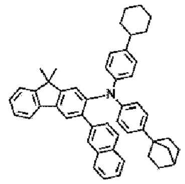

788

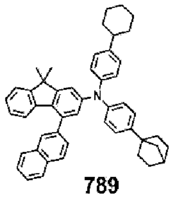

789

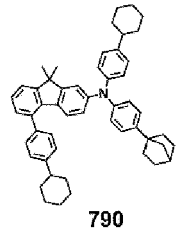

790

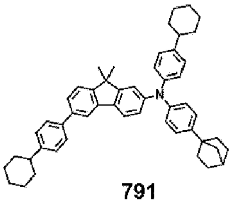

791

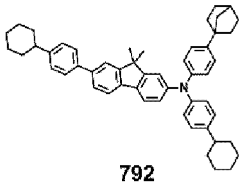

792

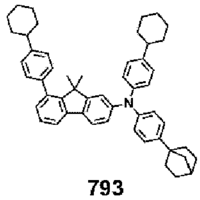

793

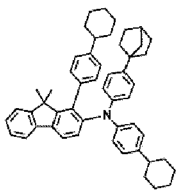

794

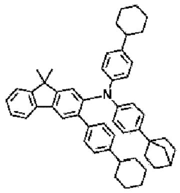

795

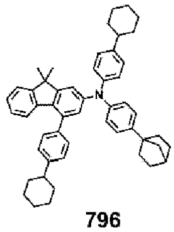

796

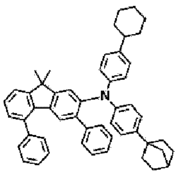

797

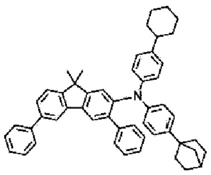

798

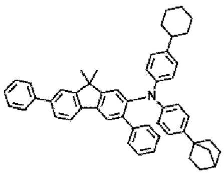

799

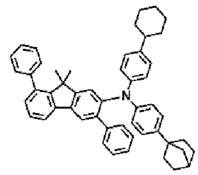

800

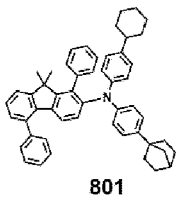

801

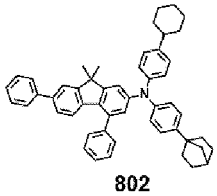

802

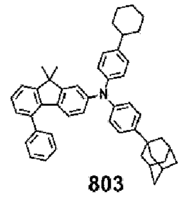

803

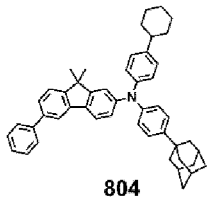
804
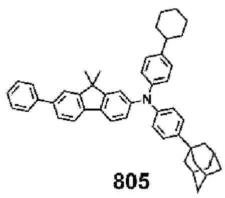
805
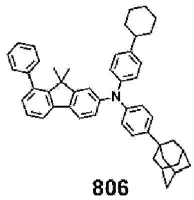
806
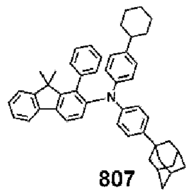
807

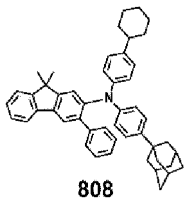
808
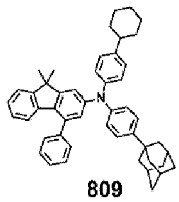
809
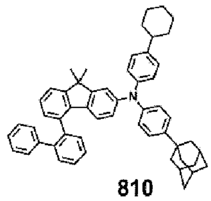
810
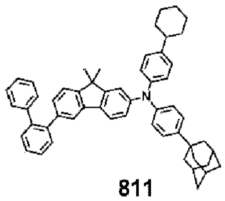
811

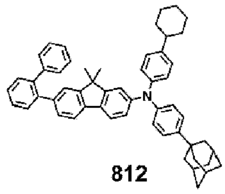
812
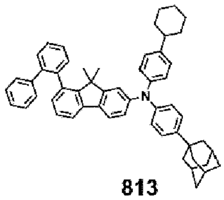
813
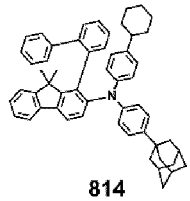
814
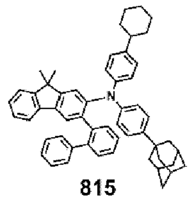
815

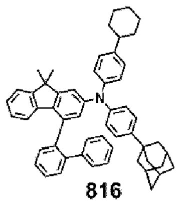
816
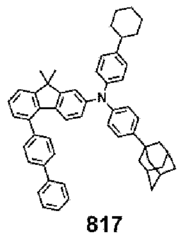
817
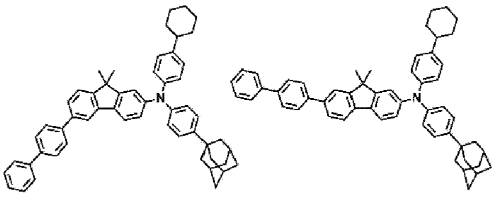
818
819

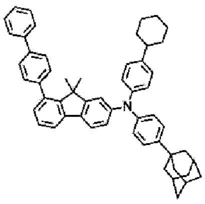
820
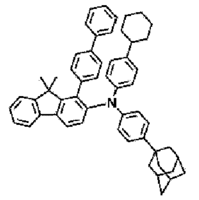
821
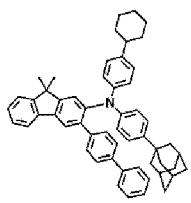
822
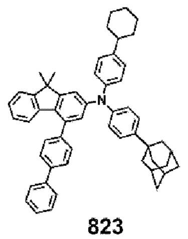
823

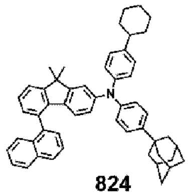
824

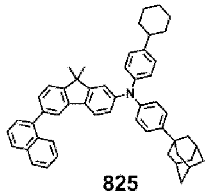
825

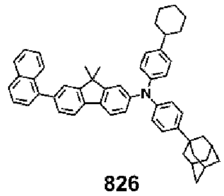
826

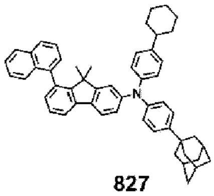
827

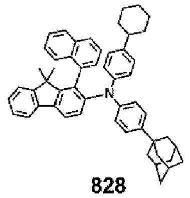
828

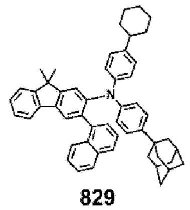
829

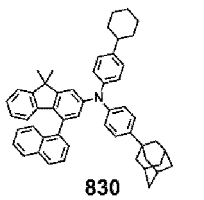
830

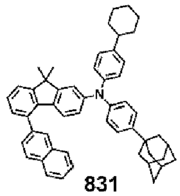
831

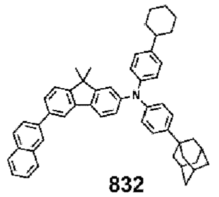
832

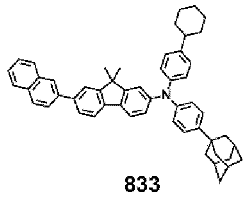
833

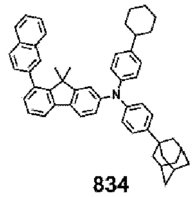
834

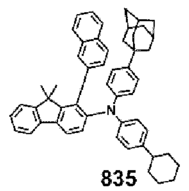
835

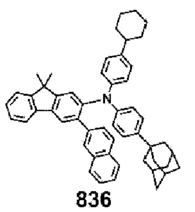
836

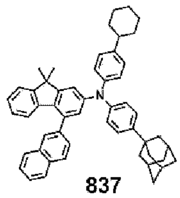
837

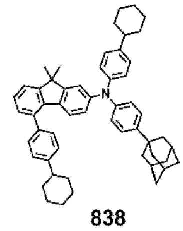
838

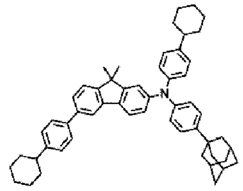
839

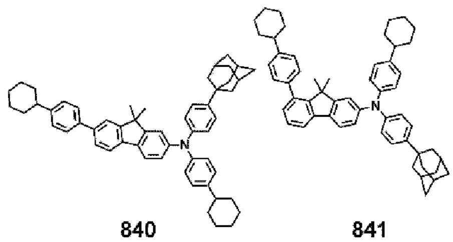
840

841

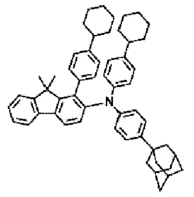
842

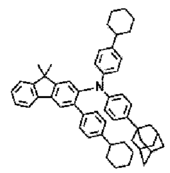
843

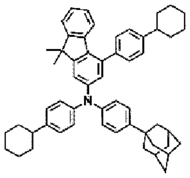
844

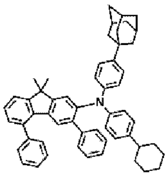
845

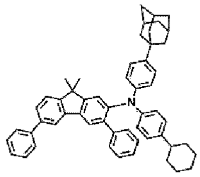
846

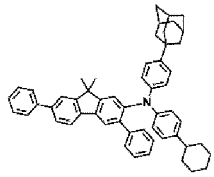
847

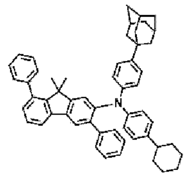
848

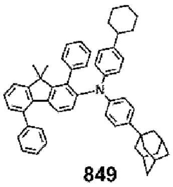
849

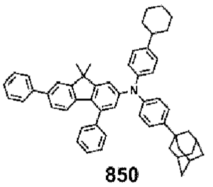
850

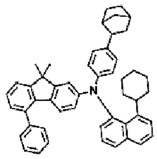
851

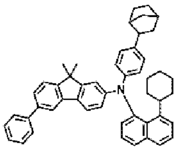
852

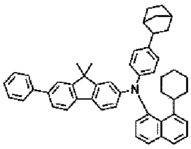
853

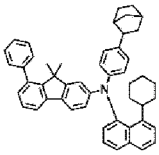
854

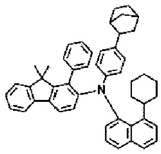
855

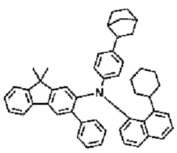
856

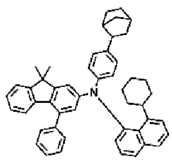
857

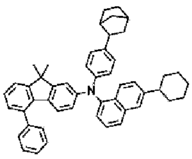
858

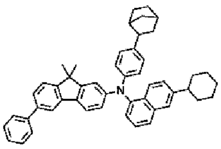
859

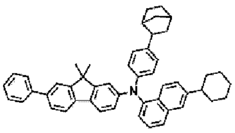
860

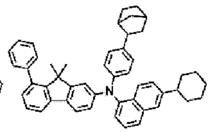
861

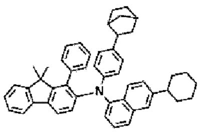
862

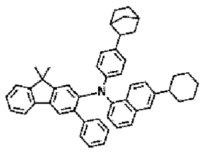
863

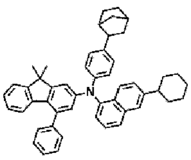
864

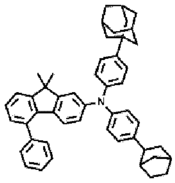
865

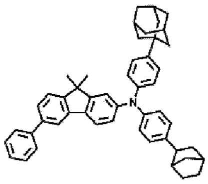
866

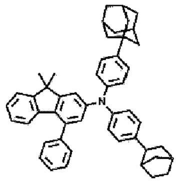
867

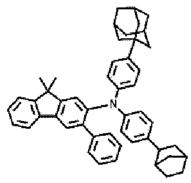
868

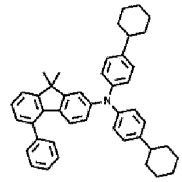
869

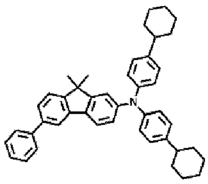
870

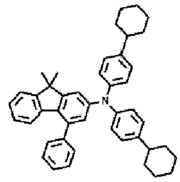
871

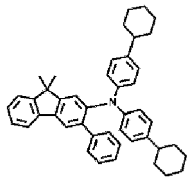
872

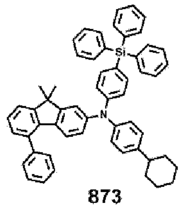
873

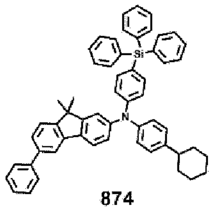
874

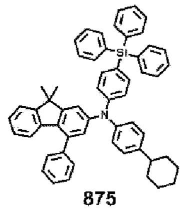
875

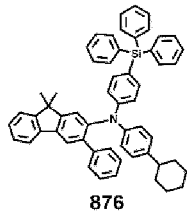
876

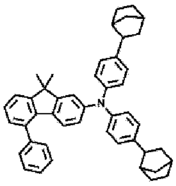
877

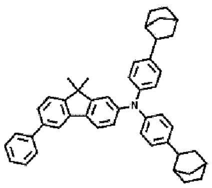
878

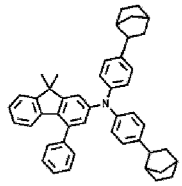
879

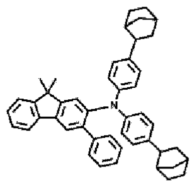
880

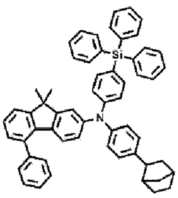
881

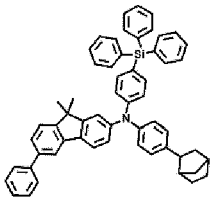
882

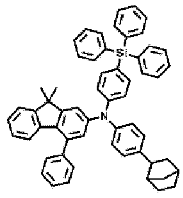
883

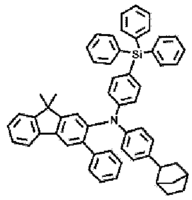
884

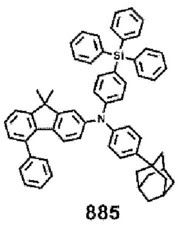
885

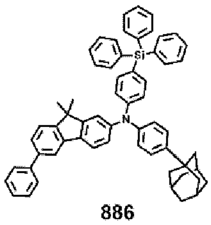
886

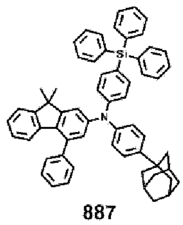
887

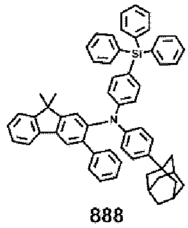
888

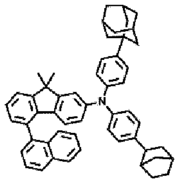
889

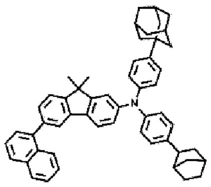
890

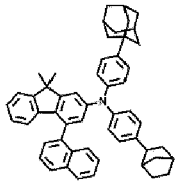
891

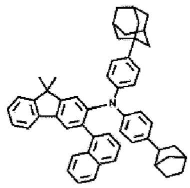
892

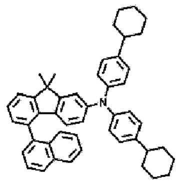
893

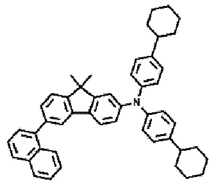
894

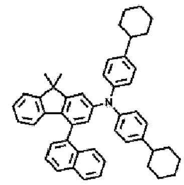
895

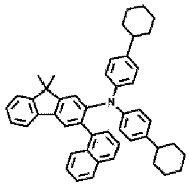
896

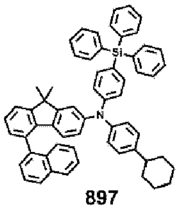
897

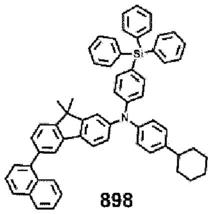
898

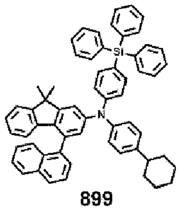
899

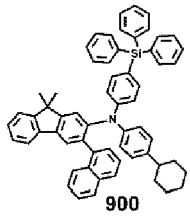
900

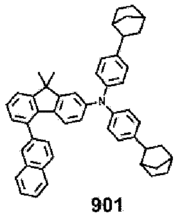
901

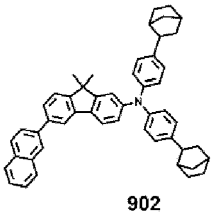
902

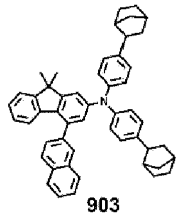
903

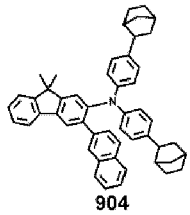
904

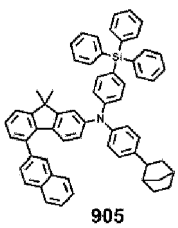
905

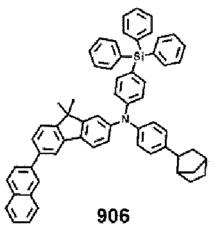
906

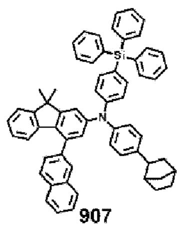
907

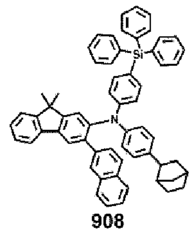
908

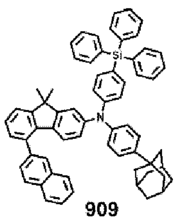
909

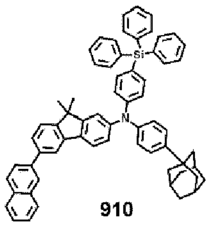
910

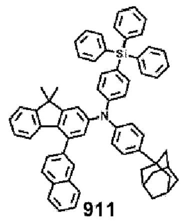
911

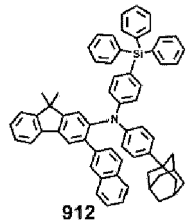
912

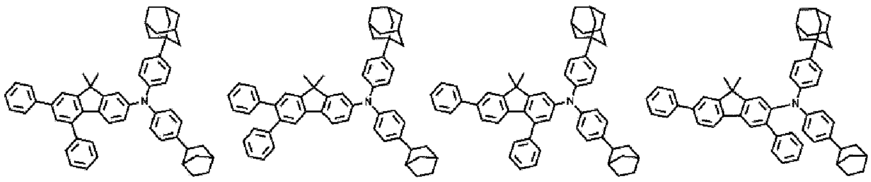

913 914 915 916

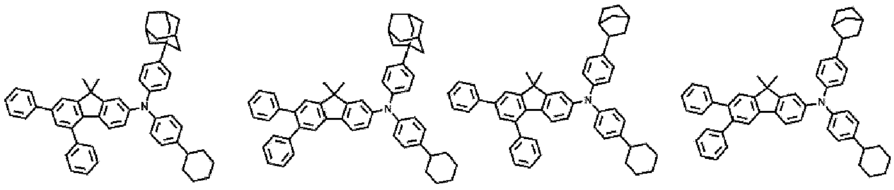

917 918 919 920

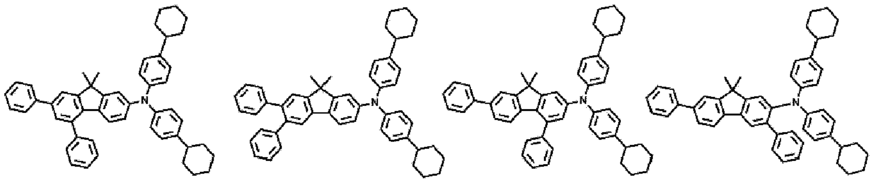

921 922 923 924

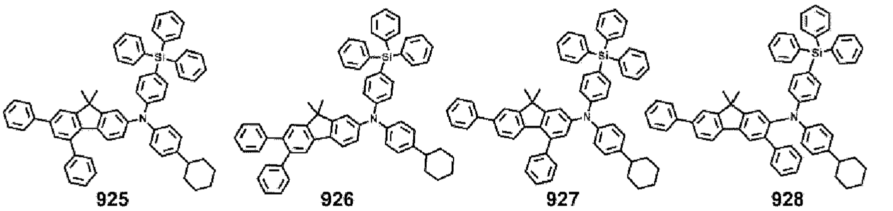

925 926 927 928

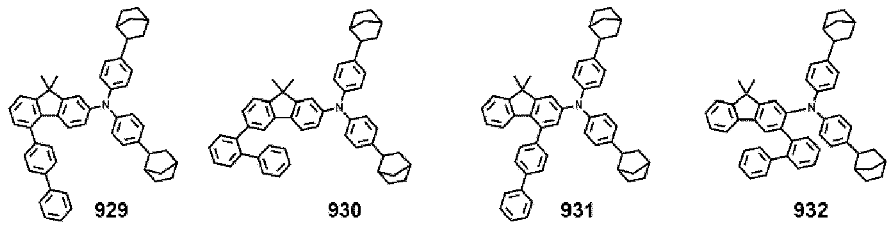

929 930 931 932

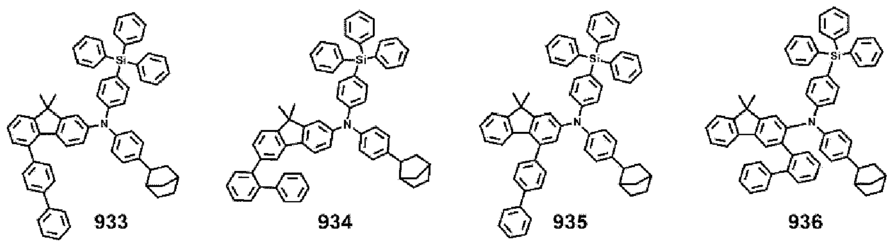

933 934 935 936

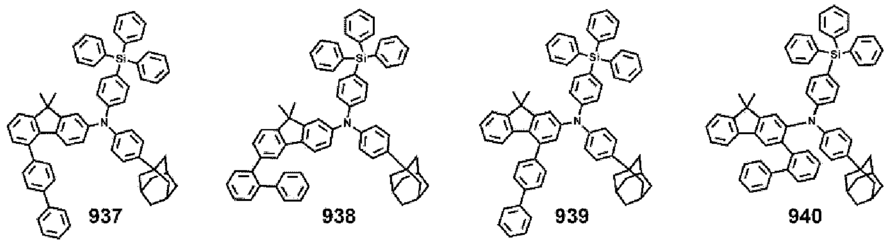

937 938 939 940

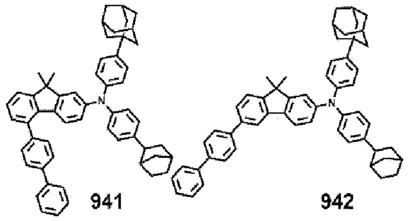
941 942

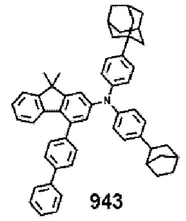
943

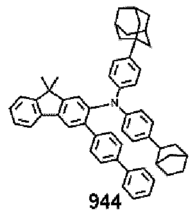
944

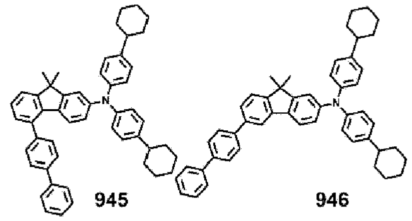
945 946

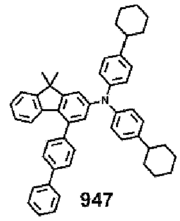
947

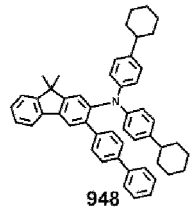
948

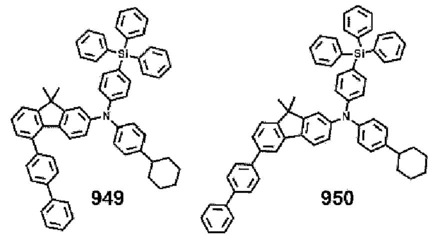
949 950

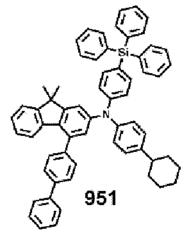
951

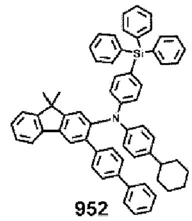
952

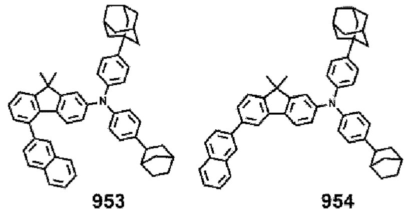
953 954

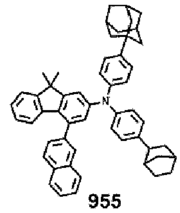
955

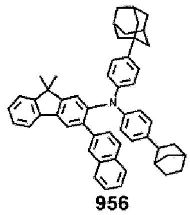
956

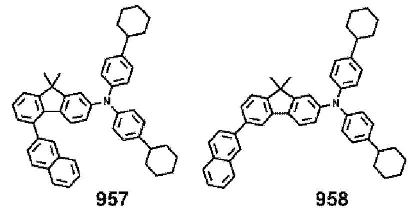
957 958

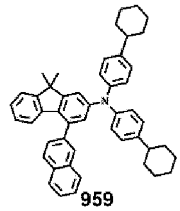
959

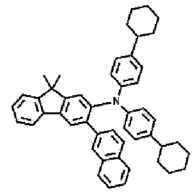
960

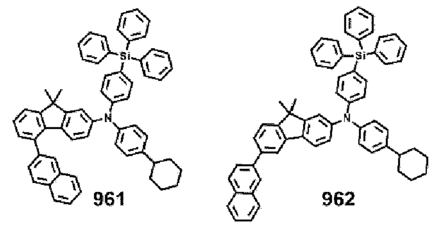
961 962

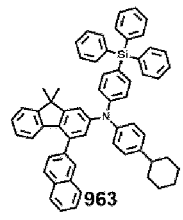
963

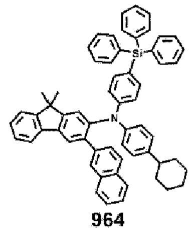
964

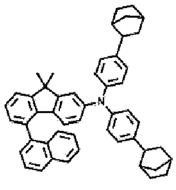
965

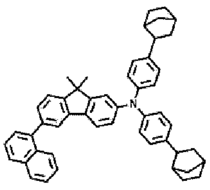
966

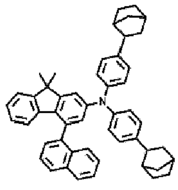
967

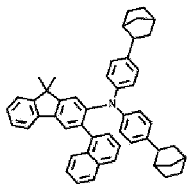
968

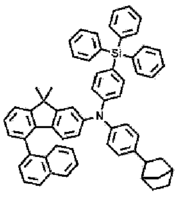
969

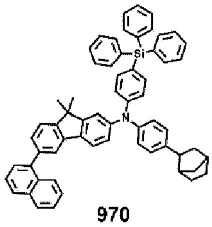
970

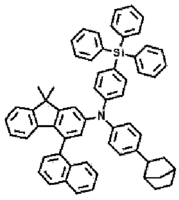
971

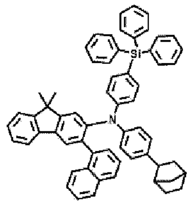
972

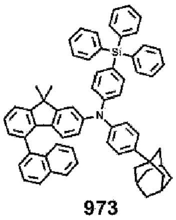
973

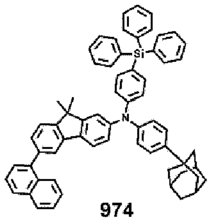
974

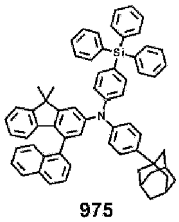
975

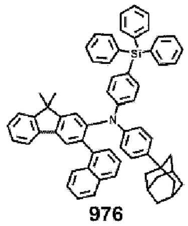
976

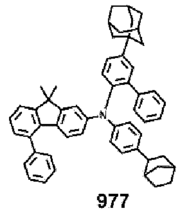
977

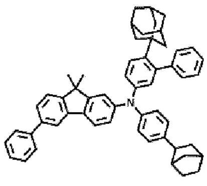
978

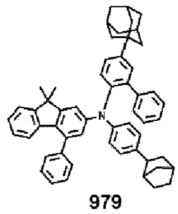
979

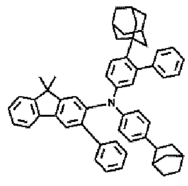
980

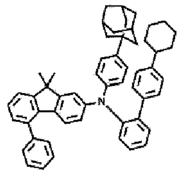
981

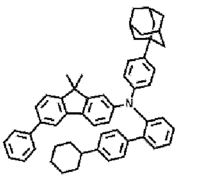
982

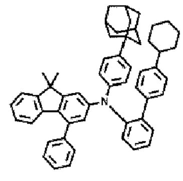
983

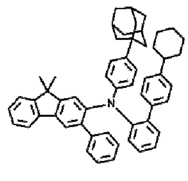
984

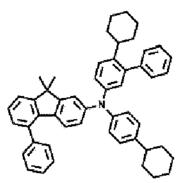
985

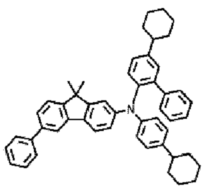
986

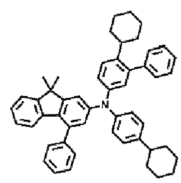
987

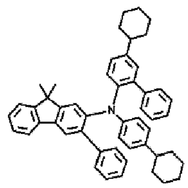
988

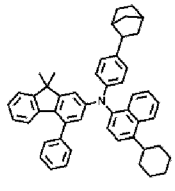
989

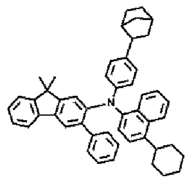
990

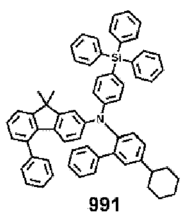
991

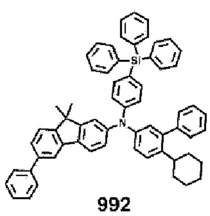
992

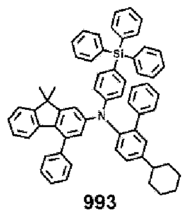
993

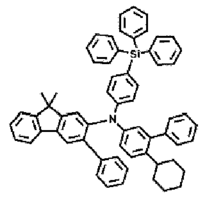
994

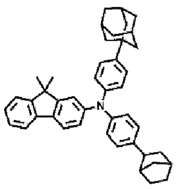
995

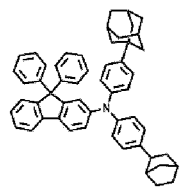
996

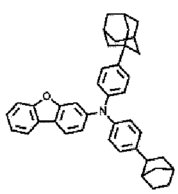
997

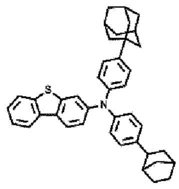
998

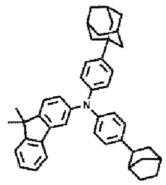
999

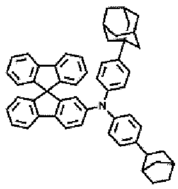
1000

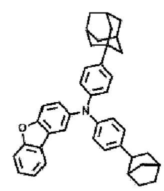
1001

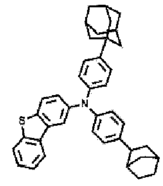
1002

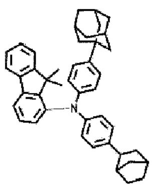
1003

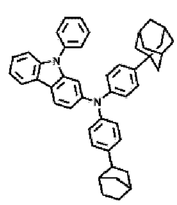
1004

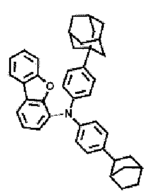
1005

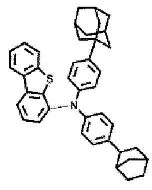
1006

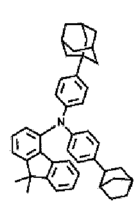
1007

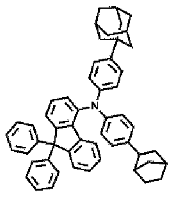
1008

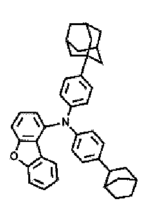
1009

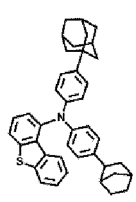
1010

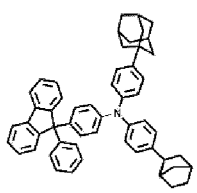
1011

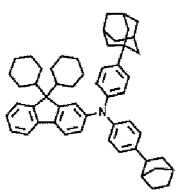
1012

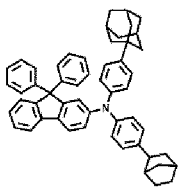
1013

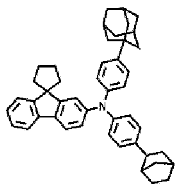
1014

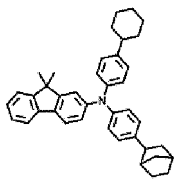

**1015**

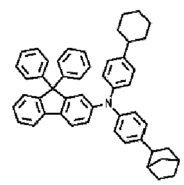

**1016**

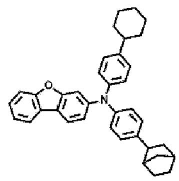

**1017**

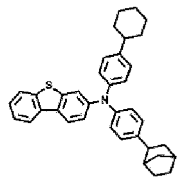

**1018**

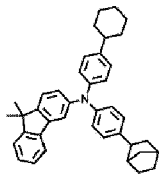

**1019**

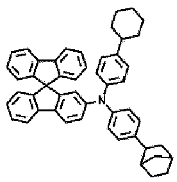

**1020**

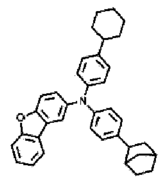

**1021**

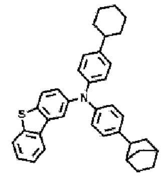

**1022**

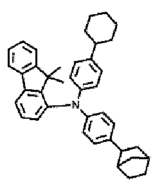

**1023**

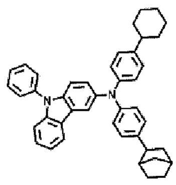

**1024**

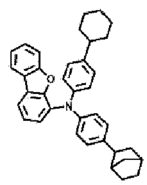

**1025**

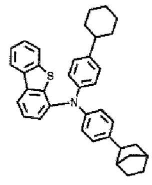

**1026**

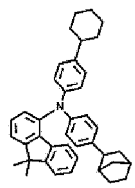

**1027**

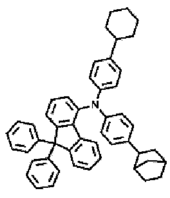

**1028**

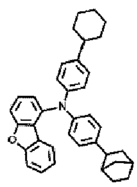

**1029**

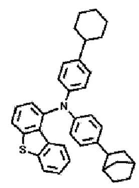

**1030**

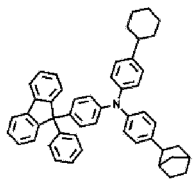

**1031**

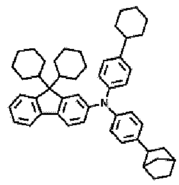

**1032**

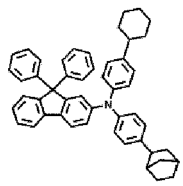

**1033**

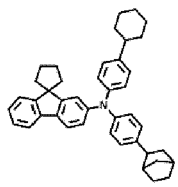

**1034**

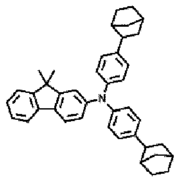
1035
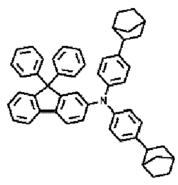
1036
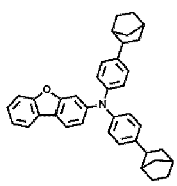
1037
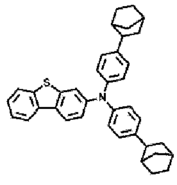
1038

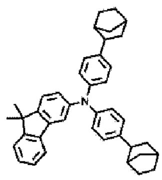
1039
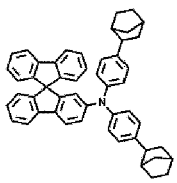
1040
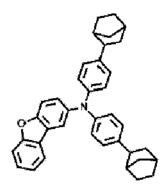
1041
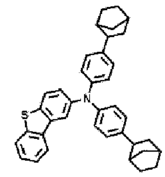
1042

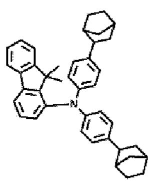
1043
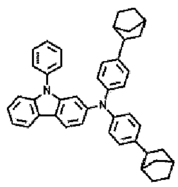
1044
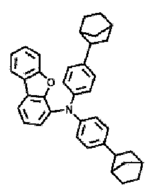
1045
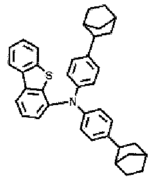
1046

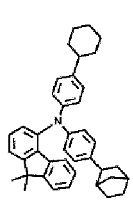
1047
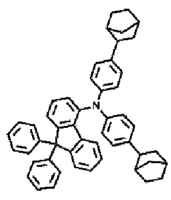
1048
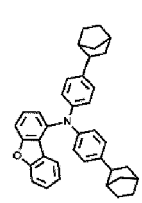
1049
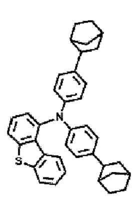
1050

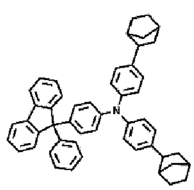
1051
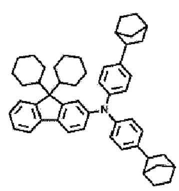
1052
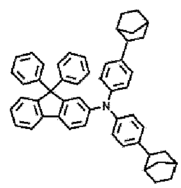
1053
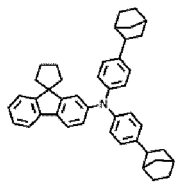
1054

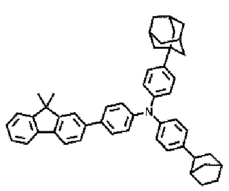
1055

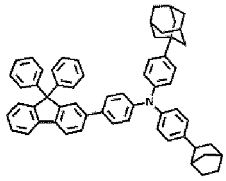
1056

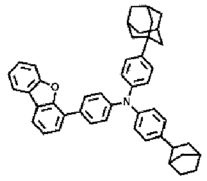
1057

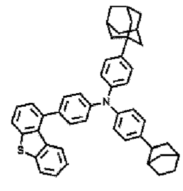
1058

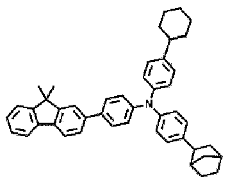
1059

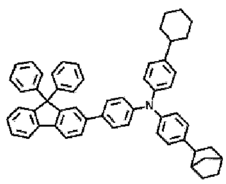
1060

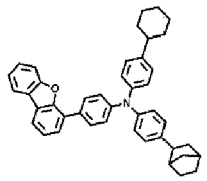
1061

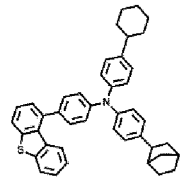
1062

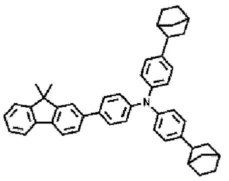
1063

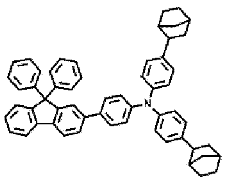
1064

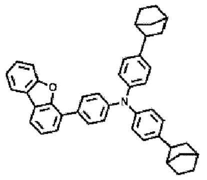
1065

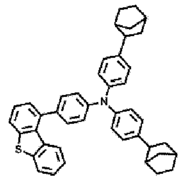
1066 .

The amine compound according to an embodiment essentially includes one of the first fused ring and has a structure essentially including a first substituent and a second substituent in a molecular structure. The first substituent and the second substituent may be each independently one substituent selected from an adamantyl group, a cyclohexyl group, a bicycloheptanyl group, a bicyclooctanyl group, and a triphenylsilyl group and may be combined with the nitrogen atom of an amine group. The amine compound according to an embodiment may include a first fused ring, a first substituent, and a second substituent, combined with an amine group. Each of the first fused ring, the first substituent, and the second substituent may be combined with the nitrogen atom of the amine group via a first to third connecting groups.

The amine compound according to an embodiment has a structure essentially including the first substituent and the second substituent selected from an adamantyl group, a cyclohexyl group, a bicycloheptanyl group, a bicyclooctanyl group, and a triphenylsilyl group, and may reduce intermolecular interaction and have low packing density. Accordingly, the amine compound of an embodiment has a low refractive index and may change the refractive index of a molecule by diversely changing the combination of the first substituent and the second substituent. In addition, the amine compound according to an embodiment includes the first substituent and the second substituent, and the deposition temperature may be reduced during forming a layer by a deposition process to improve thin layer stability and process productivity during forming the layer.

In addition, the amine compound of an embodiment may include a third substituent which is substituted at the first fused ring, or include a structure in which at least one selected from among the first substituent and the second substituent, combined with the amine group is a substituted or unsubstituted bicycloheptanyl group. For example, in the amine compound according to an embodiment, the first fused ring may be directly connected with the nitrogen atom of the amine group, and may include at least one or more third substituents as the substituent connected with the first fused ring in addition to the amine group. The third substituent may be a substituted or unsubstituted aryl group, or a substituted or unsubstituted heteroaryl group. Otherwise, the amine compound of an embodiment may essentially include a substituted or unsubstituted bicycloheptanyl group as a substituent combined with the amine group. In some embodiments, at least one selected from among the first substituent and the second substituent, combined with the amine compound of an embodiment may be a substituted or unsubstituted bicycloheptanyl group.

The amine compound according to an embodiment may diversely change the highest occupied molecular orbital (HOMO) energy level by changing the type and connecting positions of the first substituent and the second substituent, substituted at the amine group, the position of substitution of the third substituent at the first fused ring, and the number of the third substituents. Accordingly, the hole injection barrier between the first electrode EL1 and the hole transport region HTR may be changed diversely, to achieve a suitable energy level between the hole transport region HTR and the emission layer EML, so as to control to increase the excitons-production efficiency in the emission layer EML. Accordingly, if the amine compound according to an embodiment of the present disclosure is applied to the hole

transport region HTR of the light emitting device ED, a light emitting device having high efficiency, a low voltage, high luminance, and long life may be achieved.

If the amine compound of an embodiment is used in the hole transport region, external quantum efficiency may be increased by changing the refractive index change and light extraction mode between the first electrode and the second electrode. Accordingly, if the amine compound of an embodiment is used in the hole transport region, the emission efficiency of the light emitting device may be increased, and the life of the light emitting device may be improved. In addition, with excellent heat resistance and durability as described above, by including the amine compound of an embodiment as the material of the light emitting device, the life and emission efficiency of the light emitting device of an embodiment may be improved.

In some embodiments, if the light emitting device ED of an embodiment includes multiple hole transport layers HTL1, HTL2 and HTL3, each of the first hole transport layer HTL1 adjacent to the first electrode EL1 and the third hole transport layer HTL3 adjacent to the emission layer EML may include the amine compound of an embodiment, represented by Formula 1. In addition, the second hole transport layer HTL2 between the first hole transport layer HTL1 and the third hole transport layer HTL3 may include an amine derivative compound represented by Formula 10 below.

Formula 10

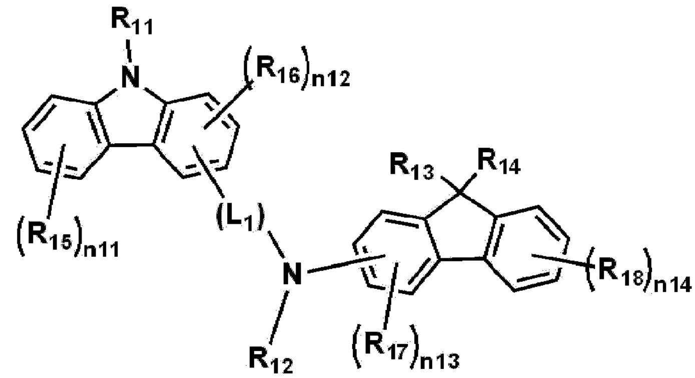

In Formula 10, $L_1$ may be a direct linkage, a substituted or unsubstituted arylene group of 6 to 30 ring-forming carbon atoms, or a substituted or unsubstituted heteroarylene group of 2 to 30 ring-forming carbon atoms.

In Formula 10, $R_{11}$ to $R_{14}$ may be each independently a substituted or unsubstituted alkyl group of 1 to 10 carbon atoms, a substituted or unsubstituted alkenyl group of 2 to 10 carbon atoms, a substituted or unsubstituted aryl group of 6 to 30 ring-forming carbon atoms, or a substituted or unsubstituted heteroaryl group of 2 to 30 ring-forming carbon atoms. Otherwise, each of $R_{11}$ to $R_{14}$ may be combined with an adjacent group to form a ring. For example, $R_{11}$ to $R_{14}$ may be each independently a substituted or unsubstituted methyl group, or a substituted or unsubstituted phenyl group.

In Formula 10, $R_{15}$ to $R_{18}$ may be each independently a hydrogen atom, a deuterium atom, a halogen atom, a substituted or unsubstituted alkyl group of 1 to 20 carbon atoms, a substituted or unsubstituted aryl group of 6 to 30 ring-forming carbon atoms, a substituted or unsubstituted heteroaryl group of 2 to 30 ring-forming carbon atoms. Otherwise, $R_{15}$ to $R_{18}$ may be combined with adjacent groups to form rings. For example, $R_{15}$ to $R_{18}$ may be each independently a hydrogen atom.

In Formula 10, n11 and n14 are each independently an integer of 0 to 4, and n12 and n13 are each independently an integer of 0 to 3.

If n11 and n14 are 0, the amine compound of an embodiment may be unsubstituted with $R_{15}$ and $R_{18}$, respectively. The case where n11 and n14 are 4, and $R_{15}$ and $R_{18}$ are all hydrogen atoms in Formula 10, may be the same as the case where n11 and n14 are 0 in Formula 10. If n11 and n14 are integers of 2 or more, each of multiple $R_{15}$ and $R_{18}$ may be the same, or at least one of multiple $R_{15}$ and $R_{18}$ may be different.

If n12 and n13 are 0, the amine compound of an embodiment may be unsubstituted with $R_{16}$ and $R_{17}$, respectively. The case where n12 and n13 are 3, and $R_{16}$ and $R_{17}$ are all hydrogen atoms in Formula 10, may be the same as the case where n12 and n13 are 0 in Formula 10. If n12 and n13 are integers of 2 or more, each of multiple $R_{16}$ and $R_{17}$ may be the same, or at least one of multiple $R_{16}$ and $R_{17}$ may be different.

In the amine derivative represented by Formula 10, $R_{12}$ may be an aryl group or a heteroaryl group. For example, $R_{12}$ may be a substituted or unsubstituted phenyl group, a substituted or unsubstituted naphthyl group, a substituted or unsubstituted biphenyl group, a substituted or unsubstituted dibenzofuran group, or a substituted or unsubstituted dibenzothiophene group.

In an embodiment, the amine derivative compound represented by Formula 10 may be represented by Formula

11-1 or Formula 11-2 below.

Formula 11-1

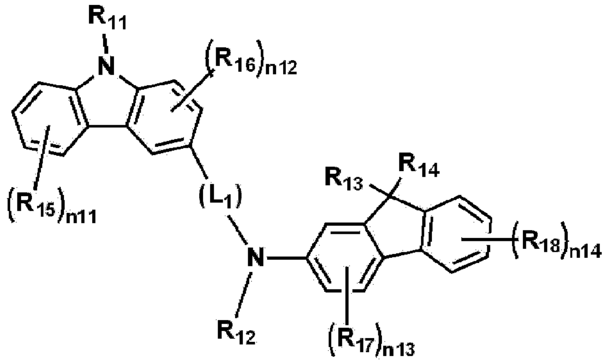

Formula 11-2

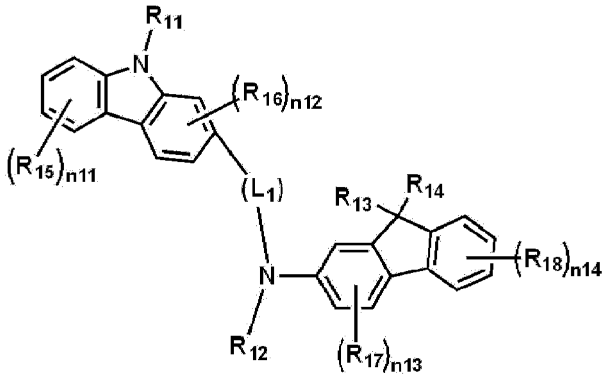

Formula 11-1 and Formula 11-2 represent cases of Formula 10 where the position connected of a carbazole moiety and $L_1$ is specified. Formula 11-1 corresponds to a case of Formula 10 where the carbon position 3 of the carbazole moiety is the position connected with $L_1$. Formula 11-2 corresponds to a case of Formula 10 where the carbon position 2 of the carbazole moiety is the position connected with $L_1$.

In Formula 11-1 and Formula 11-2, the same description of $L_1$, $R_{11}$ to $R_{14}$, $R_{15}$ to $R_{18}$, and n11 to n14 referring to Formula 10 may be applied.

The amine derivative compound represented by Formula 10 may be represented by any one selected from among the compounds represented in Compound Group 2 below. For example, the second hole transport layer HTL2 may include at least one selected from among the compounds represented in Compound Group 2 below.

Compound Group 2

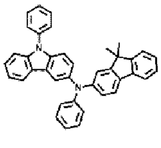
A1

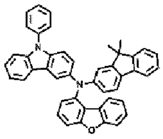
A2

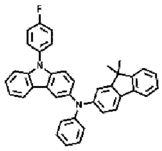
A3

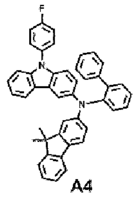
A4

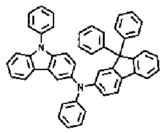
A5

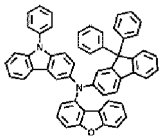
A6

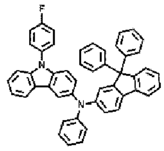
A7

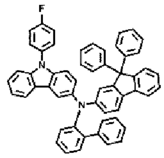
A8

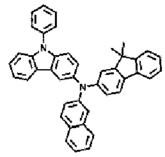
A9

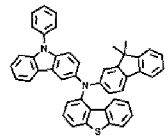
A10

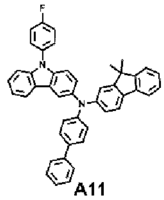
A11

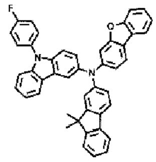
A12

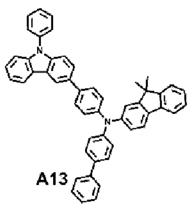
A13

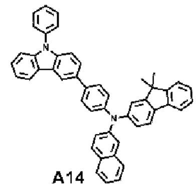
A14

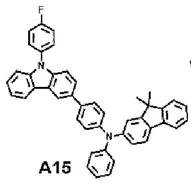
A15

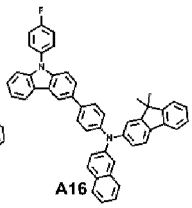
A16

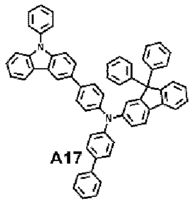
A17

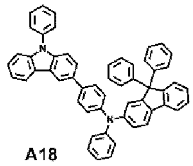
A18

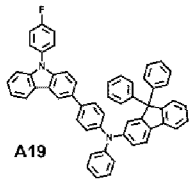
A19

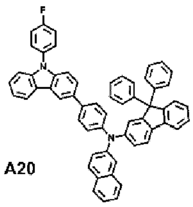
A20

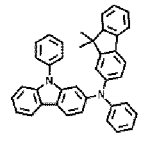
A21

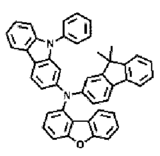
A22

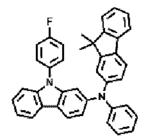
A23

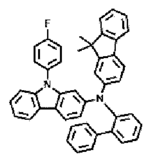
A24

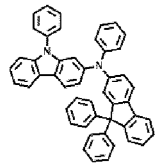
A25

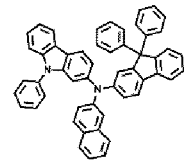
A26

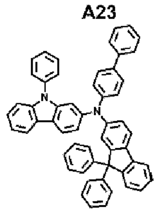
A27

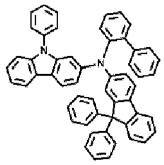
A28

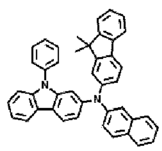
A29

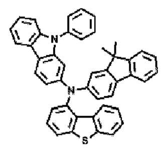
A30

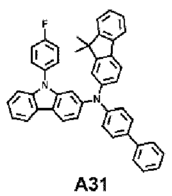
A31

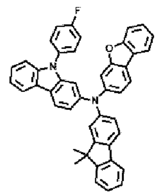
A32

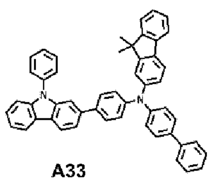
A33

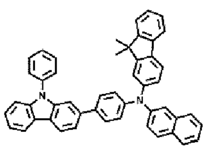
A34

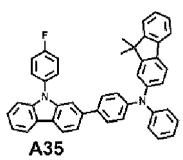
A35

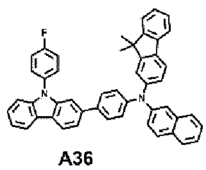
A36

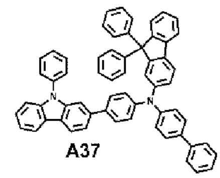
A37

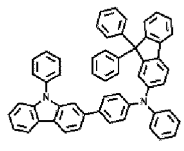
A38

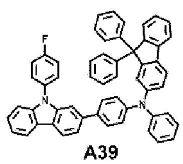
A39

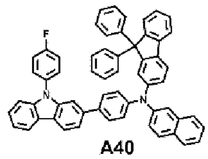
A40

.

In addition, the light emitting device ED of an embodiment may further include a material of a hole transport region further described below in the hole transport region HTR in addition to the amine compound of an embodiment and the amine derivative compound represented by Formula 10, described above.

The hole transport region HTR may include a compound represented by Formula H-1 below.

Formula H-1

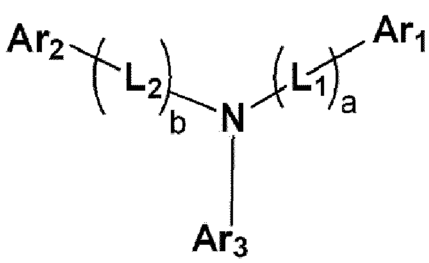

In Formula H-1 above, $L_1$ and $L_2$ may be each independently a direct linkage, a substituted or unsubstituted arylene group of 6 to 30 ring-forming carbon atoms, or a substituted or unsubstituted heteroarylene group of 2 to 30

ring-forming carbon atoms. "a" and "b" may be each independently an integer of 0 to 10. In some embodiments, if "a" or "b" is an integer of 2 or more, multiple $L_1$ and $L_2$ may be each independently a substituted or unsubstituted arylene group of 6 to 30 ring-forming carbon atoms, or a substituted or unsubstituted heteroarylene group of 2 to 30 ring-forming carbon atoms.

In Formula H-1, $Ar_1$ and $Ar_2$ may be each independently a substituted or unsubstituted aryl group of 6 to 30 ring-forming carbon atoms, or a substituted or unsubstituted heteroaryl group of 2 to 30 ring-forming carbon atoms. In addition, in Formula H-1, $Ar_3$ may be a substituted or unsubstituted aryl group of 6 to 30 ring-forming carbon atoms.

The compound represented by Formula H-1 may be a monoamine compound. Otherwise, the compound represented by Formula H-1 may be a diamine compound in which at least one selected from among $Ar_1$ to $Ar_3$ includes an amine group as a substituent. In addition, the compound represented by Formula H-1 may be a carbazole-based compound in which at least one selected from among $Ar_1$ and $Ar_2$ includes a substituted or unsubstituted carbazole group, or a fluorene-based compound in which at least one selected from among $Ar_1$ and $Ar_2$ includes a substituted or unsubstituted fluorene group.

The compound represented by Formula H-1 may be represented by any one selected from among the compounds in Compound Group H below. However, the compounds shown in Compound Group H are only examples, and the compound represented by Formula H-1 is not limited to the compounds represented in Compound Group H below.

Compound Group H

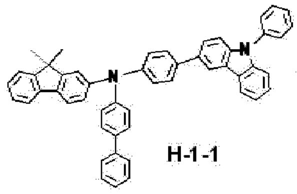
H-1-1

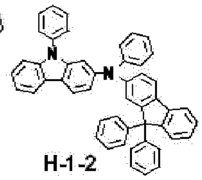
H-1-2

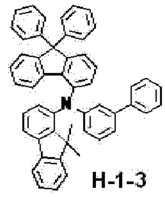
H-1-3

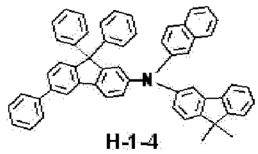
H-1-4

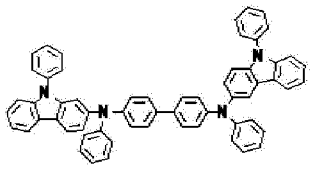
H-1-5

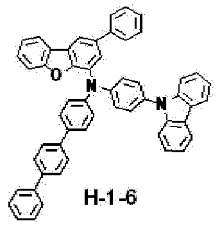
H-1-6

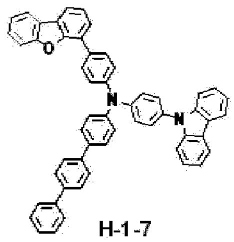
H-1-7

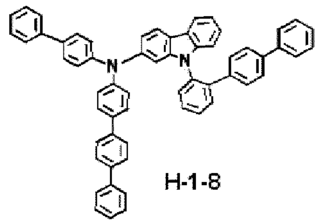
H-1-8

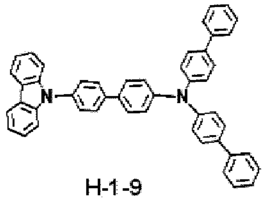
H-1-9

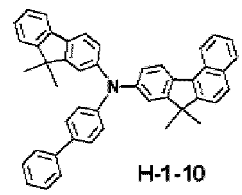
H-1-10

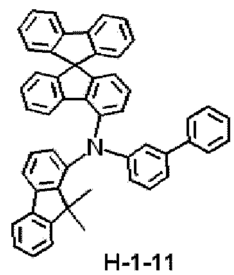
H-1-11

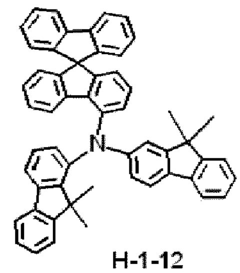
H-1-12

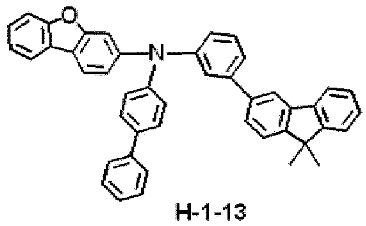
H-1-13

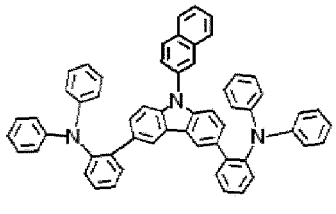
H-1-14

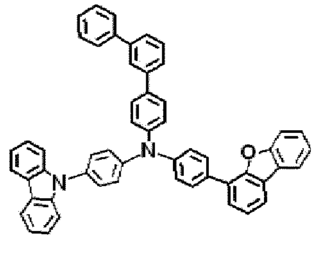
H-1-15

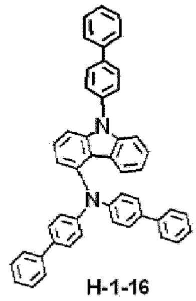
H-1-16

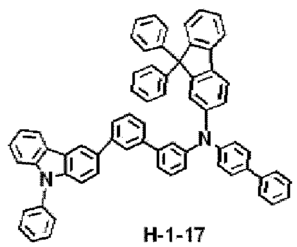
H-1-17

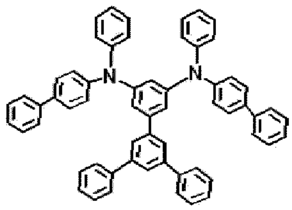
H-1-18

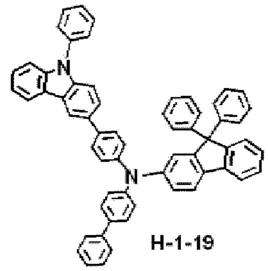
H-1-19

The hole transport region HTR may include a phthalocyanine compound such as copper phthalocyanine, $N^1,N^{1'}$-([1,1'-biphenyl]-4,4'-diyl)bis($N^1$-phenyl-$N^4,N^4$-di-m-tolylbenzene-1,4-diamine) (DNTPD), 4,4',4"-[tris(3-methylphenyl)phenylamino] triphenylamine (m-MTDATA), 4,4',4"-tris(N,N-diphenylamino)triphenylamine (TDATA), 4,4',4"-tris[N(2-naphthyl)-N-phenylamino]-triphenylamine (2-TNATA), poly(3,4-ethylenedioxythiophene)/poly(4-styrenesulfonate) (PEDOT/PSS), polyaniline/dodecylbenzenesulfonic acid (PANI/DBSA), polyaniline/camphor sulfonic acid (PANI/CSA), polyaniline/poly(4-styrenesulfonate) (PANI/PSS), N,N'-di(1-naphthalene-1-yl)-N,N'-diphenyl-benzidine (NPB), triphenylamine-containing polyetherketone (TPAPEK), 4-isopropyl-4'-methyldiphenyliodonium [tetrakis(pentafluorophenyl)borate], and dipyrazino[2,3-f:2',3'-h] quinoxaline-2,3,6,7,10,11-hexacarbonitrile (HAT-CN).

The hole transport region HTR may include carbazole derivatives such as N-phenyl carbazole and polyvinyl carbazole, fluorene-based derivatives, N,N'-bis(3-methylphenyl)-N,N'-diphenyl-[1,1'-biphenyl]-4,4'-diamine (TPD), triphenylamine-based derivatives such as 4,4',4"-tris(N-carbazolyl)triphenylamine (TCTA), N,N'-di(1-naphthalene-1-yl)-N,N'-diphenyl-benzidine (NPB), 4,4'-cyclohexylidene bis[N,N-bis(4-methylphenyl)benzeneamine] (TAPC), 4,4'-bis[N,N'-(3-tolyl)amino]-3,3'-dimethylbiphenyl (HMTPD), 1,3-bis(N-carbazolyl)benzene (mCP), etc.

In addition, the hole transport region HTR may include 9-(4-tert-butylphenyl)-3,6-bis(triphenylsilyl)-9H-carbazole (CzSi), 9-phenyl-9H-3,9'-bicarbazole (CCP), 1,3-bis(1,8-dimethyl-9H-carbazol-9-yl)benzene (mDCP), etc.

The hole transport region HTR may include the compounds of the hole transport region in at least one selected from among the hole injection layer HIL, hole transport layer HTL, and electron blocking layer EBL.

The thickness of the hole transport region HTR may be from about 100 Å to about 10,000 Å, for example, from about 100 Å to about 5,000 Å. If the hole transport region HTR includes a hole injection layer HIL, the thickness of the hole injection region HIL may be, for example, from about 30 Å to about 1,000 Å. If the hole transport region HTR includes a hole transport layer HTL, the thickness of the hole transport layer HTL may be from about 30 Å to about 1,000 Å. For example, if the hole transport region HTR includes an electron blocking layer, the thickness of the electron blocking layer EBL may be from about 10 Å to about 1,000 Å. If the thicknesses of the hole transport region HTR, the hole injection layer HIL, the hole transport layer HTL and the electron blocking layer EBL satisfy the above-described ranges, suitable or satisfactory hole transport properties may be achieved without substantial increase of a driving voltage.

The hole transport region HTR may be formed by various suitable methods such as a vacuum deposition method, a spin coating method, a cast method, a Langmuir-Blodgett (LB) method, an ink jet printing method, a laser printing method, and/or a laser induced thermal imaging (LITI) method.

The hole transport region HTR may further include a charge generating material to increase conductivity (e.g., electrical conductivity) in addition to the above-described materials. The charge generating material may be dispersed uniformly or non-uniformly in the hole transport region HTR. The charge generating material may be, for example, a p-dopant. The p-dopant may include at least one selected from metal halide compounds, quinone derivatives, metal oxides, and cyano group-containing compounds, without limitation. For example, the p-dopant may include metal halide compounds such as CuI and/or RbI, quinone derivatives such as tetracyanoquinodimethane (TCNQ) and/or 2,3,5,6-tetrafluoro-7,7',8,8-tetracyanoquinodimethane (F4-TCNQ), metal oxides such as tungsten oxide and/or molybdenum oxide, cyano group-containing compounds such as dipyrazino[2,3-f: 2',3'-h] quinoxaline-2,3,6,7,10,11-hexacarbonitrile (HATCN) and/or 4-[[2,3-bis[cyano-(4-cyano-2,3,5,6-tetrafluorophenyl)methylidene]cyclopropylidene]-cyanomethyl]-2,3,5,6-tetrafluorobenzonitrile (NDP9), etc., without limitation.

As described above, the hole transport region HTR may further include at least one selected from among a buffer layer and an electron blocking layer EBL in addition to the hole injection layer HIL and the hole transport layer HTL. The buffer layer may compensate resonance distance according to the wavelength of light emitted from an emission layer EML and may increase emission efficiency. As materials included in the buffer layer, materials which may be included in the hole transport region HTR may be used. The electron blocking layer EBL is a layer playing the role of blocking or reducing the injection of electrons from the electron transport region ETR to the hole transport region HTR.

The emission layer EML is provided on the hole transport region HTR. The emission layer EML may have a thickness of, for example, about 100 Å to about 1,000 Å or about 100 Å to about 300 Å. The emission layer EML may have a single layer formed using a single material, a single layer formed using multiple different materials, or a multilayer structure having multiple layers formed using multiple different materials.

In the light emitting device ED of an embodiment, the emission layer EML may include anthracene derivatives, pyrene derivatives, fluoranthene derivatives, chrysene derivatives, dihydrobenzanthracene derivatives, and/or triphenylene derivatives. For example, the emission layer EML may include anthracene derivatives and/or pyrene derivatives.

In the light emitting devices ED of embodiments, shown in FIG. 3 to FIG. 8, the emission layer EML may include a host and a dopant, and the emission layer EML may include a compound represented by Formula E-1 below. The compound represented by Formula E-1 below may be used as a fluorescence host material.

Formula E-1

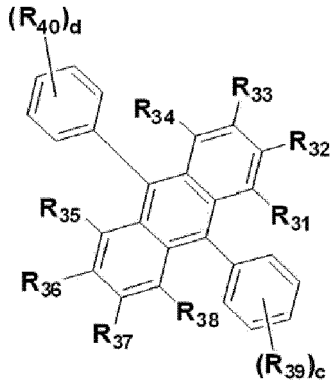

In Formula E-1, $R_{31}$ to $R_{40}$ may be each independently a hydrogen atom, a deuterium atom, a halogen atom, a substituted or unsubstituted silyl group, a substituted or unsubstituted thio group, a substituted or unsubstituted oxy group, a substituted or unsubstituted alkyl group of 1 to 10 carbon atoms, a substituted or unsubstituted alkenyl group of 2 to 10 carbon atoms, a substituted or unsubstituted aryl group of 6 to 30 ring-forming carbon atoms, or a substituted or unsubstituted heteroaryl group of 2 to 30 ring-forming carbon atoms, or may be combined with an adjacent group to form a ring. In some embodiments, $R_{31}$ to $R_{40}$ may be combined with adjacent groups to form saturated hydrocarbon rings, unsaturated hydrocarbon rings, saturated heterocycles, or unsaturated heterocycles.

In Formula E-1, "c" and "d" may be each independently an integer of 0 to 5.

Formula E-1 may be represented by any one selected from among Compound E1 to Compound E19 below.

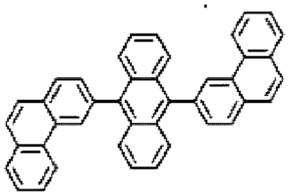

E1

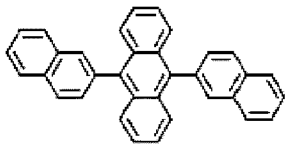

E2

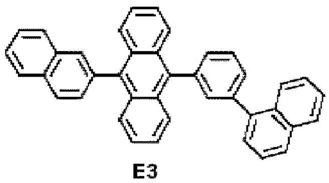

E3

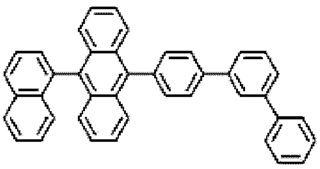

E4

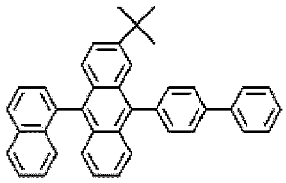

E5

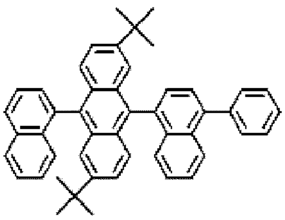

E6

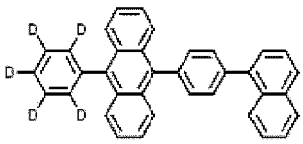

E7

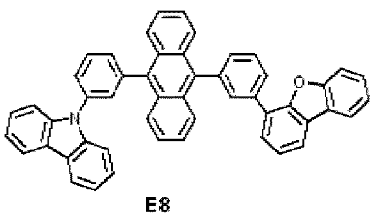

E8

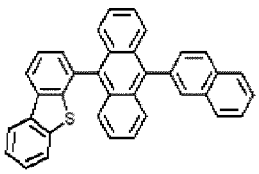

E9

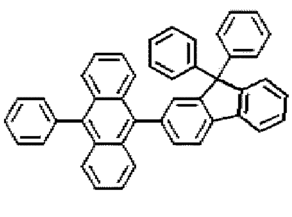

E10

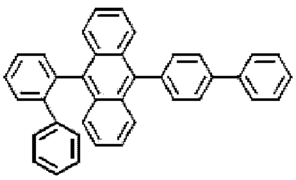

E11

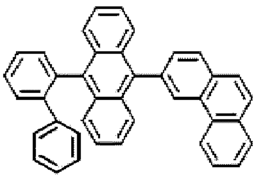

E12

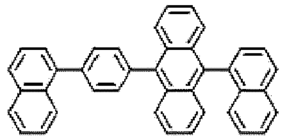

E13

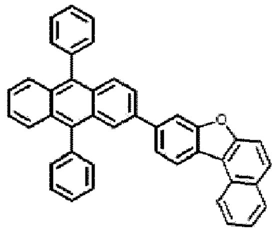

E14

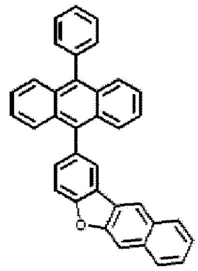

E15

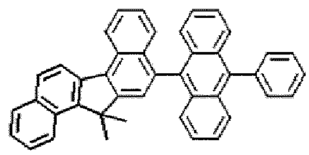

E16

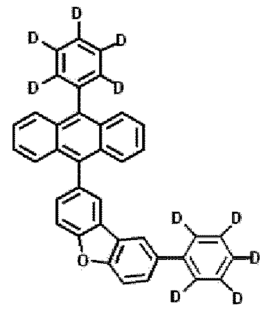

E17

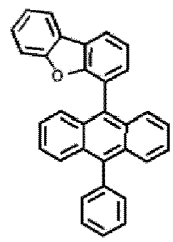

E18

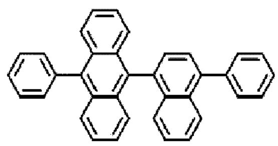

E19

In an embodiment, the emission layer EML may include a compound represented by Formula E-2a or Formula E-2b below. The compound represented by Formula E-2a or Formula E-2b below may be used as a phosphorescence host material.

Formula E-2a

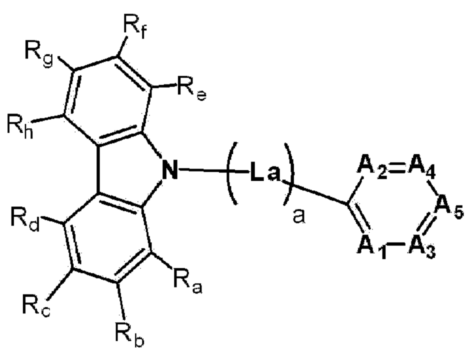

In Formula E-2a, "a" may be an integer of 0 to 10, La may be a direct linkage, a substituted or unsubstituted arylene group of 6 to 30 ring-forming carbon atoms, or a substituted or unsubstituted heteroarylene group of 2 to 30 ring-forming carbon atoms. In some embodiments, if "a" is an integer of 2 or more, multiple La may be each independently a substituted or unsubstituted arylene group of 6 to 30 ring-forming carbon atoms, or a substituted or unsubstituted heteroarylene group of 2 to 30 ring-forming carbon atoms.

In addition, in Formula E-2a, $A_1$ to $A_5$ may be each independently N or CRi. $R_a$ to $R_i$ may be each independently a hydrogen atom, a deuterium atom, a substituted or unsubstituted amine group, a substituted or unsubstituted thio group, a substituted or unsubstituted oxy group, a substituted or unsubstituted alkyl group of 1 to 20 carbon atoms, a substituted or unsubstituted alkenyl group of 2 to 20 carbon atoms, a substituted or unsubstituted aryl group of 6 to 30 ring-forming carbon atoms, or a substituted or unsubstituted heteroaryl group of 2 to 30 ring-forming carbon atoms, or may be combined with an adjacent group to form a ring. $R_a$ to $R_i$ may be combined with an adjacent group to form a hydrocarbon ring or a heterocycle including N, O, S, etc. as a ring-forming atom.

In some embodiments, in Formula E-2a, two or three selected from $A_1$ to $A_5$ may be N, and the remainder may be $CR_i$.

Formula E-2b

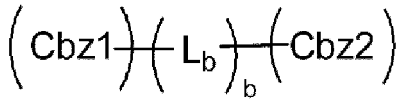

In Formula E-2b, Cbz1 and Cbz2 may be each independently an unsubstituted carbazole group, or a carbazole group substituted with an aryl group of 6 to 30 ring-forming carbon atoms. $L_b$ may be a direct linkage, a substituted or unsubstituted arylene group of 6 to 30 ring-forming carbon atoms, or a substituted or unsubstituted heteroarylene group of 2 to 30 ring-forming carbon atoms. "b" is an integer of 0 to 10, and if "b" is an integer of 2 or more, multiple $L_b$ may be each independently a substituted or unsubstituted arylene group of 6 to 30 ring-forming carbon atoms, or a substituted or unsubstituted heteroarylene group of 2 to 30 ring-forming carbon atoms.

The compound represented by Formula E-2a or Formula E-2b may be represented by any one selected from among the compounds in Compound Group E-2 below. However, the compounds shown in Compound Group E-2 below are only examples, and the compound represented by Formula E-2a or Formula E-2b is not limited to the compounds represented in Compound Group E-2 below.

Compound Group E-2

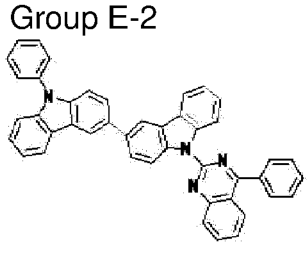
E-2-1

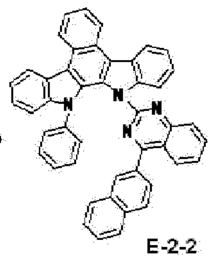
E-2-2

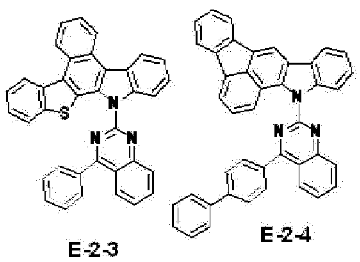
E-2-3 E-2-4

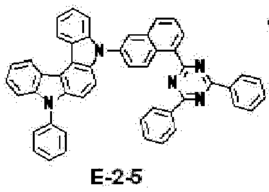
E-2-5

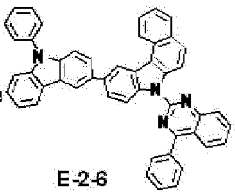
E-2-6

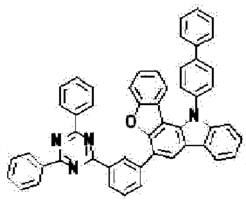
E-2-7

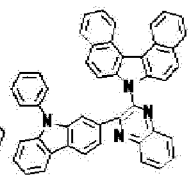
E-2-8

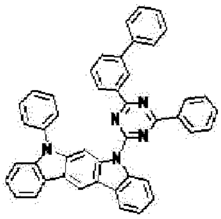
E-2-9

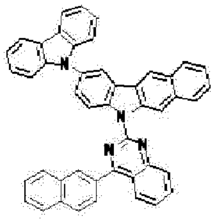
E-2-10

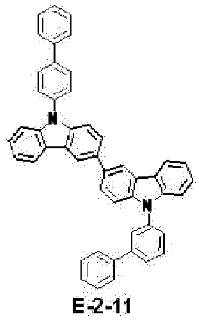
E-2-11

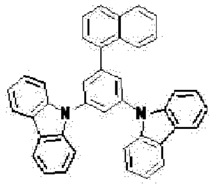
E-2-12

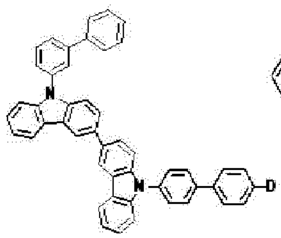
E-2-13

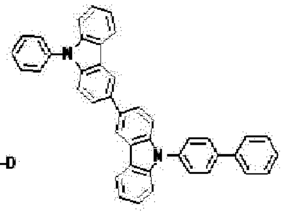
E-2-14

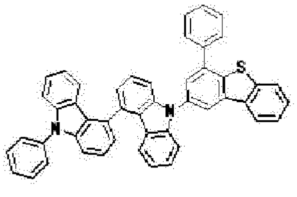
E-2-15

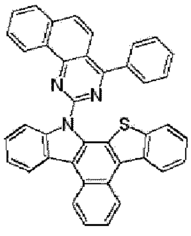
E-2-16

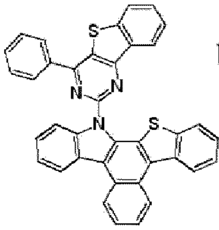
E-2-17

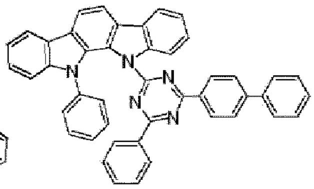
E-2-18

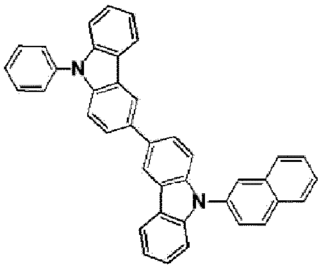
E-2-19

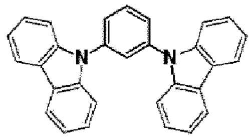
E-2-20

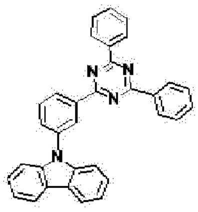
E-2-21

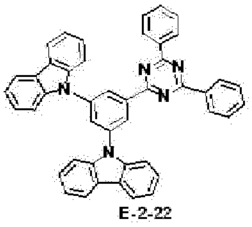
E-2-22

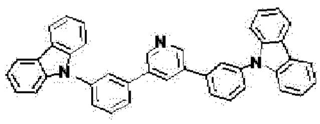
E-2-23

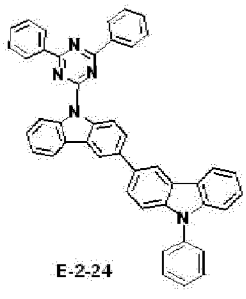
E-2-24

The emission layer EML may further include any suitable material generally used in the art as a host material. For example, the emission layer EML may include as a host material, at least one of bis (4-(9H-carbazol-9-yl) phenyl) diphenylsilane (BCPDS), (4-(1-(4-(diphenylamino) phenyl) cyclohexyl) phenyl) diphenyl-phosphine oxide (POPCPA), bis[2-(diphenylphosphino)phenyl] ether oxide (DPEPO), 4,4'-bis(carbazol-9-yl)biphenyl (CBP), 1,3-bis(carbazol-9-yl)benzene (mCP), 2,8-bis(diphenylphosphoryl)dibenzo[b,d]furan (PPF), 4,4',4"-tris(carbazol-9-yl)-triphenylamine (TCTA), or 1,3,5-tris(1-phenyl-1H-benzo[d]imidazole-2-yl)benzene (TPBi). However, an embodiment of the present disclosure is not limited thereto. For example, tris(8-hydroxyquinolino)aluminum ($Alq_3$), 9,10-di(naphthalene-2-yl)anthracene (ADN), 2-tert-butyl-9,10-di(naphth-2-yl)anthracene (TBADN), distyrylarylene (DSA), 4,4'-bis(9-carbazolyl)-2,2'-dimethyl-biphenyl (CDBP), 2-methyl-9,10-bis(naphthalen-2-yl)anthracene (MADN), hexaphenyl cyclotriphosphazene (CP1), 1,4-bis(triphenylsilyl)benzene (UGH2), hexaphenylcyclotrisiloxane ($DPSiO_3$), octaphenylcyclotetra siloxane ($DPSiO_4$), etc. may be used as the host material.

The emission layer EML may include a compound represented by Formula M-a or Formula M-b below. The compound represented by Formula M-a or Formula M-b may be used as a phosphorescence dopant material.

Formula M-a

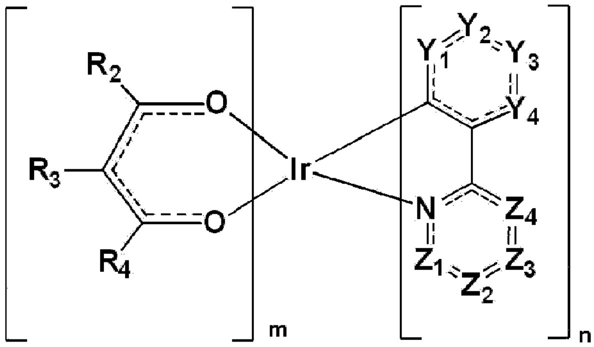

In Formula M-a, Yi to $Y_4$, and $Z_1$ to $Z_4$ may be each independently $CR_1$ or N, and $R_1$ to $R_4$ may be each independently a hydrogen atom, a deuterium atom, a substituted or unsubstituted amine group, a substituted or unsubstituted thio group, a substituted or unsubstituted oxy group, a substituted or unsubstituted alkyl group of 1 to 20 carbon atoms, a substituted or unsubstituted alkenyl group of 2 to 20 carbon atoms, a substituted or unsubstituted aryl group of 6 to 30 ring-forming carbon atoms, or a substituted or unsubstituted heteroaryl group of 2 to 30 ring-forming carbon atoms, or may be combined with an adjacent group to form a ring. In Formula M-a, "m" is 0 or 1, and "n" is 2 or 3. In Formula M-a, if "m" is 0, "n" is 3, and if "m" is 1, "n" is 2.

The compound represented by Formula M-a may be used as a phosphorescence dopant.

The compound represented by Formula M-a may be represented by any one selected from among Compounds M-a1 to M-a25 below. However, Compounds M-a1 to M-a25 below are examples, and the compound represented by Formula M-a is not limited to the compounds represented by Compounds M-a1 to M-a25 below.

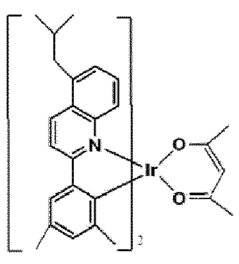

M-a1

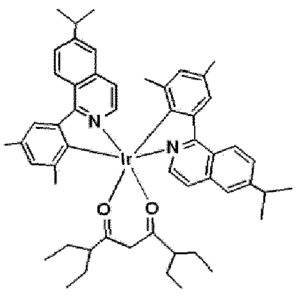

M-a2

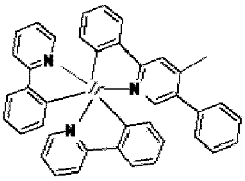
M-a3

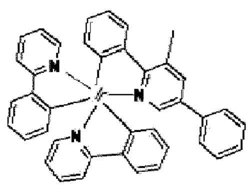
M-a4

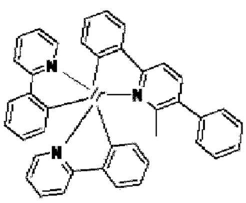
M-a5

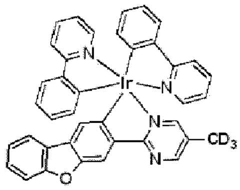
M-a6

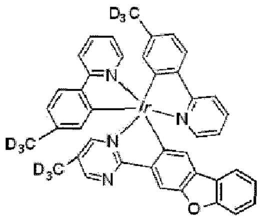
M-a7

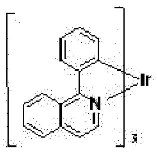
M-a8

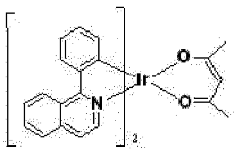
M-a9

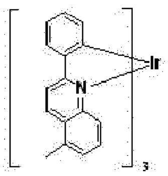
M-a10

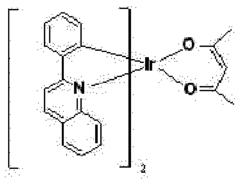
M-a11

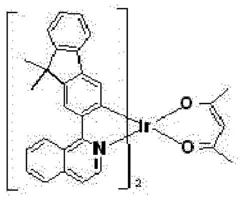
M-a12

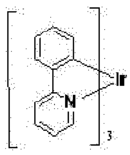
M-a13

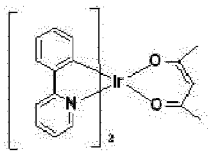
M-a14

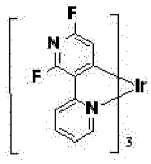
M-a15

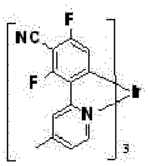
M-a16

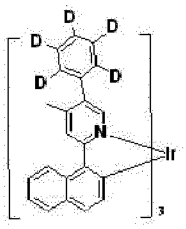
M-a17

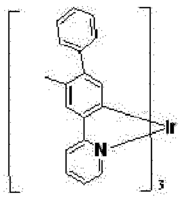
M-a18

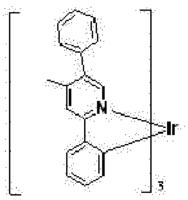
M-a19

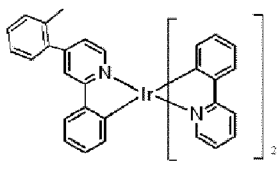
M-a20

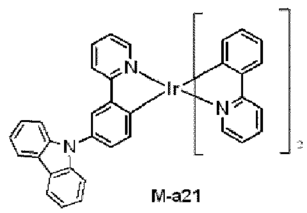
M-a21

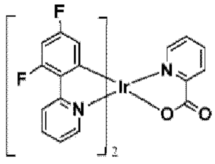

M-a22

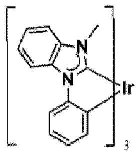

M-a23

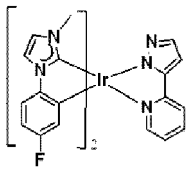

M-a24

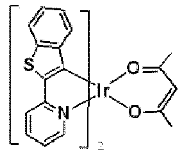

M-a25

Formula M-b

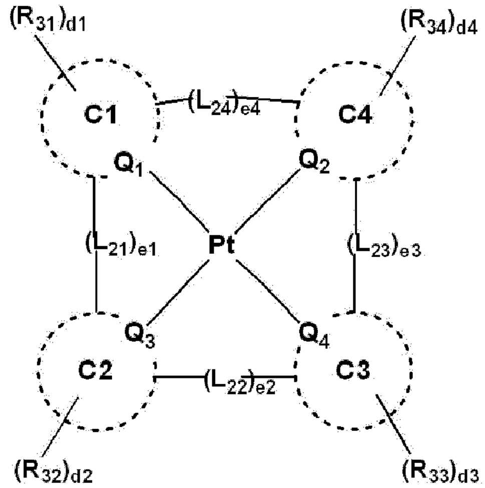

In Formula M-b, $Q_1$ to $Q_4$ are each independently C or N, C1 to C4 are each independently a substituted or unsubstituted hydrocarbon ring of 5 to 30 ring-forming carbon atoms, or a substituted or unsubstituted heterocycle of 2 to 30 ring-forming carbon atoms. $L_{21}$ to $L_{24}$ are each independently a direct linkage,

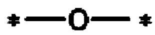,

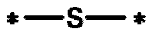, 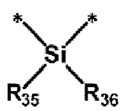, 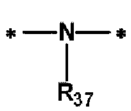, 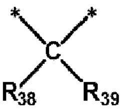, 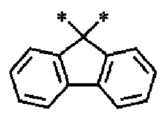,

a substituted or unsubstituted divalent alkyl group of 1 to 20 carbon atoms, a substituted or unsubstituted arylene group of 6 to 30 ring-forming carbon atoms, or a substituted or unsubstituted heteroarylene group of 2 to 30 ring-forming carbon atoms, and e1 to e4 are each independently 0 or 1. $R_{31}$ to $R_{39}$ are each independently a hydrogen atom, a deuterium atom, a halogen atom, a cyano group, a substituted or unsubstituted amine group, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms, or combined with an adjacent group to form a ring, and d1 to d4 are each independently an integer of 0 to 4.

The compound represented by Formula M-b may be used as a blue phosphorescence dopant or a green phosphorescence dopant.

The compound represented by Formula M-b may be represented by any one selected from among the compounds below. However, the compounds below are examples, and the compound represented by Formula M-b is not limited to the compounds represented below.

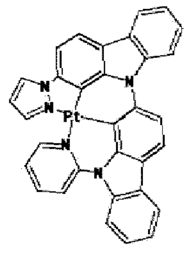

M-b-1

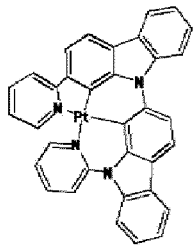

M-b-2

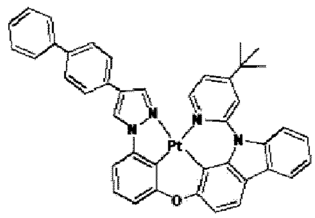

M-b-3

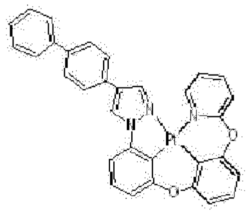

M-b-4

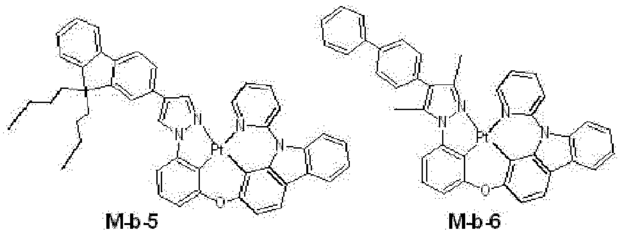

M-b-5 M-b-6

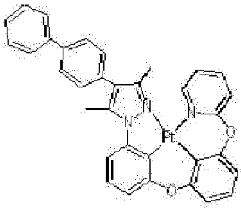

M-b-7

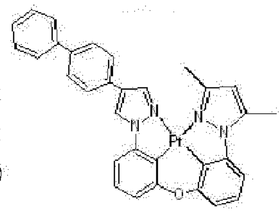

M-b-8

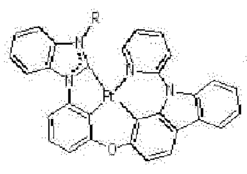

M-b-9

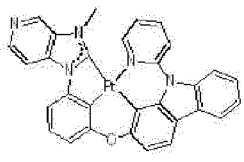

M-b-10

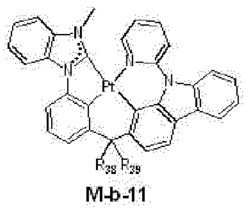

M-b-11

In the compounds above, R, $R_{38}$, and $R_{39}$ may be each independently a hydrogen atom, a deuterium atom, a halogen atom, a cyano group, a substituted or unsubstituted amine group, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms.

The emission layer EML may include any one selected from among Formula F-a to Formula F-c below. The compounds represented by Formula F-a to Formula F-c below may be used as fluorescence dopant materials.

Formula F-a

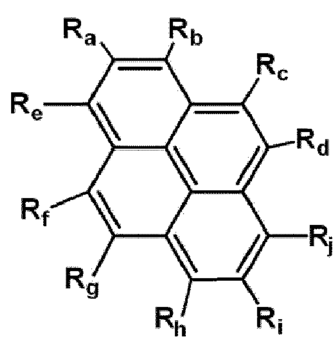

In Formula F-a, two selected from $R_a$ to $R_j$ may be each independently substituted with $*$-$NAr_1Ar_2$. The remainder not substituted with $*$-$NAr_1Ar_2$ selected from among $R_a$ to $R_j$ may be each independently a hydrogen atom, a deuterium atom, a halogen atom, a cyano group, a substituted or unsubstituted amine group, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms.

In $*$-$NAr_1Ar_2$, $Ar_1$ and $Ar_2$ may be each independently a substituted or unsubstituted aryl group of 6 to 30 ring-forming carbon atoms, or a substituted or unsubstituted heteroaryl group of 2 to 30 ring-forming carbon atoms. For example, at least one selected from among $Ar_1$ and $Ar_2$ may be a heteroaryl group including O or S as a ring-forming atom.

Formula F-b

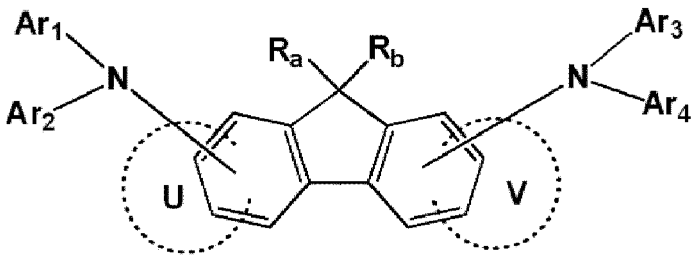

In Formula F-b, $R_a$ and $R_b$ may be each independently a hydrogen atom, a deuterium atom, a substituted or unsubstituted alkyl group of 1 to 20 carbon atoms, a substituted or unsubstituted alkenyl group of 2 to 20 carbon atoms, a substituted or unsubstituted aryl group of 6 to 30 ring-forming carbon atoms, or a substituted or unsubstituted heteroaryl group of 2 to 30 ring-forming carbon atoms, or may be combined with an adjacent group to form a ring. $Ar_1$ to $Ar_4$ may be each independently a substituted or unsubstituted aryl group of 6 to 30 ring-forming carbon atoms, or a substituted or unsubstituted heteroaryl group of 2 to 30 ring-forming carbon atoms.

In Formula F-b, U and V may be each independently a substituted or unsubstituted hydrocarbon ring of 5 to 30 ring-forming carbon atoms, or a substituted or unsubstituted heterocycle of 2 to 30 ring-forming carbon atoms.

In Formula F-b, the number of rings represented by U and V may be each independently 0 or 1. For example, in Formula F-b, if the number of U or V is 1, one ring forms a fused ring at the designated part by U or V, and if the number of U or V is 0, a ring is not present at the designated part by U or V. In some embodiments, if the number of U is 0, and the number of V is 1, or if the number of U is 1, and the number of V is 0, a fused ring having the fluorene core of Formula F-b may be a ring compound having four rings. In addition, if the number of both U and V is 0, the fused ring of Formula F-b may be a ring compound having three rings. In addition, if the number of both U and V is 1, a fused ring having the fluorene core of Formula F-b may be a ring compound having five rings.

Formula F-c

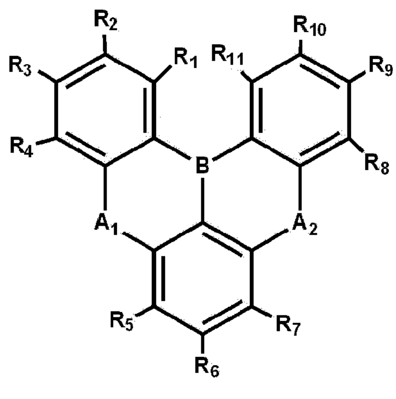

In Formula F-c, $A_1$ and $A_2$ may be each independently O, S, Se, or $NR_m$, and $R_m$ may be a hydrogen atom, a deuterium atom, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms. $R_1$ to $R_{11}$ are each independently a hydrogen atom, a deuterium atom, a halogen atom, a cyano group, a substituted or unsubstituted amine group, a substituted or unsubstituted boryl group, a substituted or unsubstituted oxy group, a substituted or unsubstituted thio group, a substituted or unsubstituted alkyl group of 1 to 20 carbon atoms, a substituted or unsubstituted aryl group of 6 to 30 ring-forming carbon atoms, or a substituted or unsubstituted heteroaryl

group of 2 to 30 ring-forming carbon atoms, or combined with an adjacent group to form a ring.

In Formula F-c, $A_1$ and $A_2$ may be each independently combined with the substituents of an adjacent ring to form a fused ring. For example, if $A_1$ and $A_2$ may be each independently $NR_m$, $A_1$ may be combined with $R_4$ or $R_5$ to form a ring. In addition, $A_2$ may be combined with $R_7$ or $R_8$ to form a ring.

In an embodiment, the emission layer EML may include any suitable dopant material generally used in the art. In some embodiments, the emission layer EML may include styryl derivatives (for example, 1,4-bis[2-(3-N-ethylcarbazoryl)vinyl]benzene (BCzVB), 4-(di-p-tolylamino)-4'-[(di-p-tolylamino)styryl]stilbene (DPAVB), N-(4-((E)-2-(6-((E)-4-(diphenylamino)styryl)naphthalen-2-yl)vinyl)phenyl)-N-phenylbenzenamine (N-BDAVBi), and 4,4'-bis[2-(4-(N,N-diphenylamino)phenyl)vinyl]biphenyl (DPAVBi)), perylene and the derivatives thereof (for example, 2,5,8,11-tetra-t-butylperylene (TBP)), pyrene and the derivatives thereof (for example, 1,1-dipyrene, 1,4-dipyrenylbenzene, and 1,4-bis(N,N-diphenylamino)pyrene), etc.

The emission layer EML may include any suitable phosphorescence dopant material generally used in the art. In some embodiments, the phosphorescence dopant may include a metal complex including iridium (Ir), platinum (Pt), osmium (Os), gold (Au), titanium (Ti), zirconium (Zr), hafnium (Hf), europium (Eu), terbium (Tb) or thulium (Tm). In some embodiments, iridium(III) bis(4,6-difluorophenylpyridinato-N,C2')picolinate (Flrpic), bis(2,4-difluorophenylpyridinato)-tetrakis(1-pyrazolyl)borate iridium(III) (Flr6), or platinum octaethyl porphyrin (PtOEP) may be used as the phosphorescence dopant. However, an embodiment of the present disclosure is not limited thereto.

The emission layer EML may include a quantum dot material. The core of the quantum dot may be selected from Group II-VI compounds, Group III-VI compounds, Group I-III-VI compounds, Group III-V compounds, Group III-II-V compounds, Group IV-VI compounds, Group IV elements, Group IV compounds, and combinations thereof.

The Group II-VI compound may be selected from the group consisting of: a binary compound selected from the group consisting of CdSe, CdTe, CdS, ZnS, ZnSe, ZnTe, ZnO, HgS, HgSe, HgTe, MgSe, MgS, and mixtures thereof; a ternary compound selected from the group consisting of CdSeS, CdSeTe, CdSTe, ZnSeS, ZnSeTe, ZnSTe, HgSeS, HgSeTe, HgSTe, CdZnS, CdZnSe, CdZnTe, CdHgS, CdHgSe, CdHgTe, HgZnS, HgZnSe, HgZnTe, MgZnSe, MgZnS, and mixtures thereof; and a quaternary compound selected from the group consisting of HgZnTeS, CdZnSeS, CdZnSeTe, CdZnSTe, CdHgSeS, CdHgSeTe, CdHgSTe, HgZnSeS, HgZnSeTe, and mixtures thereof.

The Group III-VI compound may include a binary compound such as $In_2S_3$, and $In_2Se_3$, a ternary compound such as $InGaS_3$, and $InGaSe_3$, or optional combinations thereof.

The Group I-III-VI compound may be selected from a ternary compound selected from the group consisting of AgInS, $AgInS_2$, CuInS, $CuInS_2$, $AgGaS_2$, $CuGaS_2$, $CuGaO_2$, $AgGaO_2$, $AgAlO_2$ and mixtures thereof, or a quaternary compound such as $AgInGaS_2$, and $CuInGaS_2$.

The Group III-V compound may be selected from the group consisting of a binary compound selected from the group consisting of GaN, GaP, GaAs, GaSb, AlN, AlP, AlAs, AlSb, InN, InP, InAs, InSb, and mixtures thereof, a ternary compound selected from the group consisting of GaNP, GaNAs, GaNSb, GaPAs, GaPSb, AlNP, AlNAs, AlNSb, AlPAs, AlPSb, InGaP, InAlP, InNP, InNAs, InNSb, InPAs, InPSb, and mixtures thereof, and a quaternary compound selected from the group consisting of GaAlNP, GaAlNAs, GaAlNSb, GaAlPAs, GaAlPSb, GaInNP, GaInNAs, GaInNSb, GaInPAs, GaInPSb, InAlNP, InAlNAs, InAlNSb, InAlPAs, InAlPSb, and mixtures thereof. In some embodiments, the Group III-V compound may further include a II group metal. For example, InZnP, etc. may be selected as a Group III-II-V compound.

The Group IV-VI compound may be selected from the group consisting of a binary compound selected from the group consisting of SnS, SnSe, SnTe, PbS, PbSe, PbTe, and mixtures thereof, a ternary compound selected from the group consisting of SnSeS, SnSeTe, SnSTe, PbSeS, PbSeTe, PbSTe, SnPbS, SnPbSe, SnPbTe, and mixtures thereof, and a quaternary compound selected from the group consisting of SnPbSSe, SnPbSeTe, SnPbSTe, and mixtures thereof. The Group IV element may be selected from the group consisting of Si, Ge, and a mixture thereof. The Group IV group may be a binary compound selected from the group consisting of SiC, SiGe, and a mixture thereof.

In this case, the binary compound, the ternary compound or the quaternary compound may be present at a uniform concentration in a particle or may be present at a partially different concentration distribution state in the same particle. In addition, a core/shell structure in which one quantum dot wraps another quantum dot may be possible. The interface of the core and the shell may have a concentration gradient in which the concentration of an element present in the shell is decreased along a direction toward the center.

In some embodiments, the quantum dot may have the above-described core-shell structure including a core including a nanocrystal and a shell wrapping the core. The shell of the quantum dot may play the role of a protection layer for preventing or reducing the chemical deformation or degradation of the core to maintain semiconductor properties and/or a charging layer for imparting the quantum dot having electrophoretic properties. The shell may have a single layer or a multilayer. Examples of the shell of the quantum dot may include a metal or non-metal oxide, a semiconductor compound, or combinations thereof.

For example, the metal or non-metal oxide may include a binary compound such as $SiO_2$, $Al_2O_3$, $TiO_2$, ZnO, MnO, $Mn_2O_3$, $Mn_3O_4$, CuO, FeO, $Fe_2O_3$, $Fe_3O_4$, CoO, $CO_3O_4$ and NiO, or a ternary compound such as $MgAl_2O_4$, $CoFe_2O_4$, $NiFe_2O_4$ and $CoMn_2O_4$, but an embodiment of the present disclosure is not limited thereto.

Also, the semiconductor compound may include CdS, CdSe, CdTe, ZnS, ZnSe, ZnTe, ZnSeS, ZnTeS, GaAs, GaP, GaSb, HgS, HgSe, HgTe, InAs, InP, InGaP, InSb, AlAs, AlP, AlSb, etc., but an embodiment of the present disclosure is not limited thereto.

The quantum dot may have a full width of half maximum (FWHM) of emission wavelength spectrum of about 45 nm or less, about 40 nm or less, or, for example, about 30 nm or less. Within this range, color purity or color reproducibility may be improved. In addition, light emitted via such quantum dot is emitted in all (e.g., substantially all) directions, and light view angle properties may be improved.

In addition, the shape of the quantum dot may be any generally used shapes in the art, without specific limitation. In some embodiments, the shape of spherical, pyramidal, multi-arm, or cubic nanoparticle, nanotube, nanowire, nanofiber, nanoplate particle, etc. may be used.

The quantum dot may control the color of light emitted according to the particle size of the quantum dot, and accordingly, the quantum dot may have various suitable emission colors such as blue, red and green.

In the light emitting devices ED of embodiments, as shown in FIG. 3 to FIG. 6, the electron transport region ETR is provided on the emission layer EML. The electron transport region ETR may include at least one of an electron blocking layer HBL, an electron transport layer ETL or an electron injection layer EIL. However, an embodiment of the present disclosure is not limited thereto.

The electron transport region ETR may have a single layer formed using a single material, a single layer formed using multiple different materials, or a multilayer structure having multiple layers formed using multiple different materials.

For example, the electron transport region ETR may have a single layer structure of an electron injection layer EIL or an electron transport layer ETL, or a single layer structure formed using an electron injection material and an electron transport material. Further, the electron transport region ETR may have a single layer structure formed using multiple different materials, or a structure stacked from the emission layer EML of electron transport layer ETL/electron injection layer EIL, or hole blocking layer HBL/electron transport layer ETL/electron injection layer EIL, without limitation. The thickness of the electron transport region ETR may be, for example, from about 1,000 Å to about 1,500 Å.

The electron transport region ETR may be formed using various suitable methods such as a vacuum deposition method, a spin coating method, a cast method, a Langmuir-Blodgett (LB) method, an inkjet printing method, a laser printing method, and/or a laser induced thermal imaging (LITI) method.

The electron transport region ETR may include a compound represented by Formula ET-1 below.

Formula ET-1

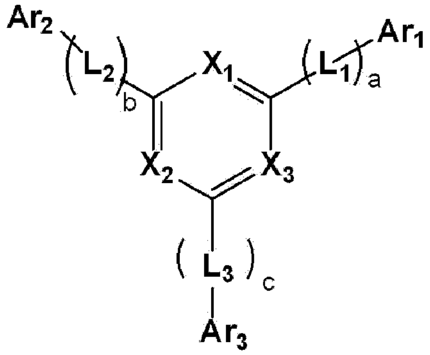

In Formula ET-1, at least one selected from among $X_1$ to $X_3$ is N, and the remainder are $CR_a$. $R_a$ may be a hydrogen atom, a deuterium atom, a substituted or unsubstituted alkyl of 1 to 20 carbon atoms, a substituted or unsubstituted aryl group of 6 to 30 ring-forming carbon atoms, or a substituted or unsubstituted heteroaryl group of 2 to 30 ring-forming carbon atoms. $Ar_1$ to $Ar_3$ may be each independently a hydrogen atom, a deuterium atom, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms.

In Formula ET-1, "a" to "c" may be each independently an integer of 0 to 10. In Formula ET-1, $L_1$ to $L_3$ may be each independently a direct linkage, a substituted or unsubstituted arylene group of 6 to 30 ring-forming carbon atoms, or a substituted or unsubstituted heteroarylene group of 2 to 30 ring-forming carbon atoms. In some embodiments, if "a" to "c" are integers of 2 or more, $L_1$ to $L_3$ may be each independently a substituted or unsubstituted arylene group of 6 to 30 ring-forming carbon atoms, or a substituted or unsubstituted heteroarylene group of 2 to 30 ring-forming carbon atoms.

The electron transport region ETR may include an anthracene-based compound. However, an embodiment of the present disclosure is not limited thereto, and the electron transport region ETR may include, for example, tris(8-hydroxyquinolinato)aluminum ($Alq_3$), 1,3,5-tri[(3-pyridyl)-phen-3-yl]benzene, 2,4,6-tris(3'-(pyridin-3-yl)biphenyl-3-yl)-1,3,5-triazine, 2-(4-(N-phenylbenzoimidazolyl-1-ylphenyl)-9,10-dinaphthylanthracene, 1,3,5-tri(1-phenyl-1H-benzo[d]imidazol-2-yl)benzene (TPBi), 2,9-dimethyl-4,7-diphenyl-1,10-phenanthroline (BCP), 4,7-diphenyl-1,10-phenanthroline (Bphen), 3-(4-biphenylyl)-4-phenyl-5-tert-butylphenyl-1,2,4-triazole (TAZ), 4-(naphthalen-1-yl)-3,5-diphenyl-4H-1,2,4-triazole (NTAZ), 2-(4-biphenylyl)-5-(4-tert-butylphenyl)-1,3,4-oxadiazole (tBu-PBD), bis(2-methyl-8-quinolinolato-N1,O8)-(1,1'-biphenyl-4-olato)aluminum(BAlq), berylliumbis(benzoquinolin-10-olate ($Bebq_2$), 9,10-di(naphthalene-2-yl)anthracene (ADN), 1,3-bis[3,5-di(pyridin-3-yl)phenyl]benzene (BmPyPhB), and mixtures thereof, without limitation.

The electron transport region ETR may include at least one selected from among Compounds ET1 to ET36 below.

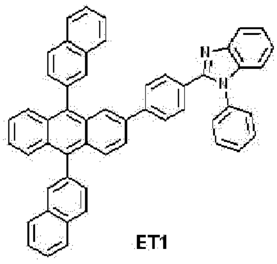

ET1

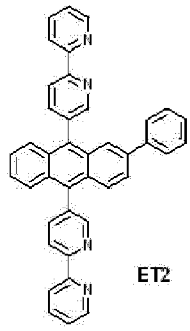

ET2

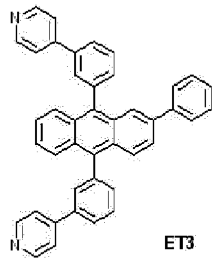

ET3

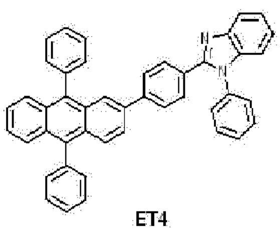

ET4

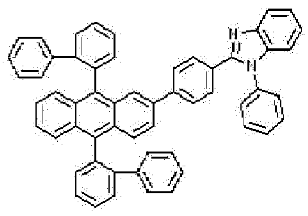

ET5

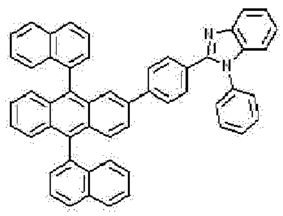

ET6

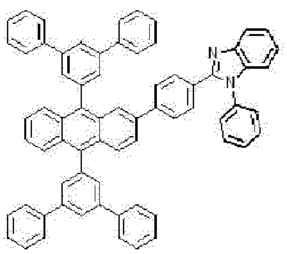

ET7

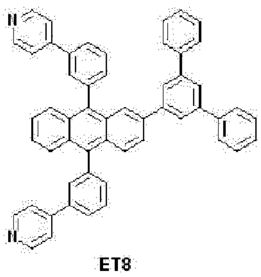

ET8

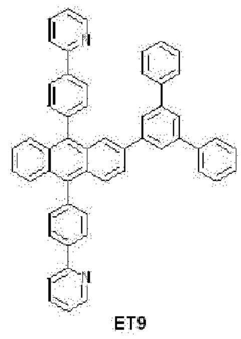

ET9

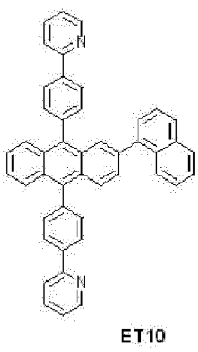

ET10

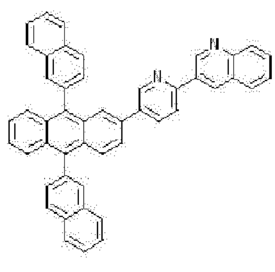

ET11

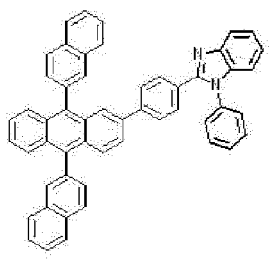

ET12

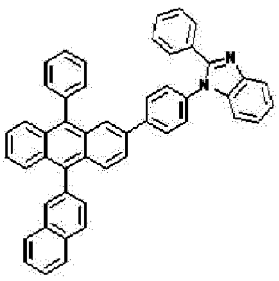

ET13

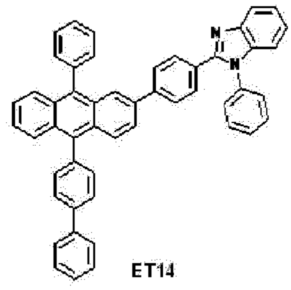

ET14

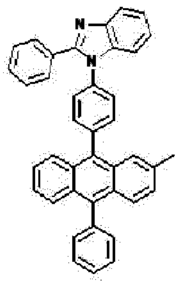

ET15

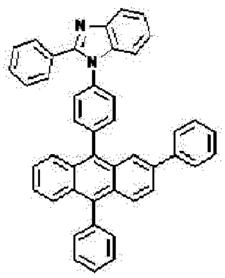

ET16

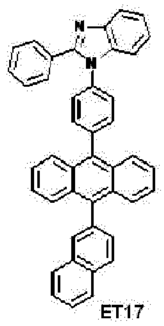

ET17

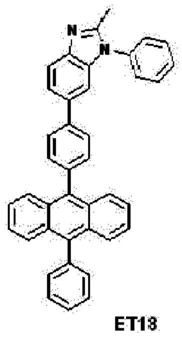

ET18

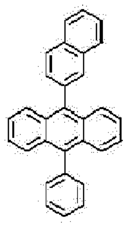

ET19

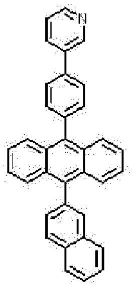

ET20

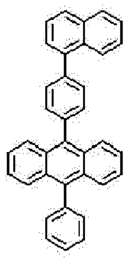

ET21

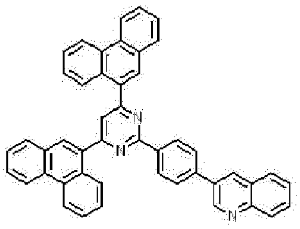

ET22

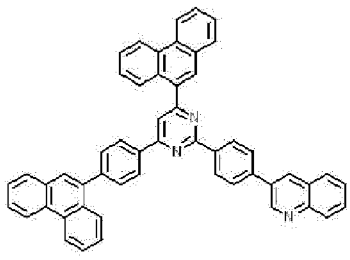

ET23

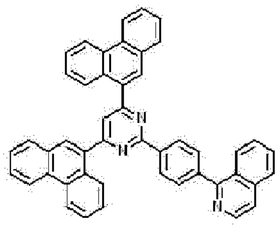

ET24

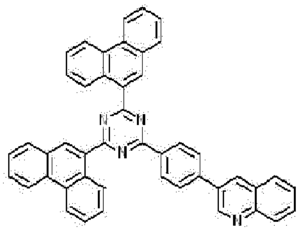

ET25

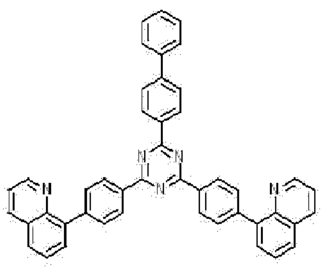

ET26

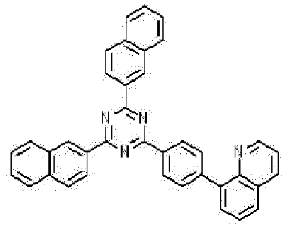

ET27

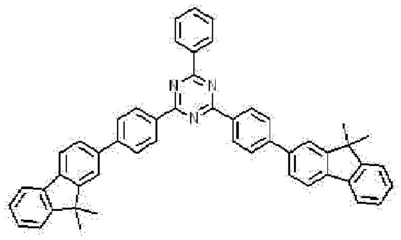

ET28

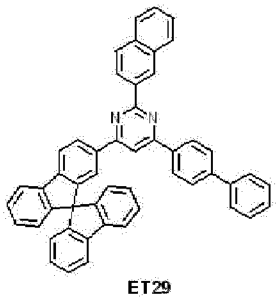

ET29

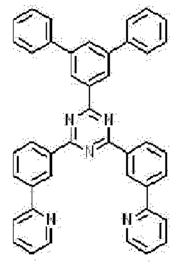

ET30

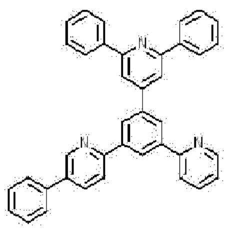

ET31

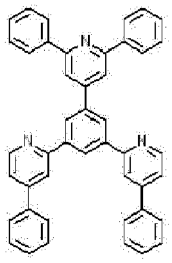

ET32

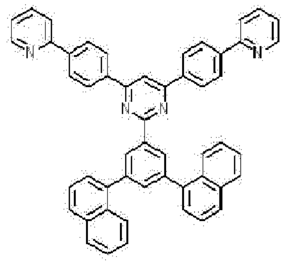

ET33

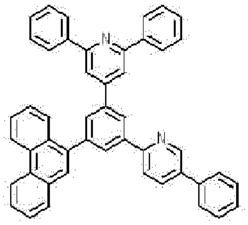

ET34

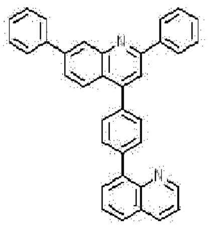

ET35

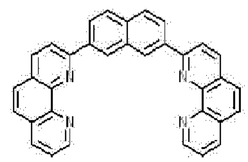

ET36

In addition, the electron transport region ETR may include a metal halide such as LiF, NaCl, CsF, RbCl, RbI, CuI and KI, a metal in lanthanides such as Yb, or a co-deposited material of the metal halide and the metal in lanthanides. For example, the electron transport region ETR may include KI:Yb, RbI:Yb, LiF:Yb, etc., as the co-deposited material. In some embodiments, the electron transport region ETR may use a metal oxide such as $Li_2O$ and BaO, or 8-hydroxy-lithium quinolate (Liq). However, an embodiment of the present disclosure is not limited thereto. The electron transport region ETR also may be formed using a mixture material of an electron transport material and an insulating organo metal salt. The organo metal salt may be a material having an energy band gap of about 4 eV or more. In some embodiments, the organo metal salt may include, for example, metal acetates, metal benzoates, metal acetoacetates, metal acetylacetonates, and/or metal stearates.

The electron transport region ETR may include at least one of 2,9-dimethyl-4,7-diphenyl-1,10-phenanthroline (BCP), diphenyl(4-(triphenylsilyl)phenyl)phosphine oxide (TSPO1), or 4,7-diphenyl-1,10-phenanthroline (Bphen) in addition to the aforementioned materials. However, an embodiment of the present disclosure is not limited thereto.

The electron transport region ETR may include the compounds of the electron transport region in at least one selected from among an electron injection layer EIL, an electron transport layer ETL, and a hole blocking layer HBL.

If the electron transport region ETR includes the electron transport layer ETL, the thickness of the electron transport layer ETL may be from about 100 Å to about 1,000 Å, for example, from about 150 Å to about 500 Å. If the thickness of the electron transport layer ETL satisfies the above-described range, suitable or satisfactory electron transport properties may be obtained without substantial increase of a driving voltage. If the electron transport region ETR includes the electron injection layer EIL, the thickness of the electron injection layer EIL may be from about 1 Å to about 100 Å, and from about 3 Å to about 90 Å. If the thickness of the electron injection layer EIL satisfies the above described range, suitable or satisfactory electron injection properties may be obtained without inducing substantial increase of a driving voltage.

The second electrode EL2 is provided on the electron transport region ETR. The second electrode EL2 may be a common electrode. The second electrode EL2 may be a cathode or an anode, but an embodiment of the present disclosure is not limited thereto. For example, if the first electrode EL1 is an anode, the second cathode EL2 may be a cathode, and if the first electrode EL1 is a cathode, the second electrode EL2 may be an anode. The second electrode

may include at least one selected from Ag, Mg, Cu, Al, Pt, Pd, Au, Ni, Nd, Ir, Cr, Li, Ca, LiF, Mo, Ti, W, In, Sn, and Zn, compounds of two or more selected therefrom, mixtures of two or more selected therefrom, or oxides thereof.

The second electrode EL2 may be a transmissive electrode, a transflective electrode or a reflective electrode. If the second electrode EL2 is the transmissive electrode, the second electrode EL2 may include a transparent metal oxide, for example, ITO, IZO, ZnO, ITZO, etc.

If the second electrode EL2 is the transflective electrode or the reflective electrode, the second electrode EL2 may include Ag, Mg, Cu, Al, Pt, Pd, Au, Ni, Nd, Ir, Cr, Li, Ca, LiF/Ca, LiF/Al, Mo, Ti, Yb, W, compounds including thereof, or mixtures thereof (for example, AgMg, AgYb, or MgAg). Otherwise, the second electrode EL2 may have a multilayered structure including a reflective layer or a transflective layer formed using the above-described materials and a transparent conductive layer formed using ITO, IZO, ZnO, ITZO, etc. For example, the second electrode EL2 may include the aforementioned metal materials, combinations of two or more metal materials selected from the aforementioned metal materials, or oxides of the aforementioned metal materials.

In some embodiments, the second electrode EL2 may be connected with an auxiliary electrode. If the second electrode EL2 is connected with the auxiliary electrode, the resistance of the second electrode EL2 may decrease.

A capping layer CPL may be further on the second electrode EL2 in the light emitting device ED of an embodiment. The capping layer CPL may include a multilayer or a single layer.

In an embodiment, the capping layer CPL may be an organic layer or an inorganic layer. For example, if the capping layer CPL includes an inorganic material, the inorganic material may include an alkali metal compound such as LiF, an alkaline earth metal compound such as $MgF_2$, SiON, SiNx, SiOy, etc.

For example, if the capping layer CPL includes an organic material, the organic material may include 2,2'-dimethyl-N,N'-di-[(1-naphthyl)-N,N'-diphenyl]-1,1'-biphenyl-4,4'-diamine($\alpha$-NPD), NPB, TPD, m-MTDATA, $Alq_3$, CuPc, N4,N4,N4',N4'-tetra(biphenyl-4-yl) biphenyl-4,4'-diamine (TPD15), 4,4',4"-tris(carbazol-9-yl) triphenylamine (TCTA), etc., an epoxy resin, and/or acrylate such as methacrylate. In addition, a capping layer CPL may include at least one selected from among Compounds P1 to P5 below, but an embodiment of the present disclosure is not limited thereto.

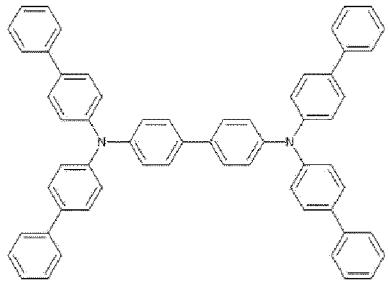

P1

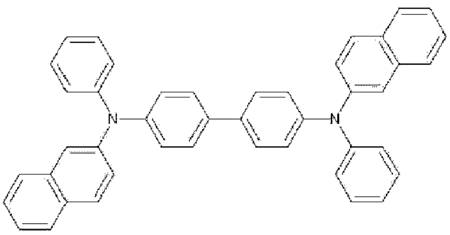

P2

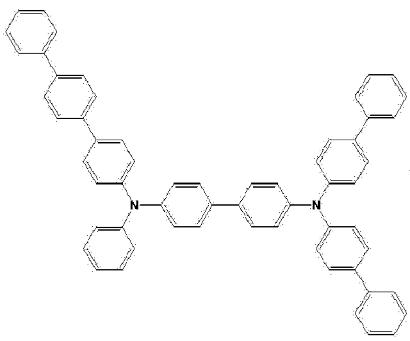

P3

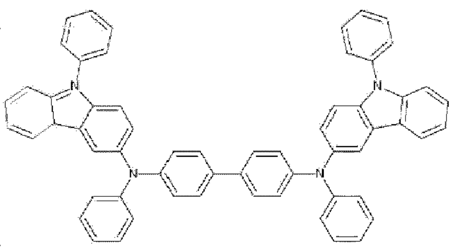

P4

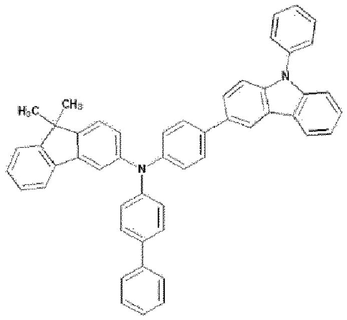

P5

In some embodiments, the refractive index of the capping layer CPL may be about 1.6 or more. For example, the refractive index of the capping layer CPL with respect to light in a wavelength range of about 550 nm to about 660 nm may be about 1.6 or more.

FIG. 9 and FIG. 10 are cross-sectional views on display apparatuses according to embodiments, respectively. In the explanation on the display apparatuses of embodiments, referring to FIG. 9 and FIG. 10, duplicative description of FIG. 1 to FIG. 8 will not be repeated here, and the different features will be explained chiefly.

Referring to FIG. 9, the display apparatus DD-a according to an embodiment may include a display panel DP including a display device layer DP-ED, a light controlling layer CCL on the display panel DP and a color filter layer CFL.

In an embodiment shown in FIG. 9, the display panel DP includes a base layer BS, a circuit layer DP-CL provided on the base layer BS and a display device layer DP-ED, and the display device layer DP-ED may include a light emitting device ED.

The light emitting device ED may include a first electrode EL1, a hole transport region HTR on the first electrode EL1, an emission layer EML on the hole transport region HTR, an electron transport region ETR on the emission layer EML, and a second electrode EL2 on the electron transport region ETR. The same structures of the light emitting devices of FIG. 3 to FIG. 8 may be applied to the structure of the light emitting device ED shown in FIG. 9.

Referring to FIG. 9, the emission layer EML may be in an opening part OH defined in a pixel definition layer PDL. For example, the emission layer EML divided by the pixel definition layer PDL and correspondingly provided to each of luminous areas PXA-R, PXA-G and PXA-B may emit light in the same (e.g., substantially the same) wavelength region. In the display apparatus DD-a of an embodiment, the emission layer EML may emit blue light. In some embodiments, the emission layer EML may be provided as a common layer for all luminous areas PXA-R, PXA-G and PXA-B.

The light controlling layer CCL may be on the display panel DP. The light controlling layer CCL may include a light converter. The light converter may be a quantum dot and/or a phosphor. The light converter may transform the wavelength of light provided and then emit light. In some embodiments, the light controlling layer CCL may be a layer including a quantum dot and/or a layer including a phosphor.

The light controlling layer CCL may include multiple light controlling parts CCP1, CCP2 and CCP3. The light controlling parts CCP1, CCP2 and CCP3 may be spaced apart from one another.

Referring to FIG. 9, a partition pattern BMP may be between the spaced apart light controlling parts CCP1, CCP2 and CCP3, but an embodiment of the present disclosure is not limited thereto. In FIG. 9, the partition pattern BMP is shown not to be overlapped with the light controlling parts CCP1, CCP2 and CCP3, but at least a portion of the edge of the light controlling parts CCP1, CCP2 and CCP3 may be overlapped with the partition pattern BMP.

The light controlling layer CCL may include a first light controlling part CCP1 including a first quantum dot QD1 that converts a first color light provided from the light emitting device ED into a second color light, a second light controlling part CCP2 including a second quantum dot QD2 that converts a first color light into a third color light, and a third light controlling part CCP3 that transmits a first color light.

In an embodiment, the first light controlling part CCP1 may provide red light which is the second color light, and the second light controlling part CCP2 may provide green light which is the third color light. The third color controlling part CCP3 may transmit and provide blue light which is the first color light provided from the light emitting device ED. For example, the first quantum dot QD1 may be a red quantum dot, and the second quantum dot QD2 may be a green quantum dot. For the quantum dots QD1 and QD2, the same description as that above may be applied.

In addition, the light controlling layer CCL may further include a scatterer SP (e.g., a light scatterer SP). The first light controlling part CCP1 may include the first quantum dot QD1 and the scatterer SP, the second light controlling part CCP2 may include the second quantum dot QD2 and the scatterer SP, and the third light controlling part CCP3 may not include a quantum dot but include the scatterer SP.

The scatterer SP may be an inorganic particle. For example, the scatterer SP may include at least one selected from among $TiO_2$, ZnO, $Al_2O_3$, $SiO_2$, and hollow silica. The scatterer SP may include at least one selected from among

$TiO_2$, ZnO, $Al_2O_3$, $SiO_2$, and hollow silica, or may be a mixture of two or more materials selected from among $TiO_2$, ZnO, $Al_2O_3$, $SiO_2$, and hollow silica.

Each of the first light controlling part CCP1, the second light controlling part CCP2, and the third light controlling part CCP3 may include base resins BR1, BR2 and BR3 that disperse the quantum dots QD1 and QD2 and the scatterer SP. In an embodiment, the first light controlling part CCP1 may include the first quantum dot QD1 and the scatterer SP dispersed in the first base resin BR1, the second light controlling part CCP2 may include the second quantum dot QD2 and the scatterer SP dispersed in the second base resin BR2, and the third light controlling part CCP3 may include the scatterer particle SP dispersed in the third base resin BR3. The base resins BR1, BR2 and BR3 are mediums in which the quantum dots QD1 and QD2 and the scatterer SP are dispersed, and may be composed of various suitable resin compositions which may be generally referred to as a binder. For example, the base resins BR1, BR2 and BR3 may be acrylic resins, urethane-based resins, silicone-based resins, epoxy-based resins, etc. The base resins BR1, BR2 and BR3 may be transparent resins. In an embodiment, the first base resin BR1, the second base resin BR2 and the third base resin BR3 may be the same or different from each other.

The light controlling layer CCL may include a barrier layer BFL1. The barrier layer BFL1 may play the role of blocking or reducing the penetration of moisture and/or oxygen (hereinafter, may be referred to as "humidity/oxygen"). The barrier layer BFL1 may be on the light controlling parts CCP1, CCP2 and CCP3 to block or reduce the exposure of the light controlling parts CCP1, CCP2 and CCP3 to humidity/oxygen. In some embodiments, the barrier layer BFL1 may cover the light controlling parts CCP1, CCP2 and CCP3. In addition, the barrier layer BFL2 may be provided between the light controlling parts CCP1, CCP2 and CCP3 and filters CF1, CF2 and CF3.

The barrier layers BFL1 and BFL2 may include at least one inorganic layer. In some embodiments, the barrier layers BFL1 and BFL2 may be formed by including an inorganic material. For example, the barrier layers BFL1 and BFL2 may be formed by including silicon nitride, aluminum nitride, zirconium nitride, titanium nitride, hafnium nitride, tantalum nitride, silicon oxide, aluminum oxide, titanium oxide, tin oxide, cerium oxide and silicon oxynitride or a metal thin film securing light transmittance. In some embodiments, the barrier layers BFL1 and BFL2 may further include an organic layer. The barrier layers BFL1 and BFL2 may be composed of a single layer or multiple layers.

In the display apparatus DD-a of an embodiment, the color filter layer CFL may be on the light controlling layer CCL. For example, the color filter layer CFL may be directly on the light controlling layer CCL. In this case, the barrier layer BFL2 may be omitted.

The color filter layer CFL may include a light blocking part BM and filters CF1, CF2 and CF3. The color filter layer CFL may include a first filter CF1 that transmits a second color light, a second filter CF2 that transmits a third color light, and a third filter CF3 that transmits a first color light. For example, the first filter CF1 may be a red filter, the second filter CF2 may be a green filter, and the third filter CF3 may be a blue filter. Each of the filters CF1, CF2 and CF3 may include a polymer photosensitive resin and a pigment and/or dye. The first filter CF1 may include a red pigment and/or dye, the second filter CF2 may include a green pigment and/or dye, and the third filter CF3 may include a blue pigment and/or dye. Embodiments of the present disclosure are not limited thereto, however, and the third filter CF3 may not include the pigment and/or dye. The third filter CF3 may include a polymer photosensitive resin and not include a pigment and/or dye. The third filter CF3 may be transparent. The third filter CF3 may be formed using a transparent photosensitive resin.

In addition, in an embodiment, the first filter CF1 and the second filter CF2 may be yellow filters. The first filter CF1 and the second filter CF2 may be provided in one body without distinction.

The light blocking part BM may be a black matrix. The light blocking part BM may be formed by including an organic light blocking material or an inorganic light blocking material including a black pigment and/or black dye. The light blocking part BM may prevent or reduce light leakage phenomenon and divide the boundaries among adjacent filters CF1, CF2 and CF3. In addition, in an embodiment, the light blocking part BM may be formed as a blue filter.

The first to third filters CF1, CF2 and CF3 may respectively correspond to a red luminous area PXA-R, green luminous area PXA-G, and blue luminous area PXA-B.

A base substrate BL may be on the color filter layer CFL. The base substrate BL may be a member providing a base surface that the color filter layer CFL, the light controlling layer CCL, etc. are on. The base substrate BL may be a glass substrate, a metal substrate, a plastic substrate, etc. However, an embodiment of the present disclosure is not limited thereto, and the base substrate BL may be an inorganic layer, an organic layer or a composite material layer. In addition, different from the drawing, the base substrate BL may be omitted in an embodiment.

FIG. 10 is a cross-sectional view showing a portion of the display apparatus according to an embodiment. In a display apparatus DD-TD of an embodiment, the light emitting device ED-BT may include multiple light emitting structures OL-B1, OL-B2 and OL-B3. The light emitting device ED-BT may include the first electrode EL1 facing the second electrode EL2, and the multiple light emitting structures OL-B1, OL-B2 and OL-B3 stacked in order in a thickness direction and provided between the first electrode EL1 and the second electrode EL2. Each of the light emitting structures OL-B1, OL-B2 and OL-B3 may include an emission layer EML (FIG. 9), and a hole transport region HTR and an electron transport region ETR with the emission layer EML (FIG. 9) therebetween.

In some embodiments, the light emitting device ED-BT included in the display apparatus DD-TD of an embodiment may be a light emitting device of a tandem structure including multiple emission layers.

In an embodiment shown in FIG. 10, light emitted from the light emitting structures OL-B1, OL-B2 and OL-B3 may all be blue light. However, an embodiment of the present disclosure is not limited thereto, and the wavelength regions of light emitted from the light emitting structures OL-B1, OL-B2 and OL-B3 may be different from each other. For example, the light emitting device ED-BT including the multiple light emitting structures OL-B1, OL-B2 and OL-B3 emitting light in different wavelength regions may emit white light.

Charge generating layers CGL1 and 2 may be between neighboring light emitting structures OL-B1, OL-B2 and OL-B3,. The charge generating layer CGL may include a p-type charge generating layer and/or an n-type charge generating layer.

Referring to FIG. 11, a display apparatus DD-b according to an embodiment may include light emitting devices ED-1, ED-2 and ED-3, formed by stacking two emission layers. Compared to the display apparatus DD-a of an embodiment, shown in FIG. 9, an embodiment shown in FIG. 11 is different in that first to third light emitting devices ED-1, ED-2 and ED-3 include two emission layers stacked in a thickness direction, each. In the first to third light emitting devices ED-1, ED-2 and ED-3, two emission layers may emit light in the same (e.g., substantially the same) wavelength region.

The first light emitting device ED-1 may include a first red emission layer EML-R1 and a second red emission layer EML-R2. The second light emitting device ED-2 may include a first green emission layer EML-G1 and a second green emission layer EML-G2. In addition, the third light emitting device ED-3 may include a first blue emission layer EML-B1 and a second blue emission layer EML-B2. Between the first red emission layer EML-R1 and the second red emission layer EML-R2, between the first green emission layer EML-G1 and the second green emission layer EML-G2, and an emission auxiliary part OG may be between the first blue emission layer EML-B1 and the second blue emission layer EML-B2.

The emission auxiliary part OG may include a single layer or a multilayer. The emission auxiliary part OG may include a charge generating layer. In some embodiments, the emission auxiliary part OG may include an electron transport region, a charge generating layer, and a hole transport region stacked in order. The emission auxiliary part OG may be provided as a common layer in all of the first to third light emitting devices ED-1, ED-2 and ED-3. However, an embodiment of the present disclosure is not limited thereto, and the emission auxiliary part OG may be patterned and provided in an opening part OH defined in a pixel definition layer PDL.

The first red emission layer EML-R1, the first green emission layer EML-G1 and the first blue emission layer EML-B1 may be between the electron transport region ETR and the emission auxiliary part OG. The second red emission layer EML-R2, the second green emission layer EML-G2 and the second blue emission layer EML-B2 may be between the emission auxiliary part OG and the hole transport region HTR.

In some embodiments, the first light emitting device ED-1 may include a first electrode EL1, a hole transport region HTR, a second red emission layer EML-R2, an emission auxiliary part OG, a first red emission layer EML-R1, an electron transport region ETR, and a second electrode EL2, stacked in order. The second light emitting device ED-2 may include a first electrode EL1, a hole transport region HTR, a second green emission layer EML-G2, an emission auxiliary part OG, a first green emission layer EML-G1, an electron transport region ETR, and a second electrode EL2, stacked in order. The third light emitting device ED-3 may include a first electrode EL1, a hole transport region HTR, a second blue emission layer EML-B2, an emission auxiliary part OG, a first blue emission layer EML-B1, an electron transport region ETR, and a second electrode EL2, stacked in order.

In some embodiments, an optical auxiliary layer PL may be on a display device layer DP-ED. The optical auxiliary layer PL may include a polarization layer. The optical auxiliary layer PL may be on a display panel DP and may control reflected light at the display panel DP by external light. Different from the drawings, the optical auxiliary layer PL may be omitted from the display apparatus according to an embodiment.

Different from FIG. 9 and FIG. 10, a display apparatus DD-c in FIG. 12 is shown to include four light emitting structures OL-B1, OL-B2, OL-B3 and OL-C1. A light emitting device ED-CT may include the first electrode EL1 facing the second electrode EL2, and first to fourth light emitting structures OL-B1, OL-B2, OL-B3 and OL-C1 stacked in order in a thickness direction between the first electrode EL1 and the second electrode EL2. Charge generating layers CGL1, CGL2 and CGL3 may be respectively between the first to fourth light emitting structures OL-B1, OL-B2, OL-B3 and OL-C1. Among the four light emitting structures, the first to third light emitting structures OL-B1, OL-B2 and OL-B3 may emit blue light, and the fourth light emitting structure OL-C1 may emit green light. However, an embodiment of the present disclosure is not limited thereto, and the first to fourth light emitting structures OL-B1, OL-B2, OL-B3 and OL-C1 may emit different wavelengths of light.

Hereinafter, the compound according to an embodiment and the light emitting device of an embodiment of the present disclosure will be particularly explained referring to examples and comparative examples. In addition, the embodiments below are only examples to assist the understanding of the subject matter of the present disclosure, and the scope of the present disclosure is not limited thereto.

## Examples

### 1. Synthesis of amine compounds

First, the synthetic method of an amine compound according to an embodiment will be explained in more detail with respect to the synthetic methods of Compounds 1, 6, 15, 28, 36, 43, 49, 145, 707, 803, 808, 851, 865, 868, 869, 872, 873, 876, 877, 880, 881, 884, 995, 996, 1024, 1035, 1044, and 1059. In addition, the synthetic methods of the amine compounds explained hereinafter are examples, and the synthetic method of the amine compound according to an embodiment of the present disclosure is not limited to the embodiments below.

### (1) Synthesis of Compound 15

Amine Compound 15 according to an embodiment may be synthesized, for example, by the reaction below.

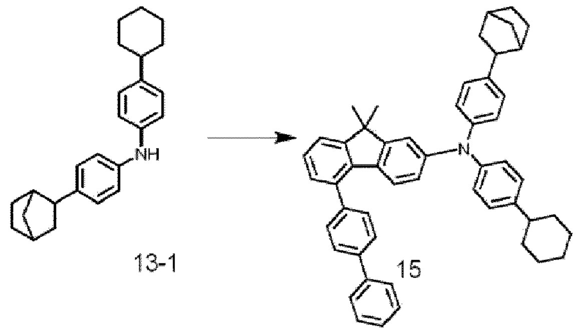

13-1 15

### Synthesis of Compound 15

Intermediate 13-1 (1 eq, 10 mmol), 5-([1,1'-biphenyl]-4-yl)-2-bromo-9,9-dimethyl-9H-fluorene (1.1 eq, 11 mmol), $Pd_2(dba)_3$ (0.03 eq, 0.3 mmol), t-BuONa (3 eq, 30 mmol), t-$Bu_3P$ (0.06 eq, 0.6 mmol), and 250 ml of toluene were put in an one-neck, round-bottom flask, followed by stirring at about 110 °C for about 2 hours. After finishing the reaction, the reaction product was worked up with $H_2O$ and ether, and an organic layer was separated by column chromatography (eluent: methylene chloride/hexane = 1/5 by volume ratio). The organic layer thus obtained was recrystallized with ether to obtain 4.83 g of Compound 15 (yield: 70%, purity ≤ 99.9%).

### (2) Synthesis of Compound 28

Amine Compound 28 according to an embodiment may be synthesized, for example, by the reaction below.

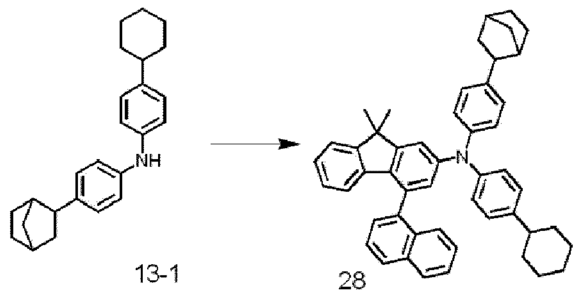

13-1 28

### Synthesis of Compound 28

Intermediate 13-1 (1 eq, 10 mmol), 2-bromo-9,9-dimethyl-4-(naphthalen-1-yl)-9H-fluorene (1.1 eq, 11 mmol), $Pd_2(dba)_3$ (0.03 eq, 0.3 mmol), t-BuONa (3 eq, 30 mmol), t-$Bu_3P$ (0.06 eq, 0.6 mmol), and 250 ml of toluene were put in an one-neck, round-bottom flask, followed by stirring at about 110 °C for about 2 hours. After finishing the reaction, the reaction product was worked up with $H_2O$ and ether, and an organic layer was separated by column chromatography (eluent: methylene chloride/hexane = 1/5 by volume ratio). The organic layer thus obtained was recrystallized with ether to obtain 4.35 g of Compound 28 (yield: 66%, purity ≥ 99.9%).

**(3) Synthesis of Compound 36**

Amine Compound 36 according to an embodiment may be synthesized, for example, by the reaction below.

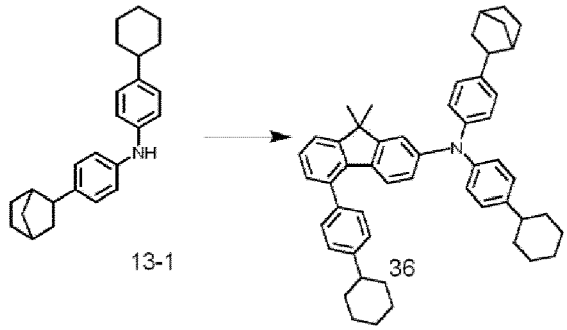

**Synthesis of Compound 36**

Intermediate 13-1 (1 eq, 10 mmol), 2-bromo-5-(4-cyclohexylphenyl)-9,9-dimethyl-9H-fluorene (1.1 eq, 11 mmol), $Pd_2(dba)_3$ (0.03 eq, 0.3 mmol), t-BuONa (3 eq, 30 mmol), t-$Bu_3P$ (0.06 eq, 0.6 mmol), and 250 ml of toluene were put in an one-neck, round-bottom flask, followed by stirring at about 110 °C for about 2 hours. After finishing the reaction, the reaction product was worked up with $H_2O$ and ether, and an organic layer was separated by column chromatography (eluent: methylene chloride/hexane = 1/5 by volume ratio). The organic layer thus obtained was recrystallized with ether to obtain 5.08 g of Compound 36 (yield: 73%, purity ≥ 99.9%).

**(4) Synthesis of Compound 43**

Amine Compound 43 according to an embodiment may be synthesized, for example, by the reaction below.

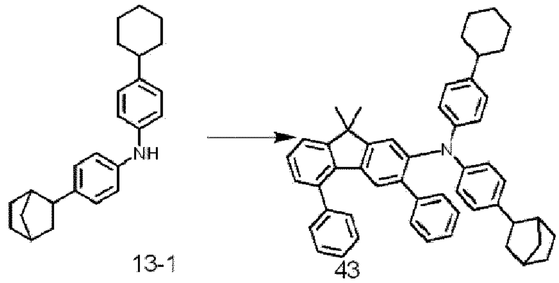

**Synthesis of Compound 43**

Intermediate 13-1 (1 eq, 10 mmol), 2-bromo-9,9-dimethyl-3,5-diphenyl-9H-fluorene (1.1 eq, 11 mmol), $Pd_2(dba)_3$ (0.03 eq, 0.3 mmol), t-BuONa (3 eq, 30 mmol), t-$Bu_3P$ (0.06 eq, 0.6 mmol), and 250 ml of toluene were put in an one-neck, round-bottom flask, followed by stirring at about 110 °C for about 2 hours. After finishing the reaction, the reaction product was worked up with $H_2O$ and ether, and an organic layer was separated by column chromatography (eluent: methylene chloride/hexane = 1/5 by volume ratio). The organic layer thus obtained was recrystallized with ether to obtain 4.75 g of Compound 43 (yield: 69%, purity ≥ 99.9%).

**(5) Synthesis of Compound 49**

Amine Compound 49 according to an embodiment may be synthesized, for example, by the reaction below.

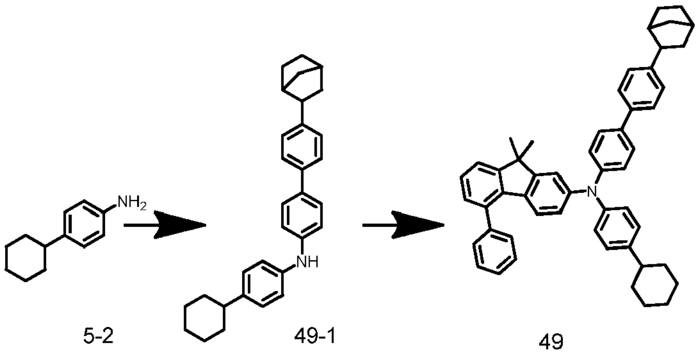

5-2 49-1 49

**Synthesis of Intermediate 49-1**

Intermediate 5-2 (1.2 eq, 22 mmol), 2-(4'-bromo-[1,1'-biphenyl]-4-yl)bicyclo[2.2.1]heptane (1 eq, 20 mmol), $Pd_2(dba)_3$ (0.03 eq, 0.6 mmol), t-BuONa (3 eq, 60 mmol), t-$Bu_3P$ (0.06 eq, 1.2 mmol), and 250 ml of toluene were put in an one-neck, round-bottom flask, followed by stirring at about 110 °C for about 2 hours. After finishing the reaction, the reaction product was worked up with $H_2O$ and ether, and an organic layer was separated by column chromatography (eluent: methylene chloride/hexane = 1/5 by volume ratio). The organic layer thus obtained was recrystallized with ether to obtain 7.3 g of Intermediate 49-1 (yield: 87%, purity ≥ 99.9%).

**Synthesis of Compound 49**

Intermediate 49-1 (1 eq, 10 mmol), 2-bromo-9,9-dimethyl-5-phenyl-9H-fluorene (1.1 eq, 11 mmol), $Pd_2(dba)_3$ (0.03 eq, 0.3 mmol), t-BuONa (3 eq, 30 mmol), t-$Bu_3P$ (0.06 eq, 0.6 mmol), and 300 ml of toluene were put in an one-neck, round-bottom flask, followed by stirring at about 110 °C for about 2 hours. After finishing the reaction, the reaction product was worked up with $H_2O$ and ether, and an organic layer was separated by column chromatography (eluent: methylene chloride/hexane = 1/5 by volume ratio). The organic layer thus obtained was recrystallized with ether to obtain 5.3 g of Compound 49 (yield: 77%, purity ≥ 99.9%).

**(6) Synthesis of Compound 145**

Amine Compound 145 according to an embodiment may be synthesized, for example, by the reaction below.

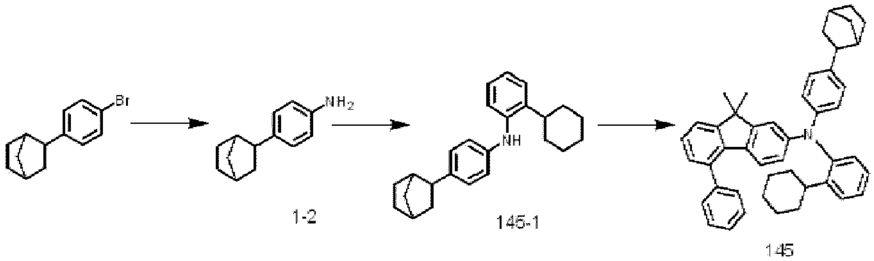

**Synthesis of Intermediate 1-2**

2-(4-Bromophenyl)bicyclo[2.2.1]heptane (1 eq, 100 mmol), CuI (3 eq, 300 mmol), ammonia solution (1 eq, 100 mmol), 100 ml of DMF were put in a seal tube, followed by stirring at about 120 °C for about 6 hours. After finishing the reaction, the reaction product was worked up with $H_2O$ and ether, and an organic layer was separated by column chromatography (eluent: methylene chloride/hexane = 1/3 by volume ratio) to obtain 9.3 g of Intermediate 1-2 (yield: 50%, purity ≥ 99.9%).

**Synthesis of Intermediate 145-1**

Intermediate 1-2 (1.2 eq, 22 mmol), 1-bromo-2-cyclohexylbenzene (1 eq, 20 mmol), $Pd_2(dba)_3$ (0.03 eq, 0.6 mmol), t-BuONa (3 eq, 60 mmol), t-$Bu_3P$ (0.06 eq, 1.2 mmol), and 250 ml of toluene were put in an one-neck, round-bottom flask, followed by stirring at about 110 °C for about 2 hours. After finishing the reaction, the reaction product was

worked up with $H_2O$ and ether, and an organic layer was separated by column chromatography (eluent: methylene chloride/hexane = 1/5 by volume ratio). The organic layer thus obtained was recrystallized with ether to obtain 4.48 g of Intermediate 145-1 (yield: 65%, purity ≥ 99.9%).

**Synthesis of Compound 145**

Intermediate 145-1 (1 eq, 10 mmol), 2-bromo-9,9-dimethyl-5-phenyl-9H-fluorene (1.1 eq, 11 mmol), $Pd_2(dba)_3$ (0.03 eq, 0.3 mmol), t-BuONa (3 eq, 30 mmol), t-$Bu_3P$ (0.06 eq, 0.6 mmol), and 250 ml of toluene were put in an one-neck, round-bottom flask, followed by stirring at about 110 °C for about 2 hours. After finishing the reaction, the reaction product was worked up with $H_2O$ and ether, and an organic layer was separated by column chromatography (eluent: methylene chloride/hexane = 1/5 by volume ratio). The organic layer thus obtained was recrystallized with ether to obtain 4.47 g of Compound 145 (yield: 73%, purity ≥ 99.9%).

**(7) Synthesis of Compound 707**

Amine Compound 707 according to an embodiment may be synthesized, for example, by the reaction below.

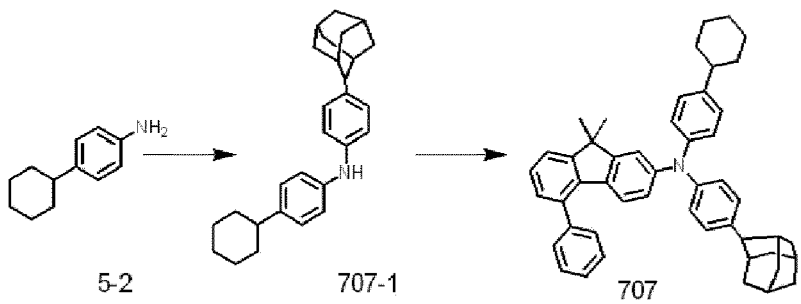

5-2 707-1 707

**Synthesis of Intermediate 707-1**

Intermediate 5-2 (1.2 eq, 22 mmol), (1R,2r,3S,5r)-2-(4-bromophenyl)adamantane (1 eq, 20 mmol), $Pd_2(dba)_3$ (0.03 eq, 0.6 mmol), t-BuONa (3 eq, 60 mmol), t-$Bu_3P$ (0.06 eq, 1.2 mmol), and 250 ml of toluene were put in an one-neck, round-bottom flask, followed by stirring at about 110 °C for about 2 hours. After finishing the reaction, the reaction product was worked up with $H_2O$ and ether, and an organic layer was separated by column chromatography (eluent: methylene chloride/hexane = 1/5 by volume ratio). The organic layer thus obtained was recrystallized with ether to obtain 6.7 g of Intermediate 707-1 (yield: 87%, purity ≥ 99.9%).

**Synthesis of Compound 707**

Intermediate 707-1 (1 eq, 10 mmol), 2-bromo-9,9-dimethyl-5-phenyl-9H-fluorene (1.1 eq, 11 mmol), $Pd_2(dba)_3$ (0.03 eq, 0.3 mmol), t-BuONa (3 eq, 30 mmol), t-$Bu_3P$ (0.06 eq, 0.6 mmol), and 300 ml of toluene were put in an one-neck, round-bottom flask, followed by stirring at about 110 °C for about 2 hours. After finishing the reaction, the reaction product was worked up with $H_2O$ and ether, and an organic layer was separated by column chromatography (eluent: methylene chloride/hexane = 1/5 by volume ratio). The organic layer thus obtained was recrystallized with ether to obtain 5.22 g of Compound 707 (yield: 80%, purity ≥ 99.9%).

**(8) Synthesis of Compound 851**

Amine Compound 851 according to an embodiment may be synthesized, for example, by the reaction below.

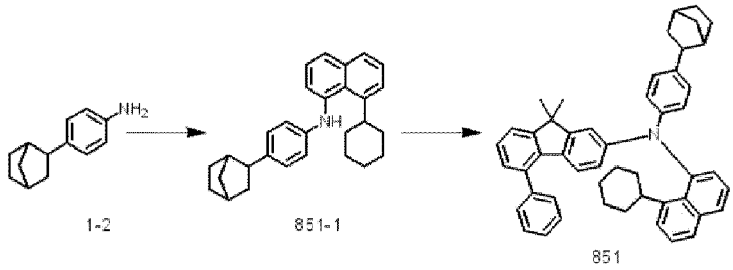


### Synthesis of Intermediate 851-1

Intermediate 1-2 (1.2 eq, 22 mmol), 1-bromo-8-cyclohexylnaphthalene (1 eq, 20 mmol), $Pd_2(dba)_3$ (0.03 eq, 0.6 mmol), t-BuONa (3 eq, 60 mmol), t-$Bu_3P$ (0.06 eq, 1.2 mmol), and 250 ml of toluene were put in an one-neck, round-bottom flask, followed by stirring at about 110 °C for about 2 hours. After finishing the reaction, the reaction product was worked up with $H_2O$ and ether, and an organic layer was separated by column chromatography (eluent: methylene chloride/hexane = 1/5 by volume ratio). The organic layer thus obtained was recrystallized with ether to obtain 4.46 g of Intermediate 851-1 (yield: 62%, purity ≥ 99.9%).

### Synthesis of Compound 851

Intermediate 851-1 (1 eq, 10 mmol), 2-bromo-9,9-dimethyl-5-phenyl-9H-fluorene (1.1 eq, 11 mmol), $Pd_2(dba)_3$ (0.03 eq, 0.3 mmol), t-BuONa (3 eq, 30 mmol), t-$Bu_3P$ (0.06 eq, 0.6 mmol), and 250 ml of toluene were put in an one-neck, round-bottom flask, followed by stirring at about 110 °C for about 2 hours. After finishing the reaction, the reaction product was worked up with $H_2O$ and ether, and an organic layer was separated by column chromatography (eluent: methylene chloride/hexane = 1/5 by volume ratio). The organic layer thus obtained was recrystallized with ether to obtain 4.11 g of Compound 851 (yield: 62%, purity ≥ 99.9%).

### (9) Synthesis of Compound 865

Amine Compound 865 according to an embodiment may be synthesized, for example, by the reaction below.

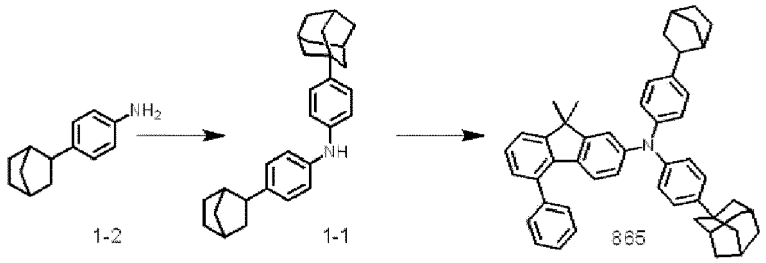


### Synthesis of Intermediate 1-1

Intermediate 1-2 (1.2 eq, 50 mmol), (1r,3R,5S)-1-(4-bromophenyl)adamantane (1 eq, 44 mmol), $Pd_2(dba)_3$ (0.03 eq, 1.5 mmol), t-BuONa (3 eq, 150 mmol), t-$Bu_3P$ (0.06 eq, 3 mmol), and 250 ml of toluene were put in an one-neck, round-bottom flask, followed by stirring at about 110 °C for about 2 hours. After finishing the reaction, the reaction product was worked up with $H_2O$ and ether, and an organic layer was separated by column chromatography (eluent: methylene chloride/hexane = 1/5 by volume ratio). The organic layer thus obtained was recrystallized with ether to obtain 16 g of Intermediate 1-1 (yield: 80%, purity ≥ 99.9%).

### Synthesis of Compound 865

Intermediate 1-1 (1 eq, 40 mmol), 2-bromo-9,9-dimethyl-5-phenyl-9H-fluorene (1.1 eq, 44 mmol), $Pd_2(dba)_3$ (0.03 eq, 1.2 mmol), t-BuONa (3 eq, 120 mmol), t-$Bu_3P$ (0.06 eq, 2.4 mmol), and 250 ml of toluene were put in an one-neck, round-bottom flask, followed by stirring at about 110 °C for about 2 hours. After finishing the reaction, the reaction product was worked up with $H_2O$ and ether, and an organic layer was separated by column chromatography (eluent: methylene chloride/hexane = 1/5 by volume ratio). The organic layer thus obtained was recrystallized with ether to obtain

20 g of Compound 865 (yield: 75%, purity ≥ 99.9%).

**(10) Synthesis of Compound 868**

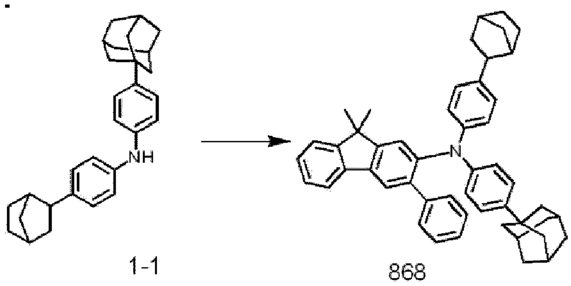

1-1

868

**Synthesis of Compound 868**

Intermediate 1-1 (1 eq, 40 mmol), 2-bromo-9,9-dimethyl-3-phenyl-9H-fluorene (1.1 eq, 44 mmol), $Pd_2(dba)_3$ (0.03 eq, 1.2 mmol), t-BuONa (3 eq, 120 mmol), t-$Bu_3P$ (0.06 eq, 2.4 mmol), and 250 ml of toluene were put in an one-neck, round-bottom flask, followed by stirring at about 110 °C for about 2 hours. After finishing the reaction, the reaction product was worked up with $H_2O$ and ether, and an organic layer was separated by column chromatography (eluent: methylene chloride/hexane = 1/5 by volume ratio). The organic layer thus obtained was recrystallized with ether to obtain 18.6 g of Compound 868 (yield: 70%, purity ≥ 99.9%).

**(11) Synthesis of Compound 803**

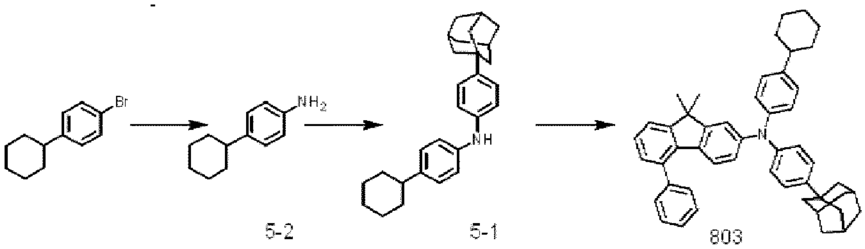

5-2 5-1 803

**Synthesis of Intermediate 5-2**

1-Bromo-4-cyclohexylbenzene (1 eq, 100 mmol), CuI (3 eq, 300 mmol), ammonia solution (1 eq, 100 mmol), 100 ml of DMF were put in a seal tube, followed by stirring at about 120 °C for about 6 hours. After finishing the reaction, the reaction product was worked up with $H_2O$ and ether, and an organic layer was separated by column chromatography (eluent: methylene chloride/hexane = 1/3 by volume ratio) to obtain 10.5 g of Intermediate 5-2 (yield: 60%, purity ≥ 99.9%).

**Synthesis of Intermediate 5-1**

Intermediate 5-2 (1.2 eq, 60 mmol), (1r,3R,5S)-1-(4-bromophenyl)adamantane (1 eq, 50 mmol), $Pd_2(dba)_3$ (0.03 eq, 1.5 mmol), t-BuONa (3 eq, 150 mmol), t-$Bu_3P$ (0.06 eq, 3 mmol), and 250 ml of toluene were put in an one-neck, round-bottom flask, followed by stirring at about 110 °C for about 2 hours. After finishing the reaction, the reaction product was worked up with $H_2O$ and ether, and an organic layer was separated by column chromatography (eluent: methylene chloride/hexane = 1/5 by volume ratio). The organic layer thus obtained was recrystallized with ether to obtain 20 g of Intermediate 5-1 (yield: 87%, purity ≥ 99.9%).

**Synthesis of Compound 803**

Intermediate 5-1 (1 eq, 52 mmol), 2-bromo-9,9-dimethyl-5-phenyl-9H-fluorene (1.1 eq, 57.2 mmol), $Pd_2(dba)_3$

(0.03 eq, 1.56 mmol), t-BuONa (3 eq, 156 mmol), t-$Bu_3P$ (0.06 eq, 3.12 mmol), and 300 ml of toluene were put in an one-neck, round-bottom flask, followed by stirring at about 110 °C for about 2 hours. After finishing the reaction, the reaction product was worked up with $H_2O$ and ether, and an organic layer was separated by column chromatography (eluent: methylene chloride/hexane = 1/5 by volume ratio). The organic layer thus obtained was recrystallized with ether to obtain 23.7 g of Compound 803 (yield: 70%, purity ≥ 99.9%).

**(12) Synthesis of Compound 808**

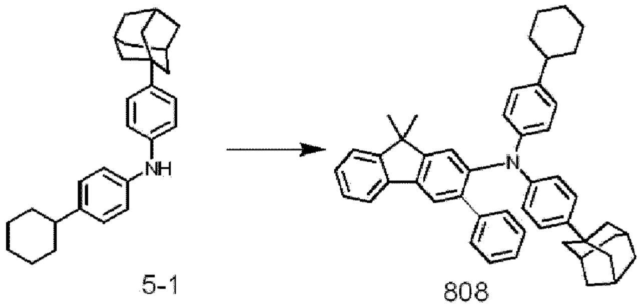

5-1 808

**Synthesis of Compound 808**

Intermediate 5-1 (1 eq, 10 mmol), 2-bromo-9,9-dimethyl-3-phenyl-9H-fluorene (1.1 eq, 11 mmol), $Pd_2(dba)_3$ (0.03 eq, 0.3 mmol), t-BuONa (3 eq, 30 mmol), t-$Bu_3P$ (0.06 eq, 0.6 mmol), and 150 ml of toluene were put in an one-neck, round-bottom flask, followed by stirring at about 110 °C for about 2 hours. After finishing the reaction, the reaction product was worked up with $H_2O$ and ether, and an organic layer was separated by column chromatography (eluent: methylene chloride/hexane = 1/5 by volume ratio). The organic layer thus obtained was recrystallized with ether to obtain 6 g of Compound 808 (yield: 90%, purity ≥ 99.9%).

**(13) Synthesis of Compound 869**

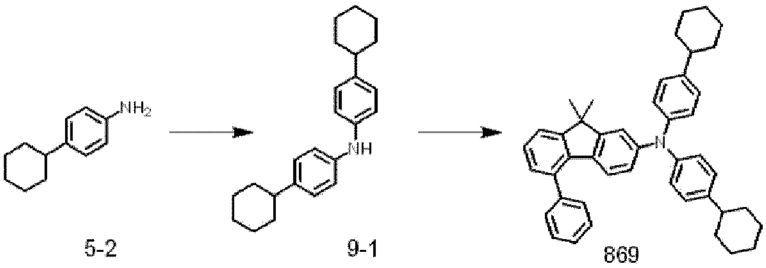

5-2 9-1 869

**Synthesis of Intermediate 9-1**

Intermediate 5-2 (1.2 eq, 30 mmol), 1-Bromo-4-cyclohexylbenzene (1 eq, 25 mmol), $Pd_2(dba)_3$ (0.03 eq, 0.75 mmol), t-BuONa (3 eq, 75 mmol), t-$Bu_3P$ (0.06 eq, 1.5 mmol), and 150 ml of toluene were put in an one-neck, round-bottom flask, followed by stirring at about 110 °C for about 2 hours. After finishing the reaction, the reaction product was worked up with $H_2O$ and ether, and an organic layer was separated by column chromatography (eluent: methylene chloride/hexane = 1/5 by volume ratio). The organic layer thus obtained was recrystallized with ether to obtain 6.8 g of Intermediate 9-1 (yield: 82%, purity ≥ 99.9%).

**Synthesis of Compound 869**

Intermediate 9-1 (1 eq, 20.5 mmol), 2-bromo-9,9-dimethyl-5-phenyl-9H-fluorene (1.1 eq, 22.55 mmol), $Pd_2(dba)_3$ (0.03 eq, 0.6 mmol), t-BuONa (3 eq, 61.5 mmol), t-$Bu_3P$ (0.06 eq, 1.23 mmol), and 150 ml of toluene were put in an one-neck, round-bottom flask, followed by stirring at about 110 °C for about 2 hours. After finishing the reaction,

the reaction product was worked up with $H_2O$ and ether, and an organic layer was separated by column chromatography (eluent: methylene chloride/hexane = 1/5 by volume ratio). The organic layer thus obtained was recrystallized with ether to obtain 12 g of Compound 869 (yield: 88%, purity ≥ 99.9%).

**(14) Synthesis of Compound 872**

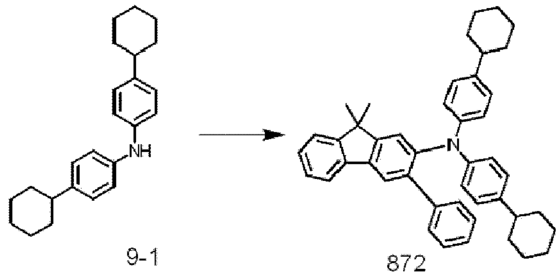

9-1 872

**Synthesis of Compound 872**

Intermediate 9-1 (1 eq, 10 mmol), 2-bromo-9,9-dimethyl-3-phenyl-9H-fluorene (1.1 eq, 11 mmol), $Pd_2(dba)_3$ (0.03 eq, 0.3 mmol), t-BuONa (3 eq, 30 mmol), t-$Bu_3P$ (0.06 eq, 0.6 mmol), and 150 ml of toluene were put in an one-neck, round-bottom flask, followed by stirring at about 110 °C for about 2 hours. After finishing the reaction, the reaction product was worked up with $H_2O$ and ether, and an organic layer was separated by column chromatography (eluent: methylene chloride/hexane = 1/5 by volume ratio). The organic layer thus obtained was recrystallized with ether to obtain 5.3 g of Compound 872 (yield: 80%, purity ≥ 99.9%).

**(15) Synthesis of Compound 1**

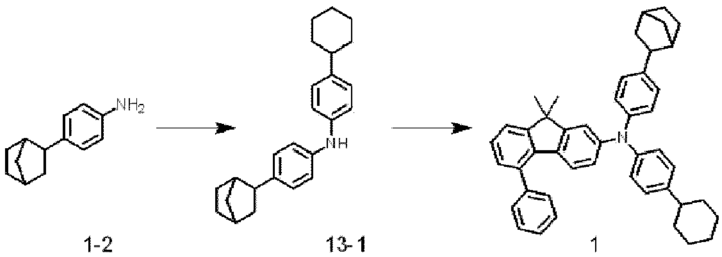

1-2 **13-1** 1

**Synthesis of Intermediate 13-1**

Intermediate 1-2 (1.2 eq, 50 mmol), 1-Bromo-4-cyclohexylbenzene (1 eq, 44 mmol), $Pd_2(dba)_3$ (0.03 eq, 1.5 mmol), t-BuONa (3 eq, 150 mmol), t-$Bu_3P$ (0.06 eq, 3 mmol), and 250 ml of toluene were put in an one-neck, round-bottom flask, followed by stirring at about 110 °C for about 2 hours. After finishing the reaction, the reaction product was worked up with $H_2O$ and ether, and an organic layer was separated by column chromatography (eluent: methylene chloride/hexane = 1/5 by volume ratio). The organic layer thus obtained was recrystallized with ether to obtain 13.8 g of Intermediate 13-1 (yield: 80%, purity ≥ 99.9%).

**Synthesis of Compound 1**

Intermediate 13-1 (1 eq, 40 mmol), 2-bromo-9,9-dimethyl-5-phenyl-9H-fluorene (1.1 eq, 44 mmol), $Pd_2(dba)_3$ (0.03 eq, 1.2 mmol), t-BuONa (3 eq, 120 mmol), t-$Bu_3P$ (0.06 eq, 2.4 mmol), and 250 ml of toluene were put in an one-neck, round-bottom flask, followed by stirring at about 110 °C for about 2 hours. After finishing the reaction, the reaction product was worked up with $H_2O$ and ether, and an organic layer was separated by column chromatography (eluent: methylene chloride/hexane = 1/5 by volume ratio). The organic layer thus obtained was recrystallized with ether to obtain 17.4 g of Compound 1 (yield: 71%, purity ≥ 99.9%).

**(16) Synthesis of Compound 6**

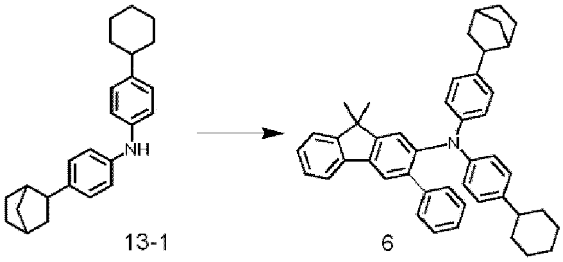

13-1 6

**Synthesis of Compound 6**

Intermediate 13-1 (1 eq, 10 mmol), 2-bromo-9,9-dimethyl-3-phenyl-9H-fluorene (1.1 eq, 11 mmol), $Pd_2(dba)_3$ (0.03 eq, 0.3 mmol), t-BuONa (3 eq, 30 mmol), t-$Bu_3P$ (0.06 eq, 0.6 mmol), and 150 ml of toluene were put in an one-neck, round-bottom flask, followed by stirring at about 110 °C for about 2 hours. After finishing the reaction, the reaction product was worked up with $H_2O$ and ether, and an organic layer was separated by column chromatography (eluent: methylene chloride/hexane = 1/5 by volume ratio). The organic layer thus obtained was recrystallized with ether to obtain 5.3 g of Compound 6 (yield: 85%, purity ≥ 99.9%).

(17) **Synthesis of Compound 873**

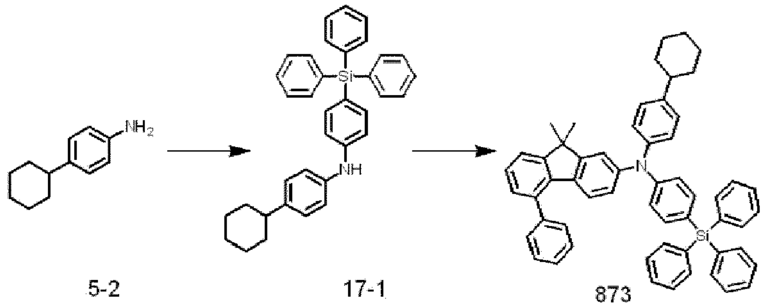

5-2 17-1 873

**Synthesis of Intermediate 17-1**

Intermediate 5-2 (1.2 eq, 30 mmol), (4-bromophenyl)triphenylsilane (1 eq, 25 mmol), $Pd_2(dba)_3$ (0.03 eq, 0.75 mmol), t-BuONa (3 eq, 75 mmol), t-$Bu_3P$ (0.06 eq, 1.5 mmol), and 150 ml of toluene were put in an one-neck, round-bottom flask, followed by stirring at about 110 °C for about 2 hours. After finishing the reaction, the reaction product was worked up with $H_2O$ and ether, and an organic layer was separated by column chromatography (eluent: methylene chloride/hexane = 1/5 by volume ratio). The organic layer thus obtained was recrystallized with ether to obtain 9.8 g of Intermediate 17-1 (yield: 77%, purity ≥ 99.9%).

**Synthesis of Compound 873**

Intermediate 17-1 (1 eq, 19 mmol), 2-bromo-9,9-dimethyl-5-phenyl-9H-fluorene (1.1 eq, 21 mmol), $Pd_2(dba)_3$ (0.03 eq, 0.57 mmol), t-BuONa (3 eq, 57 mmol), t-$Bu_3P$ (0.06 eq, 1.14 mmol), and 250 ml of toluene were put in an one-neck, round-bottom flask, followed by stirring at about 110 °C for about 2 hours. After finishing the reaction, the reaction product was worked up with $H_2O$ and ether, and an organic layer was separated by column chromatography (eluent: methylene chloride/hexane = 1/5 by volume ratio). The organic layer thus obtained was recrystallized with ether to obtain 9.6 g of Compound 873 (yield: 65%, purity ≥ 99.9%).

**(18) Synthesis of Compound 876**

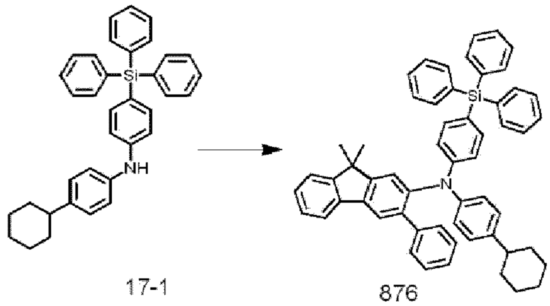

17-1 876

**(Synthesis of Compound 876)**

Intermediate 17-1 (1 eq, 10 mmol), 2-bromo-9,9-dimethyl-3-phenyl-9H-fluorene (1.1 eq, 11 mmol), $Pd_2(dba)_3$ (0.03 eq, 0.3 mmol), t-BuONa (3 eq, 30 mmol), t-$Bu_3P$ (0.06 eq, 0.6 mmol), and 150 ml of toluene were put in an one-neck, round-bottom flask, followed by stirring at about 110 °C for about 2 hours. After finishing the reaction, the reaction product was worked up with $H_2O$ and ether, and an organic layer was separated by column chromatography (eluent: methylene chloride/hexane = 1/5 by volume ratio). The organic layer thus obtained was recrystallized with ether to obtain 5.3 g of Compound 876 (yield: 80%, purity ≥ 99.9%).

**(19) Synthesis of Compound 877**

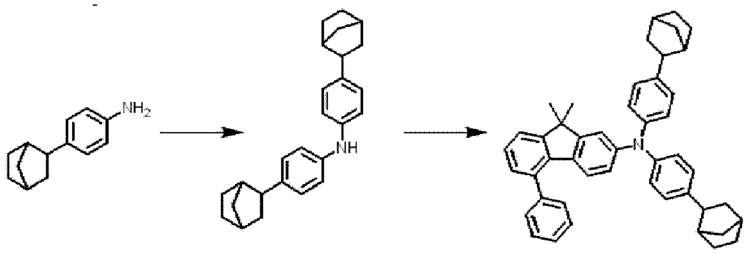

1-2 25-1 877

**(Synthesis of Intermediate 25-1)**

Intermediate 1-2 (1.2 eq, 30 mmol), 2-(4-bromophenyl)bicyclo[2.2.1]heptane (1 eq, 25 mmol), $Pd_2(dba)_3$ (0.03 eq, 0.75 mmol), t-BuONa (3 eq, 75 mmol), t-$Bu_3P$ (0.06 eq, 1.5 mmol), and 150 ml of toluene were put in an one-neck, round-bottom flask, followed by stirring at about 110 °C for about 2 hours. After finishing the reaction, the reaction product was worked up with $H_2O$ and ether, and an organic layer was separated by column chromatography (eluent: methylene chloride/hexane = 1/5 by volume ratio). The organic layer thus obtained was recrystallized with ether to obtain 6.24 g of Intermediate 25-1 (yield: 70%, purity ≥ 99.9%).

**(Synthesis of Compound 877)**

Intermediate 25-1 (1 eq, 17.5 mmol), 2-bromo-9,9-dimethyl-5-phenyl-9H-fluorene (1.1 eq, 19 mmol), $Pd_2(dba)_3$ (0.03 eq, 0.53 mmol), t-BuONa (3 eq, 53 mmol), t-$Bu_3P$ (0.06 eq, 1.05 mmol), and 250 ml of toluene were put in an one-neck, round-bottom flask, followed by stirring at about 110 °C for about 2 hours. After finishing the reaction, the reaction product was worked up with $H_2O$ and ether, and an organic layer was separated by column chromatography (eluent: methylene chloride/hexane = 1/5 by volume ratio). The organic layer thus obtained was recrystallized with ether to obtain 8.2 g of Compound 877 (yield: 75%, purity ≥ 99.9%).

(20) Synthesis of Compound 880

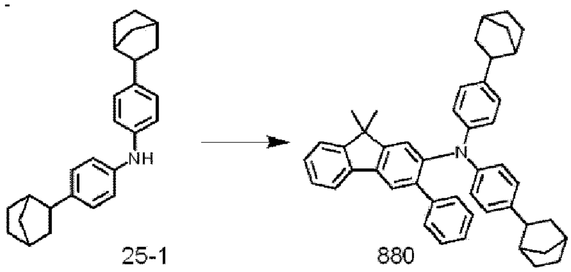

25-1 880

(Synthesis of Compound 880)

Intermediate 25-1 (1 eq, 10 mmol), 2-bromo-9,9-dimethyl-3-phenyl-9H-fluorene (1.1 eq, 11 mmol), $Pd_2(dba)_3$ (0.03 eq, 0.3 mmol), t-BuONa (3 eq, 30 mmol), t-$Bu_3P$ (0.06 eq, 0.6 mmol), and 150 ml of toluene were put in an one-neck, round-bottom flask, followed by stirring at about 110 °C for about 2 hours. After finishing the reaction, the reaction product was worked up with $H_2O$ and ether, and an organic layer was separated by column chromatography (eluent: methylene chloride/hexane = 1/5 by volume ratio). The organic layer thus obtained was recrystallized with ether to obtain 5.6 g of Compound 880 (yield: 90%, purity ≥ 99.9%).

(21) Synthesis of Compound 881

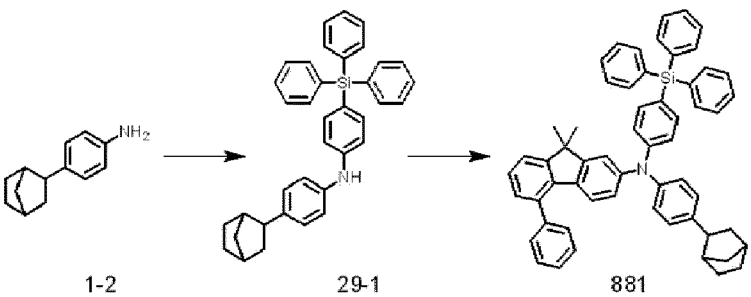

1-2 29-1 881

Synthesis of Intermediate 29-1

Intermediate 1-2 (1.2 eq, 30 mmol), (4-bromophenyl)triphenylsilane (1 eq, 25 mmol), $Pd_2(dba)_3$ (0.03 eq, 0.75 mmol), t-BuONa (3 eq, 75 mmol), t-$Bu_3P$ (0.06 eq, 1.5 mmol), and 150 ml of toluene were put in an one-neck, round-bottom flask, followed by stirring at about 110 °C for about 2 hours. After finishing the reaction, the reaction product was worked up with $H_2O$ and ether, and an organic layer was separated by column chromatography (eluent: methylene chloride/hexane = 1/5 by volume ratio). The organic layer thus obtained was recrystallized with ether to obtain 9.8 g of Intermediate 29-1 (yield: 77%, purity ≥ 99.9%).

Synthesis of Compound 881

Intermediate 29-1 (1 eq, 19 mmol), 2-bromo-9,9-dimethyl-5-phenyl-9H-fluorene (1.1 eq, 21 mmol), $Pd_2(dba)_3$ (0.03 eq, 0.57 mmol), t-BuONa (3 eq, 57 mmol), t-$Bu_3P$ (0.06 eq, 1.14 mmol), and 250 ml of toluene were put in an one-neck, round-bottom flask, followed by stirring at about 110 °C for about 2 hours. After finishing the reaction, the reaction product was worked up with $H_2O$ and ether, and an organic layer was separated by column chromatography (eluent: methylene chloride/hexane = 1/5 by volume ratio). The organic layer thus obtained was recrystallized with ether to obtain 9.6 g of Compound 881 (yield: 65%, purity ≥ 99.9%).

(22) Synthesis of Compound 884

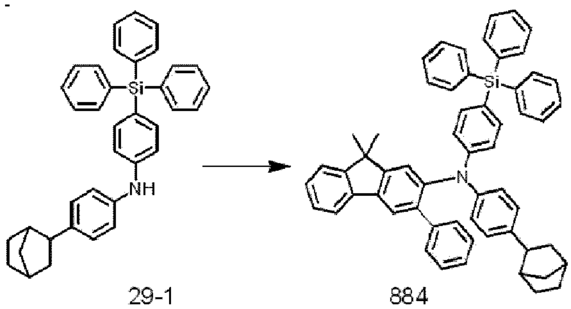

29-1 884

**Synthesis of Compound 884**

Intermediate 29-1 (1 eq, 10 mmol), 2-bromo-9,9-dimethyl-3-phenyl-9H-fluorene (1.1 eq, 11 mmol), $Pd_2(dba)_3$ (0.03 eq, 0.3 mmol), t-BuONa (3 eq, 30 mmol), t-$Bu_3P$ (0.06 eq, 0.6 mmol), and 150 ml of toluene were put in an one-neck, round-bottom flask, followed by stirring at about 110 °C for about 2 hours. After finishing the reaction, the reaction product was worked up with $H_2O$ and ether, and an organic layer was separated by column chromatography (eluent: methylene chloride/hexane = 1/5 by volume ratio). The organic layer thus obtained was recrystallized with ether to obtain 5.3 g of Compound 884 (yield: 80%, purity ≥ 99.9%).

**(23) Synthesis of Compound 995**

Amine Compound 995 may be synthesized by, for example, the reaction below.

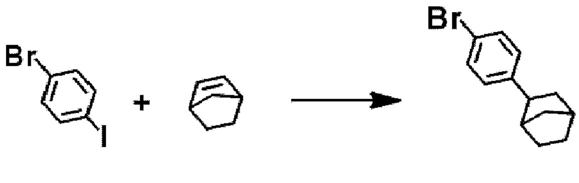

**A**

**Synthesis of Intermediate A**

1-bromo-4-iodobenzene (12.9 g, 100 mmol), bicyclo[2,2,1]hept-2-ene (23.3 g), CuI (19.5 g, 100 mmol), and $K_2CO_3$ (25.8 g, 200 mmol) were added to 200 ml of a DMF solution, followed by stirring at about 150 °C for about 96 hours. After finishing the reaction, the temperature was reduced to room temperature, and extraction with ethyl acetate/$H_2O$ was performed three times. After drying over anhydrous magnesium sulfate and separating by column chromatography with a mixture solvent of methylene chloride: hexane = 1: 10, 18.8 g (yield 80% of Intermediate A was obtained.

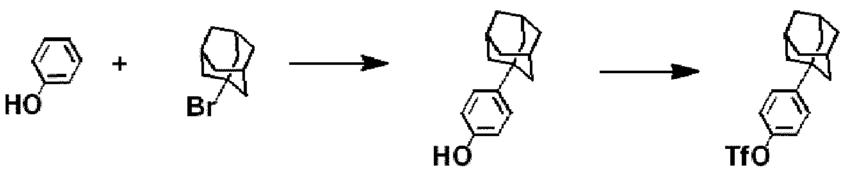

**B-1** **B**

**Synthesis of Intermediate B-1**

To 100 ml of phenol, 1-bromoadamantan (21.4 g, 100 mmol) was added and stirred at about 110 °C for about 24 hours. After finishing the reaction, the solid thus obtained was washed with $H_2O$ at about 60 °C three times. The solid was dissolved in methylene chloride, dried over anhydrous magnesium sulfate to obtain Intermediate B-1 (22 g, yield

100%).

**Synthesis of Intermediate B**

22 g of Intermediate B-1 and 10 g of $Et_3N$ were dissolved in methylene chloride, and the temperature of the solution was reduced to about 0 °C. 50 g of trifluoromethanesulfonic acid was added thereto for about 1 hour. Then, the temperature was raised to room temperature, and stirring was performed for about 4 hours. After finishing the reaction, the solid thus obtained was washed with $Et_2O/H_2O$ at about 60 °C three times. The resultant product was dried over anhydrous magnesium sulfate and separated and purified by column chromatography to obtain Intermediate B (33 g, yield 90%).

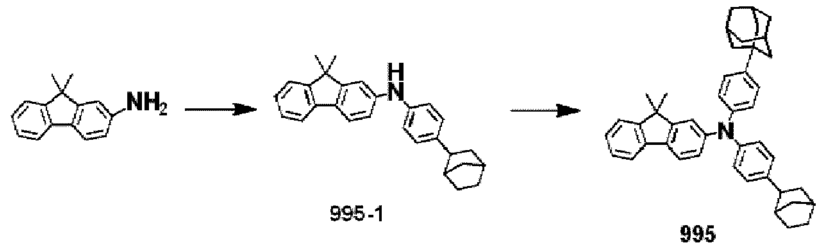

995-1

995

**Synthesis of Intermediate 995-1**

9,9-Dimethyl-9H-fluoren-2-amine (4.2 g, 20 mmol), Intermediate A (5 g, 20 mmol), $Pd_2(dba)_3$ (0.915 g, 1 mol), Sphos (0.410 g, 1 ml) and $NaO^tBu$ (3.6 g, 40 mmol) were dissolved in toluene (200 ml) and stirred at about 90 °C for about 2 hours. The resultant product was extracted with $Et_2O/H_2O$ three times. The resultant product was dried over anhydrous magnesium sulfate and separated and purified by column chromatography to obtain 6.8 g (18 mmol) of Intermediate 995-1 in a yield of 90%.

**Synthesis of Compound 995**

Compound 995 (5.3 g, 9 mmol) was obtained in a yield of 90% by using substantially the same method as the synthesis of Intermediate 995-1, except for using Intermediate 995-1 instead of 9,9-dimethyl-9H-fluoren-2-amine and using Intermediate B instead of Intermediate A.

Through confirming the molecular weight and NMR results as follows, Compound 995 was identified. [$C_{44}H_{47}N$ M+1: 590.45, $^1H$ NMR (500 MHz, $CDCl_3$) $\delta$= 7.80 (m, 2H), 7.60 (d, 1H), 7.55-7.10(m, 12H), 2.5-0.9 (m, 32H)]

**(24) Synthesis of Compound 996**

Amine Compound 996 according to an embodiment may be synthesized by, for example, the reaction below.

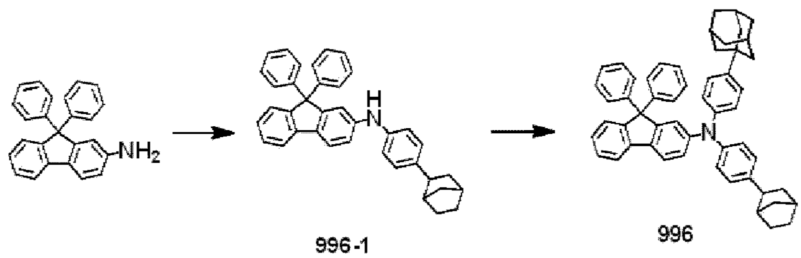

996-1

996

**Synthesis of Intermediate 996-1**

Intermediate 996-1 (9 g, 18 mmol) was obtained in a yield of 90% by using substantially the same method as the synthesis of Intermediate 995-1, except for using 9,9-diphenyl-9H-fluoren-2-amine instead of 9,9-dimethyl-9H-fluoren-2-amine.

**Synthesis of Compound 996**

Compound 996 (6.4 g, 9 mmol) was obtained in a yield of 90% by using substantially the same method as the synthesis of Compound 995, except for using Intermediate 996-1 instead of Intermediate 995-1.

Through confirming the molecular weight and NMR results as follows, Compound 996 was identified. [$C_{54}H_{51}N$ M+1: 714.55, $^1$H NMR (500 MHz, $CDCl_3$) δ= 7.80 (m, 2H), 7.60 (d, 1H), 7.55-7.10(m, 22H), 2.5-1.5 (m, 26H)]

**(25) Synthesis of Compound 1024**

Amine Compound 1024 according to an embodiment may be synthesized by, for example, the reaction below.

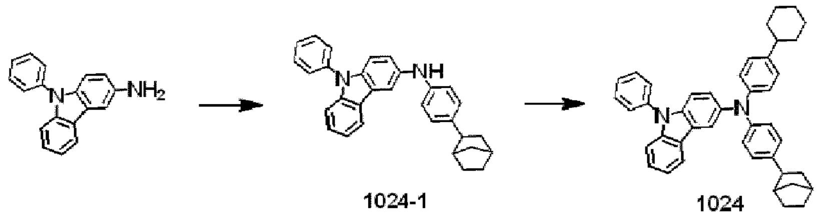

1024-1 1024

**Synthesis of Intermediate 1024-1**

Intermediate 1024-1 (7.7 g, 18 mmol) was obtained in a yield of 90% by substantially using the same method as the synthesis of Intermediate 995-1, except for using 9-phenyl-9H-carbazol-3-amine instead of 9,9-dimethyl-9H-fluoren-2-amine.

**Synthesis of Compound 1024**

Compound 1024 (5.2 g, 9 mmol) was obtained in a yield of 90% by using substantially the same method as the synthesis of Compound 995, except for using Intermediate 1024-1 instead of Intermediate 995-1 and using 1-bromo-4-cyclohexylbenzene instead of Intermediate B.

Through confirming the molecular weight and NMR results as follows, Compound 1024 was identified. [$C_{43}H_{42}N_2$ M+1: 587.33, $^1$H NMR (500 MHz, $CDCl_3$) δ= 7.80 (m, 2H), 7.60 (d, 1H), 7.55-7.10(m, 17H), 2.5-1.5 (m, 22H)]

**(26) Synthesis of Compound 1035**

Amine Compound 1035 according to an embodiment may be synthesized by, for example, the reaction below.

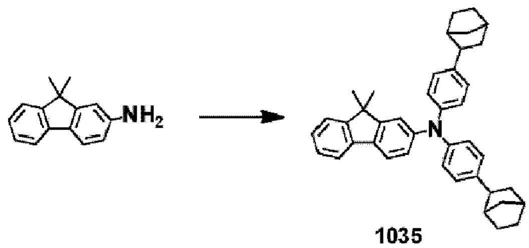

1035

Compound 1035 (5.2 g, 9 mmol) was obtained in a yield of 90% by using substantially the same method as the synthesis of Intermediate 995-1, except for using 9,9-dimethyl-9H-fluoren-2-amine (2.1 g, 10 mmol) instead of 9,9-dimethyl-9H-fluoren-2-amine (4.2 g, 20 mmol).

Through confirming the molecular weight and NMR results as follows, Compound 1035 was identified. [$C_{41}H_{43}N$ M+1: 550.52, $^1$H NMR (500 MHz, $CDCl_3$) δ= 7.80 (m, 2H), 7.60 (d, 1H), 7.55-7.10(m, 12H), 2.5-1.5 (m, 22H), 1.3 (d, 6H)]

**(27) Synthesis of Compound 1044**

Amine Compound 1044 according to an embodiment may be synthesized by, for example, the reaction below.

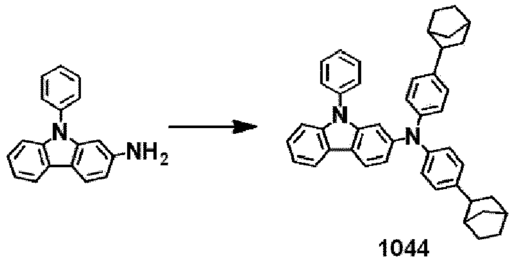

1044

Compound 1044 (5.38 g, 9 mmol) was obtained in a yield of 90% by using substantially the same method as the synthesis of Compound 995, except for using 9-phenyl-9H-carbazol-2-amine (2.6 g, 10 mmol) instead of 9,9-dimethyl-9H-fluoren-2-amine.

Through confirming the molecular weight and NMR results as follows, Compound 1044 was identified. [$C_{44}H_{42}N_2$ M+1: 599.22, $^1$H NMR (500 MHz, $CDCl_3$) $\delta$= 7.80 (m, 2H), 7.60 (d, 1H), 7.55-7.10(m, 17H), 2.5-1.5 (m, 22H)]

**(28) Synthesis of Compound 1059**

Amine Compound 1059 according to an embodiment may be synthesized by, for example, the reaction below.

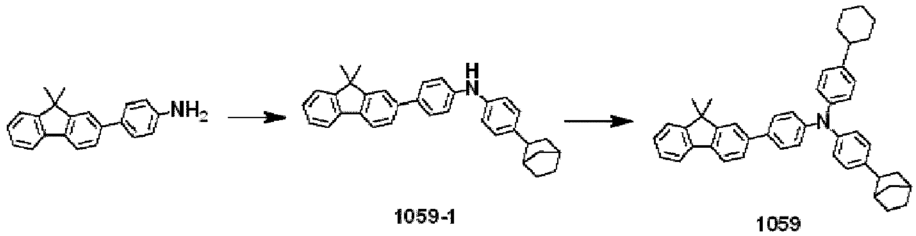

1059-1 1059

**Synthesis of Intermediate 1059-1**

Intermediate 1059-1 (8.19 g, 18 mmol) was obtained in a yield of 90% by using substantially the same method as the synthesis of Intermediate 995-1, except for using 4-(9,9-dimethyl-9H-fluoren-2-yl)aniline instead of 9,9-dimethyl-9H-fluoren-2-amine.

**Synthesis of Compound 1059**

Compound 1059 (5.5 g, 9 mmol) was obtained in a yield of 90% by using substantially the same method as the synthesis of Compound 995, except for using Intermediate 1059-1 instead of Intermediate 995-1 and using 1-bromo-4-cyclohexylbenzene instead of Intermediate B.

Through confirming the molecular weight and NMR results as follows, Compound 1059 was identified. [$C_{46}H_{47}N$ M+1: 614.44, $^1$H NMR (500 MHz, $CDCl_3$) $\delta$= 7.80 (m, 2H), 7.60 (d, 1H), 7.55-7.10(m, 16H), 2.5-1.5 (m, 22H), 1.3 (d, 6H)]

**2. Manufacture and evaluation of light emitting devices including amine compounds**

**Manufacture of light emitting devices**

A light emitting device of an embodiment, including an amine compound of an embodiment in a hole transport layer was manufactured by a method below. Light emitting devices of Examples 1 to 34 were manufactured using the amine compounds of Compounds 1, 6, 15, 28, 36, 43, 49, 145, 707, 803, 808, 851, 865, 868, 869, 872, 873, 876, 877, 880, 881, 884, 995, 996, 1024, 1035, 1044, and 1059, which are the aforementioned Example Compounds, as materials of a hole transport layer. Comparative Example 1 to Comparative Example 14 correspond to light emitting devices manufactured using Comparative Compound C1 to Comparative Compound C11 as materials of a hole transport layer.

**Example Compounds**

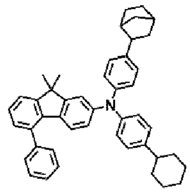
1

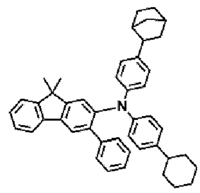
6

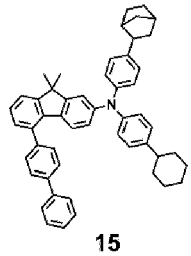
15

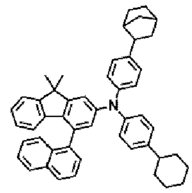
28

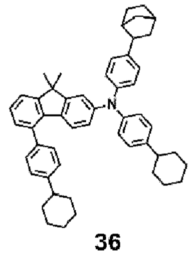
36

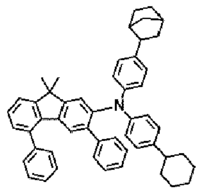
43

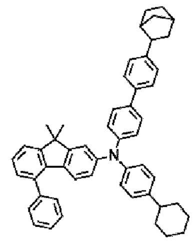
49

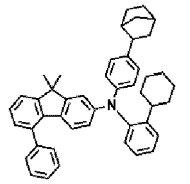
145

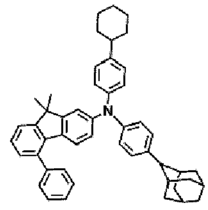
707

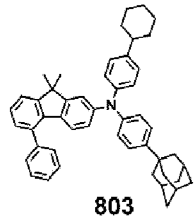
803

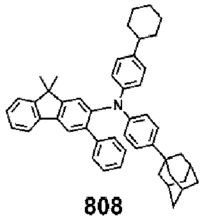
808

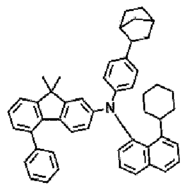
851

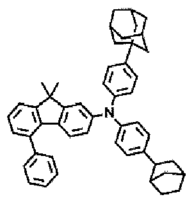
865

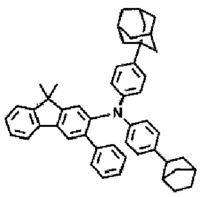
868

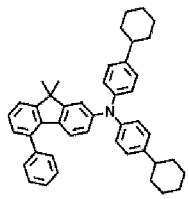
869

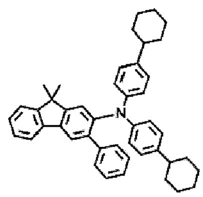
872

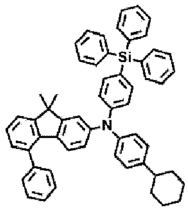
873

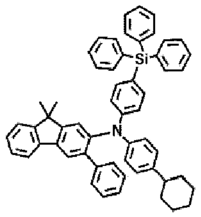
876

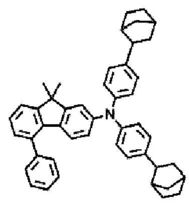
877

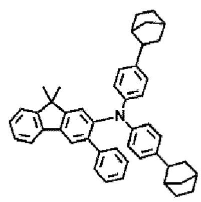
880

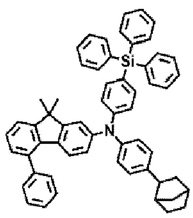
881

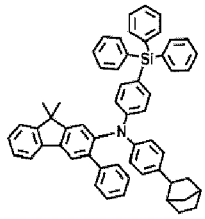
884

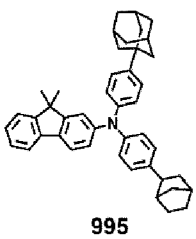
995

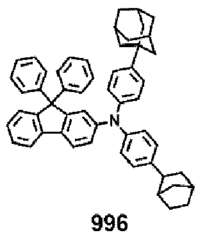
996

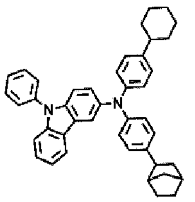
1024

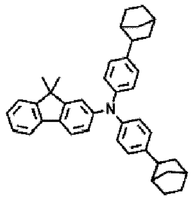
1035

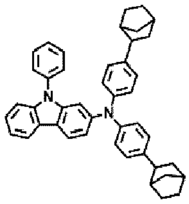
1044

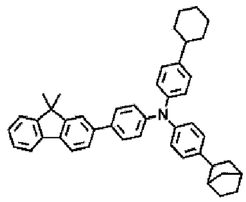
1059

Comparative Compound C1 to Comparative Compound C11 below were used for the manufacture of the devices of Comparative Example 1 to Comparative Example 14.

**Comparative Compounds**

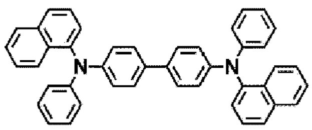
C1

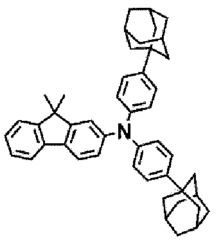
C2

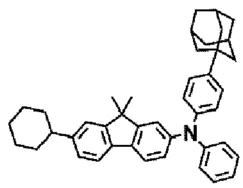
C3

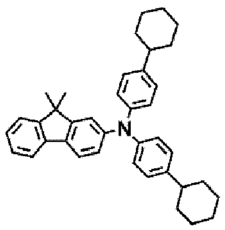
C4

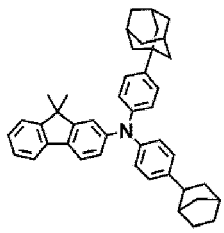
C5

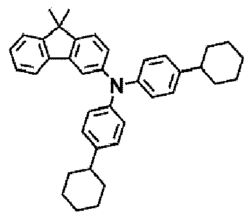
C6

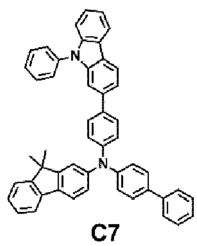
C7

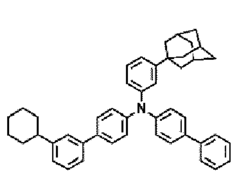
C8

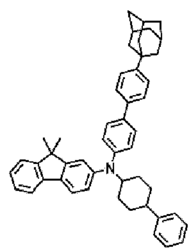
C9

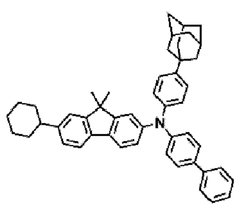
C10

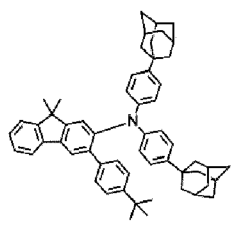
C11

### Example 1

An ITO glass substrate having a sheet resistance of 15 Ω/cm² (1200 Å) from Corning Co. was cut into a size of 50 mm x 50 mm x 0.7 mm, and washed with isopropyl alcohol and ultrapure water, cleaned with ultrasonic waves for about 5 minutes, exposed to UV for about 30 minutes and treated with ozone. Then, 4,4',4"-tris{N,-(2-naphthyl)-N-phenylamino}-triphenylamine (2-TNATA) was vacuum deposited to a thickness of about 600 Å to form a hole injection layer. After that, Example Compound 1 was vacuum deposited to a thickness of about 300 Å to form a hole transport layer.

On the hole transport layer, blue fluorescence hosts of 9,10-di(naphthalen-2-yl)anthracene (DNA) and 4,4'-bis[2-(4-(N,N-diphenylamino)phenyl)vinyl]biphenyl (DPAVBi) were co-deposited in a ratio of 98:2 to form an emission layer having a thickness of about 300 Å.

On the emission layer, an electron transport layer was formed using tris(8-hydroxyquinolino)aluminum ($Alq_3$) to a thickness of about 300 Å, and LiF was deposited to a thickness of about 10 Å to form an electron injection layer. On the electron injection layer, a second electrode having a thickness of about 3,000 Å was formed using aluminum (Al).

The compounds used for the manufacture of the light emitting devices of the Examples and Comparative Examples are shown below.

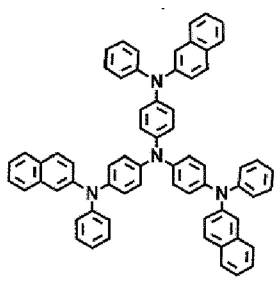

2-TNATA

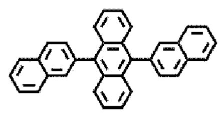

DNA

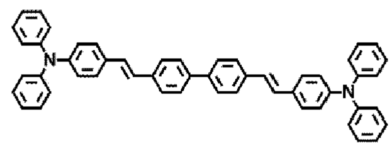

DPAVBi

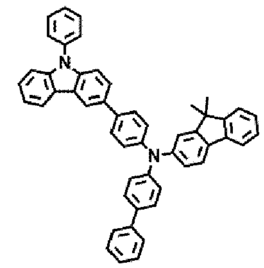

A13

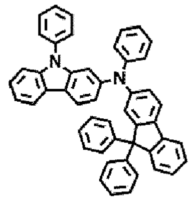

A25

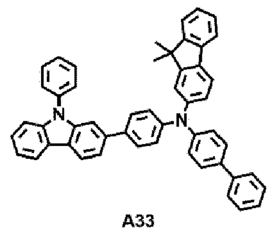

A33

Example 2

A light emitting device was manufactured by substantially the same method as Example 1, except for using Example Compound 6 instead of Example Compound 1 for forming the hole transport layer, when compared to Example 1.

### Example 3

A light emitting device was manufactured by substantially the same method as Example 1, except for using Example Compound 15 instead of Example Compound 1 for forming the hole transport layer, when compared to Example 1.

### Example 4

A light emitting device was manufactured by substantially the same method as Example 1, except for using

Example Compound 28 instead of Example Compound 1 for forming the hole transport layer, when compared to Example 1.

**Example 5**

A light emitting device was manufactured by substantially the same method as Example 1, except for using Example Compound 36 instead of Example Compound 1 for forming the hole transport layer, when compared to Example 1.

**Example 6**

A light emitting device was manufactured by substantially the same method as Example 1, except for using Example Compound 43 instead of Example Compound 1 for forming the hole transport layer, when compared to Example 1.

**Example 7**

A light emitting device was manufactured by substantially the same method as Example 1, except for using Example Compound 49 instead of Example Compound 1 for forming the hole transport layer, when compared to Example 1.

**Example 8**

A light emitting device was manufactured by substantially the same method as Example 1, except for using Example Compound 145 instead of Example Compound 1 for forming the hole transport layer, when compared to Example 1.

**Example 9**

A light emitting device was manufactured by substantially the same method as Example 1, except for using Example Compound 707 instead of Example Compound 1 for forming the hole transport layer, when compared to Example 1.

**Example 10**

A light emitting device was manufactured by substantially the same method as Example 1, except for using Example Compound 851 instead of Example Compound 1 for forming the hole transport layer, when compared to Example 1.

**Example 11**

A light emitting device was manufactured by substantially the same method as Example 1, except for using Example Compound 865 instead of Example Compound 1 for forming the hole transport layer, when compared to Example 1.

**Example 12**

A light emitting device was manufactured by substantially the same method as Example 1, except for using Example Compound 868 instead of Example Compound 1 for forming the hole transport layer, when compared to Example 1.

**Example 13**

A light emitting device was manufactured by substantially the same method as Example 1, except for using Example Compound 803 instead of Example Compound 1 for forming the hole transport layer, when compared to Example 1.

**Example 14**

A light emitting device was manufactured by substantially the same method as Example 1, except for using Example Compound 808 instead of Example Compound 1 for forming the hole transport layer, when compared to Example 1.

**Example 15**

A light emitting device was manufactured by substantially the same method as Example 1, except for using Example Compound 869 instead of Example Compound 1 for forming the hole transport layer, when compared to Example 1.

**Example 16**

A light emitting device was manufactured by substantially the same method as Example 1, except for using Example Compound 876 instead of Example Compound 1 for forming the hole transport layer, when compared to Example 1.

**Example 17**

A light emitting device was manufactured by substantially the same method as Example 1, except for using Example Compound 995 instead of Example Compound 1 for forming the hole transport layer, when compared to Example 1.

**Example 18**

A light emitting device was manufactured by substantially the same method as Example 1, except for using Example Compound 996 instead of Example Compound 1 for forming the hole transport layer, when compared to Example 1.

**Example 19**

A light emitting device was manufactured by substantially the same method as Example 1, except for using Example Compound 1024 instead of Example Compound 1 for forming the hole transport layer, when compared to Example 1.

**Example 20**

A light emitting device was manufactured by substantially the same method as Example 1, except for using Example Compound 1035 instead of Example Compound 1 for forming the hole transport layer, when compared to Example 1.

**Example 21**

A light emitting device was manufactured by substantially the same method as Example 1, except for using Example Compound 1044 instead of Example Compound 1 for forming the hole transport layer, when compared to Example 1.

**Example 22**

A light emitting device was manufactured by substantially the same method as Example 1, except for using Example Compound 1059 instead of Example Compound 1 for forming the hole transport layer, when compared to Example 1.

**Example 23**

An ITO glass substrate having a sheet resistance of 15 Ω/$cm^2$ (1200 Å) from Corning Co. was cut into a size of 50 mm x 50 mm x 0.7 mm, and washed with isopropyl alcohol and ultrapure water, cleaned with ultrasonic waves for

about 5 minutes, exposed to UV for about 30 minutes and treated with ozone. Then, 4,4',4"-tris{N,-(2-naphthyl)-N-phenylamino}-triphenylamine (2-TNATA) was vacuum deposited to a thickness of about 600 Å to form a hole injection layer.

On the hole injection layer, Example Compound 1 was vacuum deposited to a thickness of about 100-400 Å to form a first hole transport layer, Example Compound A13 was vacuum deposited on the first hole transport layer to a thickness of about 100-400 Å to form a second hole transport layer, and Example Compound 1 was vacuum deposited on the second hole transport layer to a thickness of about 100-400 Å to form a third hole transport layer..

On the hole transport layer, blue fluorescence hosts of 9,10-di(naphthalen-2-yl)anthracene (DNA) and 4,4'-bis[2-(4-(N,N-diphenylamino)phenyl)vinyl]biphenyl (DPAVBi) were co-deposited in a ratio of 98:2 to form an emission layer having a thickness of about 300 Å.

On the emission layer, an electron transport layer was formed using tris(8-hydroxyquinolino)aluminum ($Alq_3$) to a thickness of about 300 Å, and LiF was deposited to a thickness of about 10 Å to form an electron injection layer. On the electron injection layer, a second electrode having a thickness of about 3,000 Å was formed using aluminum (Al).

The compounds used for the manufacture of the light emitting devices of the Examples and Comparative Examples are shown below.

### Example 24

A light emitting device was manufactured by substantially the same method as Example 23, except for using Example Compound 6 instead of Example Compound 1 for forming the first hole transport layer and the third hole transport layer, when compared to Example 23.

### Example 25

A light emitting device was manufactured by substantially the same method as Example 23, except for using Example Compound 873 instead of Example Compound 1 for forming the first hole transport layer and the third hole transport layer, when compared to Example 23.

### Example 26

A light emitting device was manufactured by substantially the same method as Example 23, except for using Example Compound 876 instead of Example Compound 1 for forming the first hole transport layer and the third hole transport layer, when compared to Example 23.

### Example 27

A light emitting device was manufactured by substantially the same method as Example 23, except for using Example Compound 877 instead of Example Compound 1 for forming the first hole transport layer and the third hole transport layer, and using Example Compound A25 instead of Example Compound A13 for forming the second hole transport layer, when compared to Example 23.

### Example 28

A light emitting device was manufactured by substantially the same method as Example 23, except for using Example Compound 880 instead of Example Compound 1 for forming the first hole transport layer and the third hole transport layer, and using Example Compound A25 instead of Example Compound A13 for forming the second hole transport layer, when compared to Example 23.

### Example 29

A light emitting device was manufactured by substantially the same method as Example 23, except for using Example Compound 881 instead of Example Compound 1 for forming the first hole transport layer and the third hole transport layer, and using Example Compound A25 instead of Example Compound A13 for forming the second hole transport layer, when compared to Example 23.

### Example 30

A light emitting device was manufactured by substantially the same method as Example 23, except for using

Example Compound 884 instead of Example Compound 1 for forming the first hole transport layer and the third hole transport layer, and using Example Compound A25 instead of Example Compound A13 for forming the second hole transport layer, when compared to Example 23.

**Example 31**

A light emitting device was manufactured by substantially the same method as Example 23, except for using Example Compound A33 instead of Example Compound A13 for forming the second hole transport layer, when compared to Example 23.

**Example 32**

A light emitting device was manufactured by substantially the same method as Example 23, except for using Example Compound 6 instead of Example Compound 1 for forming the first hole transport layer and the third hole transport layer, and using Example Compound A33 instead of Example Compound A13 for forming the second hole transport layer, when compared to Example 23.

**Example 33**

A light emitting device was manufactured by substantially the same method as Example 23, except for using Example Compound 995 instead of Example Compound 1 for forming the first hole transport layer and the third hole transport layer, when compared to Example 23.

**Example 34**

A light emitting device was manufactured by substantially the same method as Example 23, except for using Example Compound 995 instead of Example Compound 1 for forming the first hole transport layer and the third hole transport layer, and using Example Compound A25 instead of Example Compound A13 for forming the second hole transport layer, when compared to Example 23.

**Comparative Example 1**

A light emitting device was manufactured by substantially the same method as Example 1, except for using Comparative Compound C1 instead of Example Compound 1 for forming the hole transport layer, when compared to Example 1.

**Comparative Example 2**

A light emitting device was manufactured by substantially the same method as Example 1, except for using Comparative Compound C2 instead of Example Compound 1 for forming the hole transport layer, when compared to Example 1.

**Comparative Example 3**

A light emitting device was manufactured by substantially the same method as Example 1, except for using Comparative Compound C4 instead of Example Compound 1 for forming the hole transport layer, when compared to Example 1.

**Comparative Example 4**

A light emitting device was manufactured by substantially the same method as Example 1, except for using Comparative Compound C5 instead of Example Compound 1 for forming the hole transport layer, when compared to Example 1.

**Comparative Example 5**

A light emitting device was manufactured by substantially the same method as Example 1, except for using Comparative Compound C7 instead of Example Compound 1 for forming the hole transport layer, when compared to

Example 1.

**Comparative Example 6**

A light emitting device was manufactured by substantially the same method as Example 1, except for using Comparative Compound C8 instead of Example Compound 1 for forming the hole transport layer, when compared to Example 1.

**Comparative Example 7**

A light emitting device was manufactured by substantially the same method as Example 1, except for using Comparative Compound C9 instead of Example Compound 1 for forming the hole transport layer, when compared to Example 1.

**Comparative Example 8**

A light emitting device was manufactured by substantially the same method as Example 1, except for using Comparative Compound C10 instead of Example Compound 1 for forming the hole transport layer, when compared to Example 1.

**Comparative Example 9**

A light emitting device was manufactured by substantially the same method as Example 1, except for using Comparative Compound C11 instead of Example Compound 1 for forming the hole transport layer, when compared to Example 1.

**Comparative Example 10**

A light emitting device was manufactured by substantially the same method as Example 23, except for using Comparative Compound C3 instead of Example Compound 1 for forming the first hole transport layer and the third hole transport layer, when compared to Example 23.

**Comparative Example 11**

A light emitting device was manufactured by substantially the same method as Example 23, except for using Comparative Compound C4 instead of Example Compound 1 for forming the first hole transport layer and the third hole transport layer, when compared to Example 23.

**Comparative Example 12**

A light emitting device was manufactured by substantially the same method as Example 23, except for using Comparative Compound C5 instead of Example Compound 1 for forming the first hole transport layer and the third hole transport layer, when compared to Example 23.

**Comparative Example 13**

A light emitting device was manufactured by substantially the same method as Example 23, except for using Comparative Compound C6 instead of Example Compound 1 for forming the first hole transport layer and the third hole transport layer, when compared to Example 23.

**Comparative Example 14**

A light emitting device was manufactured by substantially the same method as Example 23, except for using Comparative Compound C4 instead of Example Compound 1 for forming the first hole transport layer and the third hole transport layer, and using Example Compound A33 instead of Example Compound A13 for forming the second hole transport layer, when compared to Example 23.

**Evaluation of properties of light emitting device**

In Table 1, the evaluation results of the light emitting devices of Example 1 to Example 22, and Comparative Example 1 to Comparative Example 9 are shown. In Table 2, the evaluation results of the light emitting devices of Example 23 to Example 34, and Comparative Example 10 to Comparative Example 14 are shown. In Table 1 and Table 2, the luminance, emission efficiency and half-life of the light emitting devices manufactured are shown.

In the evaluation results on the properties of the Examples and Comparative Examples, shown in Table 1 and Table 2, the voltage and current density were measured using V7000 OLED IVL Test System, (Polaronix). The emission efficiency shows efficiency values measured at a current density of about 50 mA/cm$^{2-}$. The half-life shows half-life values measured at a current density of about 100 mA/cm$^{2-}$.

Table 1

| Device manufacturing example | Hole transport layer material | Driving voltage (V) | Current density (mA/cm$^2$) | Luminance (cd/m$^2$) | Efficiency (cd/A) | Luminous color | Half life (hr @100 mA/cm$^2$) |
|---|---|---|---|---|---|---|---|
| Example 1 | Compound 1 | 4.75 | 50 | 3500 | 7.0 | Blue | 550 |
| Example 2 | Compound 6 | 4.61 | 50 | 3450 | 6.9 | Blue | 545 |
| Example 3 | Compound 15 | 4.61 | 50 | 3440 | 6.88 | Blue | 575 |
| Example 4 | Compound 28 | 4.50 | 50 | 3400 | 6.8 | Blue | 530 |
| Example 5 | Compound 36 | 4.79 | 50 | 3500 | 7 | Blue | 540 |
| Example 6 | Compound 43 | 4.88 | 50 | 3405 | 6.81 | Blue | 520 |
| Example 7 | Compound 49 | 4.91 | 50 | 3440 | 6.88 | Blue | 545 |
| Example 8 | Compound 145 | 4.38 | 50 | 3485 | 6.97 | Blue | 535 |
| Example 9 | Compound 707 | 4.60 | 50 | 3485 | 6.97 | Blue | 545 |
| Example 10 | Compound 851 | 4.44 | 50 | 3420 | 6.84 | Blue | 535 |
| Example 11 | Compound 865 | 4.32 | 50 | 3775 | 7.55 | Blue | 520 |
| Example 12 | Compound 868 | 4.83 | 50 | 3560 | 7.12 | Blue | 510 |
| Example 13 | Compound 803 | 4.85 | 50 | 3610 | 7.22 | Blue | 510 |
| Example 14 | Compound 808 | 4.81 | 50 | 3765 | 7.53 | Blue | 555 |
| Example 15 | Compound 869 | 4.80 | 50 | 3545 | 7.09 | Blue | 540 |
| Example 16 | Compound 872 | 4.80 | 50 | 3335 | 6.67 | Blue | 560 |
| Example 17 | Compound 995 | 4.75 | 50 | 3075 | 6.15 | Blue | 520 |

(continued)

| Device manufacturing example | Hole transport layer material | Driving voltage (V) | Current density ($mA/cm^2$) | Luminance ($cd/m^2$) | Efficiency (cd/A) | Luminous color | Half life (hr @100 $mA/cm^2$) |
|---|---|---|---|---|---|---|---|
| Example 18 | Compound 996 | 4.72 | 50 | 3060 | 6.12 | Blue | 510 |
| Example 19 | Compound 1024 | 4.75 | 50 | 3010 | 6.02 | Blue | 560 |
| Example 20 | Compound 1035 | 4.73 | 50 | 2965 | 5.93 | Blue | 615 |
| Example 21 | Compound 1044 | 4.72 | 50 | 3045 | 6.09 | Blue | 570 |
| Example 22 | Compound 1059 | 4.72 | 50 | 3115 | 6.23 | Blue | 500 |
| Comparative Example 1 | Comparative Compound C1 | 7.01 | 50 | 2645 | 5.29 | Blue | 258 |
| Comparative Example 2 | Comparative Compound C2 | 6.59 | 50 | 2805 | 5.61 | Blue | 335 |
| Comparative Example 3 | Comparative Compound C4 | 6.42 | 50 | 2770 | 5.54 | Blue | 305 |
| Comparative Example 4 | Comparative Compound C5 | 6.78 | 50 | 2825 | 5.65 | Blue | 340 |
| Comparative Example 5 | Comparative Compound C7 | 6.7 | 50 | 2800 | 5.6 | Blue | 300 |
| Comparative Example 6 | Comparative Compound C8 | 6.8 | 50 | 2845 | 5.69 | Blue | 360 |
| Comparative Example 7 | Comparative Compound C9 | 6.5 | 50 | 2760 | 5.52 | Blue | 355 |
| Comparative Example 8 | Comparative Compound C10 | 6.3 | 50 | 3005 | 6.01 | Blue | 380 |
| Comparative Example 9 | Comparative Compound C11 | 7.3 | 50 | 2895 | 5.79 | Blue | 370 |

Referring to the results of Table 1, it can be seen that the Examples of the light emitting devices using the amine compound according to an embodiment of the present disclosure as the material of a hole transport layer emitted the same blue light, showed very low driving voltage values, and showed relatively higher luminance, emission efficiency and device life when compared to the Comparative Examples. The Example Compounds include a first fused ring combined with the nitrogen atom of an amine group and have a structure essentially including a first substituent and a second substituent, which are substituents selected from an adamantyl group, a cyclohexyl group, a bicycloheptanyl group, a bicyclooctanyl group, and a triphenylsilyl group. The first substituent and the second substituent are connected

with the nitrogen atom of the amine group via linkers, and the linker may be a substituted or unsubstituted arylene group, or a substituted or unsubstituted heteroarylene group, expanding the conjugation structure of the amine group. In addition, the Example Compounds may have a structure including at least one or more third substituents which are aryl groups or heteroaryl groups as substituents in addition to the amine group connected with the first fused ring, or may have a structure in which at least one selected from among the first substituent and the second substituent is a bicycloheptanyl group. The amine compound of an embodiment, having such a structure may show high thermal properties and improved hole transport capacity, and if applied to a light emitting device, the improvement of the emission efficiency and the increase of the life of the light emitting device may be accomplished. In some embodiments, the light emitting device of an embodiment includes the amine compound of an embodiment as the material of a hole transport layer of the light emitting device, and the efficiency and life of the light emitting device may be improved.

In the case of Comparative Compound C1 included in Comparative Example 1, a first fused ring substituted at an amine group was not included, and at least one substituent selected from among an adamantyl group, a cyclohexyl group, a bicycloheptanyl group, a bicyclooctanyl group, and a triphenylsilyl group was not included, and it can be seen that if applied to a light emitting device, luminance and emission efficiency were reduced, and half-life was reduced when compared to the Example Compounds.

The case of Comparative Compound C2 included in Comparative Example 2, had a structure in which an amine group was combined with a fluorenyl group, and an adamantyl group is included as a substituent, but did not include a substituent additionally connected with the fluorenyl group other than the amine group. Accordingly, it can be seen that hole transport capacity and molecular stability were deteriorated, and if applied to a light emitting device, luminance and emission efficiency were deteriorated, and half-life was reduced when compared to the Example Compounds.

When comparing Examples 2 and 5, with Comparative Examples 3 and 4, Comparative Compound C4 and Comparative Compound C5, included in Comparative Examples 3 and 4 had a structure in which a fluorenyl group was combined with an amine group, and had a structure including a cyclohexyl group, an adamantyl group, or a bicycloheptanyl group as a substituent, but it can be seen that emission efficiency was reduced, and half-life was reduced. It is thought that the results were shown because, Comparative Compound C4 and Comparative Compound C5 did not include a substituent additionally connected with the fluorenyl group in addition to the amine group, and showed degraded molecular stability when compared to the Example Compounds, and if applied to a light emitting device, heat resistance and charge transfer properties were degraded when compared to the Examples.

Comparative Compound C7 included in Comparative Example 5 included a structure in which an amine group was connected with a fluorene core, but did not include a first substituent and a second substituent as substituents connected with an amine group, as shown in the present disclosure, and it can be seen that both emission efficiency and device life were reduced when compared to the Examples.

Comparative Compound C8 included in Comparative Example 6 was a compound not including a fluorene core, and had insufficient molecular stability when compared to the Example Compounds, and it can be seen that both emission efficiency and device life were degraded when compared to the Examples.

Comparative Compound C9 included in Comparative Example 7 was a compound in which a cyclohexyl group was directly combined with an amine group, and accordingly, molecular stability was insufficient, and both emission efficiency and device life were degraded when compared to the Examples.

Comparative Compound C10 included in Comparative Example 8 included a structure in which an amine group was connected with a fluorene core but included one adamantyl group as a substituent connected with the amine group, and it can be seen that both emission efficiency and device life were degraded when compared to the Examples. The amine compounds of the Examples of the present disclosure included both a first substituent and a second substituent connected with an amine group and may show improved emission efficiency and life characteristics. In addition, the case of Comparative Compound C10 had a structure in which a cyclohexyl group was connected with a fluorene core, but with such a structure, the expansion of $\pi$-conjugation was difficult, molecular stability was deteriorated during driving, and emission efficiency and device life characteristics were deteriorated.

Comparative Compound C11 included in Comparative Example 9 included a structure in which an amine group was connected with a fluorene core, but included two adamantyl groups as substituents connected with an amine group, and it can be seen that both emission efficiency and device life were reduced when compared to the Examples.

Table 2

| Device manufacturing example | First hole transport layer | Second hole transport layer | Third hole transport layer | Driving voltage (V) | Current density ($mA/cm^2$) | Luminance (cd/m2) | Efficiency (cd/A) | Luminous color | Half life (hr) |
|---|---|---|---|---|---|---|---|---|---|
| Example 23 | Compound 1 | Compound A13 | Compound 1 | 4.56 | 50 | 3300 | 6.60 | Blue | 615 |
| Example 24 | Compound 6 | Compound A13 | Compound 6 | 4.63 | 50 | 3300 | 6.85 | Blue | 630 |
| Example 25 | Compound 873 | Compound A13 | Compound 873 | 4.69 | 50 | 3300 | 7.00 | Blue | 620 |
| Example 26 | Compound 876 | Compound A13 | Compound 876 | 4.69 | 50 | 3300 | 6.95 | Blue | 640 |
| Example 27 | Compound 877 | Compound A25 | Compound 877 | 4.59 | 50 | 3270 | 6.54 | Blue | 610 |
| Example 28 | Compound 880 | Compound A25 | Compound 880 | 4.62 | 50 | 3270 | 6.75 | Blue | 625 |
| Example 29 | Compound 881 | Compound A25 | Compound 881 | 4.66 | 50 | 3270 | 6.50 | Blue | 635 |
| Example 30 | Compound 884 | Compound A25 | Compound 884 | 4.66 | 50 | 3270 | 6.84 | Blue | 630 |
| Example 31 | Compound 1 | Compound A33 | Compound 1 | 4.3 | 50 | 3495 | 6.99 | Blue | 685 |
| Example 32 | Compound 6 | Compound A33 | Compound 6 | 4.5 | 50 | 3490 | 6.98 | Blue | 675 |
| Example 33 | Compound 995 | Compound A13 | Compound 995 | 4.72 | 50 | 3300 | 6.60 | Blue | 580 |
| Example 34 | Compound 995 | Compound A25 | Compound 995 | 4.72 | 50 | 3270 | 6.54 | Blue | 520 |
| Comparative Example 10 | Comparative Compound C3 | Compound A13 | Comparative Compound C3 | 5.66 | 50 | 3002 | 6.00 | Blue | 450 |
| Comparative Example 11 | Comparative Compound C4 | Compound A13 | Comparative Compound C4 | 5.30 | 50 | 2880 | 5.76 | Blue | 385 |
| Comparative Example 12 | Comparative Compound C5 | Compound A13 | Comparative Compound C5 | 5.49 | 50 | 2925 | 5.85 | Blue | 390 |
| Comparative Example 13 | Comparative Compound C6 | Compound A13 | Comparative Compound C6 | 5.60 | 50 | 3000 | 6.00 | Blue | 450 |
| Comparative Example 14 | Comparative Compound C4 | Compound A33 | Comparative Compound C4 | 5.6 | 50 | 3000 | 6.00 | Blue | 305 |

Referring to the results of Table 1 and Table 2, it can be seen that the Examples including multiple hole transport layers showed improved device characteristics of emission efficiency or device life when compared to the Comparative Examples.

The light emitting device of an embodiment may show improved device properties of high efficiency and long-life characteristics.

The amine compound of an embodiment may be included in the hole transport region of a light emitting device to contribute to the increase of the efficiency and life of the light emitting device.

Although the embodiments of the present disclosure have been described, it should be understood that the present disclosure should not be limited to these embodiments but various changes and modifications can be made by one ordinary skilled in the art within the spirit and scope of the appended claims, and equivalents thereof.

**Claims**

**1.** An amine compound represented by the following Formula 1:

Formula 1

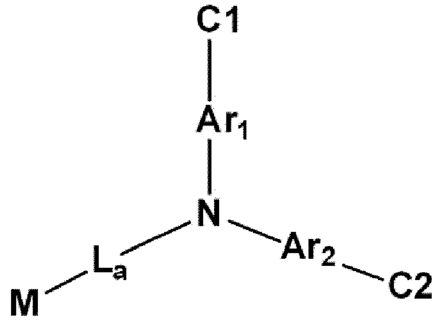

in Formula 1,

An and $Ar_2$ are each independently a substituted or unsubstituted arylene group of 6 to 30 ring-forming carbon atoms, or a substituted or unsubstituted heteroarylene group of 2 to 30 ring-forming carbon atoms,
$L_a$ is a direct linkage, a substituted or unsubstituted arylene group of 6 to 30 ring-forming carbon atoms, or a substituted or unsubstituted heteroarylene group of 2 to 30 ring-forming carbon atoms,
C1 and C2 are each independently a substituted or unsubstituted adamantyl group, a substituted or unsubstituted cyclohexyl group, a substituted or unsubstituted bicycloheptanyl group, a substituted or unsubstituted bicyclooctanyl group, or a substituted or unsubstituted triphenylsilyl group,
with the proviso that C1 and C2 cannot both be substituted or unsubstituted adamantyl groups, and
M is represented by any one selected from among the following Formula 1-a to Formula 1-c:

Formula 1-a

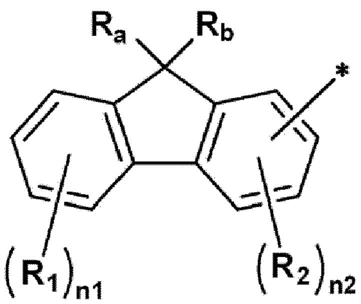

Formula 1-b

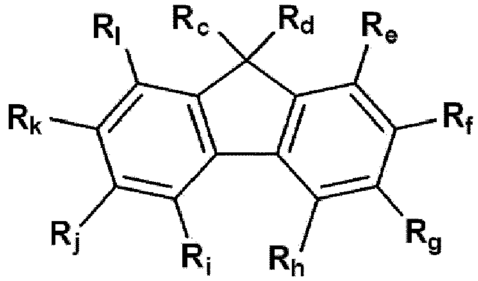

Formula 1-c

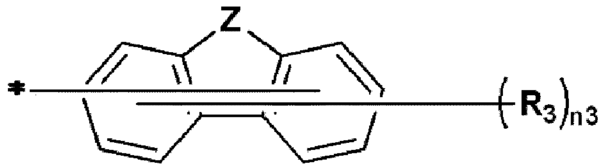

in Formula 1-a to Formula 1-c,

Z is $NR_4$, O, or S,
$R_1$ and $R_2$ are each independently a substituted or unsubstituted aryl group of 6 to 30 ring-forming carbon atoms, or a substituted or unsubstituted heteroaryl group of 2 to 30 ring-forming carbon atoms, and
$R_a$ to $R_l$, $R_3$, and $R_4$ are each independently a hydrogen atom, a deuterium atom, a halogen atom, a substituted or unsubstituted alkyl group of 1 to 20 carbon atoms, a substituted or unsubstituted alkenyl group of 2 to 20 carbon atoms, a substituted or unsubstituted aryl group of 6 to 30 ring-forming carbon atoms, or a substituted or unsubstituted heteroaryl group of 2 to 30 ring-forming carbon atoms, or combined with an adjacent group to form a ring,
in Formula 1-b, any one selected from among $R_c$ to $R_l$ is a part connected with Formula 1,
in Formula 1-a and Formula 1-c, -* is a part connected with Formula 1,
in a case where M in Formula 1 is represented by Formula 1-b or Formula 1-c, at least one selected from among C1 and C2 in Formula 1 is a bicycloheptanyl group,
n1 is an integer of 0 to 4,
n2 is an integer of 0 to 3,
where the sum of n1 and n2 is 1 or more, and
n3 is an integer of 0 to 7.

2. The amine compound of claim 1, wherein the amine compound represented by Formula 1 is represented by any one selected from among the following Formula 2-1 to Formula 2-8:

Formula 2-1

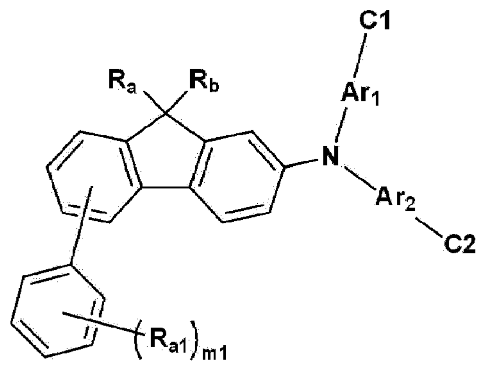

Formula 2-2

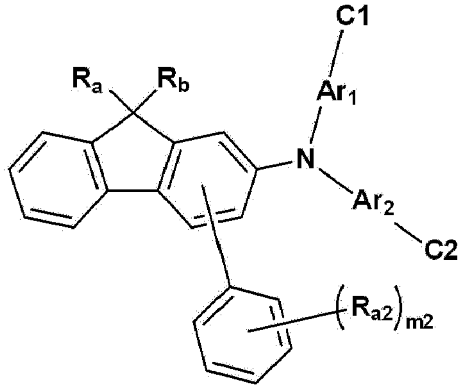

Formula 2-3

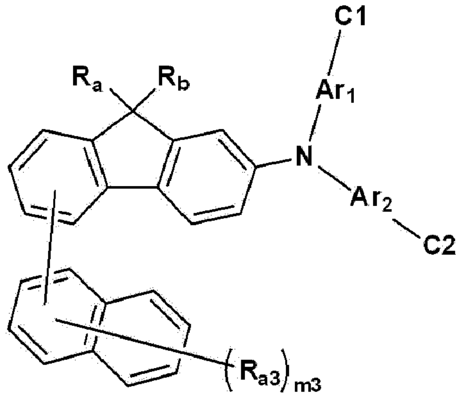

Formula 2-4

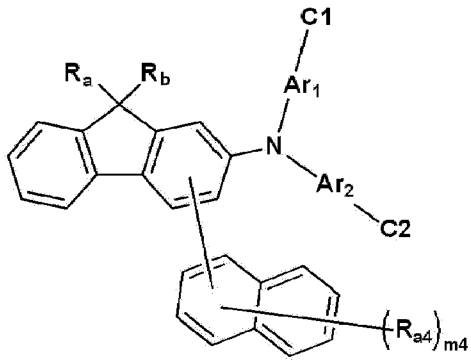

Formula 2-5

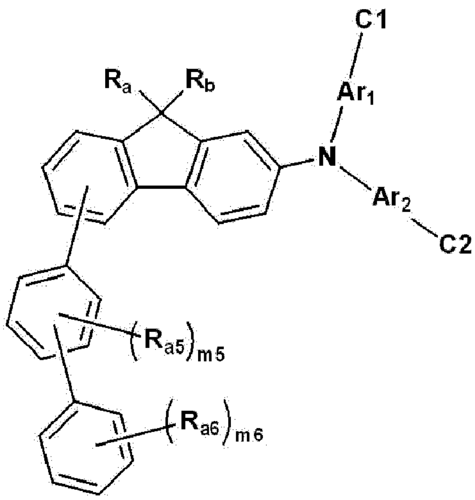

Formula 2-6

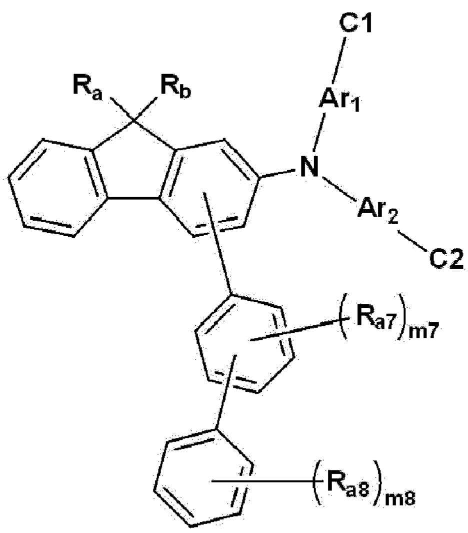

Formula 2-7

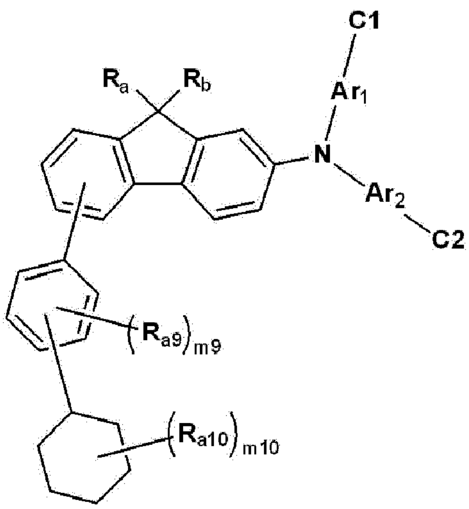

Formula 2-8

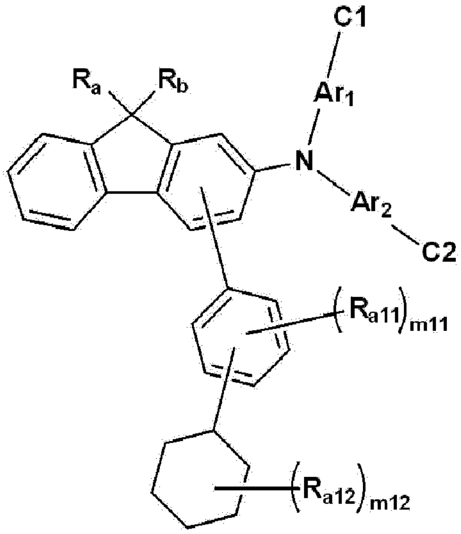

in Formula 2-1 to Formula 2-8,

$R_{a1}$ to $R_{a12}$ are each independently a hydrogen atom, a deuterium atom, a halogen atom, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms,
m1, m2, m6, and m8" are each independently an integer of 0 to 5,
m3 and m4 are each independently an integer of 0 to 7,
m5, m7, m9, and m11 are each independently an integer of 0 to 4,
m10, and m12 are each independently an integer of 0 to 11, and
$R_a$, $R_b$, $Ar_1$, $Ar_2$, C1 and C2 are the same as defined with respect to Formula 1 and Formula 1-a.

**3.** The amine compound of claim 1, wherein the amine compound represented by Formula 1 is represented by the following Formula 3-1 or Formula 3-2:

Formula 3-1

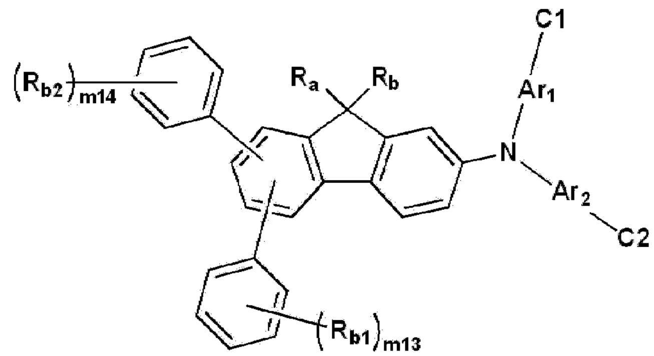

Formula 3-2

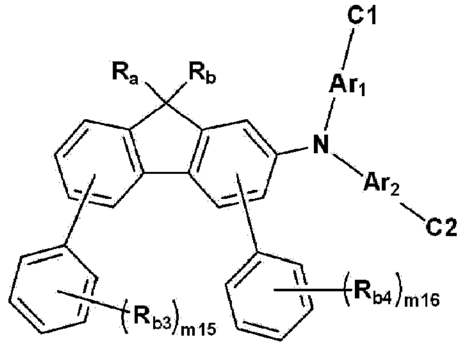

in Formula 3-1 and Formula 3-2,

$R_{b1}$ to $R_{b4}$ are each independently a hydrogen atom, a deuterium atom, a halogen atom, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms,
m13 to m16 are each independently an integer of 0 to 5, and
$R_a$, $R_b$, $Ar_1$, $Ar_2$, C1 and C2 are the same as defined with respect to Formula 1 and Formula 1-a.

**4.** The amine compound of claim 1, wherein the amine compound represented by Formula 1 is represented by any one selected from among the following Formula 5-1 to Formula 5-3:

Formula 5-1

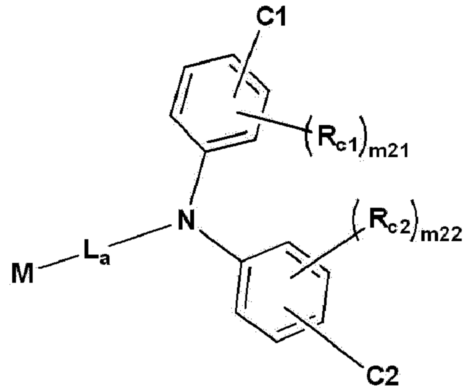

Formula 5-2

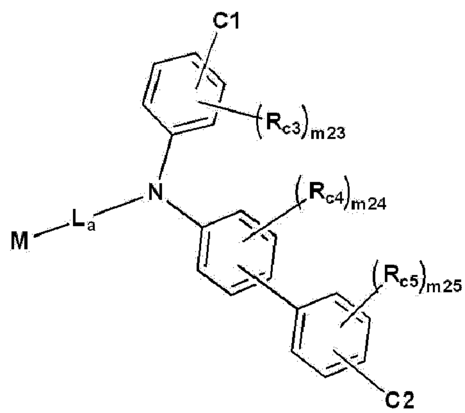

Formula 5-3

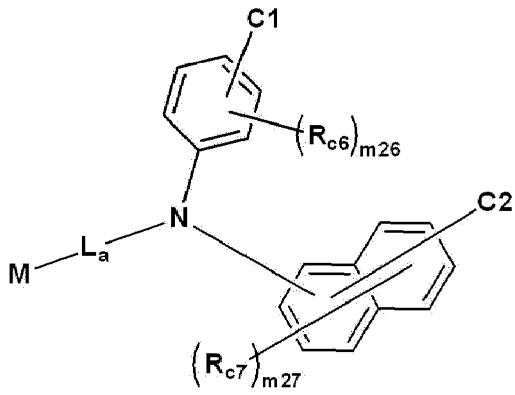

in Formula 5-1 to Formula 5-3,

$R_{c1}$ to $R_{c7}$ are each independently a hydrogen atom, a deuterium atom, a halogen atom, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms,
m21 to m26 are each independently an integer of 0 to 4,
m27 is an integer of 0 to 6, and
M, $L_a$, C1, and C2 are the same as defined with respect to Formula 1.

**5.** The amine compound of claim 1, wherein the amine compound represented by Formula 1 is represented by any one selected from among the following Formula 8-1 to Formula 8-5:

Formula 8-1

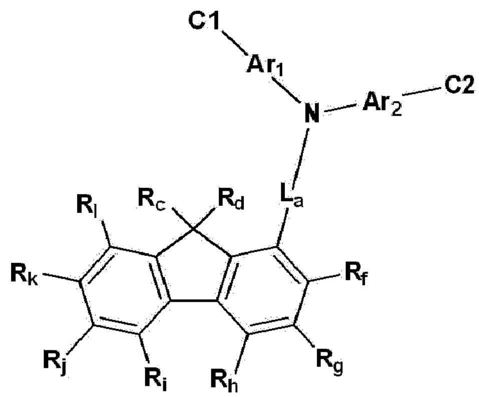

Formula 8-2

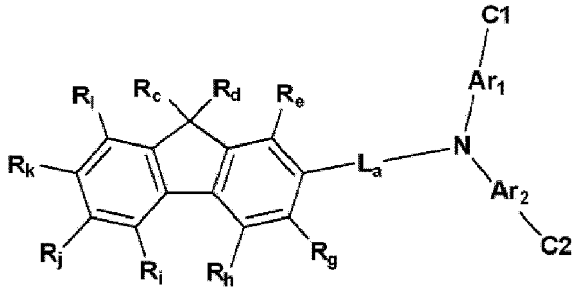

Formula 8-3

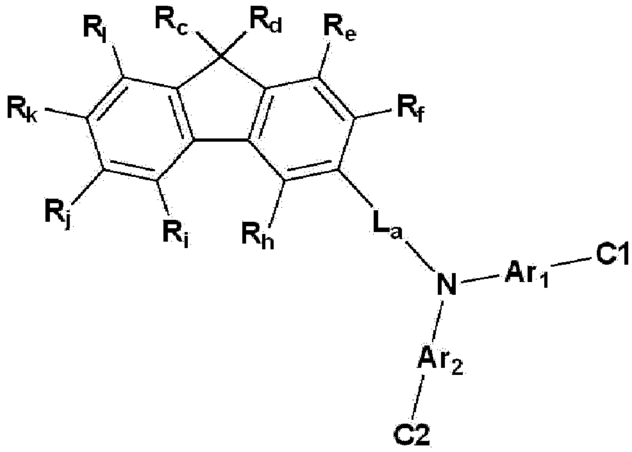

Formula 8-4

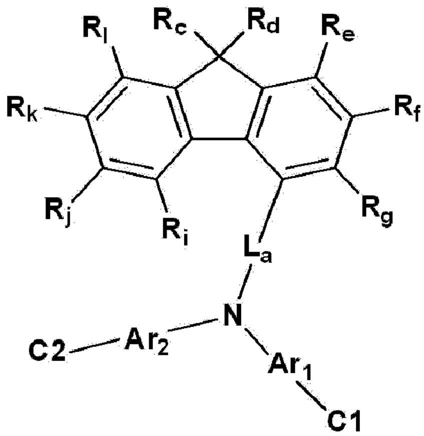

Formula 8-5

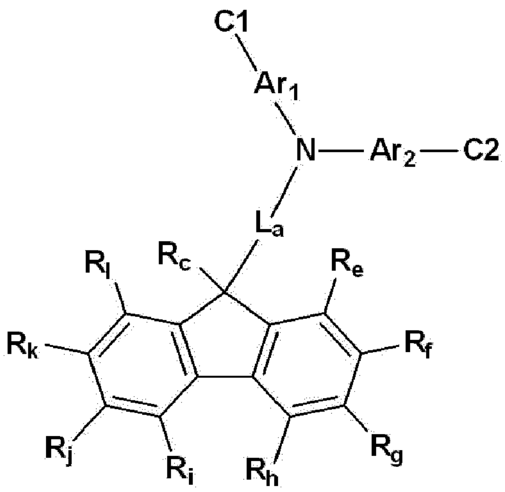

in Formula 8-1 to Formula 8-5,

at least one among C1 and C2 is a substituted or unsubstituted bicycloheptanyl group, and
$Ar_1$, $Ar_2$, $L_a$, C1, C2, and $R_c$ to $R_l$ are the same as defined with respect to Formula 1 and Formula 1-b.

6. The amine compound of claim 1, wherein the amine compound represented by Formula 1 is represented by any one selected from among the following Formula 9-1 to Formula 9-4:

Formula 9-1

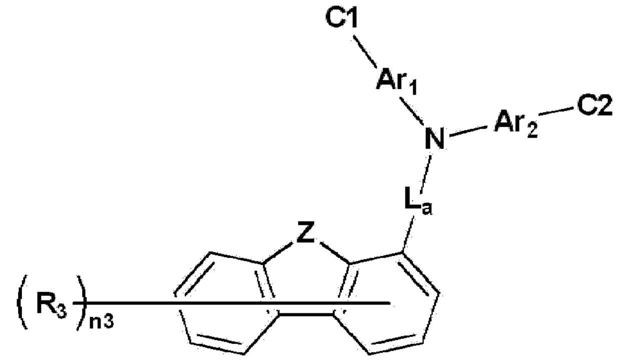

Formula 9-2

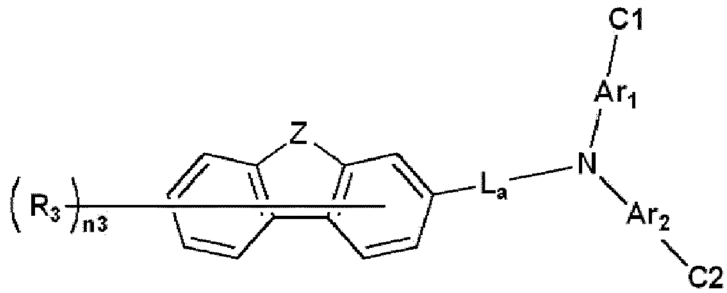

Formula 9-3

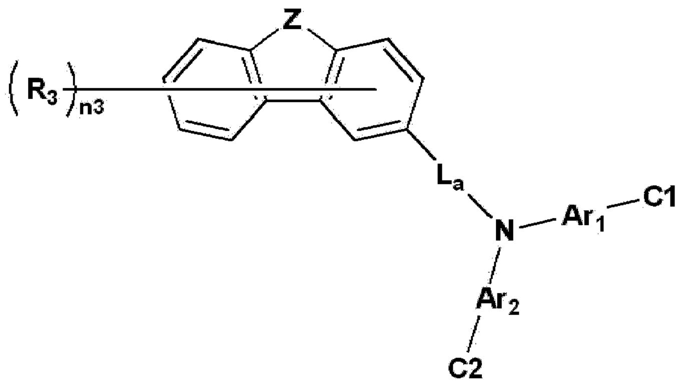

Formula 9-4

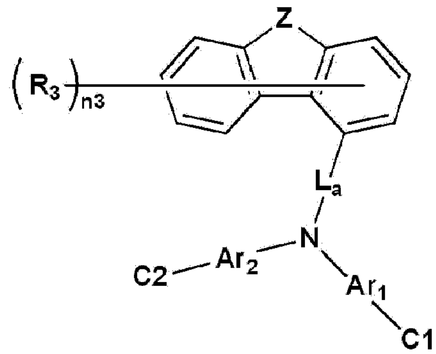

in Formula 9-1 to Formula 9-4,

at least one among C1 and C2 is a substituted or unsubstituted bicycloheptanyl group, and
Z, $R_3$, $Ar_1$, $Ar_2$, $L_a$, n3, C1, and C2 are the same as defined with respect to Formula 1 and Formula 1-c.

**7.** The amine compound of claim 1, wherein the amine compound is at least one selected from compounds in the following Compound Group 1:

Compound Group 1

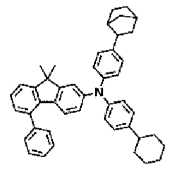
1

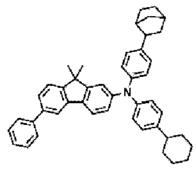
2

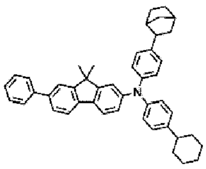
3

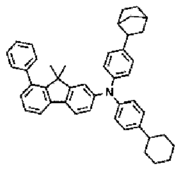
4

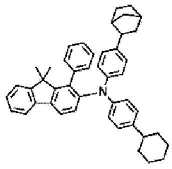
5

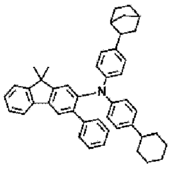
6

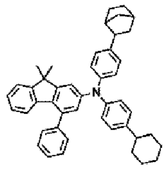
7

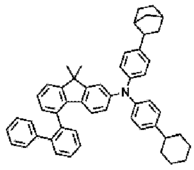
8

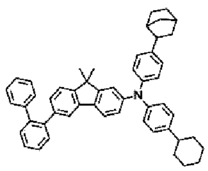
9

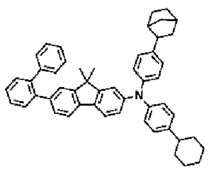
10

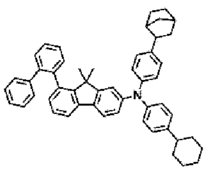
11

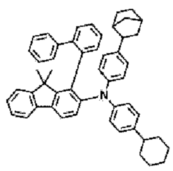
12

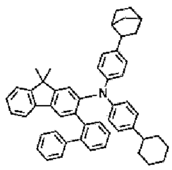
13

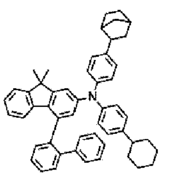
14

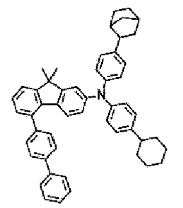
15

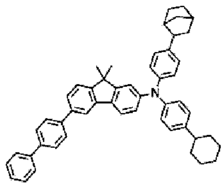
16

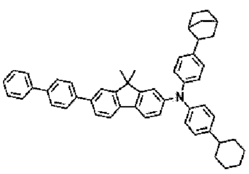
17

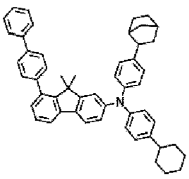
18

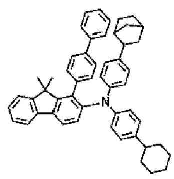
19

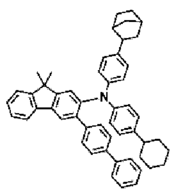
20

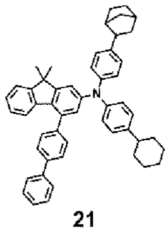 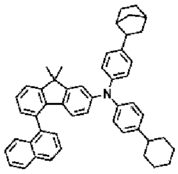 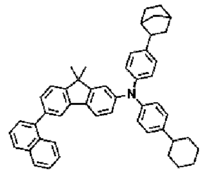 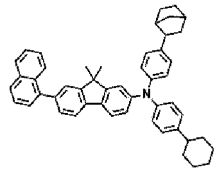

21 22 23 24

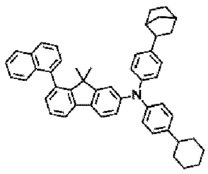 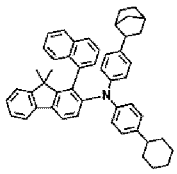 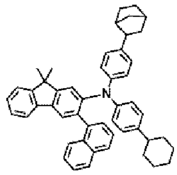 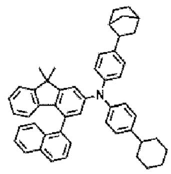

25 26 27 28

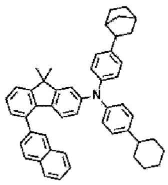 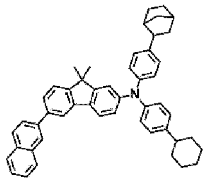 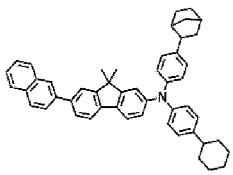 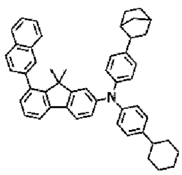

29 30 31 32

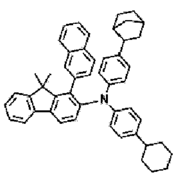 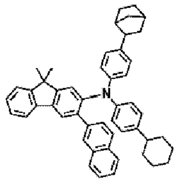 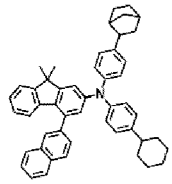 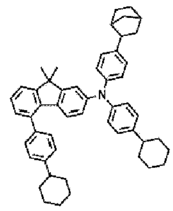

33 34 35 36

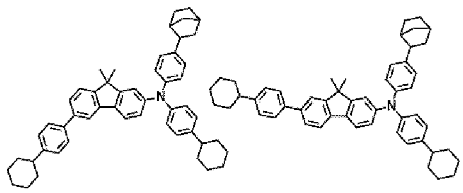 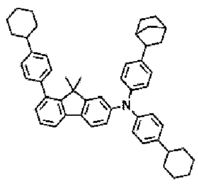 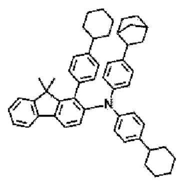

37 38 39 40

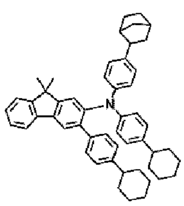
41

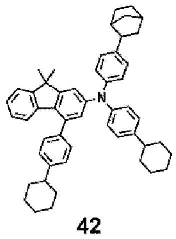
42

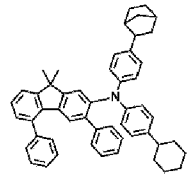
43

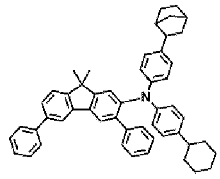
44

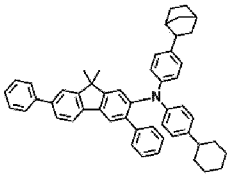
45

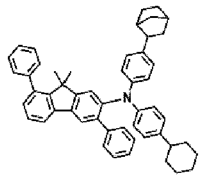
46

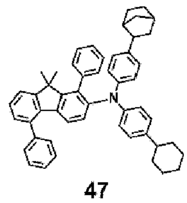
47

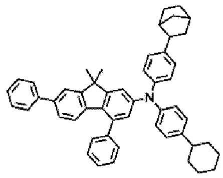
48

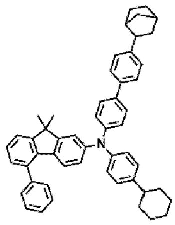
49

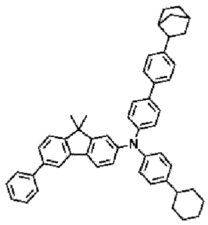
50

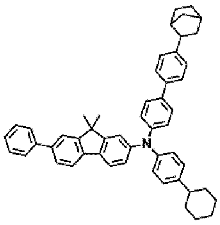
51

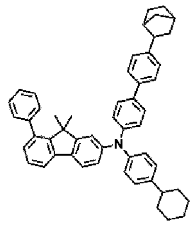
52

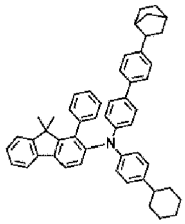
53

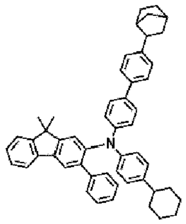
54

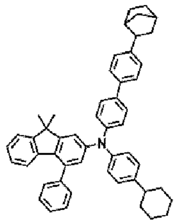
55

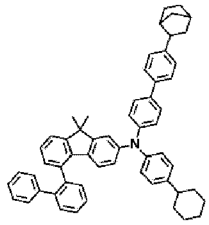
56

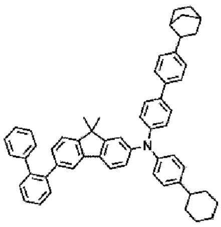
57

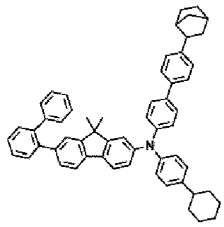
58

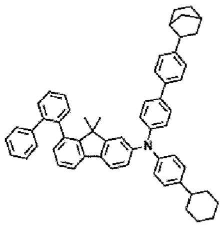
59

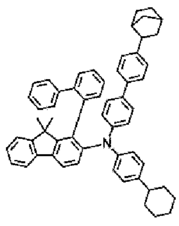

60

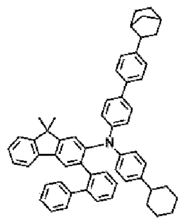

61

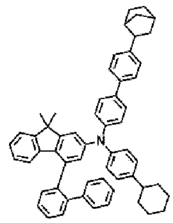

62

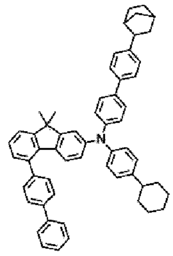

63

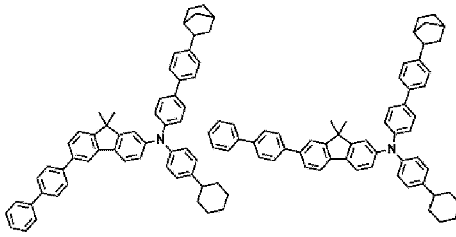

64

65

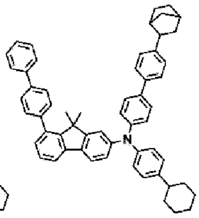

66

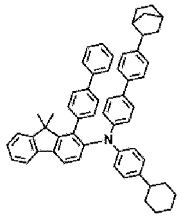

67

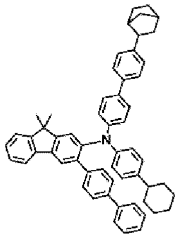

68

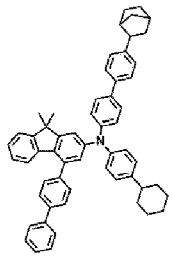

69

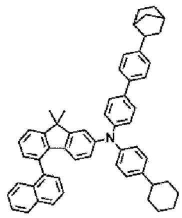

70

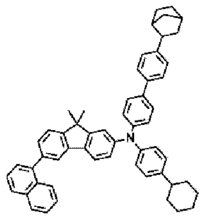

71

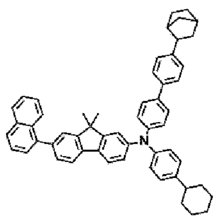

72

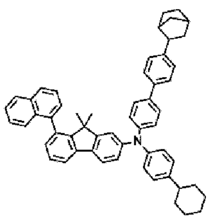

73

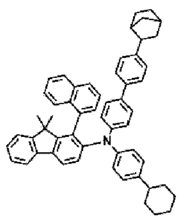

74

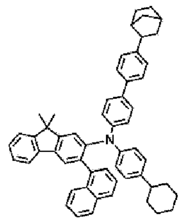

75

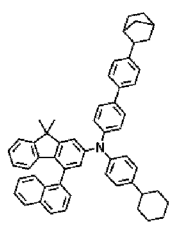

76

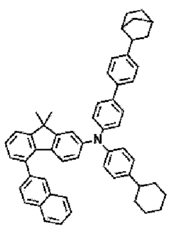

77

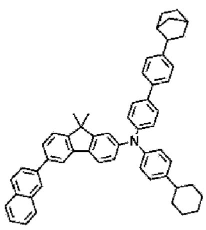

78

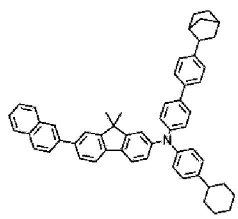

79

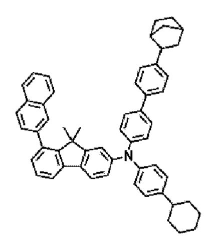
80

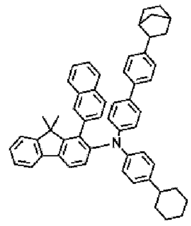
81

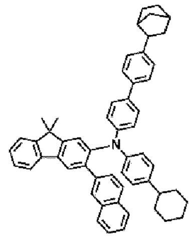
82

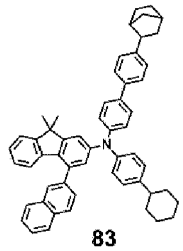
83

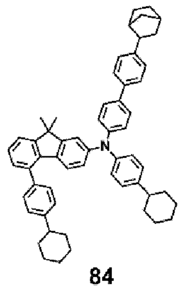
84

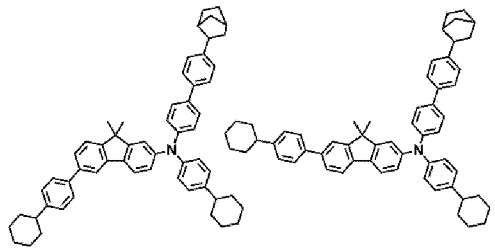
85

86

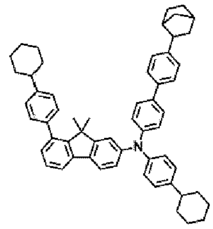
87

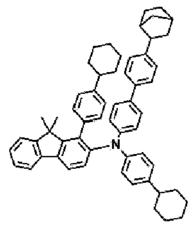
88

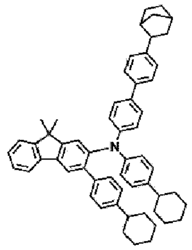
89

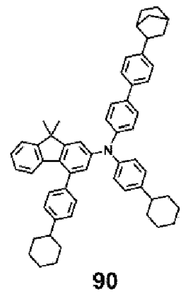
90

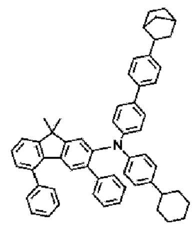
91

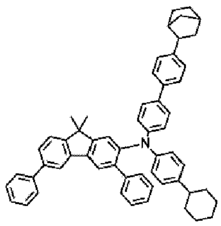
92

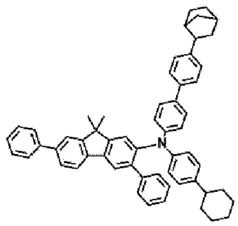
93

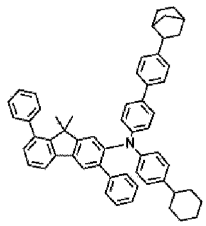
94

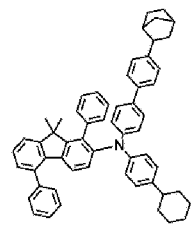
95

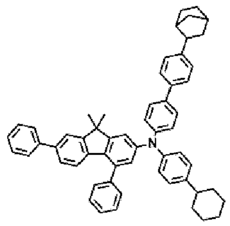
96

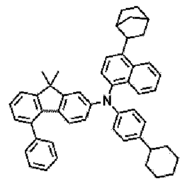
97

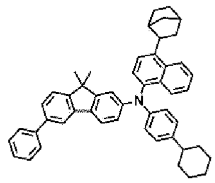
98

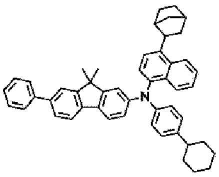
99

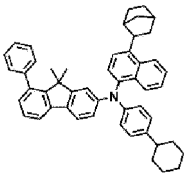
100

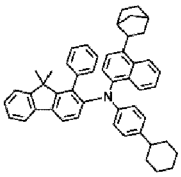
101

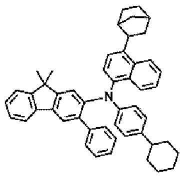
102

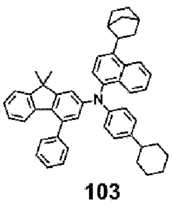
103

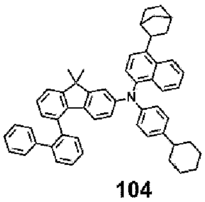
104

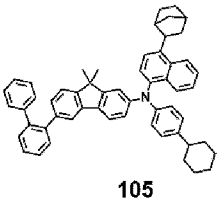
105

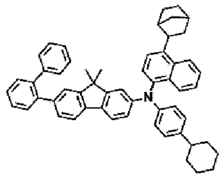
106

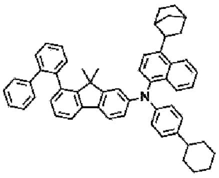
107

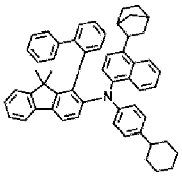
108

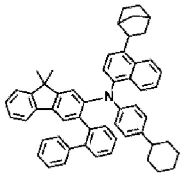
109

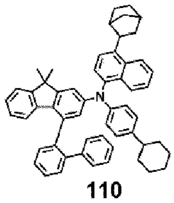
110

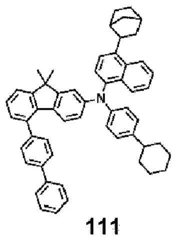
111

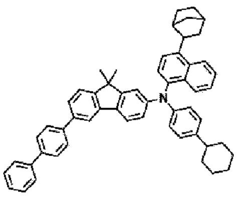
112

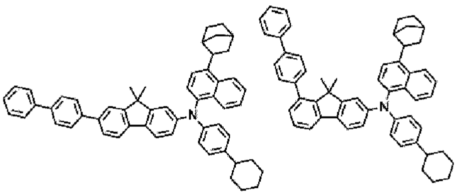
113

114

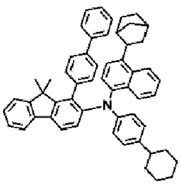
115

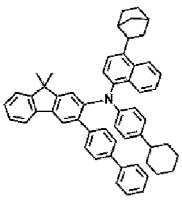
116

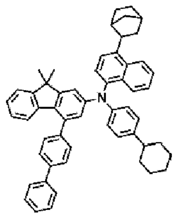
117

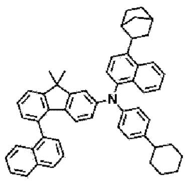
118

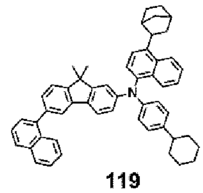
119

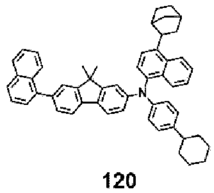
120

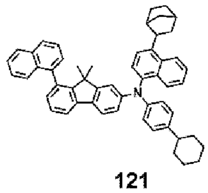
121

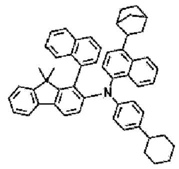
122

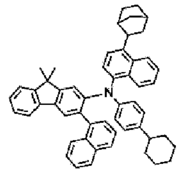
123

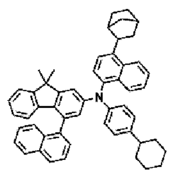
124

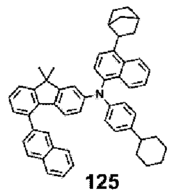
125

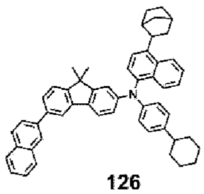
126

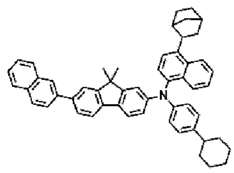
127

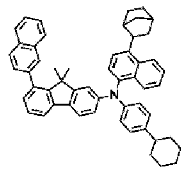
128

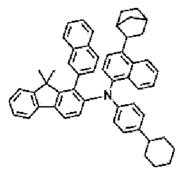
129

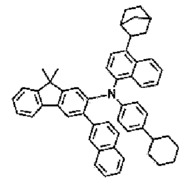
130

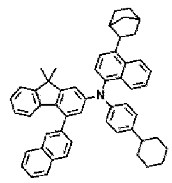
131

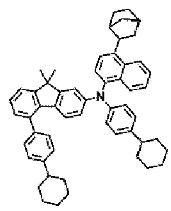
132

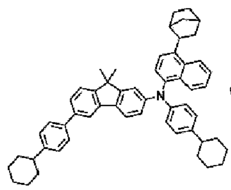
133

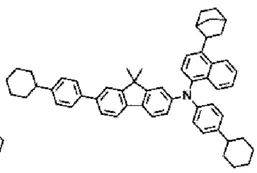
134

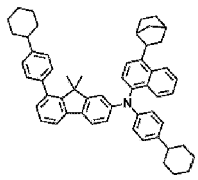
135

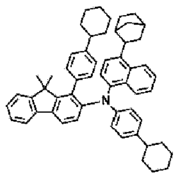
136

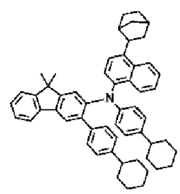
137

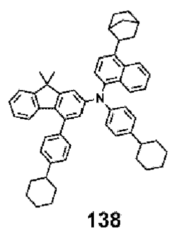
138

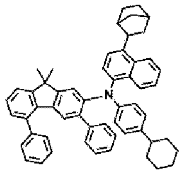

139

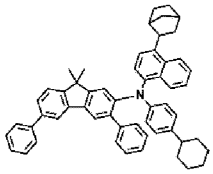

140

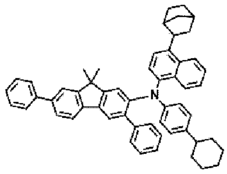

141

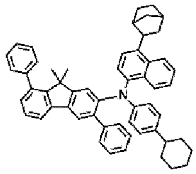

142

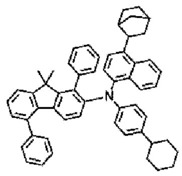

143

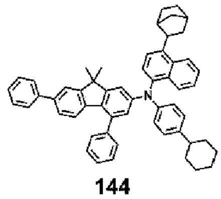

144

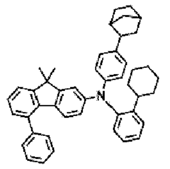

145

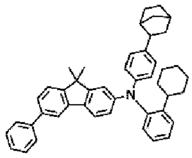

146

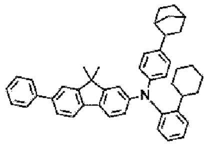

147

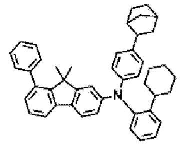

148

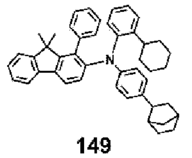

149

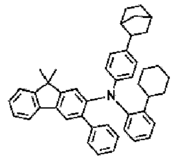

150

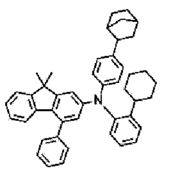

151

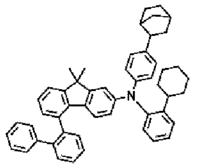

152

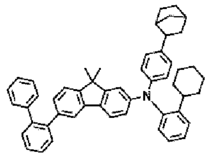

153

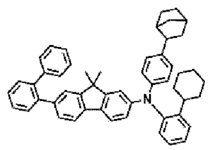

154

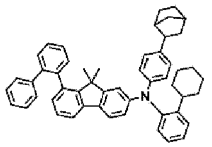

155

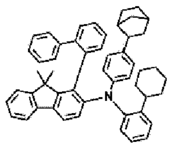

156

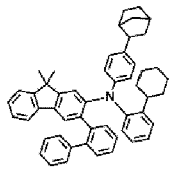

157

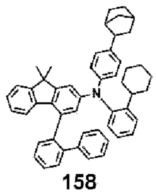
158

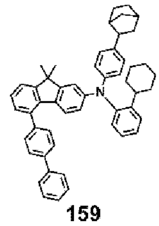
159

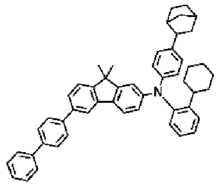
160

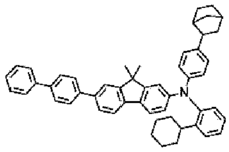
161

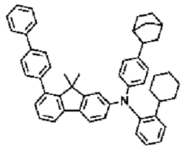
162

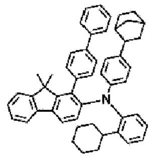
163

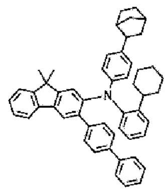
164

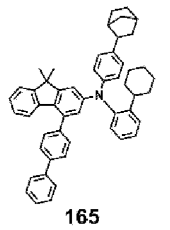
165

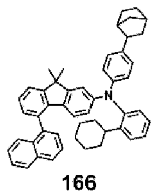
166

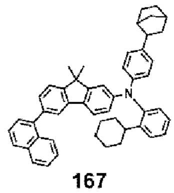
167

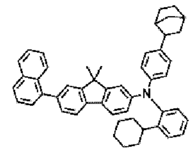
168

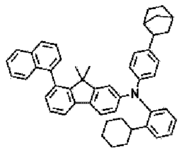
169

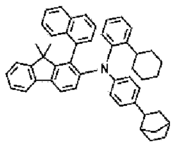
170

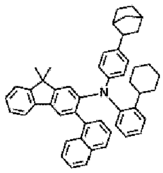
171

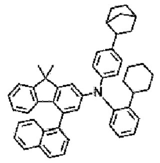
172

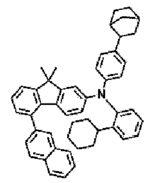
173

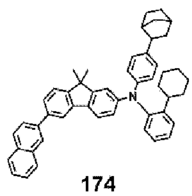
174

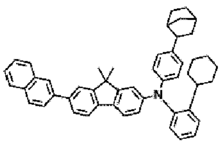
175

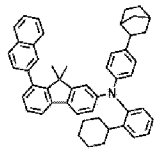
176

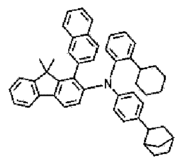
177

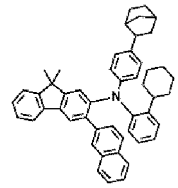

178

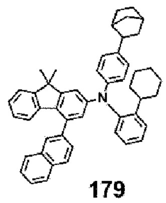

179

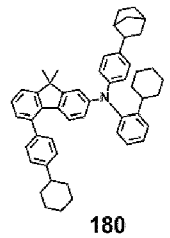

180

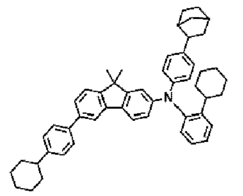

181

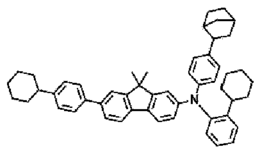

182

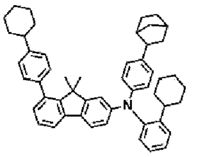

183

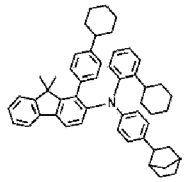

184

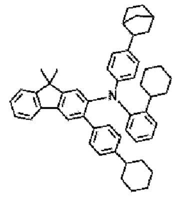

185

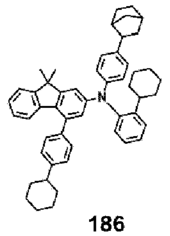

186

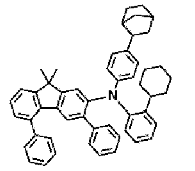

187

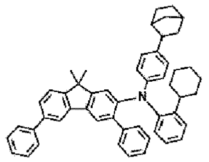

188

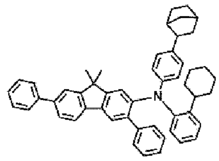

189

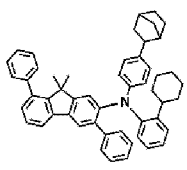

190

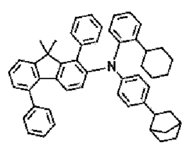

191

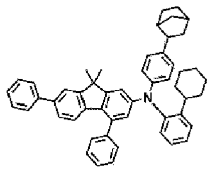

192

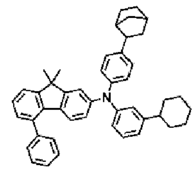

193

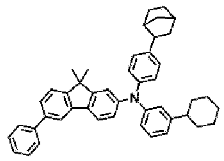

194

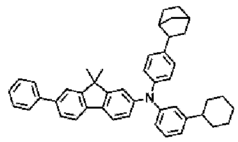

195

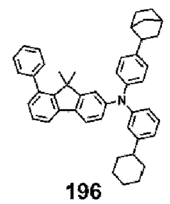

196

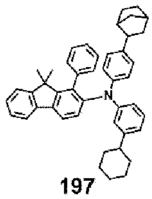
197

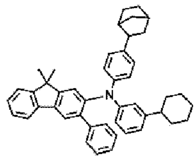
198

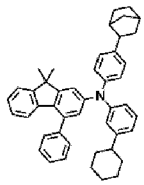
199

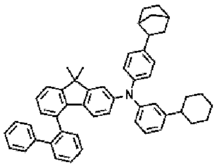
200

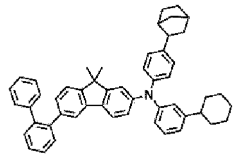
201

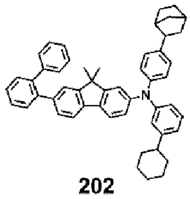
202

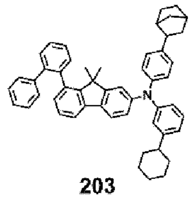
203

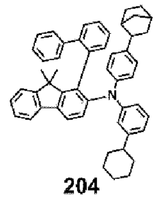
204

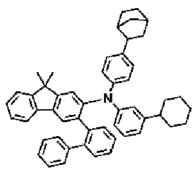
205

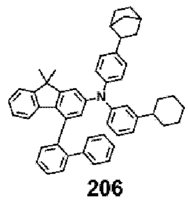
206

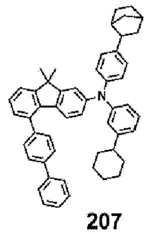
207

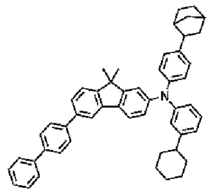
208

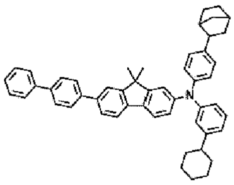
209

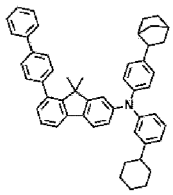
210

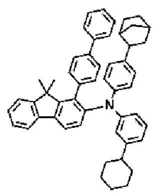
211

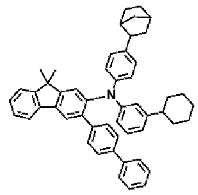
212

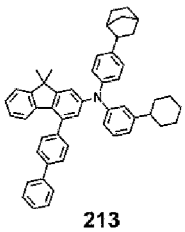
213

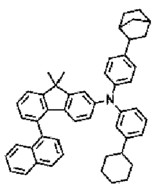
214

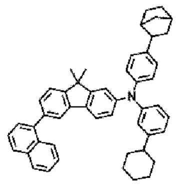
215

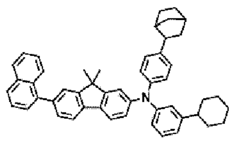
216

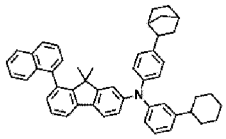
**217**

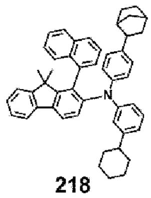
**218**

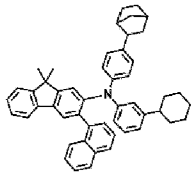
**219**

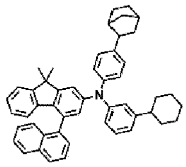
**220**

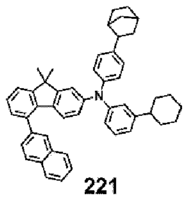
**221**

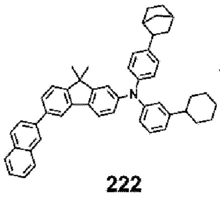
**222**

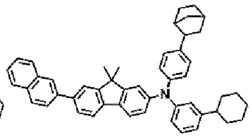
**223**

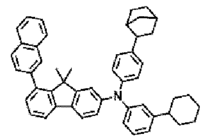
**224**

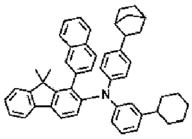
**225**

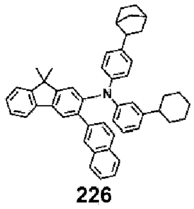
**226**

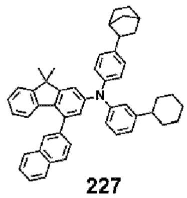
**227**

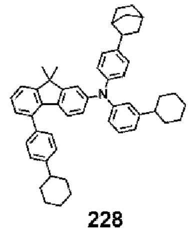
**228**

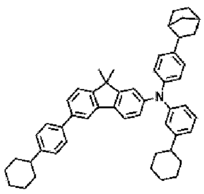
**229**

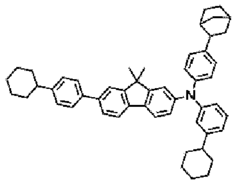
**230**

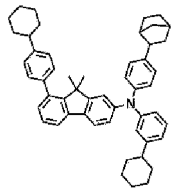
**231**

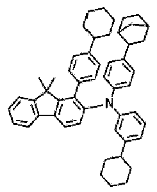
**232**

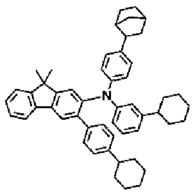
**233**

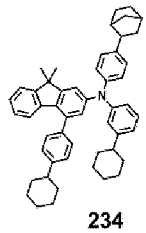
**234**

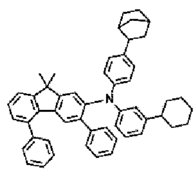
**235**

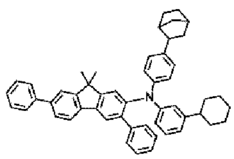
236
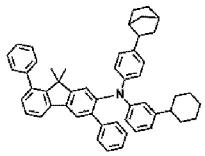
237
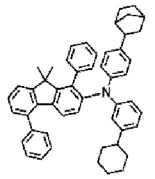
238
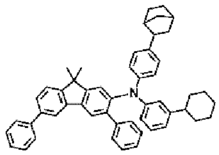
239

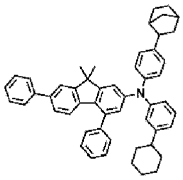
240
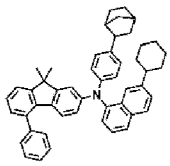
241
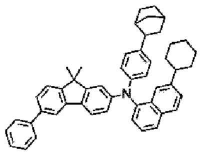
242
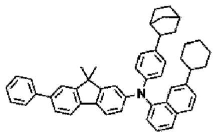
243

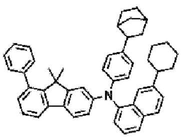
244
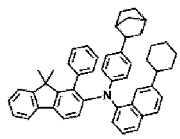
245
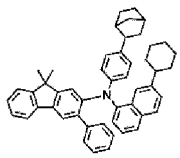
246
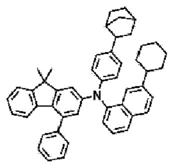
247

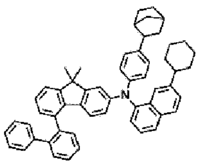
248
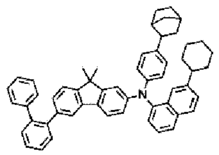
249
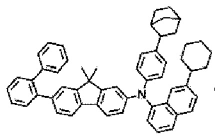
250
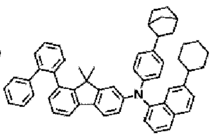
251

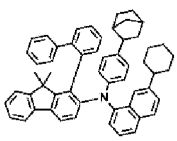
252
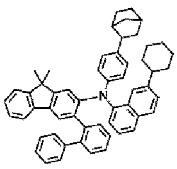
253
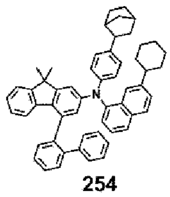
254
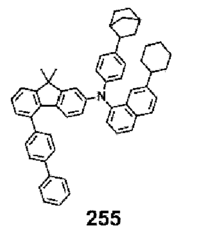
255

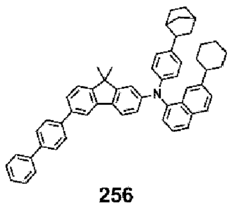 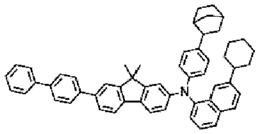 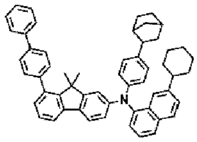 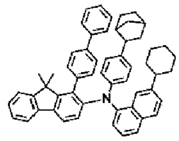

256 257 258 259

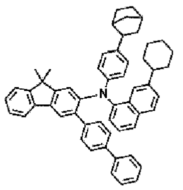 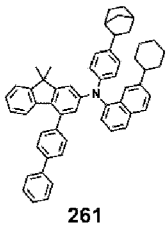 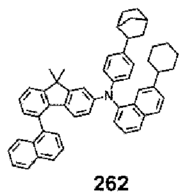 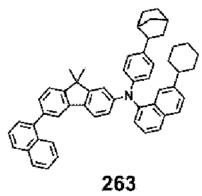

260 261 262 263

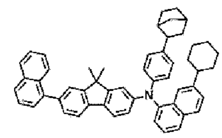 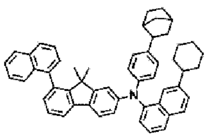 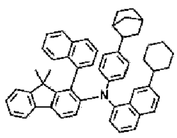 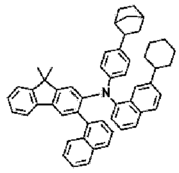

264 265 266 267

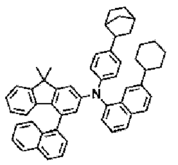 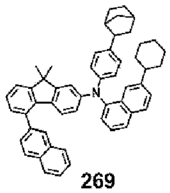 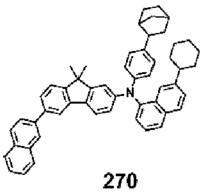 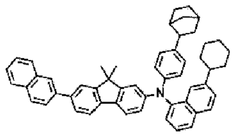

268 269 270 271

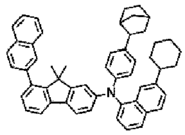 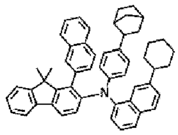 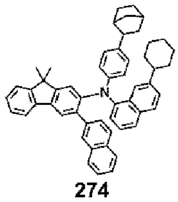 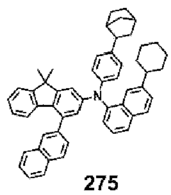

272 273 274 275

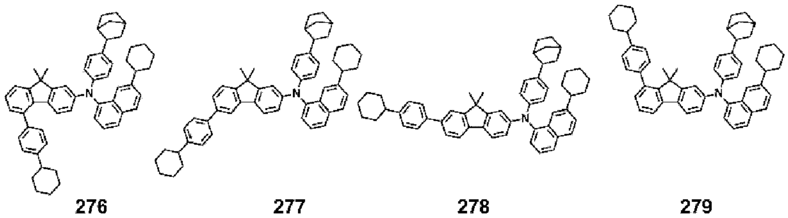

276 277 278 279

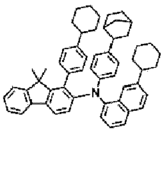

280

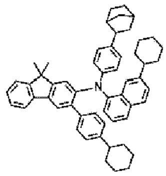

281

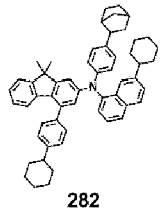

282

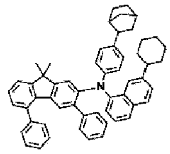

283

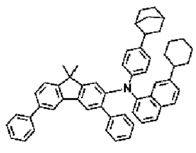

284

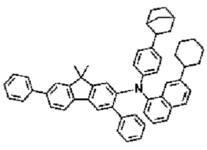

285

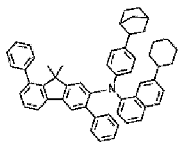

286

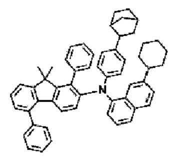

287

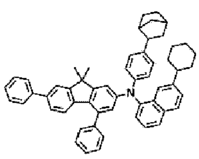

288

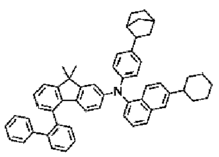

289

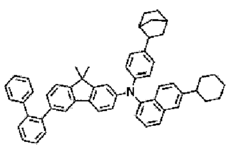

290

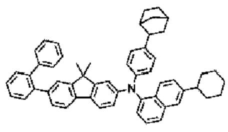

291

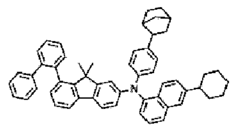

292

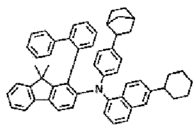

293

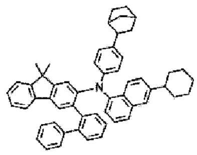

294

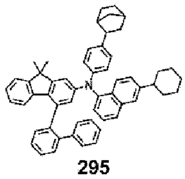

295

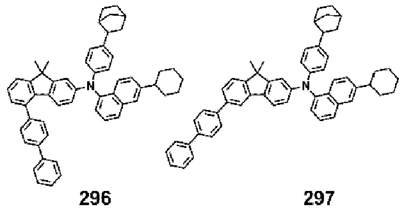

296 297

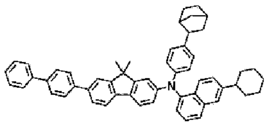

298

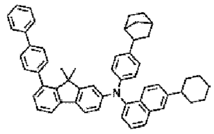

299

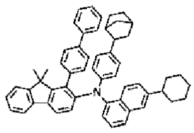

300

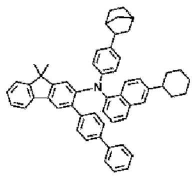

301

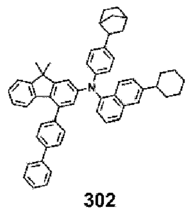

302

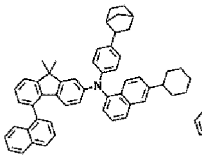

303

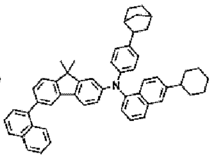

304

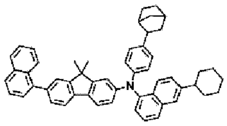

305

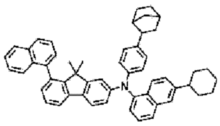

306

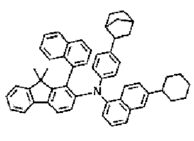

307

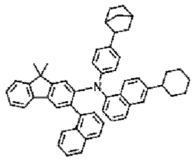

308

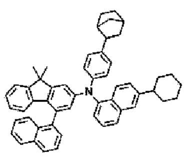

309

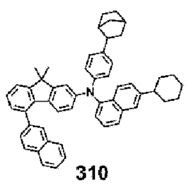

310

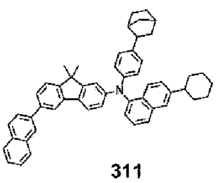

311

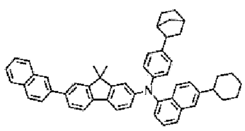

312

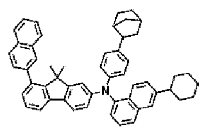

313

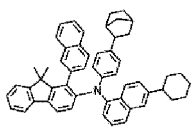

314

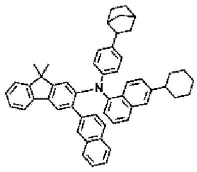

315

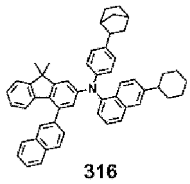

316

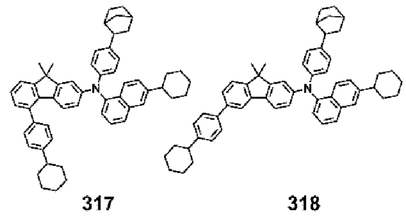

317 318

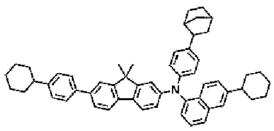

319

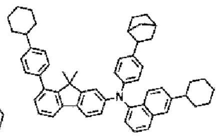

320

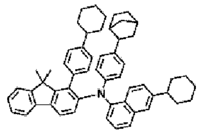

321

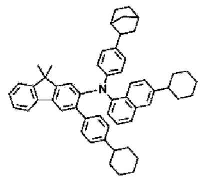

322

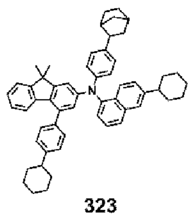

323

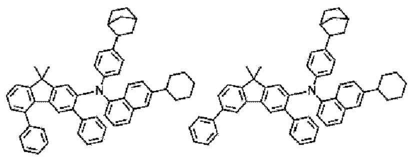

324 325

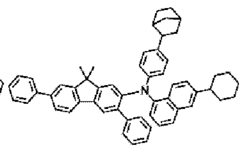

326

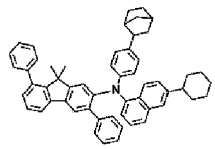

327

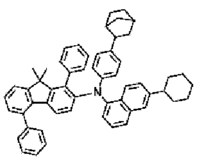

328

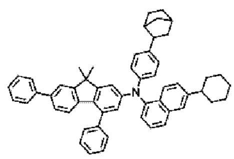

329

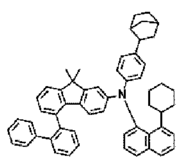

330

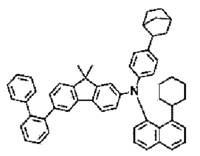

331

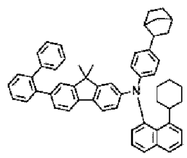

332

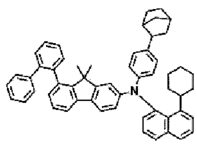

333

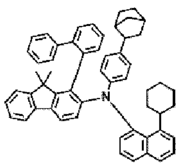
334
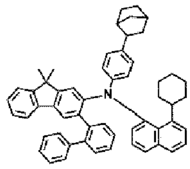
335
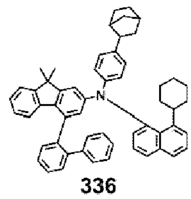
336
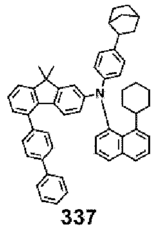
337

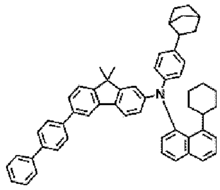
338
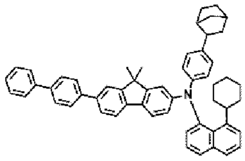
339
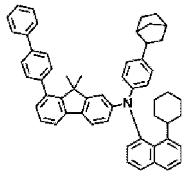
340
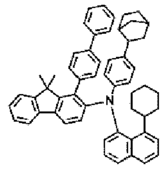
341

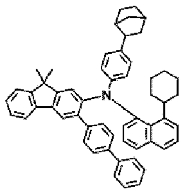
342
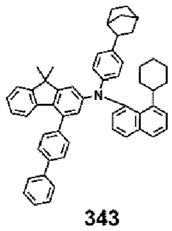
343
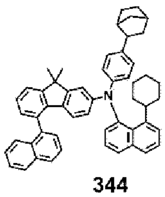
344
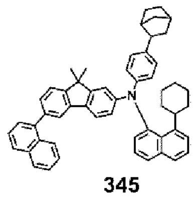
345

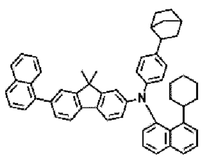
346
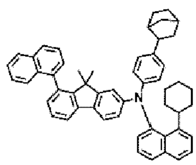
347
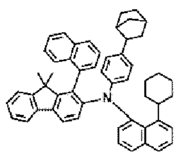
348
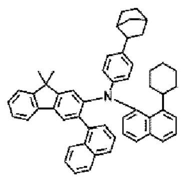
349

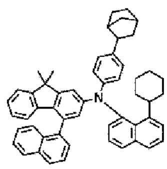
350
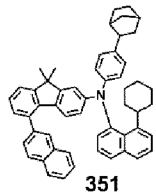
351
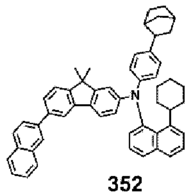
352
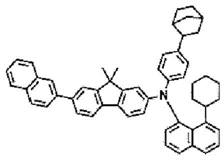
353

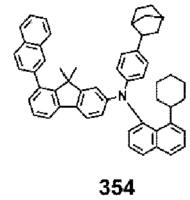
354

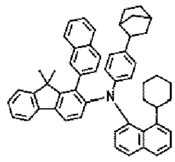
355

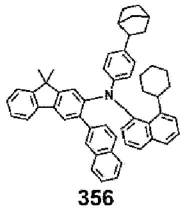
356

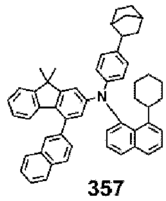
357

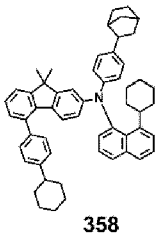
358

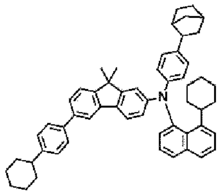
359

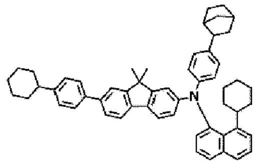
360

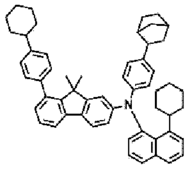
361

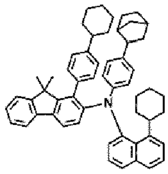
362

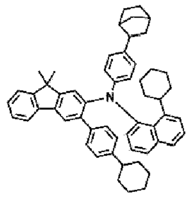
363

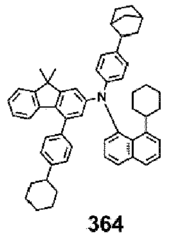
364

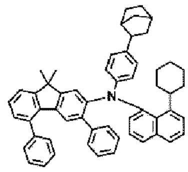
365

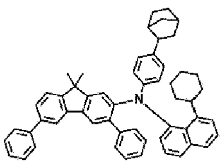
366

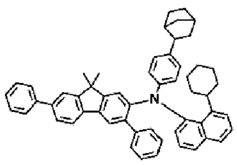
367

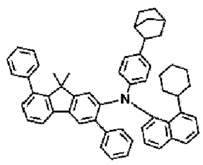
368

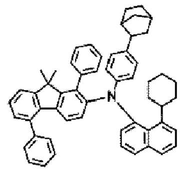
369

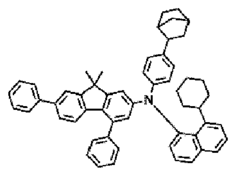
370

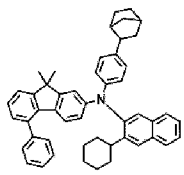
371

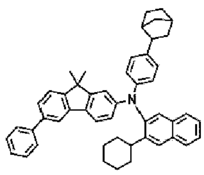
372

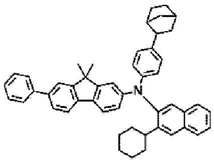
373
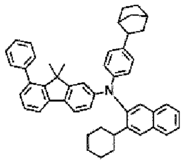
374
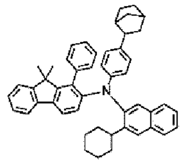
375
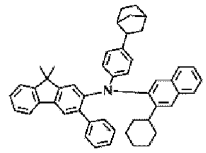
376

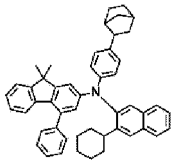
377
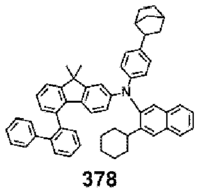
378
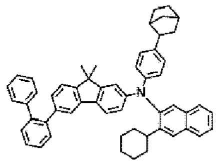
379
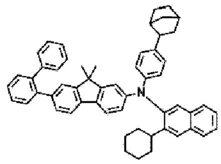
380

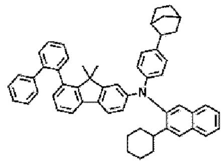
381
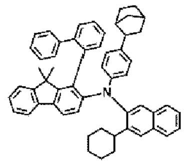
382
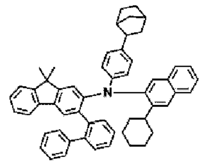
383
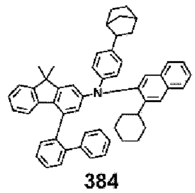
384

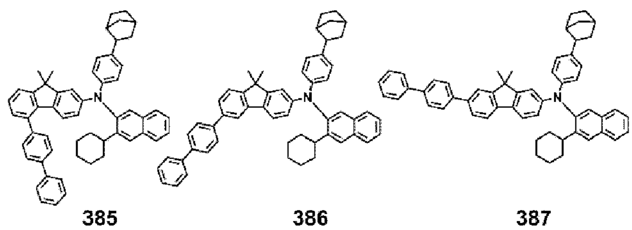
385
386
387
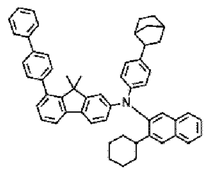
388

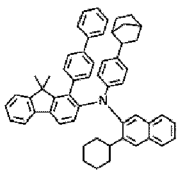
389
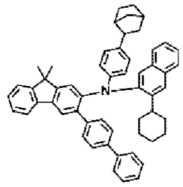
390
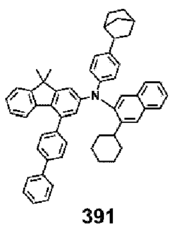
391
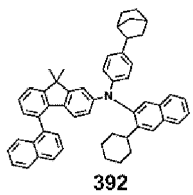
392

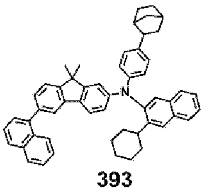
393

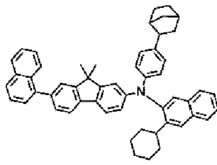
394

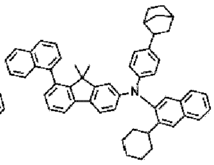
395

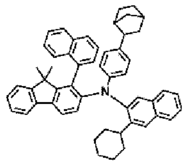
396

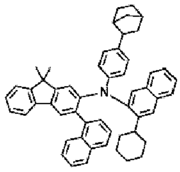
397

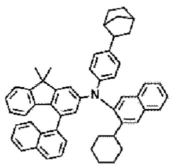
398

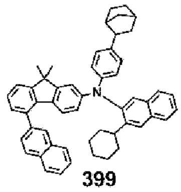
399

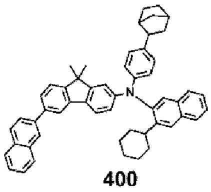
400

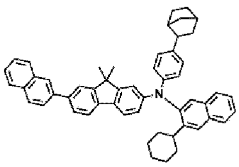
401

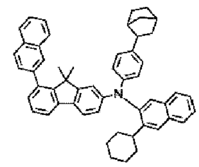
402

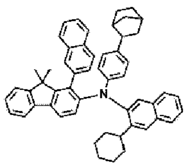
403

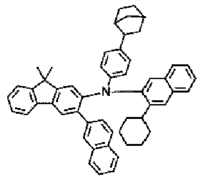
404

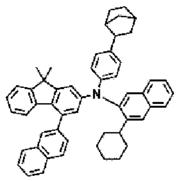
405

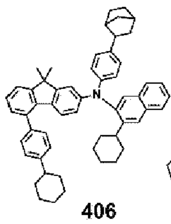
406

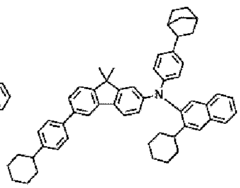
407

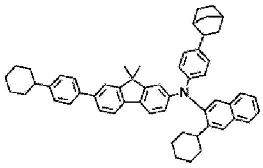
408

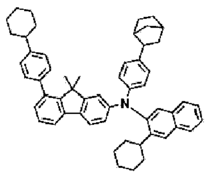
409

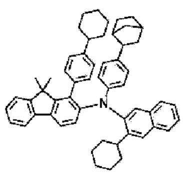
410

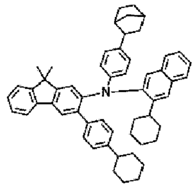
411

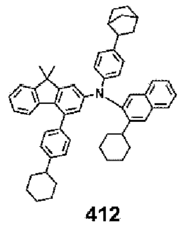
412

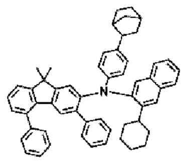

413

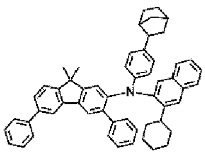

414

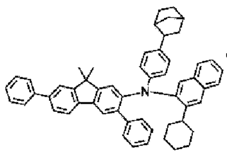

415

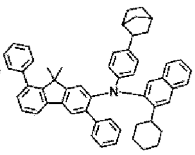

416

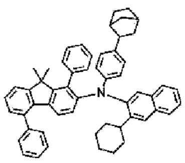

417

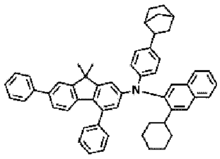

418

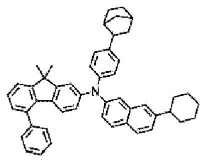

419

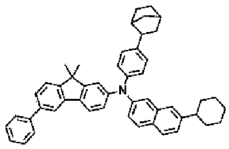

420

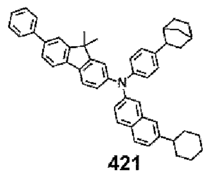

421

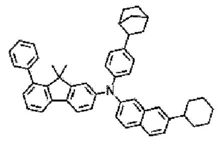

422

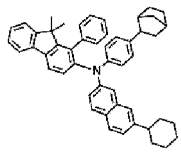

423

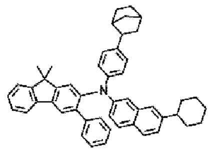

424

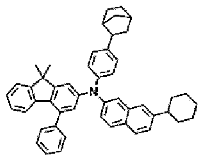

425

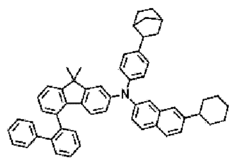

426

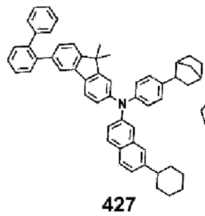

427

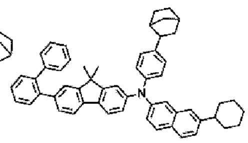

428

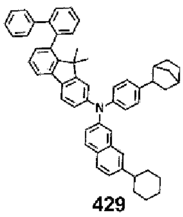

429

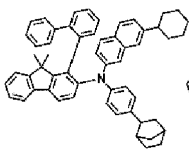

430

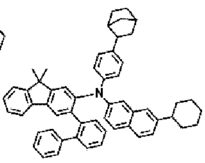

431

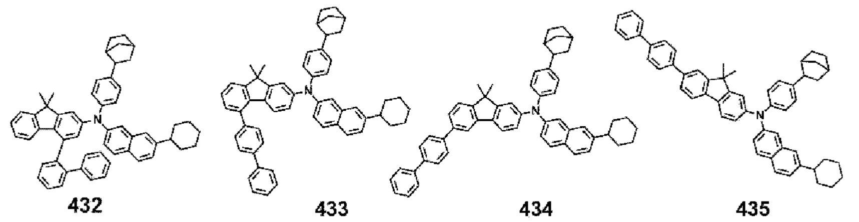

432 433 434 435

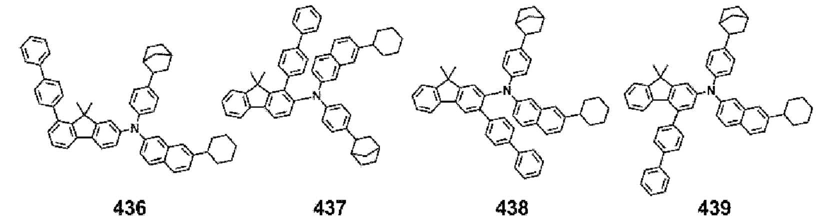

436 437 438 439

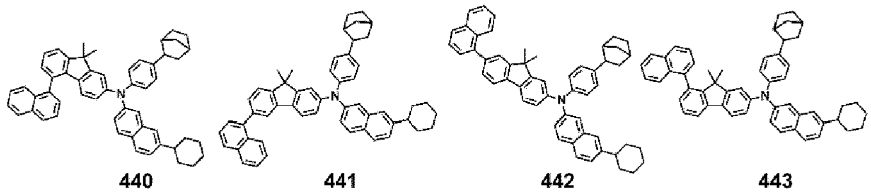

440 441 442 443

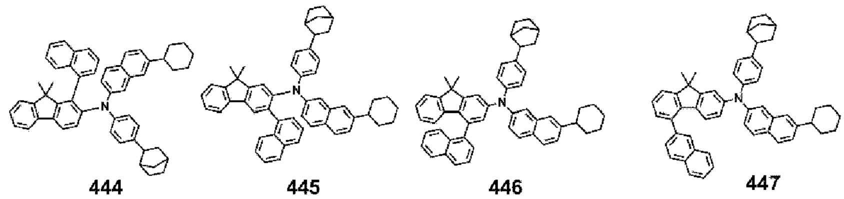

444 445 446 447

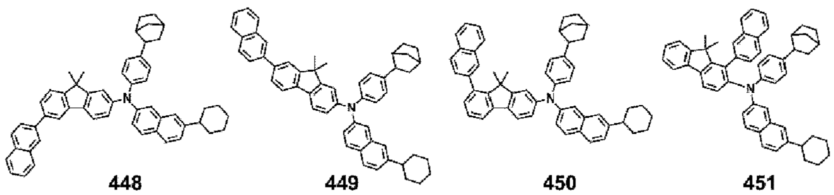

448 449 450 451

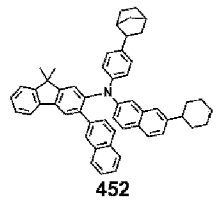

452

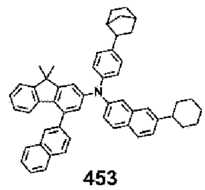

453

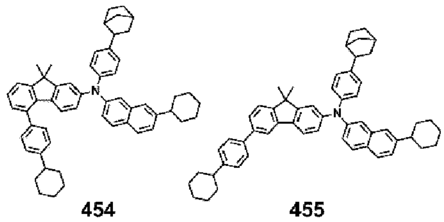

454 455

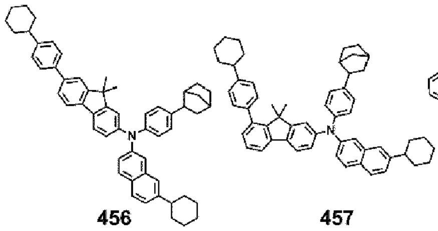

456 457

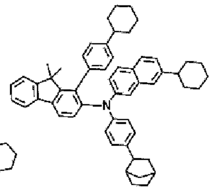

458

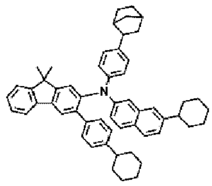

459

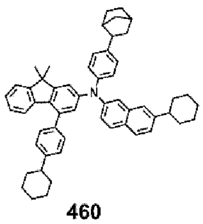

460

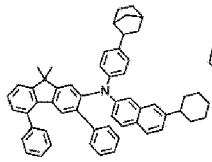

461

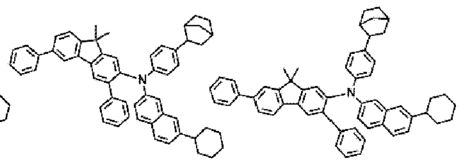

462 463

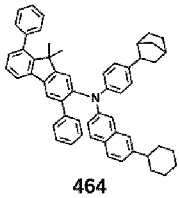

464

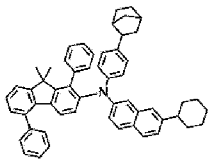

465

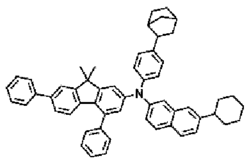

466

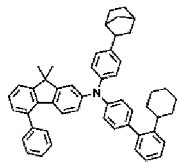

467

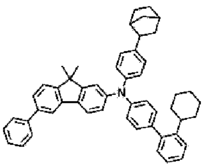

468

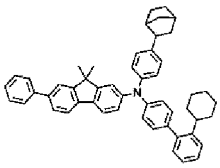

469

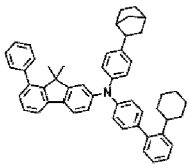

470

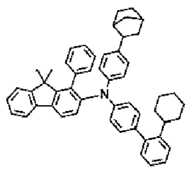 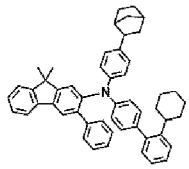 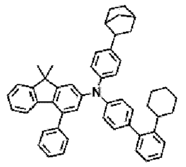 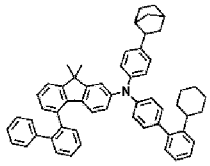

471 472 473 474

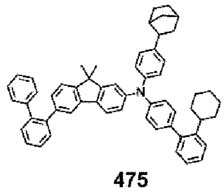 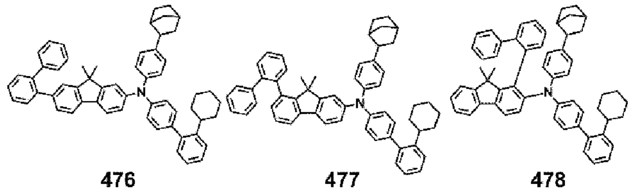

475 476 477 478

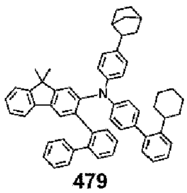 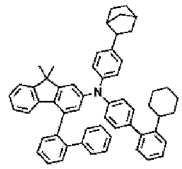 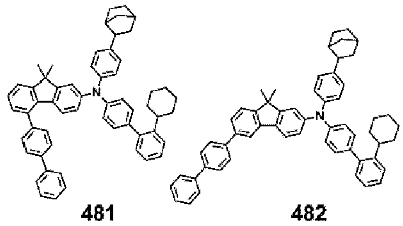

479 480 481 482

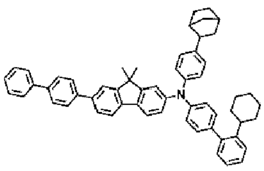 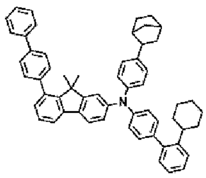 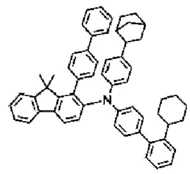 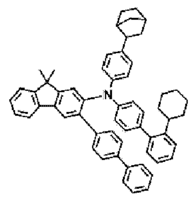

483 484 485 486

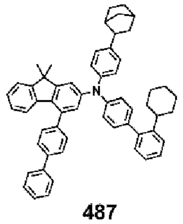 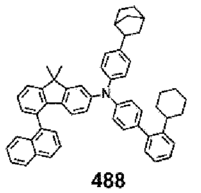 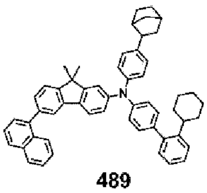 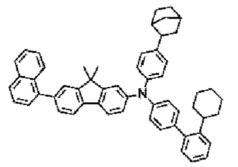

487 488 489 490

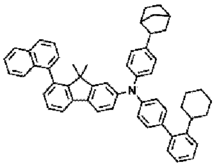
491
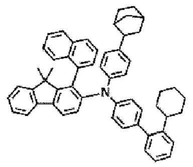
492
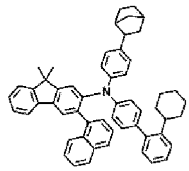
493
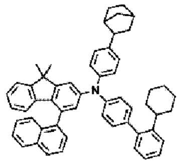
494

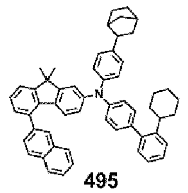
495
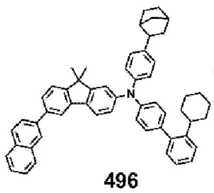
496
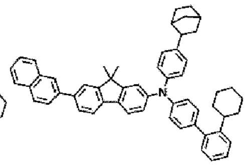
497
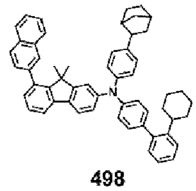
498

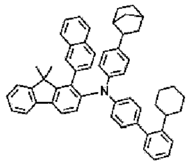
499
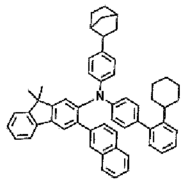
500
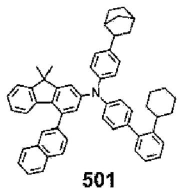
501
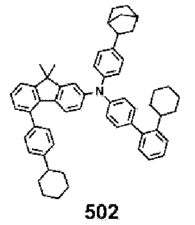
502

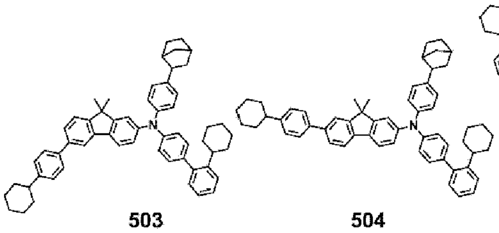
503
504
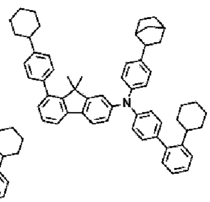
505
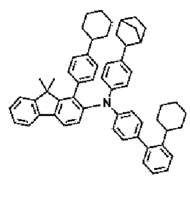
506

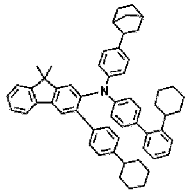
507
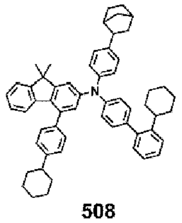
508
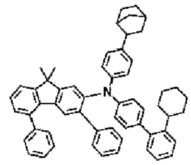
509

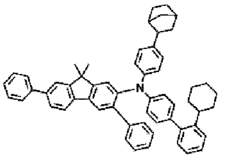

510

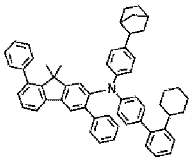

511

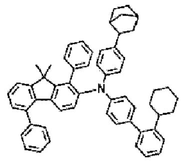

512

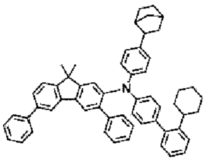

513

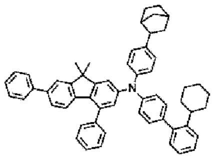

514

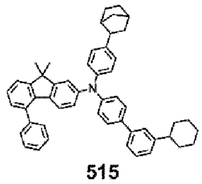

515

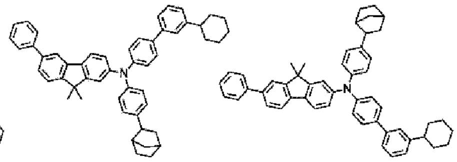

516 517

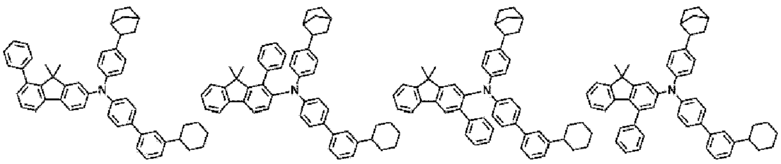

518 519 520 521

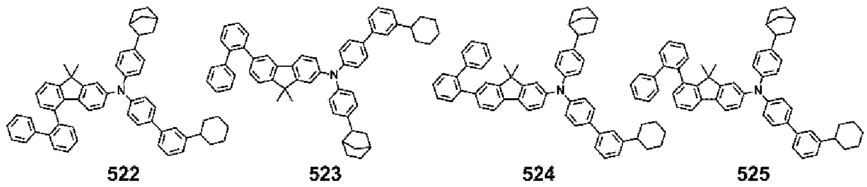

522 523 524 525

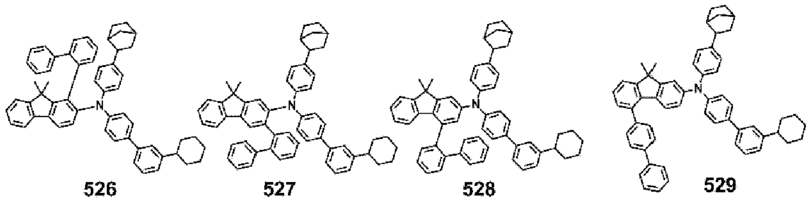

526 527 528 529

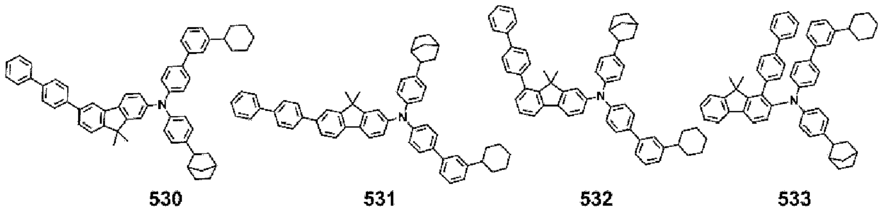

530 531 532 533

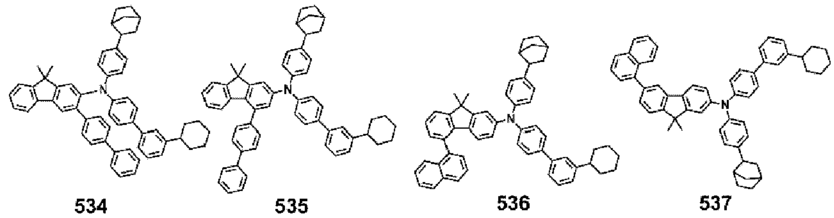

534 535 536 537

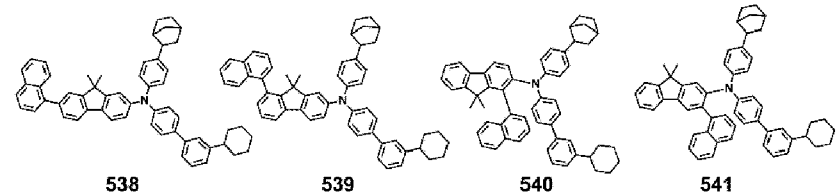

538 539 540 541

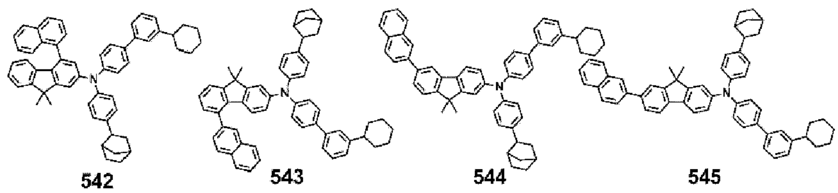

542 543 544 545

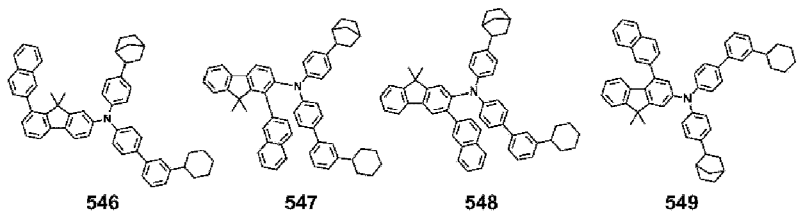

546 547 548 549

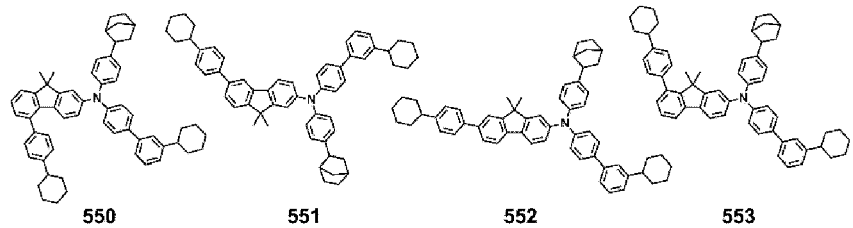

550 551 552 553

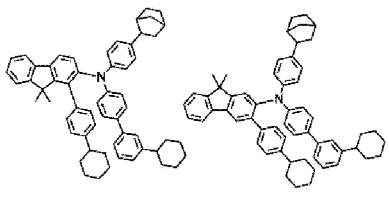

554 555

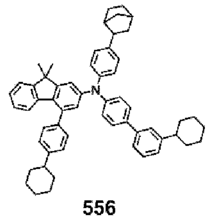

556

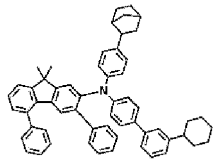

557

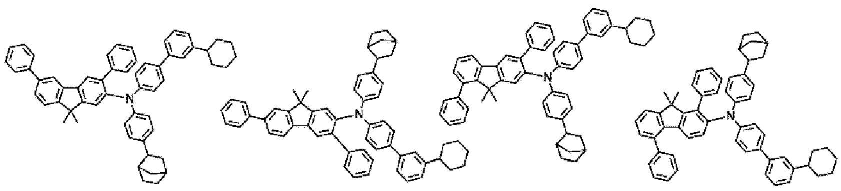

558 559 560 561

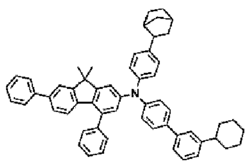

562 563

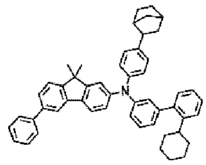

564

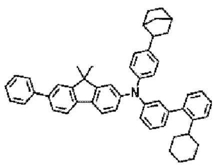

565

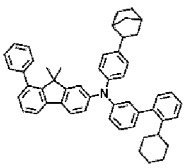

566

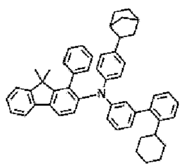

567

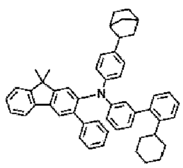

568

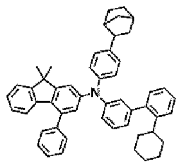

569

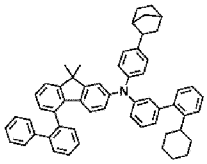

570

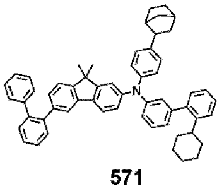

571

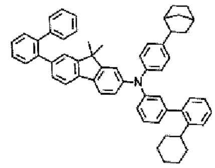

572

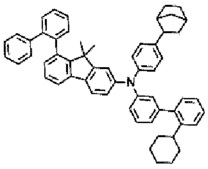

573

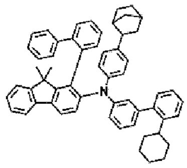

574

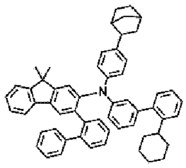

575

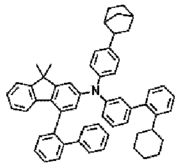

576

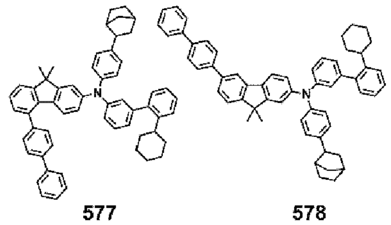

577

578

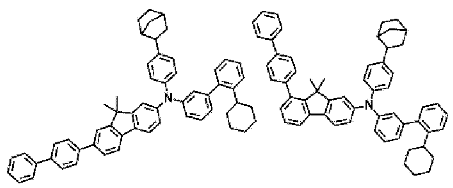

579

580

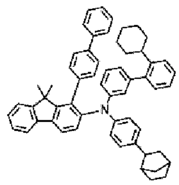

581

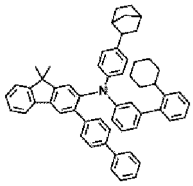

582

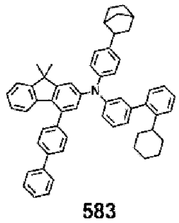

583

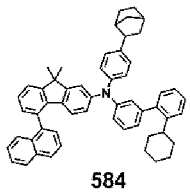

584

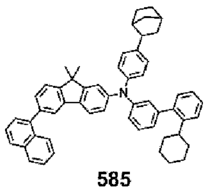

585

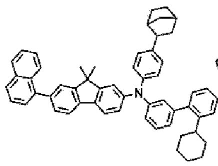

586

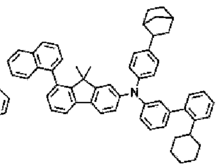

587

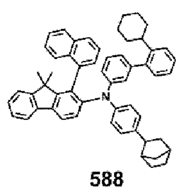

588

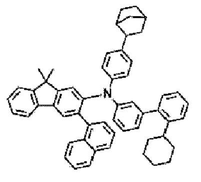
589
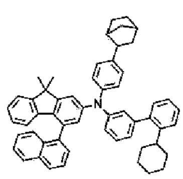
590
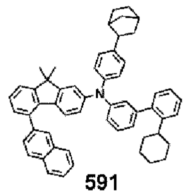
591
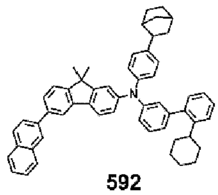
592

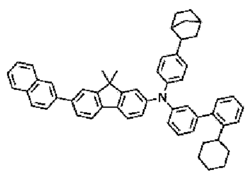
593
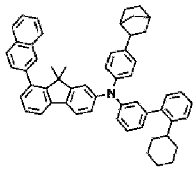
594
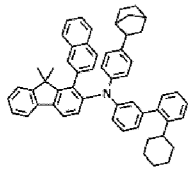
595
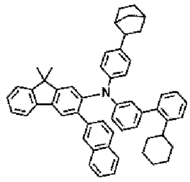
596

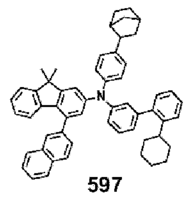
597
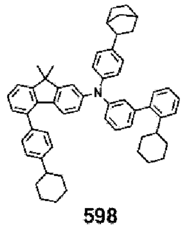
598
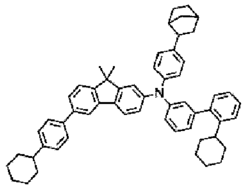
599
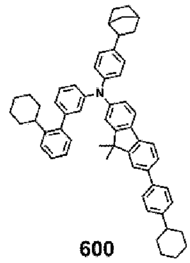
600

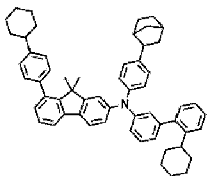
601
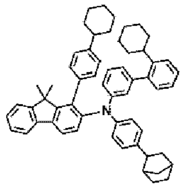
602
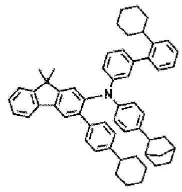
603
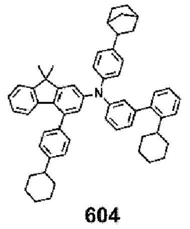
604

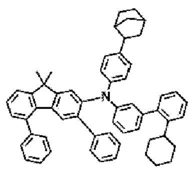
605
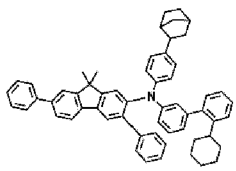
606
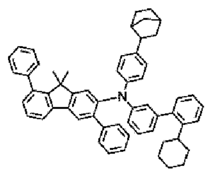
607

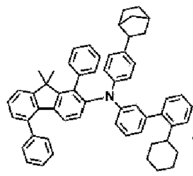
608
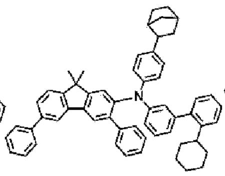
609
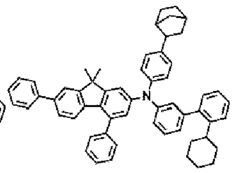
610
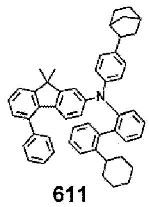
611
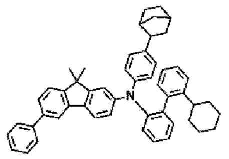
612
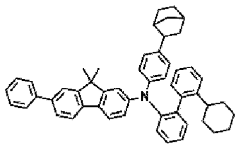
613
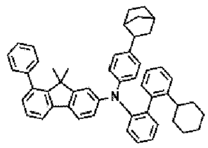
614
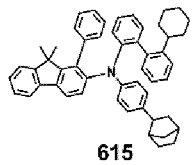
615
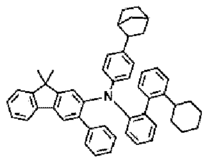
616
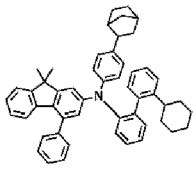
617
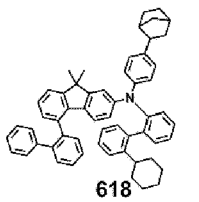
618
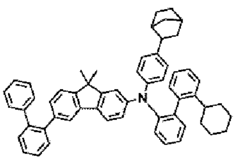
619
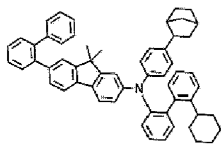
620
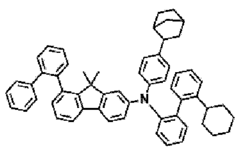
621
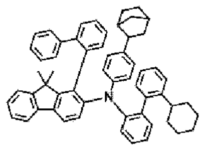
622
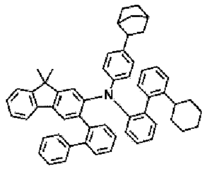
623
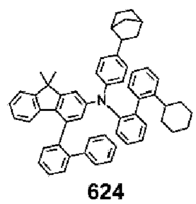
624
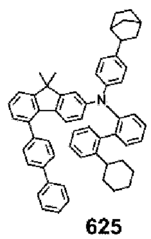
625
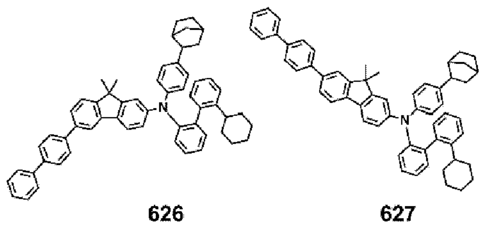
626

627

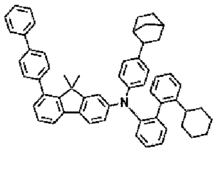

628

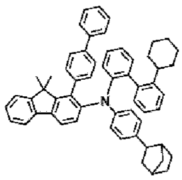

629

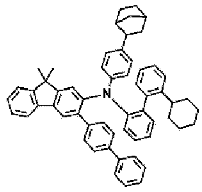

630

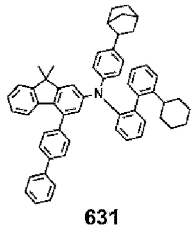

631

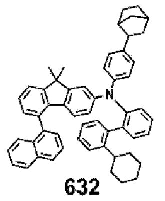

632

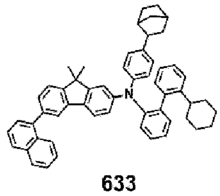

633

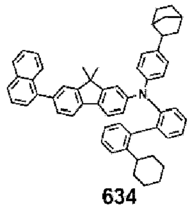

634

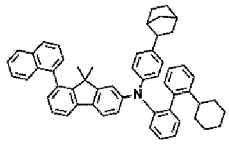

635

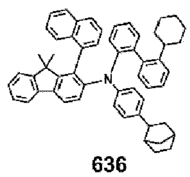

636

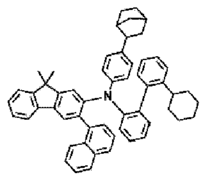

637

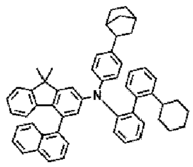

638

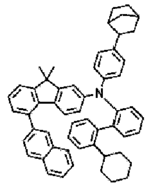

639

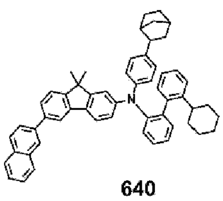

640

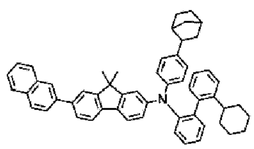

641

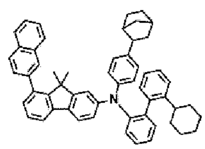

642

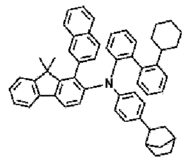

643

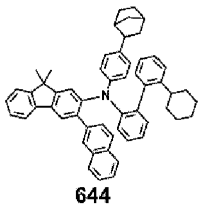

644

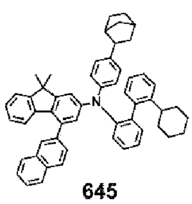

645

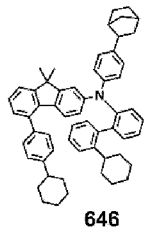

646

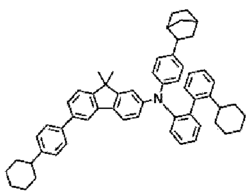

647

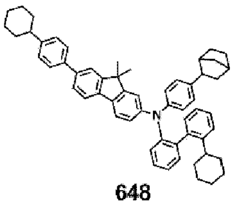
648

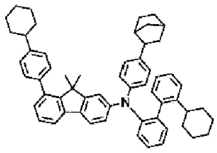
649

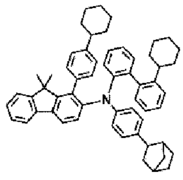
650

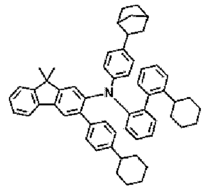
651

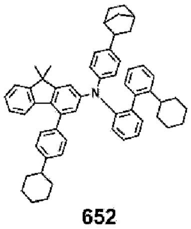
652

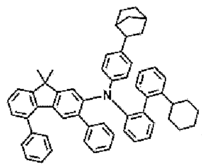
653

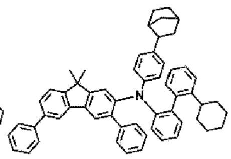
654

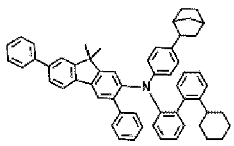
655

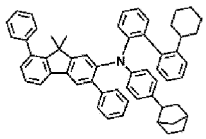
656

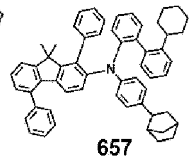
657

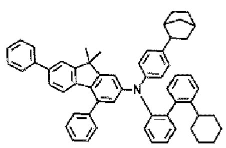
658

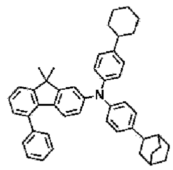
659

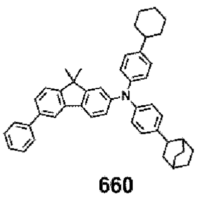
660

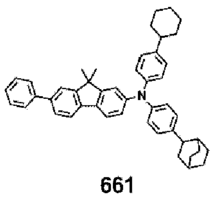
661

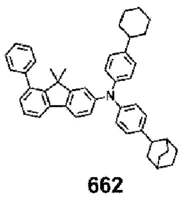
662

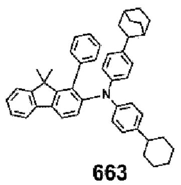
663

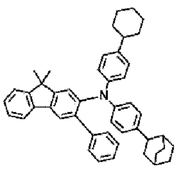
664

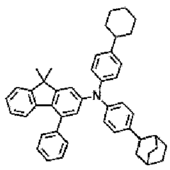
665

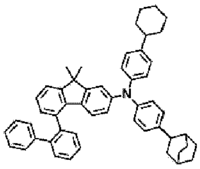
666

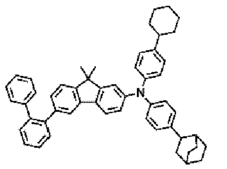
667

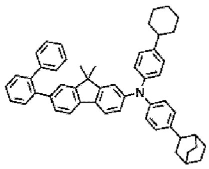
668

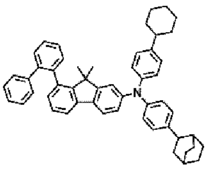
669

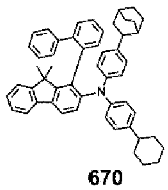
670

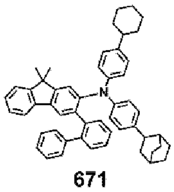
671

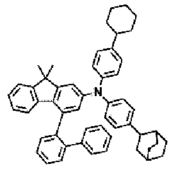
672

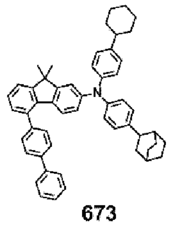
673

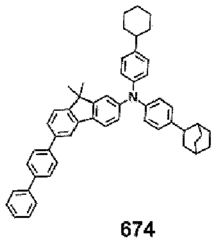
674

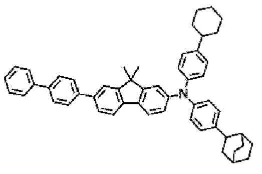
675

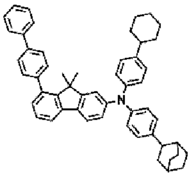
676

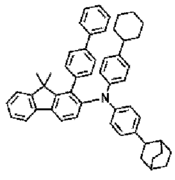
677

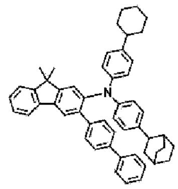
678

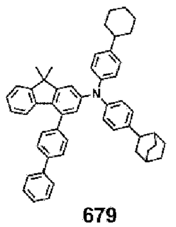
679

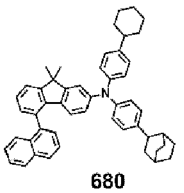
680

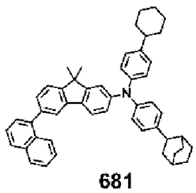
681

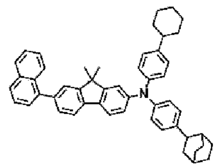
682

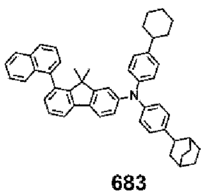
683

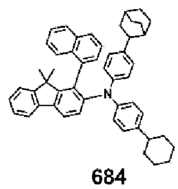
684

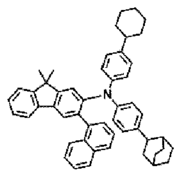
685

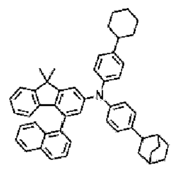
686

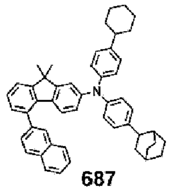
687
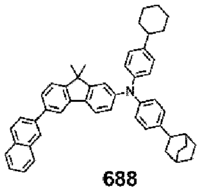
688
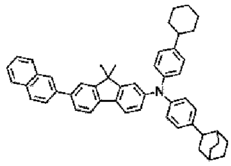
689
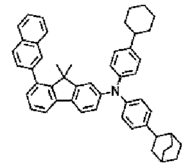
690

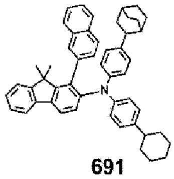
691
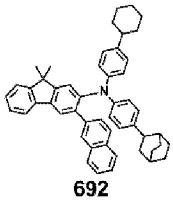
692
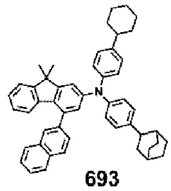
693
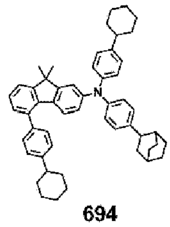
694

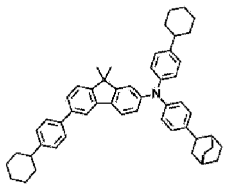
695
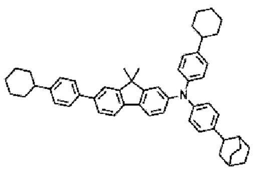
696
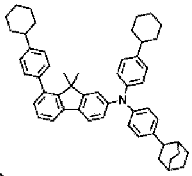
697
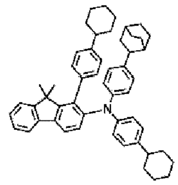
698

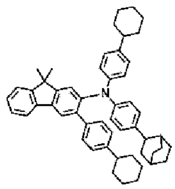
699
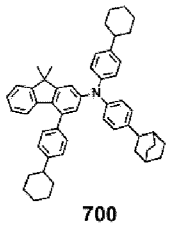
700
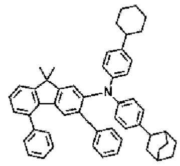
701
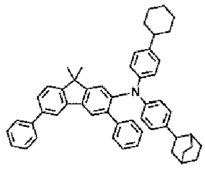
702

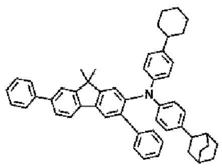
703
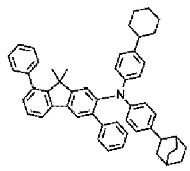
704
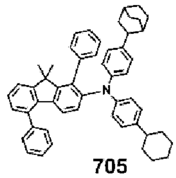
705

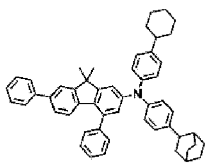
706
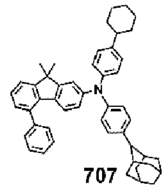
707
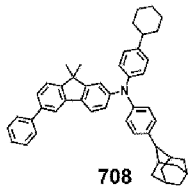
708
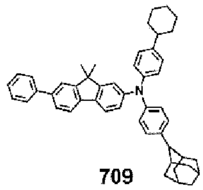
709

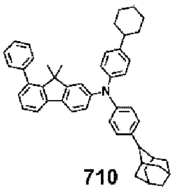
710
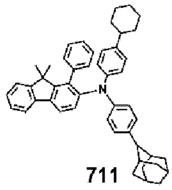
711
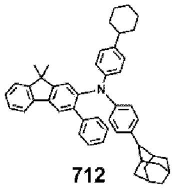
712
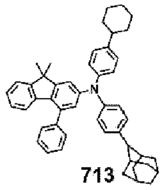
713

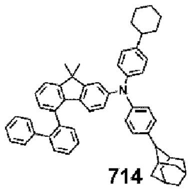
714
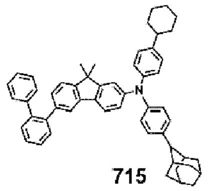
715
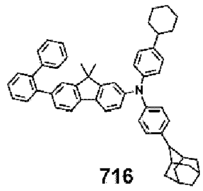
716
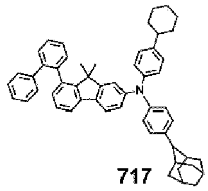
717

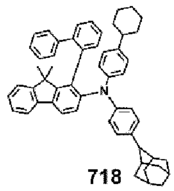
718
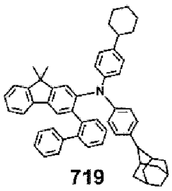
719
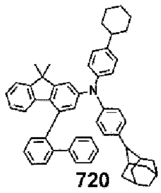
720
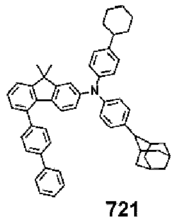
721

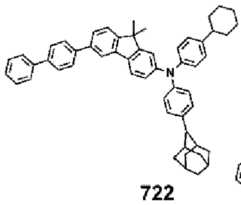
722
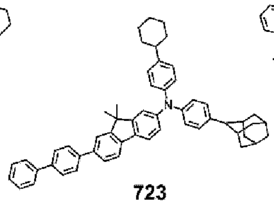
723
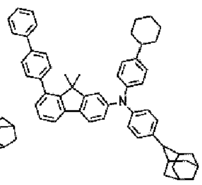
724
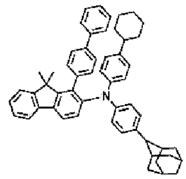
725

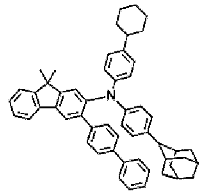 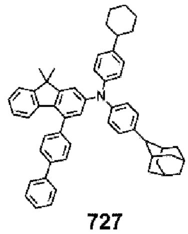 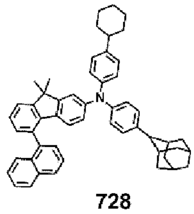 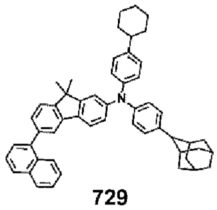

726 727 728 729

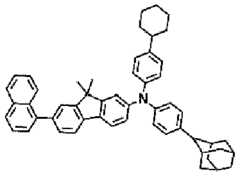 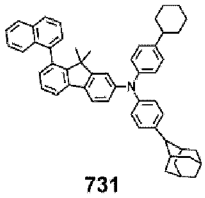 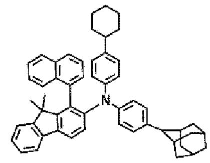 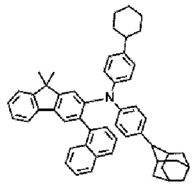

730 731 732 733

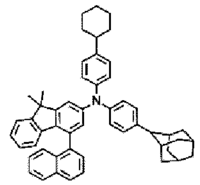 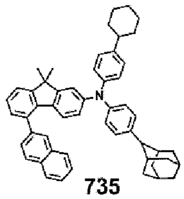 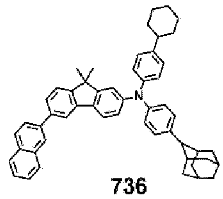 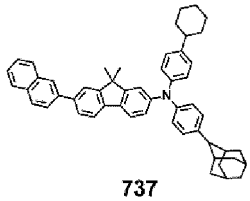

734 735 736 737

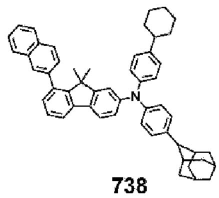 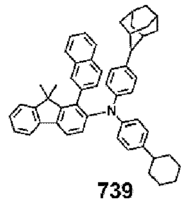 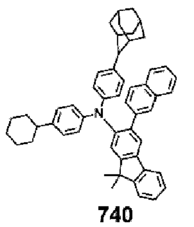 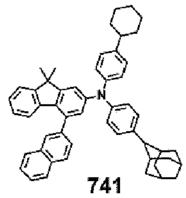

738 739 740 741

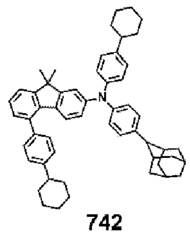 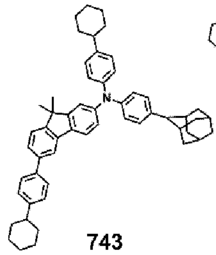 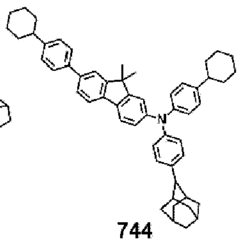 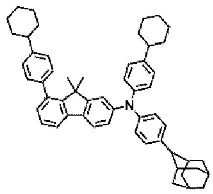

742 743 744 745

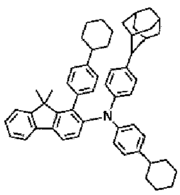
746
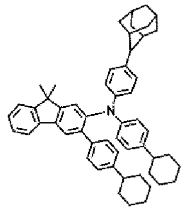
747
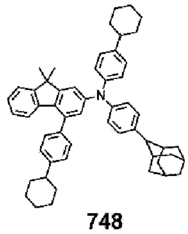
748
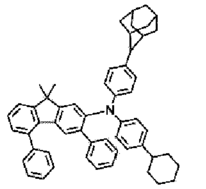
749

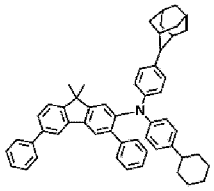
750
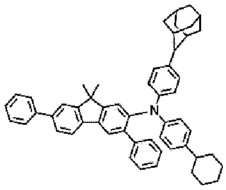
751
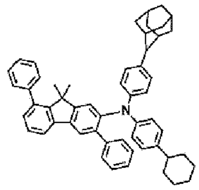
752
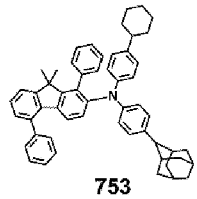
753

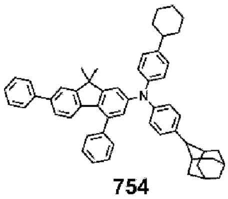
754
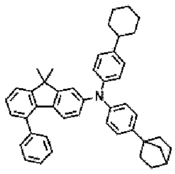
755
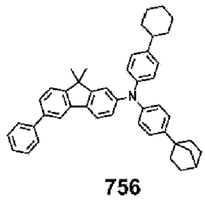
756
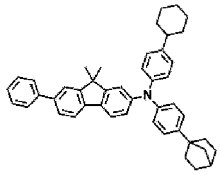
757

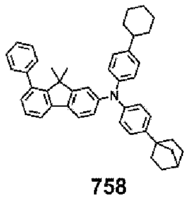
758
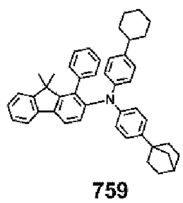
759
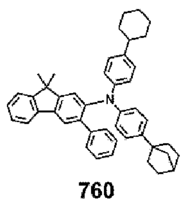
760
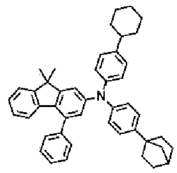
761

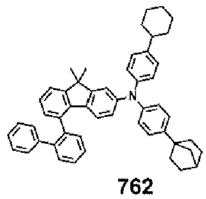
762
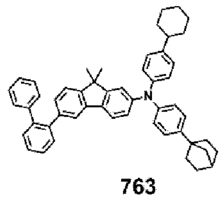
763
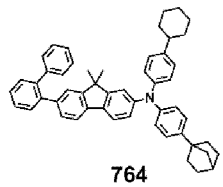
764

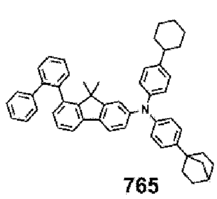
765

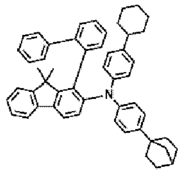
766

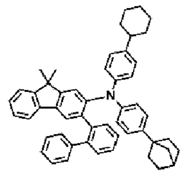
767

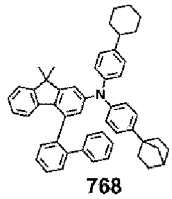
768

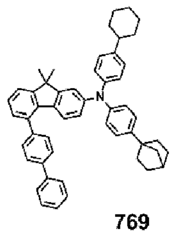
769

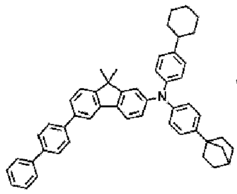
770

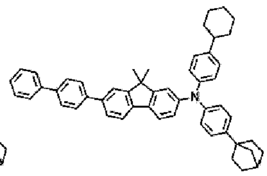
771

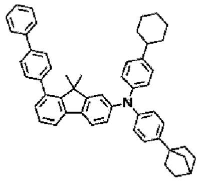
772

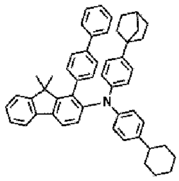
773

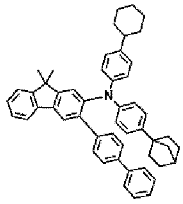
774

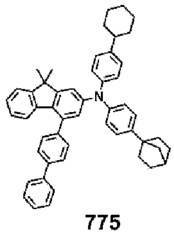
775

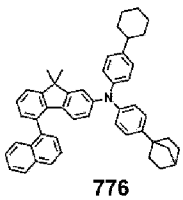
776

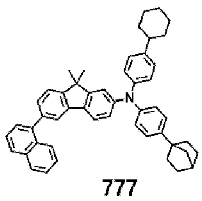
777

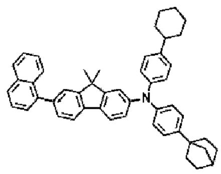
778

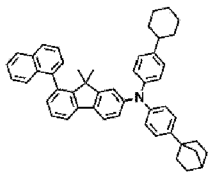
779

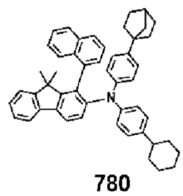
780

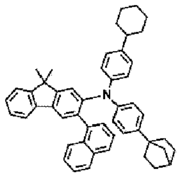
781

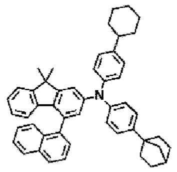
782

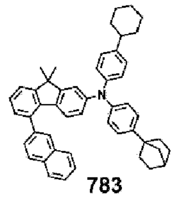
783

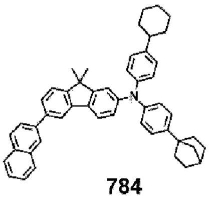
784

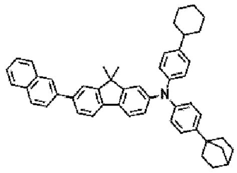
785

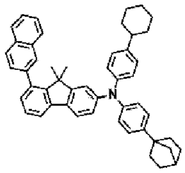
786

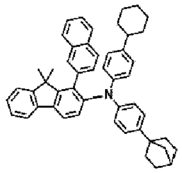
787

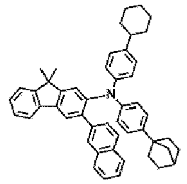
788

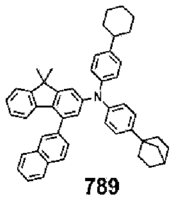
789

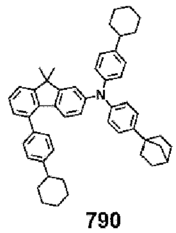
790

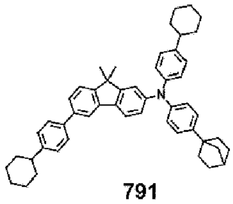
791

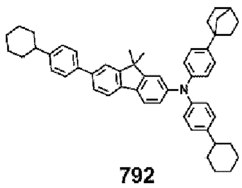
792

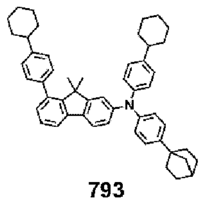
793

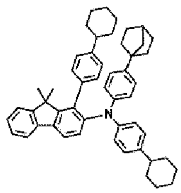
794

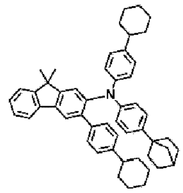
795

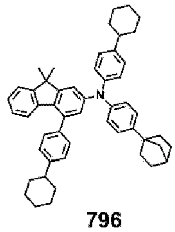
796

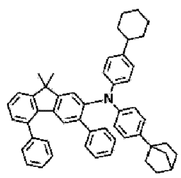
797

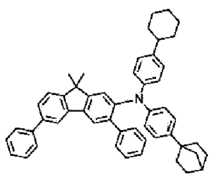
798

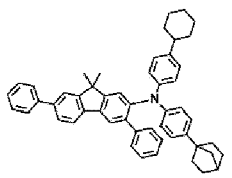
799

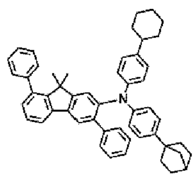
800

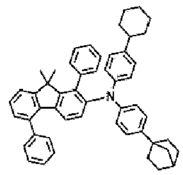
801

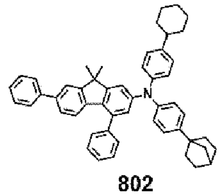
802

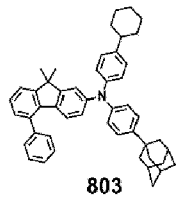
803

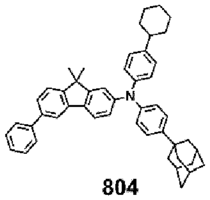
804
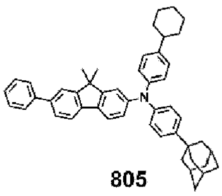
805
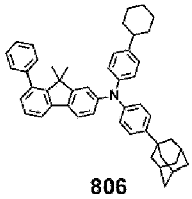
806
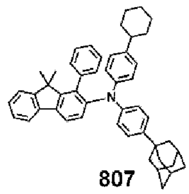
807

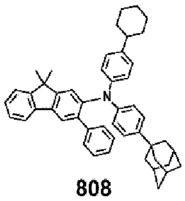
808
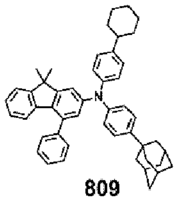
809
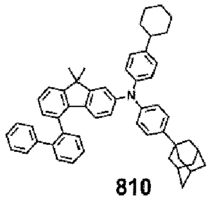
810
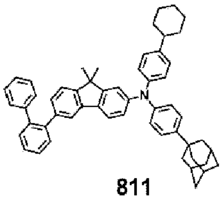
811

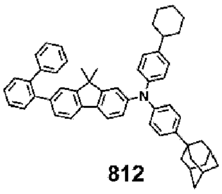
812
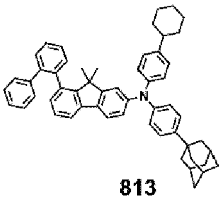
813
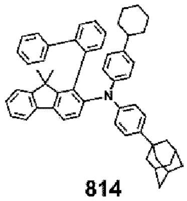
814
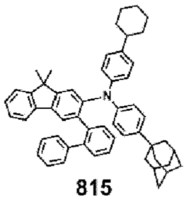
815

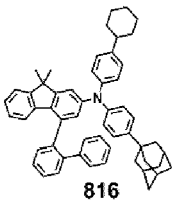
816
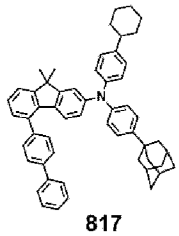
817
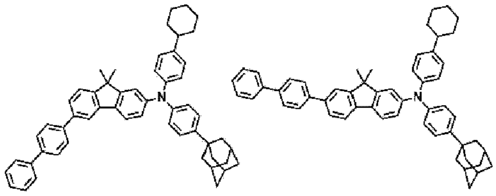
818

819

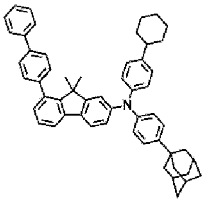
820
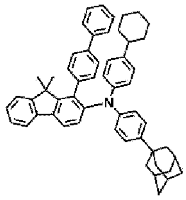
821
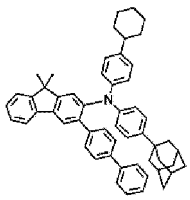
822
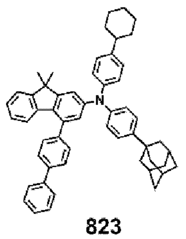
823

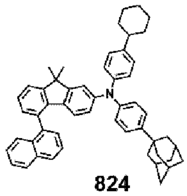
824
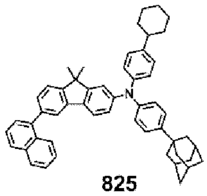
825
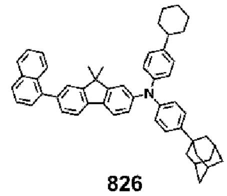
826
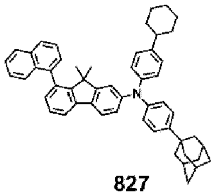
827

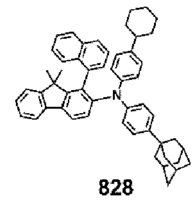
828
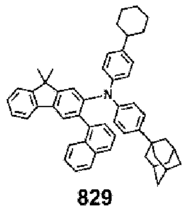
829
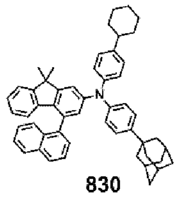
830
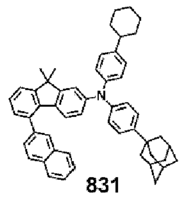
831

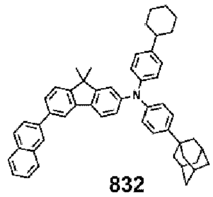
832
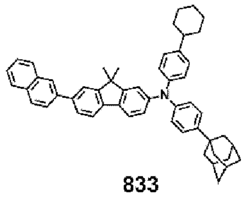
833
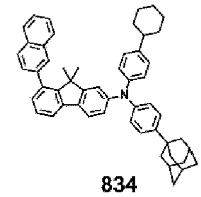
834
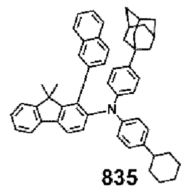
835

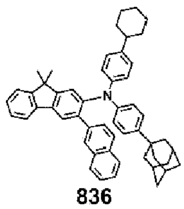
836
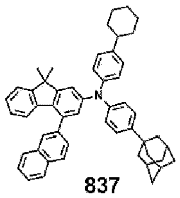
837
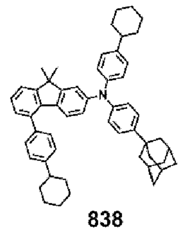
838
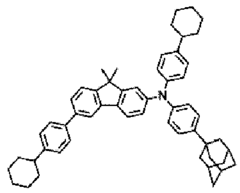
839

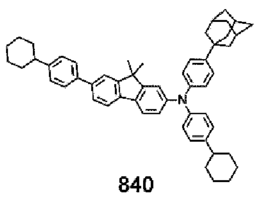
840
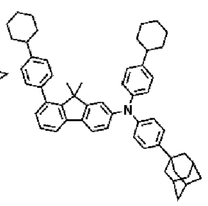
841
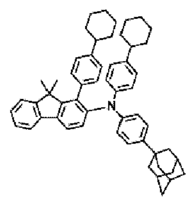
842
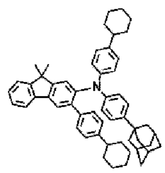
843

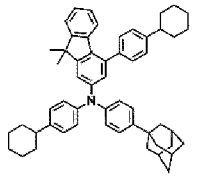
844
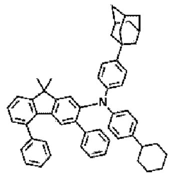
845
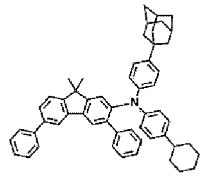
846
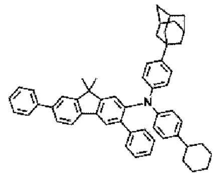
847

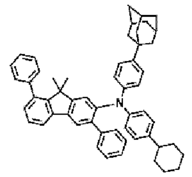
848
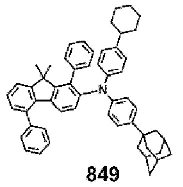
849
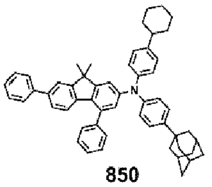
850
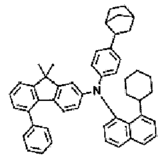
851

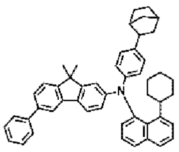
852
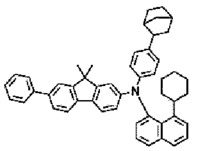
853
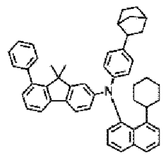
854
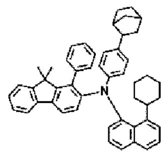
855

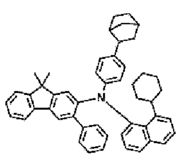
856
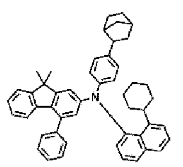
857
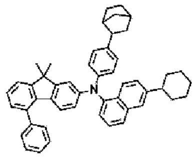
858
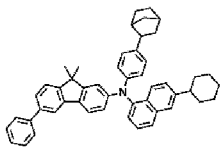
859

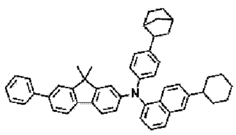
860
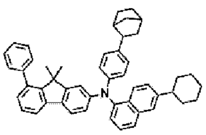
861
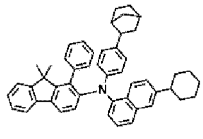
862
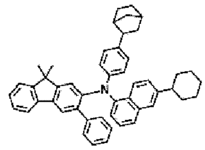
863

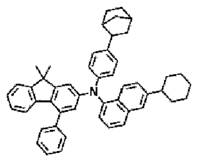
864

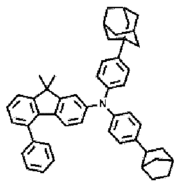
865
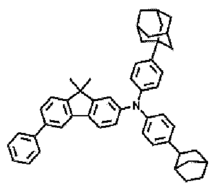
866
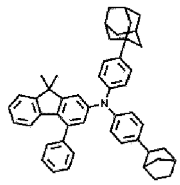
867
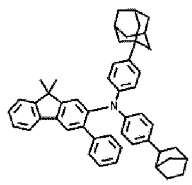
868

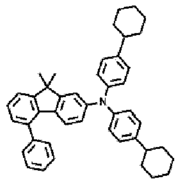
869
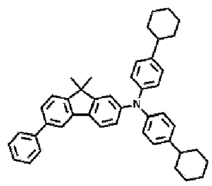
870
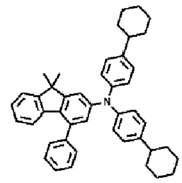
871
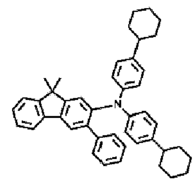
872

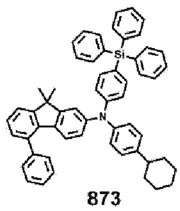
873
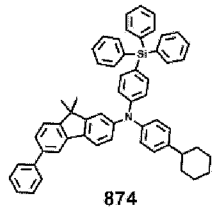
874
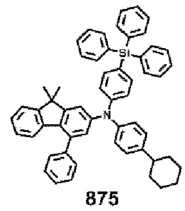
875
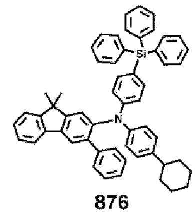
876

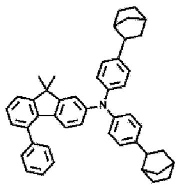
877
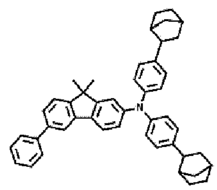
878
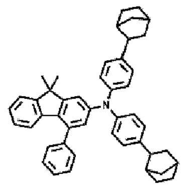
879
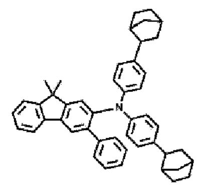
880

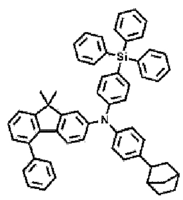
881
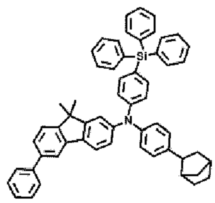
882
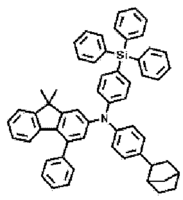
883
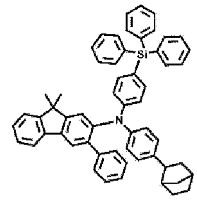
884

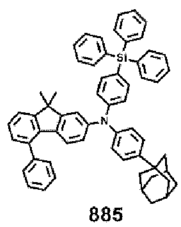
885
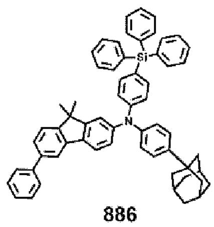
886
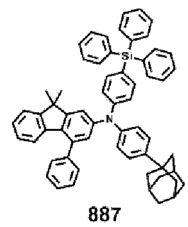
887
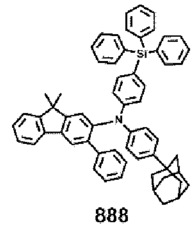
888

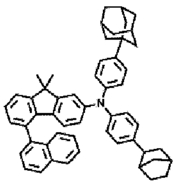
889
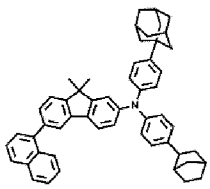
890
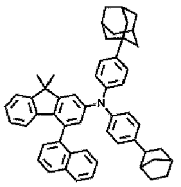
891
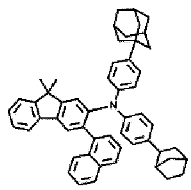
892
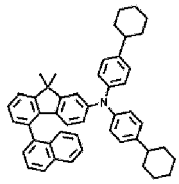
893
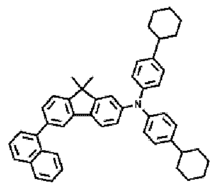
894
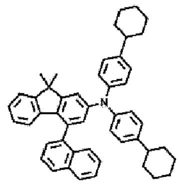
895
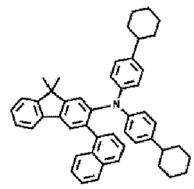
896
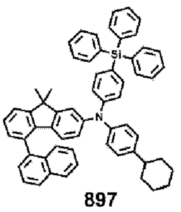
897
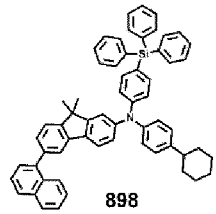
898
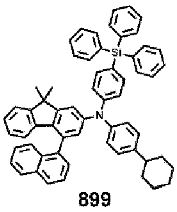
899
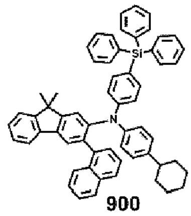
900
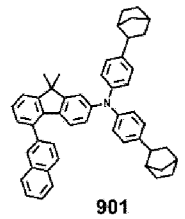
901
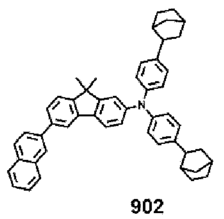
902
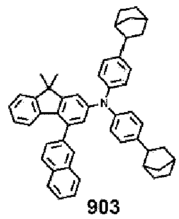
903
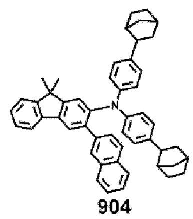
904
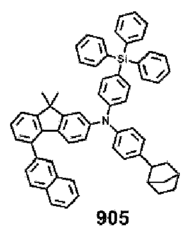
905
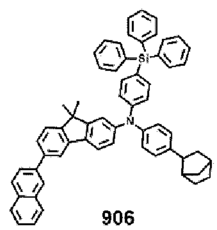
906
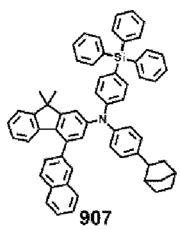
907
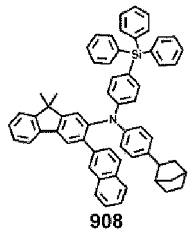
908
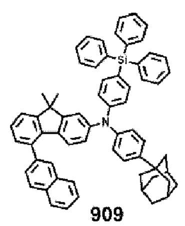
909
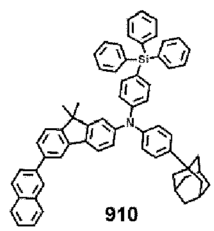
910
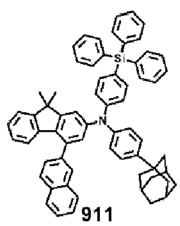
911
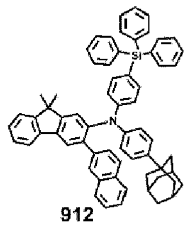
912

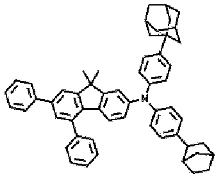
913

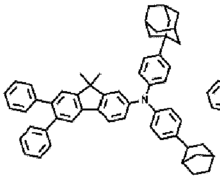
914

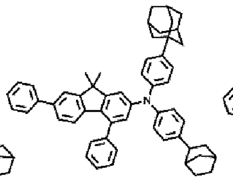
915

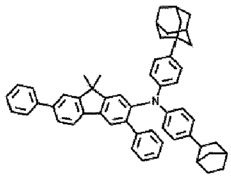
916

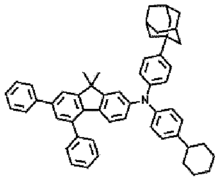
917

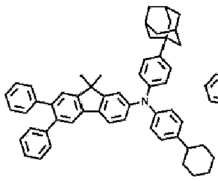
918

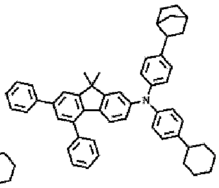
919

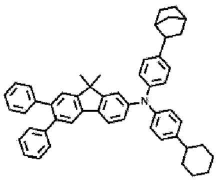
920

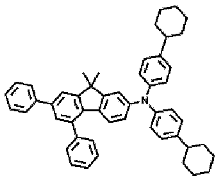
921

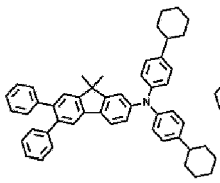
922

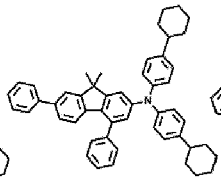
923

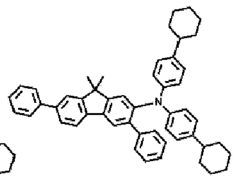
924

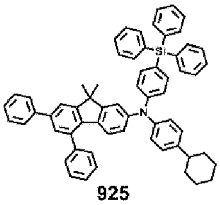
925

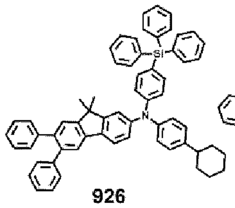
926

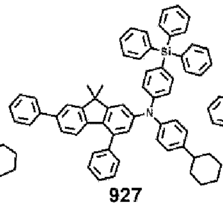
927

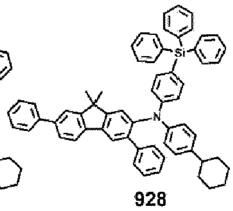
928

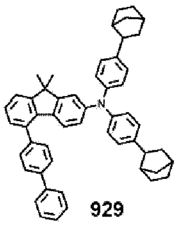
929

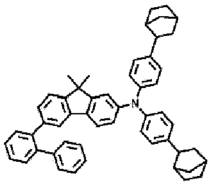
930

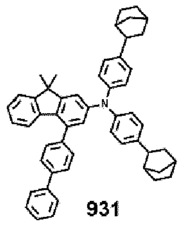
931

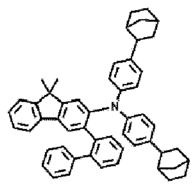
932

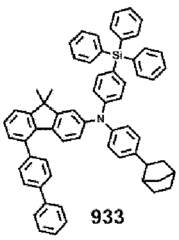
933

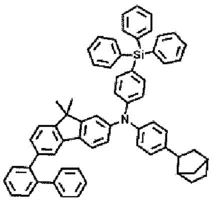
934

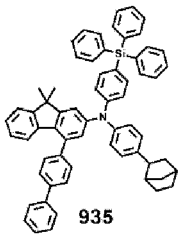
935

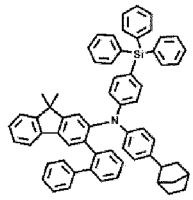
936

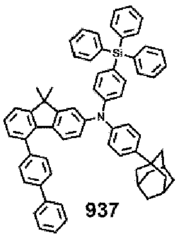
937

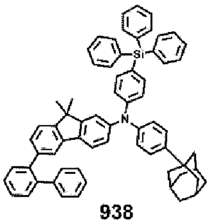
938

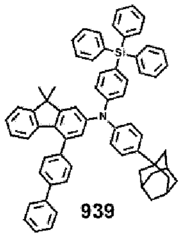
939

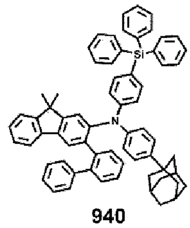
940

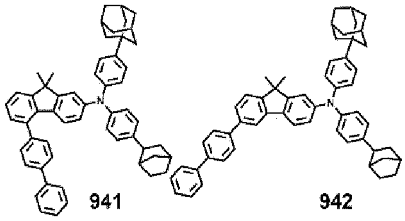

941

942

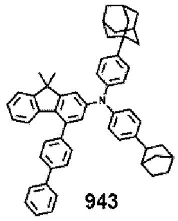

943

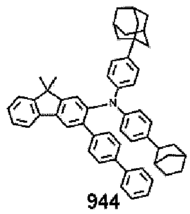

944

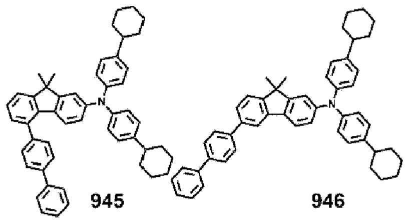

945

946

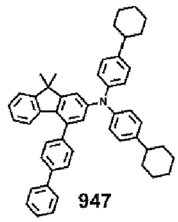

947

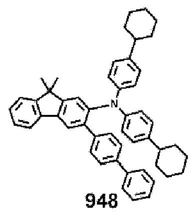

948

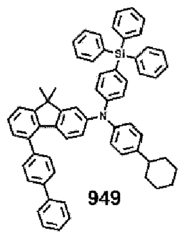

949

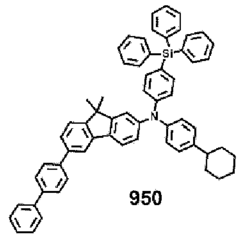

950

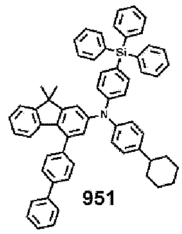

951

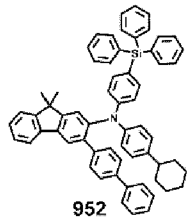

952

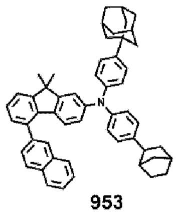

953

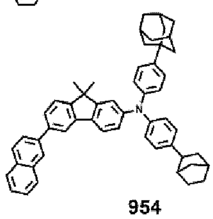

954

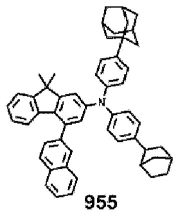

955

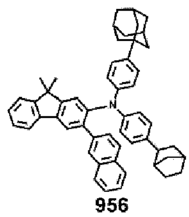

956

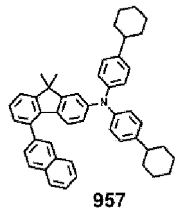

957

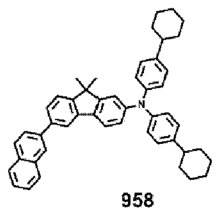

958

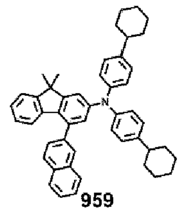

959

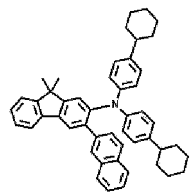

960

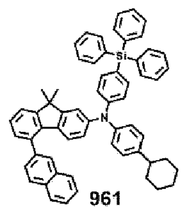

961

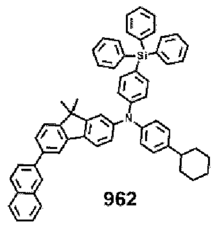

962

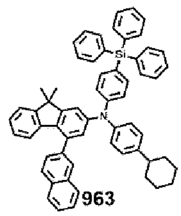

963

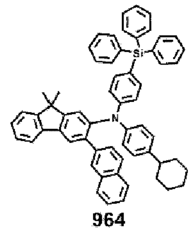

964

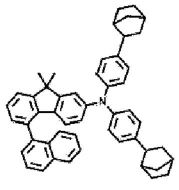
965

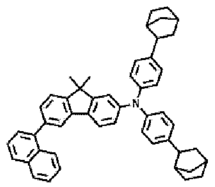
966

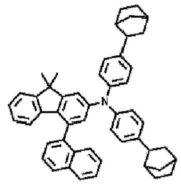
967

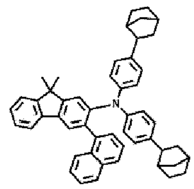
968

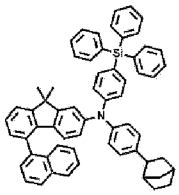
969

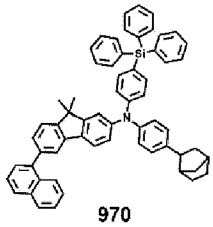
970

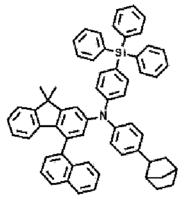
971

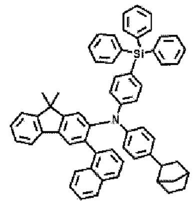
972

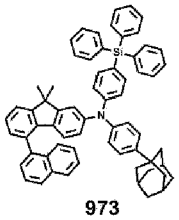
973

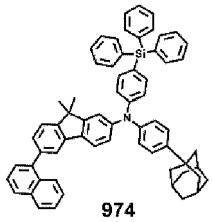
974

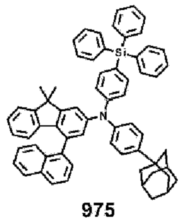
975

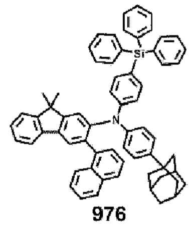
976

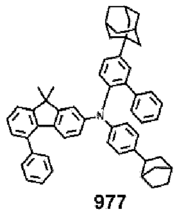
977

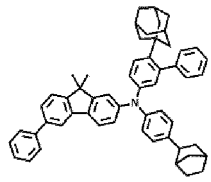
978

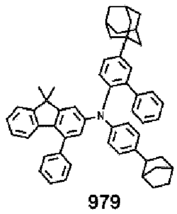
979

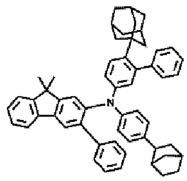
980

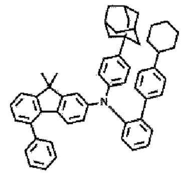
981

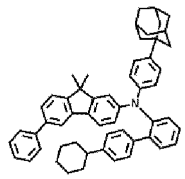
982

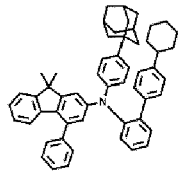
983

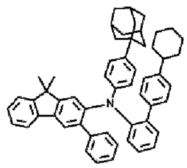
984

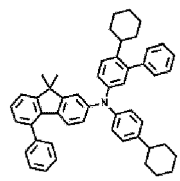
985

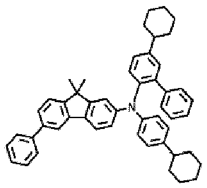
986

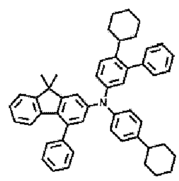
987

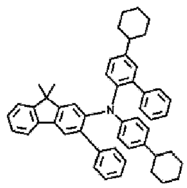
988

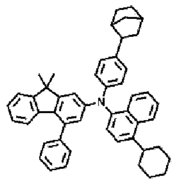
989

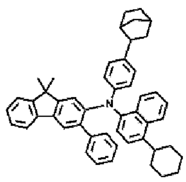
990

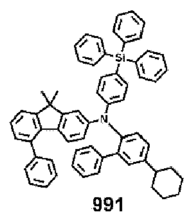
991

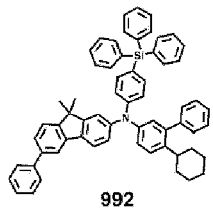
992

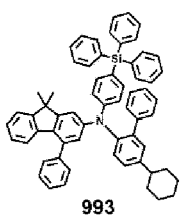
993

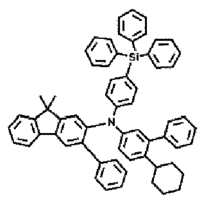
994

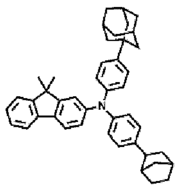 995

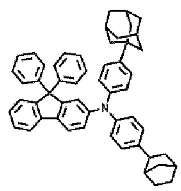 996

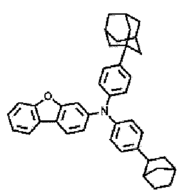 997

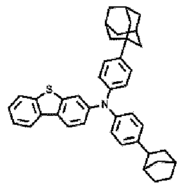 998

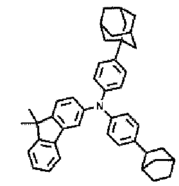 999

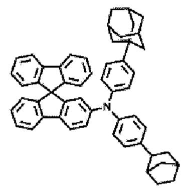 1000

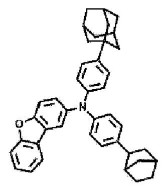 1001

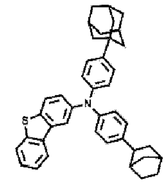 1002

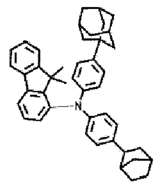 1003

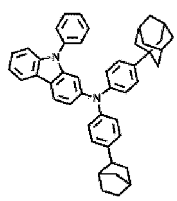 1004

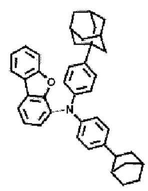 1005

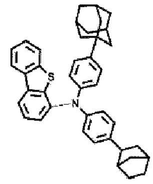 1006

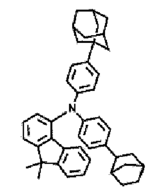 1007

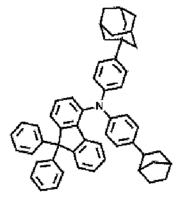 1008

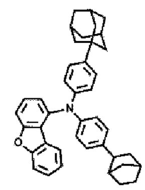 1009

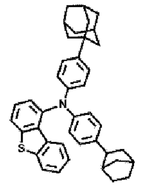 1010

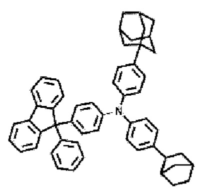 1011

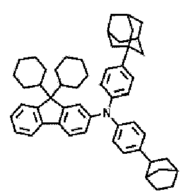 1012

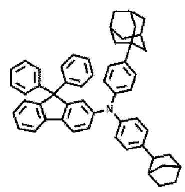 1013

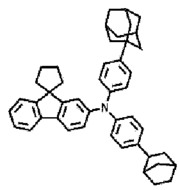 1014

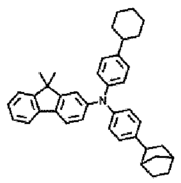
1015

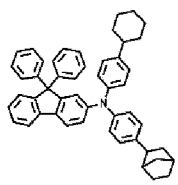
1016

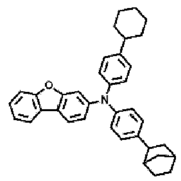
1017

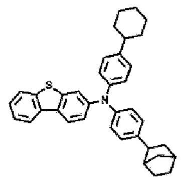
1018

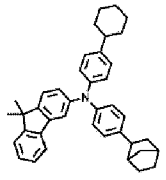
1019

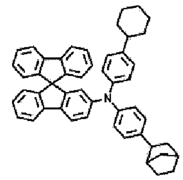
1020

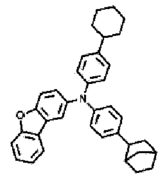
1021

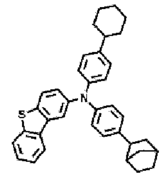
1022

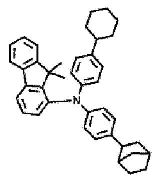
1023

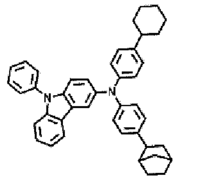
1024

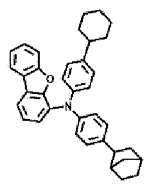
1025

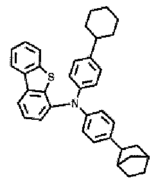
1026

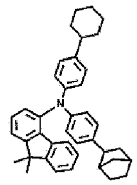
1027

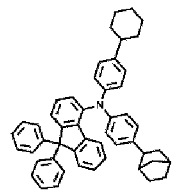
1028

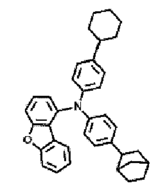
1029

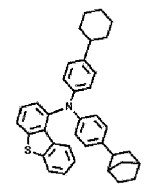
1030

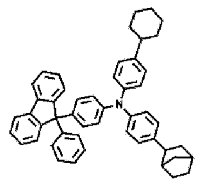
1031

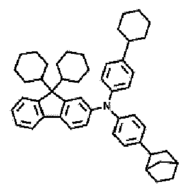
1032

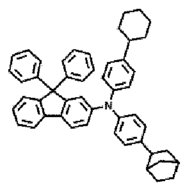
1033

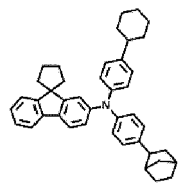
1034

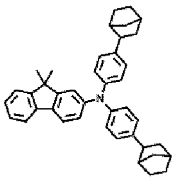
1035

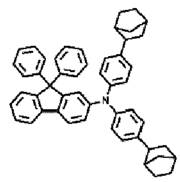
1036

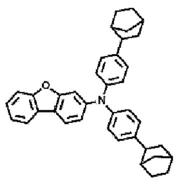
1037

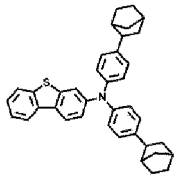
1038

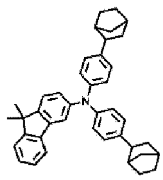
1039

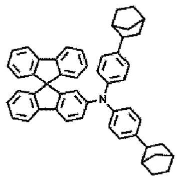
1040

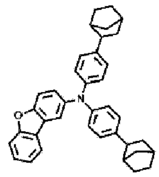
1041

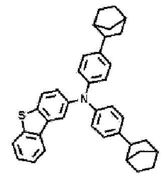
1042

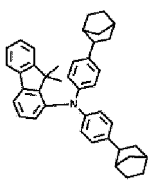
1043

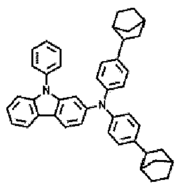
1044

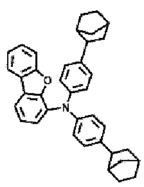
1045

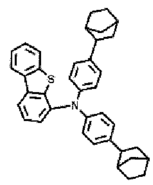
1046

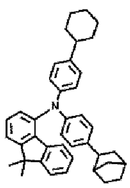
1047

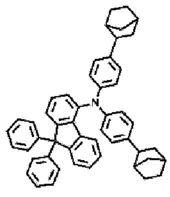
1048

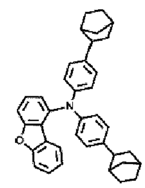
1049

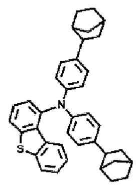
1050

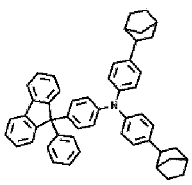
1051

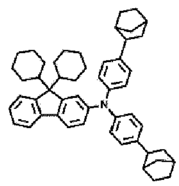
1052

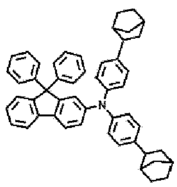
1053

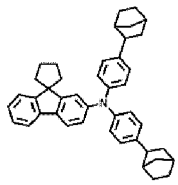
1054

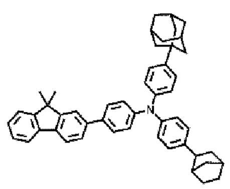

1055

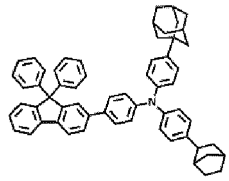

1056

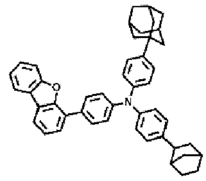

1057

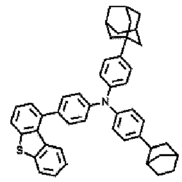

1058

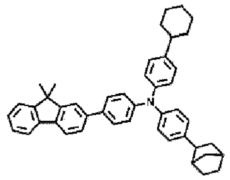

1059

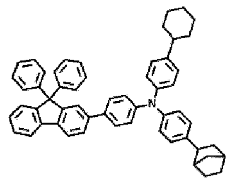

1060

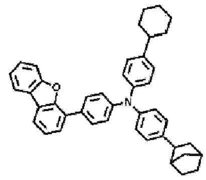

1061

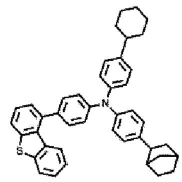

1062

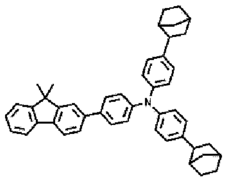

1063

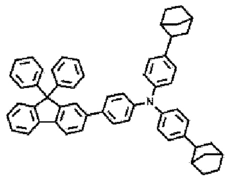

1064

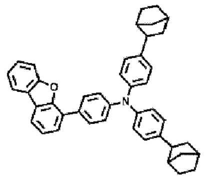

1065

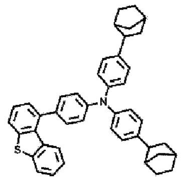

1066 .

**8.** A light emitting device, comprising:

a first electrode;
a second electrode on the first electrode; and
at least one functional layer between the first electrode and the second electrode, wherein the at least one functional layer comprises an amine compound according to any one of claims 1 to 7.

**9.** The light emitting device of claim 8, wherein the at least one functional layer comprises an emission layer, a hole transport region between the first electrode and the emission layer, and an electron transport region between the emission layer and second electrode, and
the hole transport region comprises the amine compound.

**10.** The light emitting device of claim 8, wherein the at least one functional layer comprises an emission layer, a first hole transport layer between the first electrode and the emission layer, a second hole transport layer between the first hole transport layer and the emission layer, and an electron transport region between the emission layer and the second electrode, and
the first hole transport layer comprises the amine compound.

**11.** The light emitting device of claim 8, wherein the at least one functional layer further comprises a third hole transport layer between the second hole transport layer and the emission layer, and
the third hole transport layer comprises the amine compound.

**12.** The light emitting device of any of claims 8 to 11, wherein the second hole transport layer comprises an amine derivative compound represented by the following Formula 10:

Formula 10

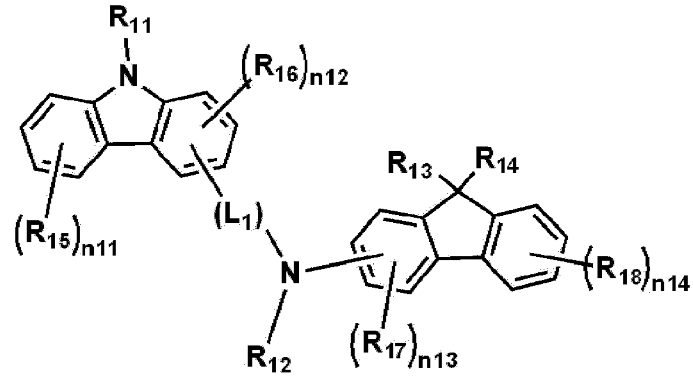

in Formula 10,

$L_1$ is a direct linkage, a substituted or unsubstituted arylene group of 6 to 30 ring-forming carbon atoms, or a substituted or unsubstituted heteroarylene group of 2 to 30 ring-forming carbon atoms,
$R_{11}$ to $R_{14}$ are each independently a substituted or unsubstituted alkyl group of 1 to 10 carbon atoms, a substituted or unsubstituted alkenyl group of 2 to 10 carbon atoms, a substituted or unsubstituted aryl group of 6 to 30 ring-forming carbon atoms, or a substituted or unsubstituted heteroaryl group of 2 to 30 ring-forming carbon atoms, or combined with an adjacent group to form a ring,
$R_{15}$ to $R_{18}$ are each independently a hydrogen atom, a deuterium atom, a halogen atom, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms, or combined with an adjacent group to form a ring,
n11 and n14 are each independently an integer of 0 to 4, and
n12 and n13 are each independently an integer of 0 to 3.

**13.** The light emitting device of claim 8, wherein the at least one functional layer comprises an emission layer, a hole transport layer between the first electrode and the emission layer, and a hole transport auxiliary layer between the hole transport layer and the emission layer, and

the hole transport auxiliary layer comprises a first hole transport auxiliary layer on the hole transport layer, and a second hole transport auxiliary layer on the first hole transport auxiliary layer, and
the first hole transport auxiliary layer comprises the amine compound.

**14.** The light emitting device of claim 13, wherein a refractive index of the first hole transport auxiliary layer in a wavelength range of about 450 nm to about 700 nm is about 1.55 to about 1.80, and
a refractive index of the second hole transport auxiliary layer in a wavelength range of about 450 nm to about 700 nm is about 1.65 to about 1.90.

**15.** The light emitting device of claim 13 or claim 14, wherein an absolute value of the highest occupied molecular orbital (HOMO) energy level of the second hole transport auxiliary layer is greater than an absolute value of the HOMO energy level of the first hole transport auxiliary layer.

FIG. 1

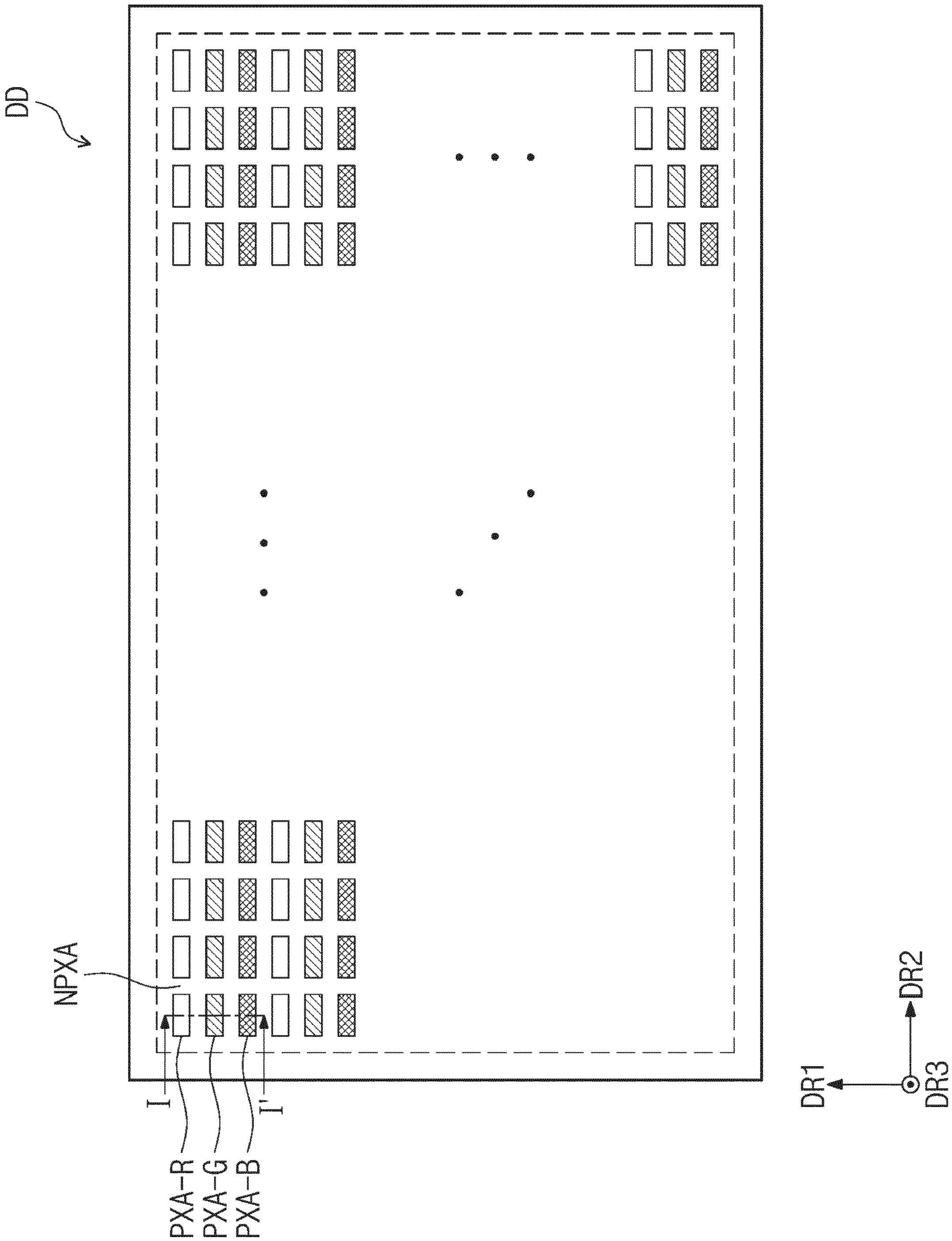

FIG. 2

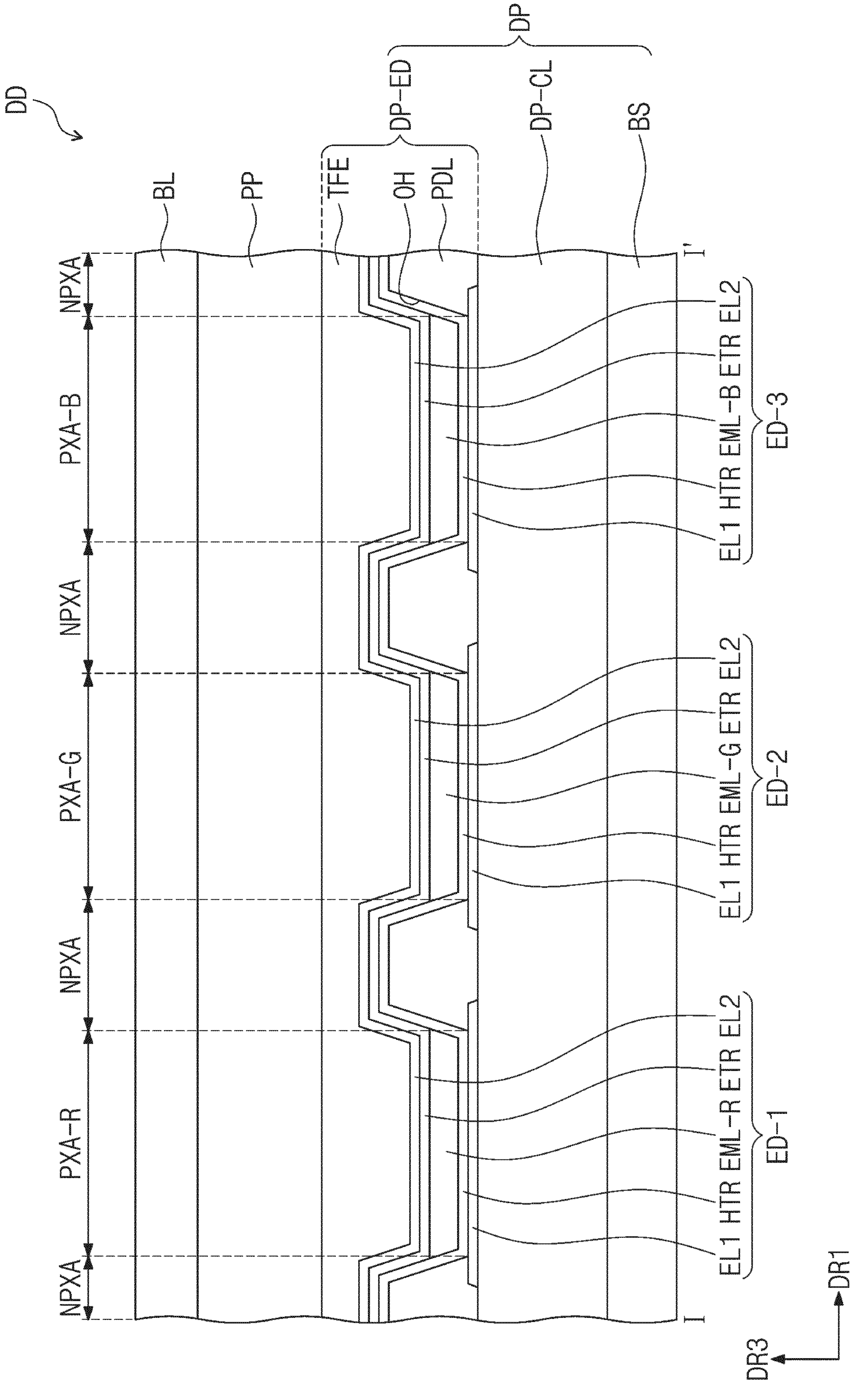
DD
DP
DP-ED
DP-CL
BS
BL
PP
TFE
OH
PDL
NPXA
PXA-B
NPXA
PXA-G
NPXA
PXA-R
NPXA
I'
I
EL1 HTR EML-B ETR EL2
ED-3
EL1 HTR EML-G ETR EL2
ED-2
EL1 HTR EML-R ETR EL2
ED-1
DR1
DR3

FIG. 3

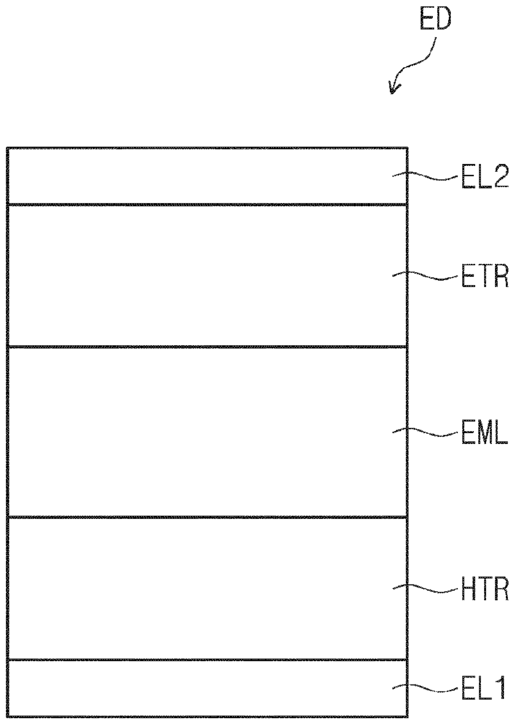


FIG. 4

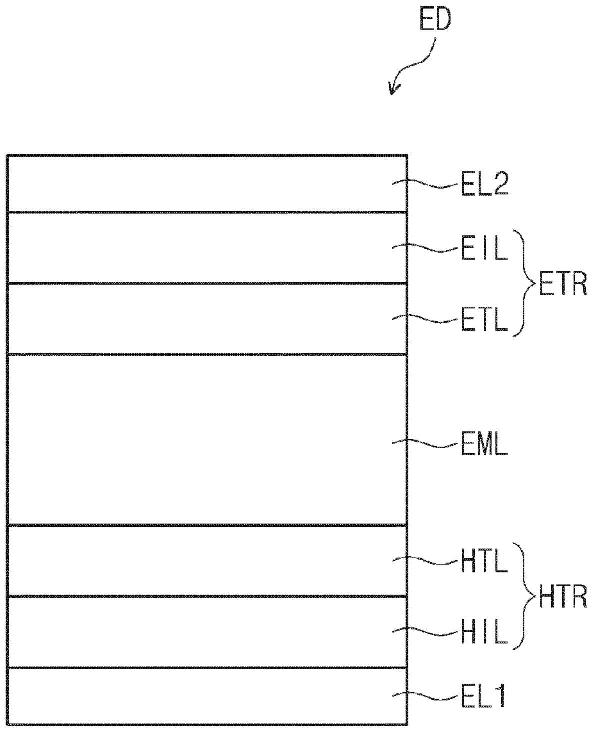

FIG. 5

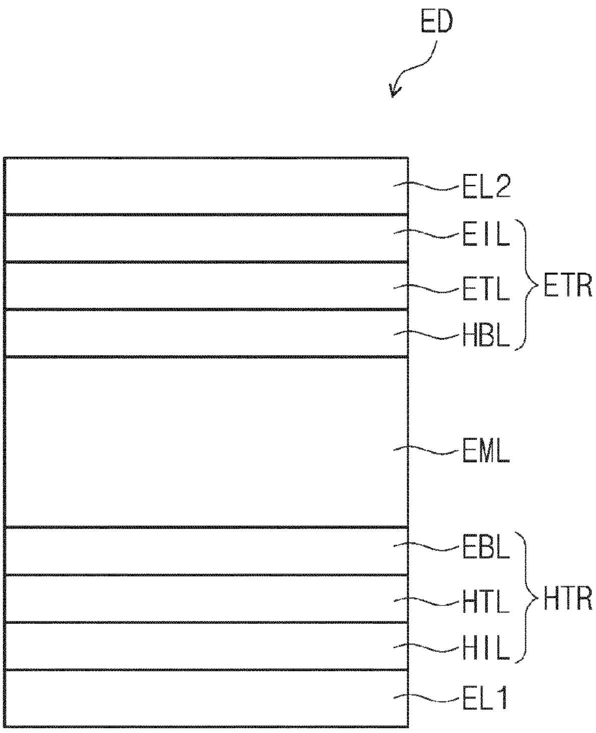
ED
EL2
EIL
ETL
HBL
ETR
EML
EBL
HTL
HIL
HTR
EL1

FIG. 6

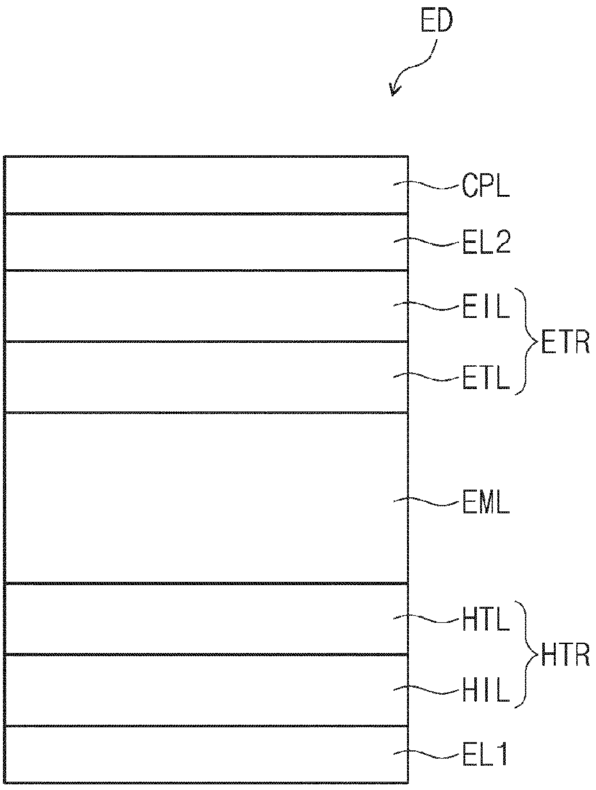
ED
CPL
EL2
EIL
ETL
ETR
EML
HTL
HIL
HTR
EL1

FIG. 7

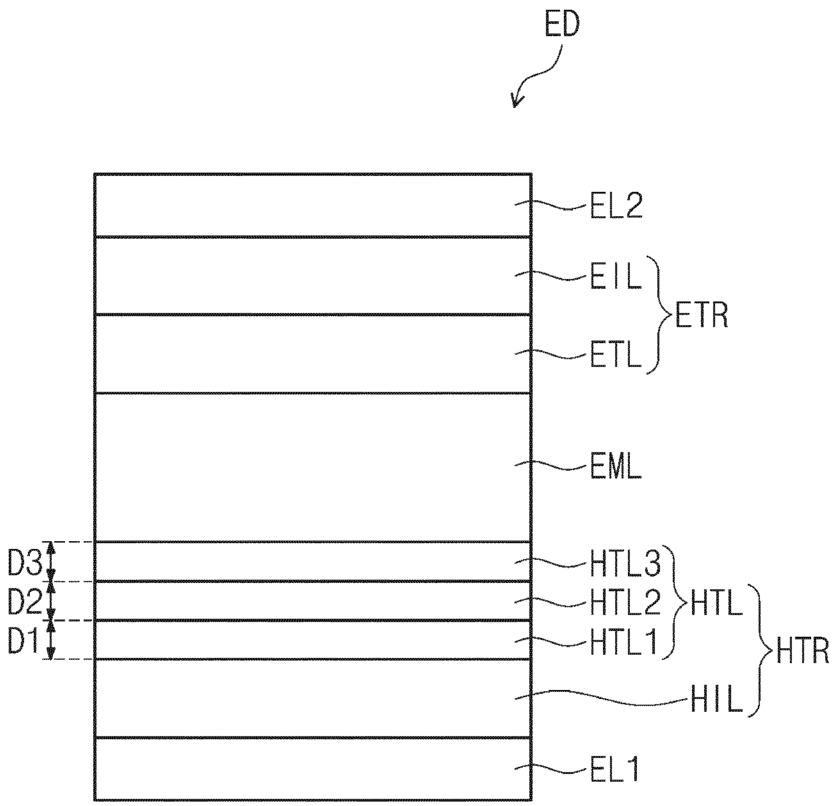

FIG. 8

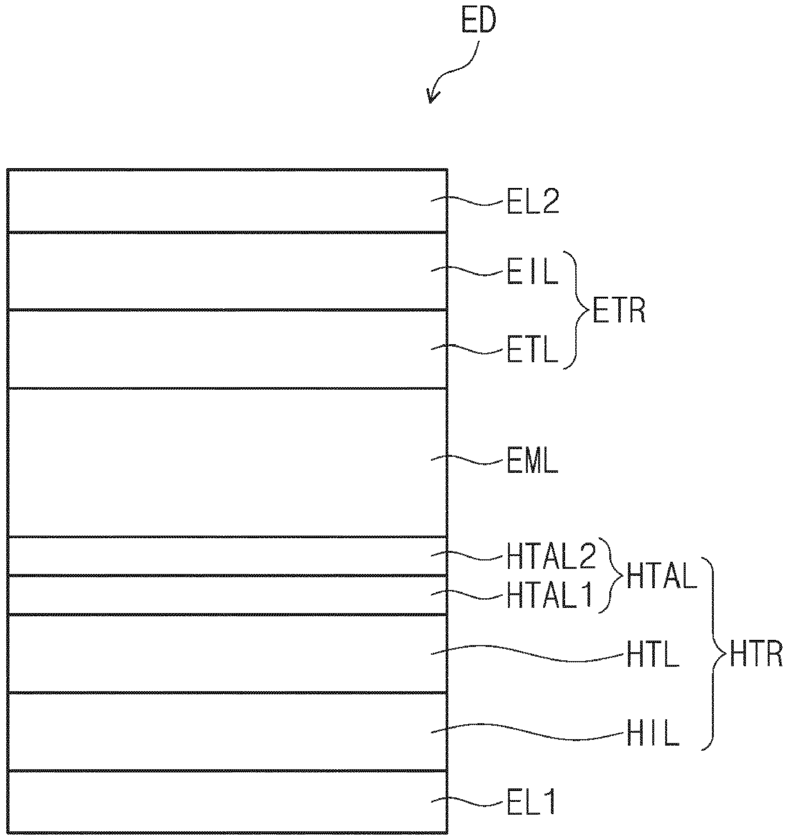

FIG. 9

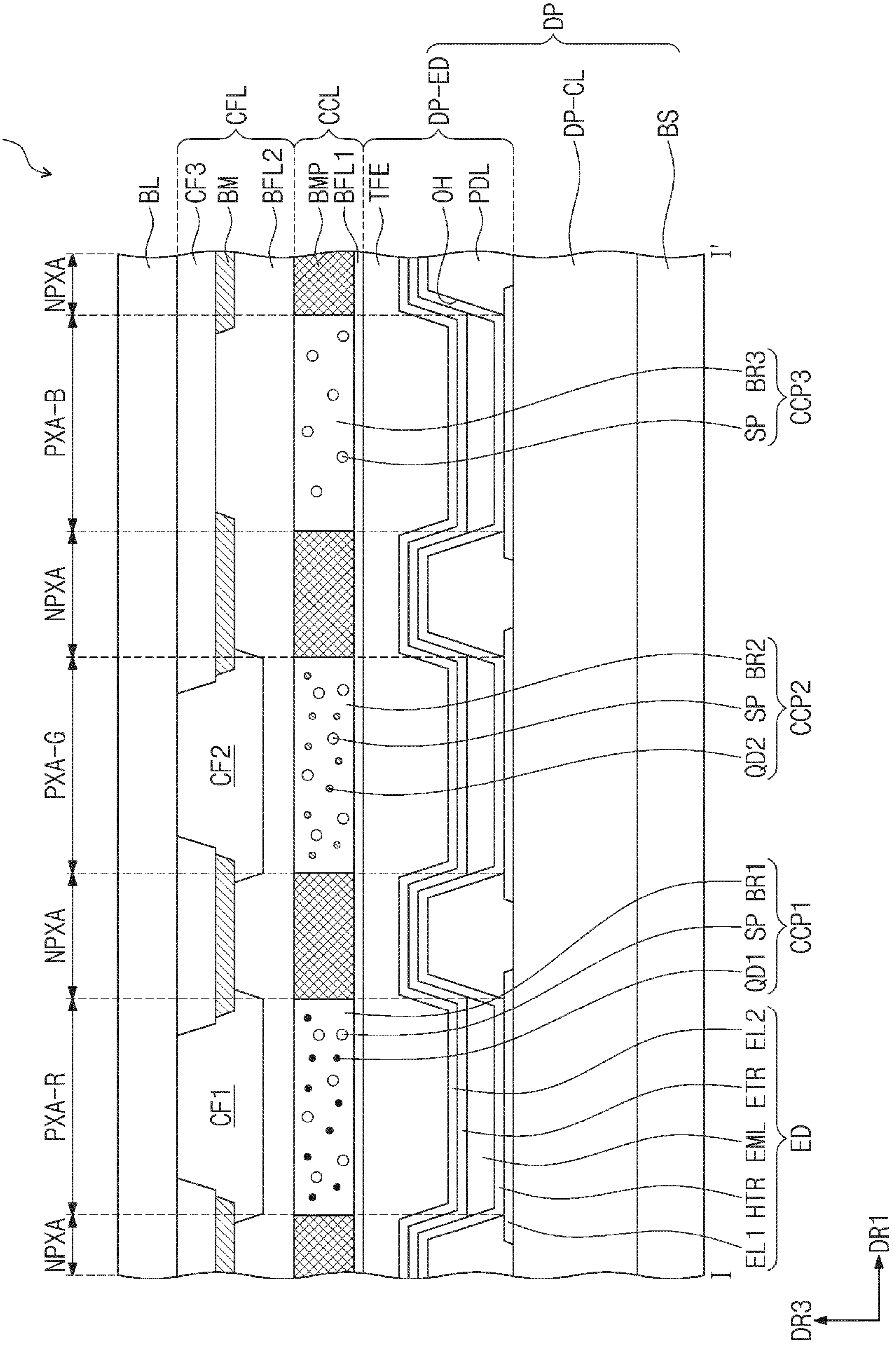
DD-a
DP
CFL
CCL
DP-ED
DP-CL
BS
BL
CF3
BM
BFL2
BMP
BFL1
TFE
OH
PDL
NPXA
PXA-B
NPXA
PXA-G
NPXA
PXA-R
NPXA
CF2
CF1
SP BR3
CCP3
QD2 SP BR2
CCP2
QD1 SP BR1
CCP1
EL1 HTR EML ETR EL2
ED
I
I'
DR3
DR1

FIG. 10

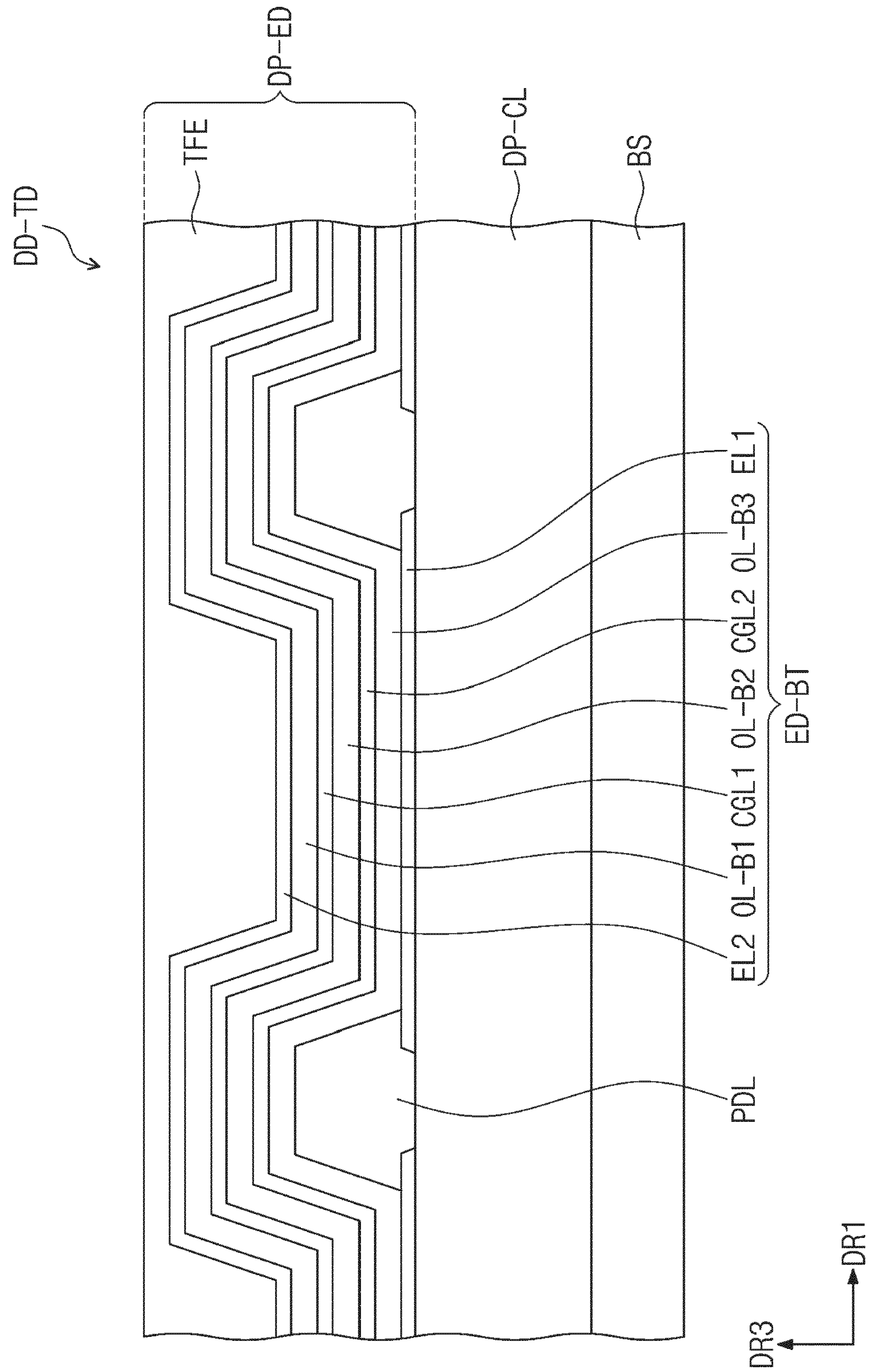
DD-TD
DP-ED
TFE
DP-CL
BS
EL1
OL-B3
CGL2
OL-B2
CGL1
OL-B1
EL2
ED-BT
PDL
DR1
DR3

FIG. 11

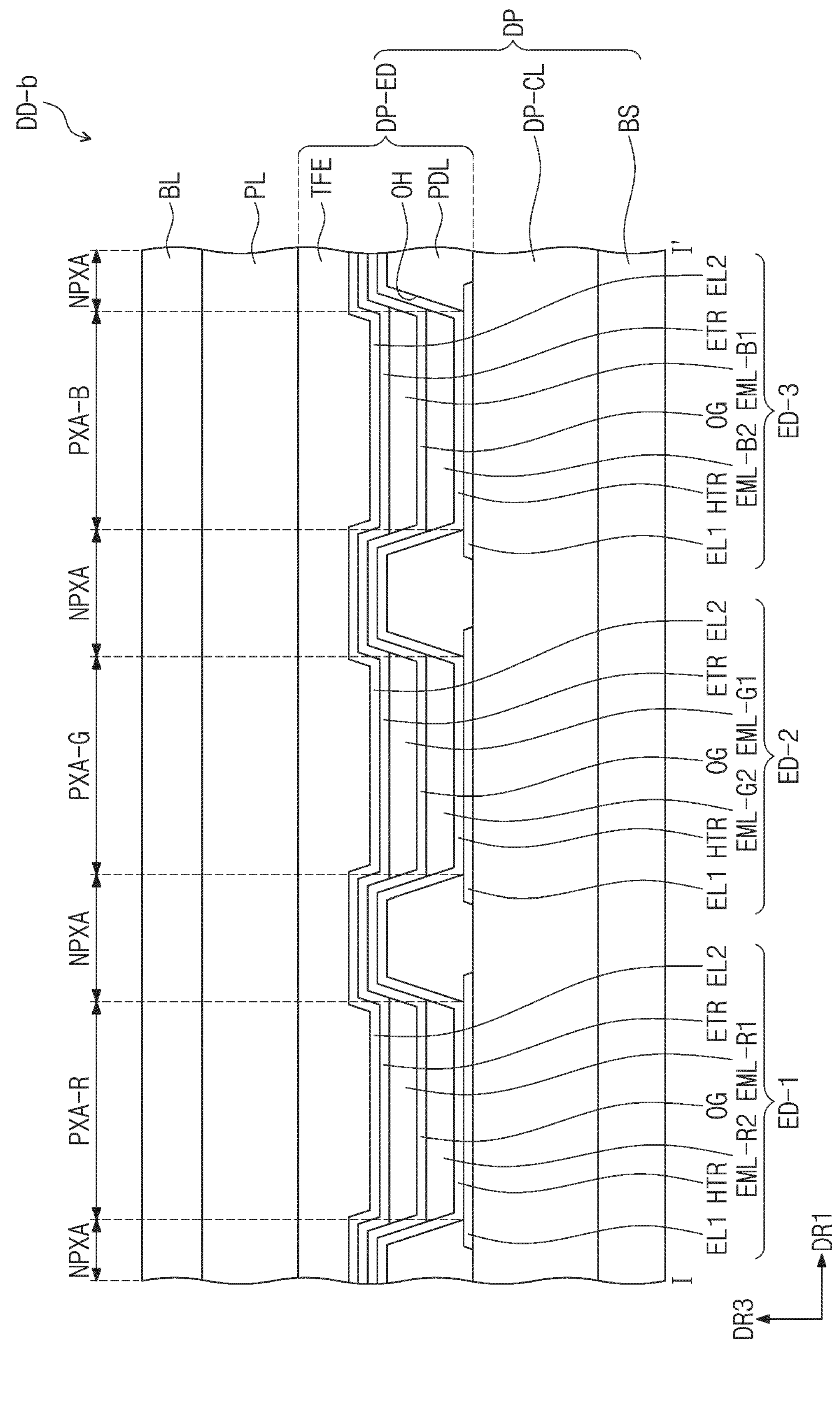
DD-b
DP
DP-ED
DP-CL
BS
BL
PL
TFE
OH
PDL
I
I'
NPXA
PXA-R
PXA-G
PXA-B
EL1 HTR EML-R2 OG EML-R1 ETR EL2
ED-1
EL1 HTR EML-G2 OG EML-G1 ETR EL2
ED-2
EL1 HTR EML-B2 OG EML-B1 ETR EL2
ED-3
DR1
DR3

FIG. 12

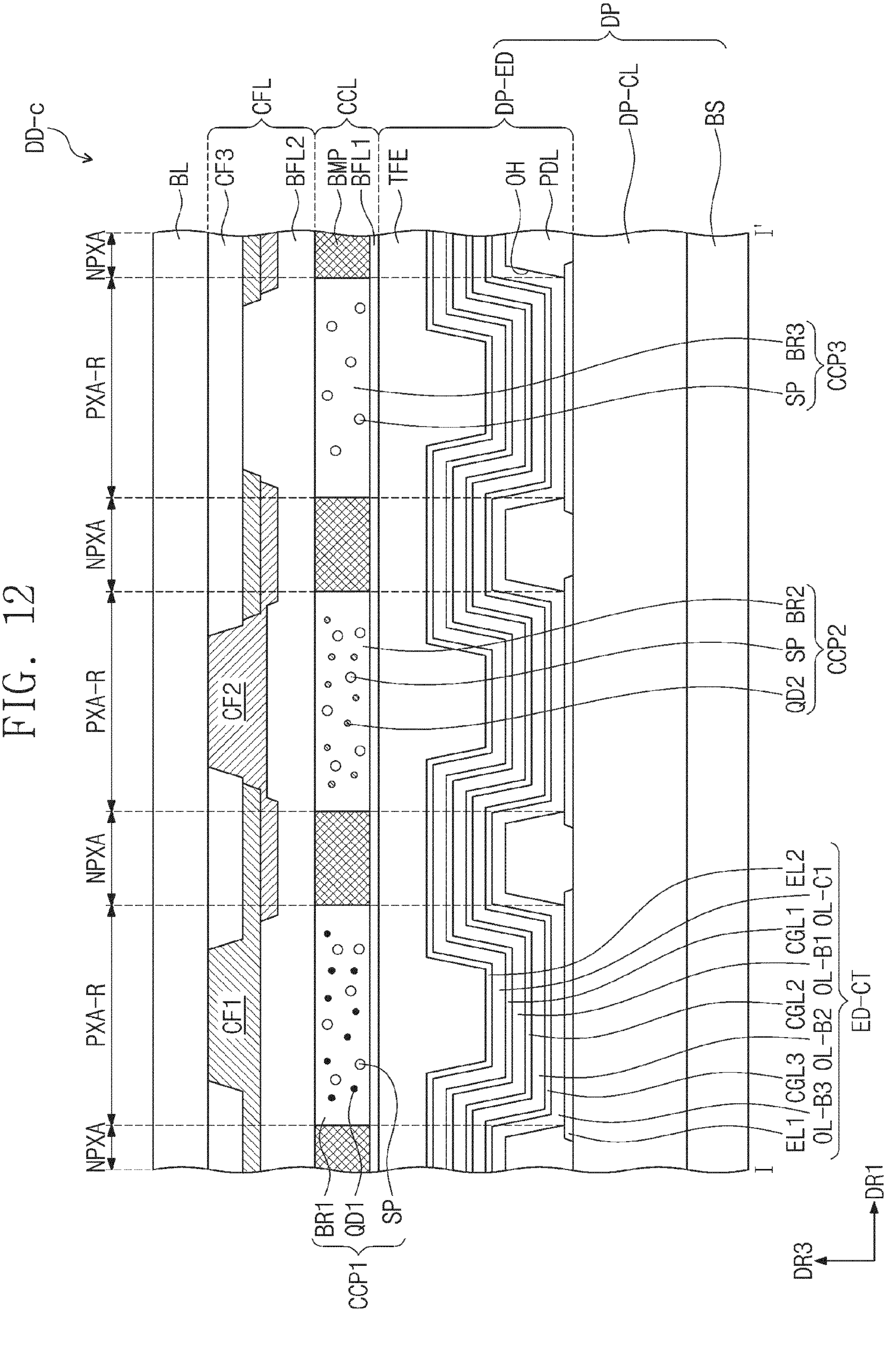
DD-c
BL
CF3
BFL2
CFL
BMP
BFL1
CCL
TFE
OH
PDL
DP-ED
DP-CL
BS
DP
NPXA
PXA-R
NPXA
PXA-R
NPXA
PXA-R
NPXA
CF1
CF2
BR1
QD1
SP
CCP1
SP BR3
CCP3
QD2 SP BR2
CCP2
EL2
CGL1 OL-C1
CGL2 OL-B1
CGL3 OL-B2
EL1 OL-B3
ED-CT
I
I'
DR1
DR3

Europäisches Patentamt
European Patent Office
Office européen des brevets

# EUROPEAN SEARCH REPORT

Application Number
EP 22 20 4291

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P<br>A,P | EP 4 043 432 A1 (SAMSUNG DISPLAY CO LTD [KR]) 17 August 2022 (2022-08-17)<br>* compounds 1, 2, 30, 41, 50 and 65 on page 51;<br>claims 1,12-15 * | 1-10,12<br>11,13-15 | INV.<br>C07D209/88<br>C07D307/91<br>C07D333/76<br>C07C211/61<br>H10K50/15 |
| X<br>Y | CN 110 845 394 B (CHANGCHUN HAIPURUNSI TECH CO LTD) 1 January 2021 (2021-01-01)<br>* compounds 312-316 on page 26; device example 9, tables 1 and 2 on page 34;<br>table 3 on page 38 * | 1,5,6,8<br>2,3 | |
| Y | EP 3 855 524 A1 (SAMSUNG DISPLAY CO LTD [KR]) 28 July 2021 (2021-07-28)<br>* paragraphs [0120] - [0126]; claims 1-3,10-15; figure 6 * | 2,3,11,<br>13,14 | |
| Y | KR 2021 0096954 A (LG CHEMICAL LTD [KR]) 6 August 2021 (2021-08-06)<br>* last but one compound on page 18; last but one compound on page 21; 8th and 15th compound on page 27; last compound on page 28;<br>claims 1,9,10 * | 2,3,11,<br>13,14 | TECHNICAL FIELDS SEARCHED (IPC)<br>C07C<br>C07D<br>H10K |
| Y | KR 2021 0092869 A (LG CHEMICAL LTD [KR]) 27 July 2021 (2021-07-27)<br>* compounds containing two adamantyl groups on pages 14-18, 21, 26, 31, 35, 39, 40, 42, 49, 51 and 53;<br>claims 1,11 * | 2,3,11,<br>13,14 | |

The present search report has been drawn up for all claims

1

| Place of search | Date of completion of the search | Examiner |
|---|---|---|
| Munich | 28 February 2023 | Guspanová, Jana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 22 20 4291

This annex lists the patent family members relating to the patent documents cited in the above-mentioned European search report. The members are as contained in the European Patent Office EDP file on
The European Patent Office is in no way liable for these particulars which are merely given for the purpose of information.

28-02-2023

| Patent document cited in search report | Publication date | Patent family member(s) | Publication date |
|---|---|---|---|
| EP 4043432 A1 | 17-08-2022 | CN 114853616 A | 05-08-2022 |
| | | EP 4043432 A1 | 17-08-2022 |
| | | JP 2022119217 A | 16-08-2022 |
| | | KR 102355848 B1 | 10-02-2022 |
| | | KR 20220112678 A | 11-08-2022 |
| | | US 2022255003 A1 | 11-08-2022 |
| CN 110845394 B | 01-01-2021 | NONE | |
| EP 3855524 A1 | 28-07-2021 | CN 113224246 A | 06-08-2021 |
| | | EP 3855524 A1 | 28-07-2021 |
| | | US 2021234098 A1 | 29-07-2021 |
| KR 20210096954 A | 06-08-2021 | NONE | |
| KR 20210092869 A | 27-07-2021 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020210146503
- KR 1020220001805